# EUROPEAN PATENT SPECIFICATION

(11) **EP 1 151 088 B1**
(45) Date of publication and mention of the grant of the patent: **30.08.2006**
(21) Application number: 00901049.7
(22) Date of filing: 21.01.2000
(51) Int. Cl.: C12N 9/16

(54) **IMPROVED PHYTASES**
VERBESSERTE PHYTASEN
PHYTASES AMELIOREES

(30) Priority: 22.01.1999 DK 990092; 21.09.1999 DK 991340
(43) Date of publication of application: 07.11.2001
(73) Proprietor: Novozymes A/S, 2880 Bagsvaerd (DK)
(72) Inventor: LEHMANN, Martin, Princeton, NJ 07043 (US); LASSEN,Soren, Flensted, DK-2100 Copenhagen (DK)
(86) International application number: PCT/DK2000/000025
(87) International publication number: WO 2000/043503

(56) References cited:
- EP-A1- 0 420 358
- EP-A1- 0 422 697
- EP-A2- 0 897 985
- WO-A1-97/35016
- WO-A1-99/48380
- WO-A1-99/49022

### SEQUENCE LISTING

<110> Novo Nordisk A/S
<120> Improved phytases
<130> seqlist171299
<140>
   <141>
<150> DK 99/00092
   <151> 1999-01-22
<150> DK 99/01340
   <151> 1999-09-21
<160> 89
<170> PatentIn Ver. 2.1
<210> 1
   <211> 440
   <212> PRT
   <213> Aspergillus terreus 9A-1
<400> 1
   Lys His Ser Asp Cys Asn Ser Val Asp His Gly Tyr Gln Cys Phe Pro
     1               5                  10                  15
   Glu Leu Ser His Lys Trp Gly Leu Tyr Ala Pro Tyr Phe Ser Leu Gln
                20                  25                  30
   Asp Glu Ser Pro Phe Pro Leu Asp Val Pro Glu Asp Cys His Ile Thr
            35                  40                  45
   Phe Val Gln Val Leu Ala Arg His Gly Ala Arg Ser Pro Thr His Ser
        50                  55                  60
   Lys Thr Lys Ala Tyr Ala Ala Thr Ile Ala Ala Ile Gln Lys Ser Ala
    65                  70                  75                  80
   Thr Ala Phe Pro Gly Lys Tyr Ala Phe Leu Gln Ser Tyr Asn Tyr Ser
                    85                  90                  95
   Leu Asp Ser Glu Glu Leu Thr Pro Phe Gly Arg Asn Gln Leu Arg Asp
               100                 105                 110
   Leu Gly Ala Gln Phe Tyr Glu Arg Tyr Asn Ala Leu Thr Arg His Ile
           115                 120                 125
   Asn Pro Phe Val Arg Ala Thr Asp Ala Ser Arg Val His Glu Ser Ala
       130                 135                 140
   Glu Lys Phe Val Glu Gly Phe Gln Thr Ala Arg Gln Asp Asp His His
   145 150 155 160
   Ala Asn Pro His Gln Pro Ser Pro Arg Val Asp Val Ala Ile Pro Glu
   165 170 175
   Gly Ser Ala Tyr Asn Asn Thr Leu Glu His Ser Leu Cys Thr Ala Phe
   180 185 190
   Glu Ser Ser Thr Val Gly Asp Asp Ala Val Ala Asn Phe Thr Ala Val
   195 200 205
   Phe Ala Pro Ala Ile Ala Gln Arg Leu Glu Ala Asp Leu Pro Gly Val
   210 215 220
   Gln Leu Ser Thr Asp Asp Val Val Asn Leu Met Ala Met Cys Pro Phe
   225 230 235 240
   Glu Thr Val Ser Leu Thr Asp Asp Ala His Thr Leu Ser Pro Phe Cys
   245 250 255
   Asp Leu Phe Thr Ala Thr Glu Trp Thr Gln Tyr Asn Tyr Leu Leu Ser
   260 265 270
   Leu Asp Lys Tyr Tyr Gly Tyr Gly Gly Gly Asn Pro Leu Gly Pro Val
   275 280 285
   Gln Gly Val Gly Trp Ala Asn Glu Leu Met Ala Arg Leu Thr Arg Ala
   290 295 300
   Pro Val His Asp His Thr Cys Val Asn Asn Thr Leu Asp Ala Ser Pro
   305 310 315 320
   Ala Thr Phe Pro Leu Asn Ala Thr Leu Tyr Ala Asp Phe Ser His Asp
   325 330 335
   Ser Asn Leu Val Ser Ile Phe Trp Ala Leu Gly Leu Tyr Asn Gly Thr
   340 345 350
   Ala Pro Leu Ser Gln Thr Ser Val Glu Ser Val Ser Gln Thr Asp Gly
   355 360 365
   Tyr Ala Ala Ala Trp Thr Val Pro Phe Ala Ala Arg Ala Tyr Val Glu
   370 375 380
   Met Met Gln Cys Arg Ala Glu Lys Glu Pro Leu Val Arg Val Leu Val
   385 390 395 400
   Asn Asp Arg Val Met Pro Leu His Gly Cys Pro Thr Asp Lys Leu Gly
   405 410 415
   Arg Cys Lys Arg Asp Ala Phe Val Ala Gly Leu Ser Phe Ala Gln Ala
   420 425 430
   Gly Gly Asn Trp Ala Asp Cys Phe
   435 440
<210> 2
   <211> 440
   <212> PRT
   <213> Aspergillus terreus cbs
<400> 2
   Asn His Ser Asp Cys Thr Ser Val Asp Arg Gly Tyr Gln Cys Phe Pro
   1               5                   10                  15
   Glu Leu Ser His Lys Trp Gly Leu Tyr Ala Pro Tyr Phe Ser Leu Gln
               20                  25                  30
   Asp Glu Ser Pro Phe Pro Leu Asp Val Pro Asp Asp Cys His Ile Thr
           35                  40                  45
   Phe Val Gln Val Leu Ala Arg His Gly Ala Arg Ser Pro Thr Asp Ser
       50                  55                  60
   Lys Thr Lys Ala Tyr Ala Ala Thr Ile Ala Ala Ile Gln Lys Asn Ala
   65                  70                  75                  80
   Thr Ala Leu Pro Gly Lys Tyr Ala Phe Leu Lys Ser Tyr Asn Tyr Ser
                   85                  90                  95
   Met Gly Ser Glu Asn Leu Thr Pro Phe Gly Arg Asn Gln Leu Gln Asp
               100                 105                 110
   Leu Gly Ala Gln Phe Tyr Arg Arg Tyr Asp Thr Leu Thr Arg His Ile
           115                 120                 125
   Asn Pro Phe Val Arg Ala Ala Asp Ser Ser Arg Val His Glu Ser Ala
       130                 135                 140
   Glu Lys Phe Val Glu Gly Phe Gln Asn Ala Arg Gln Gly Asp Pro His
   145                 150                 155                 160
   Ala Asn Pro His Gln Pro Ser Pro Arg Val Asp Val Val Ile Pro Glu
                   165                 170                 175
   Gly Thr Ala Tyr Asn Asn Thr Leu Glu His Ser Ile Cys Thr Ala Phe
               180                 185                 190
   Glu Ala Ser Thr Val Gly Asp Ala Ala Ala Asp Asn Phe Thr Ala Val
           195                 200                 205
   Phe Ala Pro Ala Ile Ala Lys Arg Leu Glu Ala Asp Leu Pro Gly Val
       210                 215                 220
   Gln Leu Ser Ala Asp Asp Val Val Asn Leu Met Ala Met Cys Pro Phe
   225                 230                 235                 240
   Glu Thr Val Ser Leu Thr Asp Asp Ala His Thr Leu Ser Pro Phe Cys
   245 250 255
   Asp Leu Phe Thr Ala Ala Glu Trp Thr Gln Tyr Asn Tyr Leu Leu Ser
   260 265 270
   Leu Asp Lys Tyr Tyr Gly Tyr Gly Gly Gly Asn Pro Leu Gly Pro Val
   275 280 285
   Gln Gly Val Gly Trp Ala Asn Glu Leu Ile Ala Arg Leu Thr Arg Ser
   290 295 300
   Pro Val His Asp His Thr Cys Val Asn Asn Thr Leu Asp Ala Asn Pro
   305 310 315 320
   Ala Thr Phe Pro Leu Asn Ala Thr Leu Tyr Ala Asp Phe Ser His Asp
   325 330 335
   Ser Asn Leu Val Ser Ile Phe Trp Ala Leu Gly Leu Tyr Asn Gly Thr
   340 345 350
   Lys Pro Leu Ser Gln Thr Thr Val Glu Asp Ile Thr Arg Thr Asp Gly
   355 360 365
   Tyr Ala Ala Ala Trp Thr Val Pro Phe Ala Ala Arg Ala Tyr Ile Glu
   370 375 380
   Met Met Gln Cys Arg Ala Glu Lys Gln Pro Leu Val Arg Val Leu Val
   385 390 395 400
   Asn Asp Arg Val Met Pro Leu His Gly Cys Ala Val Asp Asn Leu Gly
   405 410 415
   Arg Cys Lys Arg Asp Asp Phe Val Glu Gly Leu Ser Phe Ala Arg Ala
   420 425 430
   Gly Gly Asn Trp Ala Glu Cys Phe
   435 440
<210> 3
   <211> 441
   <212> PRT
   <213> Aspergillus niger var. awamori
<400> 3
   Asn Gln Ser Thr Cys Asp Thr Val Asp Gln Gly Tyr Gln Cys Phe Ser
   1 5 10 15
   Glu Thr Ser His Leu Trp Gly Gln Tyr Ala Pro Phe Phe Ser Leu Ala
   20 25 30
   Asn Glu Ser Ala Ile Ser Pro Asp Val Pro Ala Gly Cys Arg Val Thr
   35 40 45
   Phe Ala Gln Val Leu Ser Arg His Gly Ala Arg Tyr Pro Thr Glu Ser
   50 55 60
   Lys Gly Lys Lys Tyr Ser Ala Leu Ile Glu Glu Ile Gln Gln Asn Val
   65 70 75 80
   Thr Thr Phe Asp Gly Lys Tyr Ala Phe Leu Lys Thr Tyr Asn Tyr Ser
   85 90 95
   Leu Gly Ala Asp Asp Leu Thr Pro Phe Gly Glu Gln Glu Leu Val Asn
   100 105 110
   Ser Gly Ile Lys Phe Tyr Gln Arg Tyr Glu Ser Leu Thr Arg Asn Ile
   115 120 125
   Ile Pro Phe Ile Arg Ser Ser Gly Ser Ser Arg Val Ile Ala Ser Gly
   130 135 140
   Glu Lys Phe Ile Glu Gly Phe Gln Ser Thr Lys Leu Lys Asp Pro Arg
   145 150 155 160
   Ala Gln Pro Gly Gln Ser Ser Pro Lys Ile Asp Val Val Ile Ser Glu
   165 170 175
   Ala Ser Ser Ser Asn Asn Thr Leu Asp Pro Gly Thr Cys Thr Val Phe
   180 185 190
   Glu Asp Ser Glu Leu Ala Asp Thr Val Glu Ala Asn Phe Thr Ala Thr
   195 200 205
   Phe Ala Pro Ser Ile Arg Gln Arg Leu Glu Asn Asp Leu Ser Gly Val
   210 215 220
   Thr Leu Thr Asp Thr Glu Val Thr Tyr Leu Met Asp Met Cys Ser Phe
   225 230 235 240
   Asp Thr Ile Ser Thr Ser Thr Val Asp Thr Lys Leu Ser Pro Phe Cys
   245 250 255
   Asp Leu Phe Thr His Asp Glu Trp Ile His Tyr Asp Tyr Leu Gln Ser
   260 265 270
   Leu Lys Lys Tyr Tyr Gly His Gly Ala Gly Asn Pro Leu Gly Pro Thr
   275 280 285
   Gln Gly Val Gly Tyr Ala Asn Glu Leu Ile Ala Arg Leu Thr His Ser
   290 295 300
   Pro Val His Asp Asp Thr Ser Ser Asn His Thr Leu Asp Ser Asn Pro
   305 310 315 320
   Ala Thr Phe Pro Leu Asn Ser Thr Leu Tyr Ala Asp Phe Ser His Asp
                   325                 330                 335
   Asn Gly Ile Ile Ser Ile Leu Phe Ala Leu Gly Leu Tyr Asn Gly Thr
               340                 345                 350
   Lys Pro Leu Ser Thr Thr Thr Val Glu Asn Ile Thr Gln Thr Asp Gly
           355                 360                 365
   Phe Ser Ser Ala Trp Thr Val Pro Phe Ala Ser Arg Leu Tyr Val Glu
       370                 375                 380
   Met Met Gln Cys Gln Ala Glu Gln Glu Pro Leu Val Arg Val Leu Val
   385                 390                 395                 400
   Asn Asp Arg Val Val Pro Leu His Gly Cys Pro Ile Asp Ala Leu Gly
                   405                 410                 415
   Arg Cys Thr Arg Asp Ser Phe Val Arg Gly Leu Ser Phe Ala Arg Ser
               420                 425                 430
   Gly Gly Asp Trp Ala Glu Cys Ser Ala
           435                 440
<210> 4
   <211> 441
   <212> PRT
   <213> Aspergillus niger T213
<400> 4
   Asn Gln Ser Ser Cys Asp Thr Val Asp Gln Gly Tyr Gln Cys Phe Ser
   1               5                   10                  15
   Glu Thr Ser His Leu Trp Gly Gln Tyr Ala Pro Phe Phe Ser Leu Ala
               20                  25                  30
   Asn Glu Ser Val Ile Ser Pro Asp Val Pro Ala Gly Cys Arg Val Thr
           35                  40                  45
   Phe Ala Gln Val Leu Ser Arg His Gly Ala Arg Tyr Pro Thr Glu Ser
       50                  55                  60
   Lys Gly Lys Lys Tyr Ser Ala Leu Ile Glu Glu Ile Gln Gln Asn Val
   65                  70                  75                  80
   Thr Thr Phe Asp Gly Lys Tyr Ala Phe Leu Lys Thr Tyr Asn Tyr Ser
                   85                  90                  95
   Leu Gly Ala Asp Asp Leu Thr Pro Phe Gly Glu Gln Glu Leu Val Asn
               100                 105                 110
   Ser Gly Ile Lys Phe Tyr Gln Arg Tyr Glu Ser Leu Thr Arg Asn Ile
           115                 120                 125
   Ile Pro Phe Ile Arg Ser Ser Gly Ser Ser Arg Val Ile Ala Ser Gly
       130                 135                 140
   Glu Lys Phe Ile Glu Gly Phe Gln Ser Thr Lys Leu Lys Asp Pro Arg
   145                 150                 155                 160
   Ala Gln Pro Gly Gln Ser Ser Pro Lys Ile Asp Val Val Ile Ser Glu
                   165                 170                 175
   Ala Ser Ser Ser Asn Asn Thr Leu Asp Pro Gly Thr Cys Thr Val Phe
               180                 185                 190
   Glu Asp Ser Glu Leu Ala Asp Thr Val Glu Ala Asn Phe Thr Ala Thr
           195                 200                 205
   Phe Ala Pro Ser Ile Arg Gln Arg Leu Glu Asn Asp Leu Ser Gly Val
       210                 215                 220
   Thr Leu Thr Asp Thr Glu Val Thr Tyr Leu Met Asp Met Cys Ser Phe
   225                 230                 235                 240
   Asp Thr Ile Ser Thr Ser Thr Val Asp Thr Lys Leu Ser Pro Phe Cys
                   245                 250                 255
   Asp Leu Phe Thr His Asp Glu Trp Ile His Tyr Asp Tyr Leu Arg Ser
               260                 265                 270
   Leu Lys Lys Tyr Tyr Gly His Gly Ala Gly Asn Pro Leu Gly Pro Thr
           275                 280                 285
   Gln Gly Val Gly Tyr Ala Asn Glu Leu Ile Ala Arg Leu Thr His Ser
       290                 295                 300
   Pro Val His Asp Asp Thr Ser Ser Asn His Thr Leu Asp Ser Asn Pro
   305                 310                 315                 320
   Ala Thr Phe Pro Leu Asn Ser Thr Leu Tyr Ala Asp Phe Ser His Asp
                   325                 330                 335
   Asn Gly Ile Ile Ser Ile Leu Phe Ala Leu Gly Leu Tyr Asn Gly Thr
               340                 345                 350
   Lys Pro Leu Ser Thr Thr Thr Val Glu Asn Ile Thr Gln Thr Asp Gly
           355                 360                 365
   Phe Ser Ser Ala Trp Thr Val Pro Phe Ala Ser Arg Leu Tyr Val Glu
       370                 375                 380
   Met Met Gln Cys Gln Ala Glu Gln Glu Pro Leu Val Arg Val Leu Val
   385                 390                 395                 400
   Asn Asp Arg Val Val Pro Leu His Gly Cys Pro Ile Asp Ala Leu Gly
                   405                 410                 415
   Arg Cys Thr Arg Asp Ser Phe Val Arg Gly Leu Ser Phe Ala Arg Ser
   420 425 430
   Gly Gly Asp Trp Ala Glu Cys Phe Ala
   435 440
<210> 5
   <211> 441
   <212> PRT
   <213> Aspergillus niger NRRL3135
<400> 5
   Asn Gln Ser Ser Cys Asp Thr Val Asp Gln Gly Tyr Gln Cys Phe Ser
   1 5 10 15
   Glu Thr Ser His Leu Trp Gly Gln Tyr Ala Pro Phe Phe Ser Leu Ala
   20 25 30
   Asn Glu Ser Val Ile Ser Pro Glu Val Pro Ala Gly Cys Arg Val Thr
   35 40 45
   Phe Ala Gln Val Leu Ser Arg His Gly Ala Arg Tyr Pro Thr Asp Ser
   50 55 60
   Lys Gly Lys Lys Tyr Ser Ala Leu Ile Glu Glu Ile Gln Gln Asn Ala
   65 70 75 80
   Thr Thr Phe Asp Gly Lys Tyr Ala Phe Leu Lys Thr Tyr Asn Tyr Ser
   85 90 95
   Leu Gly Ala Asp Asp Leu Thr Pro Phe Gly Glu Gln Glu Leu Val Asn
   100 105 110
   Ser Gly Ile Lys Phe Tyr Gln Arg Tyr Glu Ser Leu Thr Arg Asn Ile
   115 120 125
   Val Pro Phe Ile Arg Ser Ser Gly Ser Ser Arg Val Ile Ala Ser Gly
   130 135 140
   Lys Lys Phe Ile Glu Gly Phe Gln Ser Thr Lys Leu Lys Asp Pro Arg
   145 150 155 160
   Ala Gln Pro Gly Gln Ser Ser Pro Lys Ile Asp Val Val Ile Ser Glu
   165 170 175
   Ala Ser Ser Ser Asn Asn Thr Leu Asp Pro Gly Thr Cys Thr Val Phe
   180 185 190
   Glu Asp Ser Glu Leu Ala Asp Thr Val Glu Ala Asn Phe Thr Ala Thr
   195 200 205
   Phe Val Pro Ser Ile Arg Gln Arg Leu Glu Asn Asp Leu Ser Gly Val
       210                 215                 220
   Thr Leu Thr Asp Thr Glu Val Thr Tyr Leu Met Asp Met Cys Ser Phe
   225                 230                 235                 240
   Asp Thr Ile Ser Thr Ser Thr Val Asp Thr Lys Leu Ser Pro Phe Cys
                   245                 250                 255
   Asp Leu Phe Thr His Asp Glu Trp Ile Asn Tyr Asp Tyr Leu Gln Ser
               260                 265                 270
   Leu Lys Lys Tyr Tyr Gly His Gly Ala Gly Asn Pro Leu Gly Pro Thr
           275                 280                 285
   Gln Gly Val Gly Tyr Ala Asn Glu Leu Ile Ala Arg Leu Thr His Ser
       290                 295                 300
   Pro Val His Asp Asp Thr Ser Ser Asn His Thr Leu Asp Ser Ser Pro
   305                 310                 315                 320
   Ala Thr Phe Pro Leu Asn Ser Thr Leu Tyr Ala Asp Phe Ser His Asp
                   325                 330                 335
   Asn Gly Ile Ile Ser Ile Leu Phe Ala Leu Gly Leu Tyr Asn Gly Thr
               340                 345                 350
   Lys Pro Leu Ser Thr Thr Thr Val Glu Asn Ile Thr Gln Thr Asp Gly
           355                 360                 365
   Phe Ser Ser Ala Trp Thr Val Pro Phe Ala Ser Arg Leu Tyr Val Glu
       370                 375                 380
   Met Met Gln Cys Gln Ala Glu Gln Glu Pro Leu Val Arg Val Leu Val
   385                 390                 395                 400
   Asn Asp Arg Val Val Pro Leu His Gly Cys Pro Val Asp Ala Leu Gly
                   405                 410                 415
   Arg Cys Thr Arg Asp Ser Phe Val Arg Gly Leu Ser Phe Ala Arg Ser
               420                 425                 430
   Gly Gly Asp Trp Ala Glu Cys Phe Ala
           435                 440
<210> 6
   <211> 440
   <212> PRT
   <213> Aspergillus fumigatus 13073
<400> 6
   Gly Ser Lys Ser Cys Asp Thr Val Asp Leu Gly Tyr Gln Cys Ser Pro
    1               5                  10                  15
   Ala Thr Ser His Leu Trp Gly Gln Tyr Ser Pro Phe Phe Ser Leu Glu
                   20                  25                  30
   Asp Glu Leu Ser Val Ser Ser Lys Leu Pro Lys Asp Cys Arg Ile Thr
               35                  40                  45
   Leu Val Gln Val Leu Ser Arg His Gly Ala Arg Tyr Pro Thr Ser Ser
       50                  55                  60
   Lys Ser Lys Lys Tyr Lys Lys Leu Val Thr Ala Ile Gln Ala Asn Ala
   65                  70                  75                  80
   Thr Asp Phe Lys Gly Lys Phe Ala Phe Leu Lys Thr Tyr Asn Tyr Thr
                   85                  90                  95
   Leu Gly Ala Asp Asp Leu Thr Pro Phe Gly Glu Gln Gln Leu Val Asn
               100                 105                 110
   Ser Gly  Ile Lys Phe Tyr Gln Arg Tyr Lys Ala Leu Ala Arg Ser Val
           115                 120                 125
   Val Pro  Phe Ile Arg Ala Ser Gly Ser Asp Arg Val Ile Ala Ser Gly
       130                 135                 140
   Glu Lys  Phe Ile Glu Gly Phe Gln Gln Ala Lys Leu Ala Asp Pro Gly
   145                 150                 155                 160
   Ala Thr Asn Arg Ala Ala Pro Ala Ile Ser Val Ile Ile Pro Glu Ser
                   165                 170                 175
   Glu Thr Phe Asn Asn Thr Leu Asp His Gly Val Cys Thr Lys Phe Glu
               180                 185                 190
   Ala Ser Gln Leu Gly Asp Glu Val Ala Ala Asn Phe Thr Ala Leu Phe
           195                 200                 205
   Ala Pro Asp Ile Arg Ala Arg Ala Glu Lys His Leu Pro Gly Val Thr
       210                 215                 220
   Leu Thr Asp Glu Asp Val Val Ser Leu Met Asp Met Cys Ser Phe Asp
   225                 230                 235                 240
   Thr Val Ala Arg Thr Ser Asp Ala Ser Gln Leu Ser Pro Phe Cys Gln
                   245                 250                 255
   Leu Phe Thr His Asn Glu Trp Lys Lys Tyr Asn Tyr Leu Gln Ser Leu
               260                 265                 270
   Gly Lys Tyr Tyr Gly Tyr Gly Ala Gly Asn Pro Leu Gly Pro Ala Gln
           275                 280                 285
   Gly Ile Gly Phe Thr Asn Glu Leu Ile Ala Arg Leu Thr Arg Ser Pro
       290                 295                 300
   Val Gln Asp His Thr Ser Thr Asn Ser Thr Leu Val Ser Asn Pro Ala
   305 310 315 320
   Thr Phe Pro Leu Asn Ala Thr Met Tyr Val Asp Phe Ser His Asp Asn
   325 330 335
   Ser Met Val Ser Ile Phe Phe Ala Leu Gly Leu Tyr Asn Gly Thr Glu
   340 345 350
   Pro Leu Ser Arg Thr Ser Val Glu Ser Ala Lys Glu Leu Asp Gly Tyr
   355 360 365
   Ser Ala Ser Trp Val Val Pro Phe Gly Ala Arg Ala Tyr Phe Glu Thr
   370 375 380
   Met Gln Cys Lys Ser Glu Lys Glu Pro Leu Val Arg Ala Leu Ile Asn
   385 390 395 400
   Asp Arg Val Val Pro Leu His Gly Cys Asp Val Asp Lys Leu Gly Arg
   405 410 415
   Cys Lys Leu Asn Asp Phe Val Lys Gly Leu Ser Trp Ala Arg Ser Gly
   420 425 430
   Gly Asn Trp Gly Glu Cys Phe Ser
   435 440
<210> 7
   <211> 440
   <212> PRT
   <213> Aspergillus fumigatus 32722
<400> 7
   Gly Ser Lys Ser Cys Asp Thr Val Asp Leu Gly Tyr Gln Cys Ser Pro
   1 5 10 15
   Ala Thr Ser His Leu Trp Gly Gln Tyr Ser Pro Phe Phe Ser Leu Glu
   20 25 30
   Asp Glu Leu Ser Val Ser Ser Lys Leu Pro Lys Asp Cys Arg Ile Thr
   35 40 45
   Leu Val Gln Val Leu Ser Arg His Gly Ala Arg Tyr Pro Thr Ser Ser
   50 55 60
   Lys Ser Lys Lys Tyr Lys Lys Leu Val Thr Ala Ile Gln Ala Asn Ala
   65 70 75 80
   Thr Asp Phe Lys Gly Lys Phe Ala Phe Leu Lys Thr Tyr Asn Tyr Thr
   85 90 95
   Leu Gly Ala Asp Asp Leu Thr Pro Phe Gly Glu Gln Gln Leu Val Asn
               100                 105                 110
   Ser Gly Ile Lys Phe Tyr Gln Arg Tyr Lys Ala Leu Ala Arg Ser Val
           115                 120                 125
   Val Pro Phe Ile Arg Ala Ser Gly Ser Asp Arg Val Ile Ala Ser Gly
       130                 135                 140
   Glu Lys Phe Ile Glu Gly Phe Gln Gln Ala Lys Leu Ala Asp Pro Gly
   145                 150                 155                 160
   Ala Thr Asn Arg Ala Ala Pro Ala Ile Ser Val Ile Ile Pro Glu Ser
                   165                 170                 175
   Glu Thr Phe Asn Asn Thr Leu Asp His Gly Val Cys Thr Lys Phe Glu
               180                 185                 190
   Ala Ser Gln Leu Gly Asp Glu Val Ala Ala Asn Phe Thr Ala Leu Phe
           195                 200                 205
   Ala Pro Asp Ile Arg Ala Arg Ala Glu Lys His Leu Pro Gly Val Thr
       210                 215                 220
   Leu Thr Asp Glu Asp Val Val Ser Leu Met Asp Met Cys Ser Phe Asp
   225                 230                 235                 240
   Thr Val Ala Arg Thr Ser Asp Ala Ser Gln Leu Ser Pro Phe Cys Gln
                   245                 250                 255
   Leu Phe Thr His Asn Glu Trp Lys Lys Tyr Asn Tyr Leu Gln Ser Leu
               260                 265                 270
   Gly Lys Tyr Tyr Gly Tyr Gly Ala Gly Asn Pro Leu Gly Pro Ala Gln
           275                 280                 285
   Gly Ile Gly Phe Thr Asn Glu Leu Ile Ala Arg Leu Thr Arg Ser Pro
       290                 295                 300
   Val Gln Asp His Thr Ser Thr Asn Ser Thr Leu Val Ser Asn Pro Ala
   305                 310                 315                 320
   Thr Phe Pro Leu Asn Ala Thr Met Tyr Val Asp Phe Ser His Asp Asn
                   325                 330                 335
   Ser Met Val Ser Ile Phe Phe Ala Leu Gly Leu Tyr Asn Gly Thr Gly
               340                 345                 350
   Pro Leu Ser Arg Thr Ser Val Glu Ser Ala Lys Glu Leu Asp Gly Tyr
           355                 360                 365
   Ser Ala Ser Trp Val Val Pro Phe Gly Ala Arg Ala Tyr Phe Glu Thr
       370                 375                 380
   Met Gln Cys Lys Ser Glu Lys Glu Pro Leu Val Arg Ala Leu Ile Asn
   385 390 395 400
   Asp Arg Val Val Pro Leu His Gly Cys Asp Val Asp Lys Leu Gly Arg
   405 410 415
   Cys Lys Leu Asn Asp Phe Val Lys Gly Leu Ser Trp Ala Arg Ser Gly
   420 425 430
   Gly Asn Trp Gly Glu Cys Phe Ser
   435 440
<210> 8
   <211> 440
   <212> PRT
   <213> Aspergillus fumigatus 58128
<400> 8
   Gly Ser Lys Ser Cys Asp Thr Val Asp Leu Gly Tyr Gln Cys Ser Pro
   1 5 10 15
   Ala Thr Ser His Leu Trp Gly Gln Tyr Ser Pro Phe Phe Ser Leu Glu
   20 25 30
   Asp Glu Leu Ser Val Ser Ser Lys Leu Pro Lys Asp Cys Arg Ile Thr
   35 40 45
   Leu Val Gln Val Leu Ser Arg His Gly Ala Arg Tyr Pro Thr Ser Ser
   50 55 60
   Lys Ser Lys Lys Tyr Lys Lys Leu Val Thr Ala Ile Gln Ala Asn Ala
   65 70 75 80
   Thr Asp Phe Lys Gly Lys Phe Ala Phe Leu Lys Thr Tyr Asn Tyr Thr
   85 90 95
   Leu Gly Ala Asp Asp Leu Thr Pro Phe Gly Glu Gln Gln Leu Val Asn
   100 105 110
   Ser Gly Ile Lys Phe Tyr Gln Arg Tyr Lys Ala Leu Ala Arg Ser Val
   115 120 125
   Val Pro Phe Ile Arg Ala Ser Gly Ser Asp Arg Val Ile Ala Ser Gly
   130 135 140
   Glu Lys Phe Ile Glu Gly Phe Gln Gln Ala Lys Leu Ala Asp Pro Gly
   145 150 155 160
   Ala Thr Asn Arg Ala Ala Pro Ala Ile Ser Val Ile Ile Pro Glu Ser
   165 170 175
   Glu Thr Phe Asn Asn Thr Leu Asp His Gly Val Cys Thr Lys Phe Glu
   180 185 190
   Ala Ser Gln Leu Gly Asp Glu Val Ala Ala Asn Phe Thr Ala Leu Phe
           195                 200                 205
   Ala Pro Asp Ile Arg Ala Arg Ala Glu Lys His Leu Pro Gly Val Thr
       210                 215                 220
   Leu Thr Asp Glu Asp Val Val Ser Leu Met Asp Met Cys Ser Phe Asp
   225                 230                 235                 240
   Thr Val Ala Arg Thr Ser Asp Ala Ser Gln Leu Ser Pro Phe Cys Gln
                   245                 250                 255
   Leu Phe Thr His Asn Glu Trp Lys Lys Tyr Asn Tyr Leu Gln Ser Leu
               260                 265                 270
   Gly Lys Tyr Tyr Gly Tyr Gly Ala Gly Asn Pro Leu Gly Pro Ala Gln
           275                 280                 285
   Gly Ile Gly Phe Thr Asn Glu Leu Ile Ala Arg Leu Thr Arg Ser Pro
       290                 295                 300
   Val Gln Asp His Thr Ser Thr Asn Ser Thr Leu Val Ser Asn Pro Ala
   305                 310                 315                 320
   Thr Phe Pro Leu Asn Ala Thr Met Tyr Val Asp Phe Ser His Asp Asn
                   325                 330                 335
   Ser Met Val Ser Ile Phe Phe Ala Leu Gly Leu Tyr Asn Gly Thr Glu
               340                 345                 350
   Pro Leu Ser Arg Thr Ser Val Glu Ser Ala Lys Glu Leu Asp Gly Tyr
           355                 360                 365
   Ser Ala Ser Trp Val Val Pro Phe Gly Ala Arg Ala Tyr Phe Glu Thr
       370                 375                 380
   Met Gln Cys Lys Ser Glu Lys Glu Ser Leu Val Arg Ala Leu Ile Asn
   385                 390                 395                 400
   Asp Arg Val Val Pro Leu His Gly Cys Asp Val Asp Lys Leu Gly Arg
                   405                 410                 415
   Cys Lys Leu Asn Asp Phe Val Lys Gly Leu Ser Trp Ala Arg Ser Gly
               420                 425                 430
   Gly Asn Trp Gly Glu Cys Phe Ser
           435                 440
<210> 9
   <211> 440
   <212> PRT
   <213> Aspergillus fumigatus 26906
<400> 9
   Gly Ser Lys Ser Cys Asp Thr Val Asp Leu Gly Tyr Gln Cys Ser Pro
    1               5                  10                  15
   Ala Thr Ser His Leu Trp Gly Gln Tyr Ser Pro Phe Phe Ser Leu Glu
               20                  25                  30
   Asp Glu Leu Ser Val Ser Ser Lys Leu Pro Lys Asp Cys Arg Ile Thr
           35                  40                  45
   Leu Val Gln Val Leu Ser Arg His Gly Ala Arg Tyr Pro Thr Ser Ser
       50                  55                  60
   Lys Ser Lys Lys Tyr Lys Lys Leu Val Thr Ala Ile Gln Ala Asn Ala
   65                  70                  75                  80
   Thr Asp Phe Lys Gly Lys Phe Ala Phe Leu Lys Thr Tyr Asn Tyr Thr
                   85                  90                  95
   Leu Gly Ala Asp Asp Leu Thr Ala Phe Gly Glu Gln Gln Leu Val Asn
               100                 105                 110
   Ser Gly Ile Lys Phe Tyr Gln Arg Tyr Lys Ala Leu Ala Arg Ser Val
           115                 120                 125
   Val Pro Phe Ile Arg Ala Ser Gly Ser Asp Arg Val Ile Ala Ser Gly
       130                 135                 140
   Glu Lys Phe Ile Glu Gly Phe Gln Gln Ala Lys Leu Ala Asp Pro Gly
   145                 150                 155                 160
   Ala Thr Asn Arg Ala Ala Pro Ala Ile Ser Val Ile Ile Pro Glu Ser
                   165                 170                 175
   Glu Thr Phe Asn Asn Thr Leu Asp His Gly Val Cys Thr Lys Phe Glu
               180                 185                 190
   Ala Ser Gln Leu Gly Asp Glu Val Ala Ala Asn Phe Thr Ala Leu Phe
           195                 200                 205
   Ala Pro Asp Ile Arg Ala Arg Ala Lys Lys His Leu Pro Gly Val Thr
       210                 215                 220
   Leu Thr Asp Glu Asp Val Val Ser Leu Met Asp Met Cys Ser Phe Asp
   225                 230                 235                 240
   Thr Val Ala Arg Thr Ser Asp Ala Ser Gln Leu Ser Pro Phe Cys Gln
                   245                 250                 255
   Leu Phe Thr His Asn Glu Trp Lys Lys Tyr Asn Tyr Leu Gln Ser Leu
               260                 265                 270
   Gly Lys Tyr Tyr Gly Tyr Gly Ala Gly Asn Pro Leu Gly Pro Ala Gln
           275                 280                 285
   Gly Ile Gly Phe Thr Asn Glu Leu Ile Ala Arg Leu Thr Arg Ser Pro
       290                 295                 300
   Val Gln Asp His Thr Ser Thr Asn Ser Thr Leu Val Ser Asn Pro Ala
   305                 310                 315                 320
   Thr Phe Pro Leu Asn Ala Thr Met Tyr Val Asp Phe Ser His Asp Asn
                   325                 330                 335
   Ser Met Val Ser Ile Phe Phe Ala Leu Gly Leu Tyr Asn Gly Thr Glu
               340                 345                 350
   Pro Leu Ser Arg Thr Ser Val Glu Ser Ala Lys Glu Leu Asp Gly Tyr
           355                 360                 365
   Ser Ala Ser Trp Val Val Pro Phe Gly Ala Arg Ala Tyr Phe Glu Thr
       370                 375                 380
   Met Gln Cys Lys Ser Glu Lys Glu Pro Leu Val Arg Ala Leu Ile Asn
   385                 390                 395                 400
   Asp Arg Val Val Pro Leu His Gly Cys Asp Val Asp Lys Leu Gly Arg
                   405                 410                 415
   Cys Lys Leu Asn Asp Phe Val Lys Gly Leu Ser Trp Ala Arg Ser Gly
               420                 425                 430
   Gly Asn Trp Gly Glu Cys Phe Ser
           435                 440
<210> 10
   <211> 440
   <212> PRT
   <213> Aspergillus fumigatus 32239
<400> 10
   Gly Ser Lys Ala Cys Asp Thr Val Glu Leu Gly Tyr Gln Cys Ser Pro
    1               5                  10                  15
   Gly Thr Ser His Leu Trp Gly Gln Tyr Ser Pro Phe Phe Ser Leu Glu
               20                  25                  30
   Asp Glu Leu Ser Val Ser Ser Asp Leu Pro Lys Asp Cys Arg Val Thr
           35                  40                  45
   Phe Val Gln Val Leu Ser Arg His Gly Ala Arg Tyr Pro Thr Ala Ser
       50                  55                  60
   Lys Ser Lys Lys Tyr Lys Lys Leu Val Thr Ala Ile Gln Lys Asn Ala
   65                  70                  75                  80
   Thr Glu Phe Lys Gly Lys Phe Ala Phe Leu Glu Thr Tyr Asn Tyr Thr
                   85                  90                  95
   Leu Gly Ala Asp Asp Leu Thr Pro Phe Gly Glu Gln Gln Met Val Asn
               100                 105                 110
   Ser Gly Ile Lys Phe Tyr Gln Lys Tyr Lys Ala Leu Ala Gly Ser Val
           115                 120                 125
   Val Pro Phe Ile Arg Ser Ser Gly Ser Asp Arg Val Ile Ala Ser Gly
       130                 135                 140
   Glu Lys Phe Ile Glu Gly Phe Gln Gln Ala Asn Val Ala Asp Pro Gly
   145                 150                 155                 160
   Ala Thr Asn Arg Ala Ala Pro Val Ile Ser Val Ile Ile Pro Glu Ser
                   165                 170                 175
   Glu Thr Tyr Asn Asn Thr Leu Asp His Ser Val Cys Thr Asn Phe Glu
               180                 185                 190
   Ala Ser Glu Leu Gly Asp Glu Val Glu Ala Asn Phe Thr Ala Leu Phe
           195                 200                 205
   Ala Pro Ala Ile Arg Ala Arg Ile Glu Lys His Leu Pro Gly Val Gln
       210                 215                 220
   Leu Thr Asp Asp Asp Val Val Ser Leu Met Asp Met Cys Ser Phe Asp
   225                 230                 235                 240
   Thr Val Ala Arg Thr Ala Asp Ala Ser Glu Leu Ser Pro Phe Cys Ala
                   245                 250                 255
   Ile Phe Thr His Asn Glu Trp Lys Lys Tyr Asp Tyr Leu Gln Ser Leu
               260                 265                 270
   Gly Lys Tyr Tyr Gly Tyr Gly Ala Gly Asn Pro Leu Gly Pro Ala Gln
           275                 280                 285
   Gly Ile Gly Phe Thr Asn Glu Leu Ile Ala Arg Leu Thr Asn Ser Pro
       290                 295                 300
   Val Gln Asp His Thr Ser Thr Asn Ser Thr Leu Asp Ser Asp Pro Ala
   305                 310                 315                 320
   Thr Phe Pro Leu Asn Ala Thr Ile Tyr Val Asp Phe Ser His Asp Asn
                   325                 330                 335
   Gly Met Ile Pro Ile Phe Phe Ala Met Gly Leu Tyr Asn Gly Thr Glu
               340                 345                 350
   Pro Leu Ser Gln Thr Ser Glu Glu Ser Thr Lys Glu Ser Asn Gly Tyr
           355                 360                 365
   Ser Ala Ser Trp Ala Val Pro Phe Gly Ala Arg Ala Tyr Phe Glu Thr
   370 375 380
   Met Gln Cys Lys Ser Glu Lys Glu Pro Leu Val Arg Ala Leu Ile Asn
   385 390 395 400
   Asp Arg Val Val Pro Leu His Gly Cys Ala Val Asp Lys Leu Gly Arg
   405 410 415
   Cys Lys Leu Lys Asp Phe Val Lys Gly Leu Ser Trp Ala Arg Ser Gly
   420 425 430
   Gly Asn Ser Glu Gln Ser Phe Ser
   435 440
<210> 11
   <211> 439
   <212> PRT
   <213> Emericella nidulans
<400> 11
   Gln Asn His Ser Cys Asn Thr Ala Asp Gly Gly Tyr Gln Cys Phe Pro
   1 5 10 15
   Asn Val Ser His Val Trp Gly Gln Tyr Ser Pro Tyr Phe Ser Ile Glu
   20 25 30
   Gln Glu Ser Ala Ile Ser Glu Asp Val Pro His Gly Cys Glu Val Thr
   35 40 45
   Phe Val Gln Val Leu Ser Arg His Gly Ala Arg Tyr Pro Thr Glu Ser
   50 55 60
   Lys Ser Lys Ala Tyr Ser Gly Leu Ile Glu Ala Ile Gln Lys Asn Ala
   65 70 75 80
   Thr Ser Phe Trp Gly Gln Tyr Ala Phe Leu Glu Ser Tyr Asn Tyr Thr
   85 90 95
   Leu Gly Ala Asp Asp Leu Thr Ile Phe Gly Glu Asn Gln Met Val Asp
   100 105 110
   Ser Gly Ala Lys Phe Tyr Arg Arg Tyr Lys Asn Leu Ala Arg Lys Asn
   115 120 125
   Thr Pro Phe Ile Arg Ala Ser Gly Ser Asp Arg Val Val Ala Ser Ala
   130 135 140
   Glu Lys Phe Ile Asn Gly Phe Arg Lys Ala Gln Leu His Asp His Gly
   145 150 155 160
   Ser Gly Gln Ala Thr Pro Val Val Asn Val Ile Ile Pro Glu Ile Asp
   165 170 175
   Gly Phe Asn Asn Thr Leu Asp His Ser Thr Cys Val Ser Phe Glu Asn
   180 185 190
   Asp Glu Arg Ala Asp Glu Ile Glu Ala Asn Phe Thr Ala Ile Met Gly
   195 200 205
   Pro Pro Ile Arg Lys Arg Leu Glu Asn Asp Leu Pro Gly Ile Lys Leu
   210 215 220
   Thr Asn Glu Asn Val Ile Tyr Leu Met Asp Met Cys Ser Phe Asp Thr
   225 230 235 240
   Met Ala Arg Thr Ala His Gly Thr Glu Leu Ser Pro Phe Cys Ala Ile
   245 250 255
   Phe Thr Glu Lys Glu Trp Leu Gln Tyr Asp Tyr Leu Gln Ser Leu Ser
   260 265 270
   Lys Tyr Tyr Gly Tyr Gly Ala Gly Ser Pro Leu Gly Pro Ala Gln Gly
   275 280 285
   Ile Gly Phe Thr Asn Glu Leu Ile Ala Arg Leu Thr Gln Ser Pro Val
   290 295 300
   Gln Asp Asn Thr Ser Thr Asn His Thr Leu Asp Ser Asn Pro Ala Thr
   305 310 315 320
   Phe Pro Leu Asp Arg Lys Leu Tyr Ala Asp Phe Ser His Asp Asn Ser
   325 330 335
   Met Ile Ser Ile Phe Phe Ala Met Gly Leu Tyr Asn Gly Thr Gln Pro
   340 345 350
   Leu Ser Met Asp Ser Val Glu Ser Ile Gln Glu Met Asp Gly Tyr Ala
   355 360 365
   Ala Ser Trp Thr Val Pro Phe Gly Ala Arg Ala Tyr Phe Glu Leu Met
   370 375 380
   Gln Cys Glu Lys Lys Glu Pro Leu Val Arg Val Leu Val Asn Asp Arg
   385 390 395 400
   Val Val Pro Leu His Gly Cys Ala Val Asp Lys Phe Gly Arg Cys Thr
   405 410 415
   Leu Asp Asp Trp Val Glu Gly Leu Asn Phe Ala Arg Ser Gly Gly Asn
   420 425 430
   Trp Lys Thr Cys Phe Thr Leu
   435
<210> 12
   <211> 443
   <212> PRT
   <213> Talaromyces thermophilus
<400> 12
   Asp Ser His Ser Cys Asn Thr Val Glu Gly Gly Tyr Gln Cys Arg Pro
    1               5                  10                  15
   Glu Ile Ser His Ser Trp Gly Gln Tyr Ser Pro Phe Phe Ser Leu Ala
               20                  25                  30
   Asp Gln Ser Glu Ile Ser Pro Asp Val Pro Gln Asn Cys Lys Ile Thr
           35                  40                  45
   Phe Val Gln Leu Leu Ser Arg His Gly Ala Arg Tyr Pro Thr Ser Ser
       50                  55                  60
   Lys Thr Glu Leu Tyr Ser Gln Leu Ile Ser Arg Ile Gln Lys Thr Ala
   65                  70                  75                  80
   Thr Ala Tyr Lys Gly Tyr Tyr Ala Phe Leu Lys Asp Tyr Arg Tyr Gln
                   85                  90                  95
   Leu Gly Ala Asn Asp Leu Thr Pro Phe Gly Glu Asn Gln Met Ile Gln
               100                 105                 110
   Leu Gly Ile Lys Phe Tyr Asn His Tyr Lys Ser Leu Ala Arg Asn Ala
           115                 120                 125
   Val Pro Phe Val Arg Cys Ser Gly Ser Asp Arg Val Ile Ala Ser Gly
       130                 135                 140
   Arg Leu Phe Ile Glu Gly Phe Gln Ser Ala Lys Val Leu Asp Pro His
   145                 150                 155                 160
   Ser Asp Lys His Asp Ala Pro Pro Thr Ile Asn Val Ile Ile Glu Glu
                   165                 170                 175
   Gly Pro Ser Tyr Asn Asn Thr Leu Asp Thr Gly Ser Cys Pro Val Phe
               180                 185                 190
   Glu Asp Ser Ser Gly Gly His Asp Ala Gln Glu Lys Phe Ala Lys Gln
           195                 200                 205
   Phe Ala Pro Ala Ile Leu Glu Lys Ile Lys Asp His Leu Pro Gly Val
       210                 215                 220
   Asp Leu Ala Val Ser Asp Val Pro Tyr Leu Met Asp Leu Cys Pro Phe
   225                 230                 235                 240
   Glu Thr Leu Ala Arg Asn His Thr Asp Thr Leu Ser Pro Phe Cys Ala
                   245                 250                 255
   Leu Ser Thr Gln Glu Glu Trp Gln Ala Tyr Asp Tyr Tyr Gln Ser Leu
   260 265 270
   Gly Lys Tyr Tyr Gly Asn Gly Gly Gly Asn Pro Leu Gly Pro Ala Gln
   275 280 285
   Gly Val Gly Phe Val Asn Glu Leu Ile Ala Arg Met Thr His Ser Pro
   290 295 300
   Val Gln Asp Tyr Thr Thr Val Asn His Thr Leu Asp Ser Asn Pro Ala
   305 310 315 320
   Thr Phe Pro Leu Asn Ala Thr Leu Tyr Ala Asp Phe Ser His Asp Asn
   325 330 335
   Thr Met Thr Ser Ile Phe Ala Ala Leu Gly Leu Tyr Asn Gly Thr Ala
   340 345 350
   Lys Leu Ser Thr Thr Glu Ile Lys Ser Ile Glu Glu Thr Asp Gly Tyr
   355 360 365
   Ser Ala Ala Trp Thr Val Pro Phe Gly Gly Arg Ala Tyr Ile Glu Met
   370 375 380
   Met Gln Cys Asp Asp Ser Asp Glu Pro Val Val Arg Val Leu Val Asn
   385 390 395 400
   Asp Arg Val Val Pro Leu His Gly Cys Glu Val Asp Ser Leu Gly Arg
   405 410 415
   Cys Lys Arg Asp Asp Phe Val Arg Gly Leu Ser Phe Ala Arg Gln Gly
   420 425 430
   Gly Asn Trp Glu Gly Cys Tyr Ala Ala Ser Glu
   435 440
<210> 13
   <211> 466
   <212> PRT
   <213> Myceliophthora thermophila
<400> 13
   Glu Ser Arg Pro Cys Asp Thr Pro Asp Leu Gly Phe Gln Cys Gly Thr
   1 5 10 15
   Ala Ile Ser His Phe Trp Gly Gln Tyr Ser Pro Tyr Phe Ser Val Pro
   20 25 30
   Ser Glu Leu Asp Ala Ser Ile Pro Asp Asp Cys Glu Val Thr Phe Ala
   35 40 45
   Gln Val Leu Ser Arg His Gly Ala Arg Ala Pro Thr Leu Lys Arg Ala
        50                  55                  60
   Ala Ser Tyr Val Asp Leu Ile Asp Arg Ile His His Gly Ala Ile Ser
    65                  70                  75                  80
   Tyr Gly Pro Gly Tyr Glu Phe Leu Arg Thr Tyr Asp Tyr Thr Leu Gly
                    85                  90                  95
   Ala Asp Glu Leu Thr Arg Thr Gly Gln Gln Gln Met Val Asn Ser Gly
               100                 105                 110
   Ile Lys Phe Tyr Arg Arg Tyr Arg Ala Leu Ala Arg Lys Ser Ile Pro
           115                 120                 125
   Phe Val Arg Thr Ala Gly Gln Asp Arg Val Val His Ser Ala Glu Asn
       130                 135                 140
   Phe Thr Gln Gly Phe His Ser Ala Leu Leu Ala Asp Arg Gly Ser Thr
   145                 150                 155                 160
   Val Arg Pro Thr Leu Pro Tyr Asp Met Val Val Ile Pro Glu Thr Ala
                   165                 170                 175
   Gly Ala Asn Asn Thr Leu His Asn Asp Leu Cys Thr Ala Phe Glu Glu
               180                 185                 190
   Gly Pro Tyr Ser Thr Ile Gly Asp Asp Ala Gln Asp Thr Tyr Leu Ser
           195                 200                 205
   Thr Phe Ala Gly Pro Ile Thr Ala Arg Val Asn Ala Asn Leu Pro Gly
       210                 215                 220
   Ala Asn Leu Thr Asp Ala Asp Thr Val Ala Leu Met Asp Leu Cys Pro
   225                 230                 235                 240
   Phe Glu Thr Val Ala Ser Ser Ser Ser Asp Pro Ala Thr Ala Asp Ala
                   245                 250                 255
   Gly Gly Gly Asn Gly Arg Pro Leu Ser Pro Phe Cys Arg Leu Phe Ser
               260                 265                 270
   Glu Ser Glu Trp Arg Ala Tyr Asp Tyr Leu Gln Ser Val Gly Lys Trp
           275                 280                 285
   Tyr Gly Tyr Gly Pro Gly Asn Pro Leu Gly Pro Thr Gln Gly Val Gly
       290                 295                 300
   Phe Val Asn Glu Leu Leu Ala Arg Leu Ala Gly Val Pro Val Arg Asp
   305                 310                 315                 320
   Gly Thr Ser Thr Asn Arg Thr Leu Asp Gly Asp Pro Arg Thr Phe Pro
                   325                 330                 335
   Leu Gly Arg Pro Leu Tyr Ala Asp Phe Ser His Asp Asn Asp Met Met
   340 345 350
   Gly Val Leu Gly Ala Leu Gly Ala Tyr Asp Gly Val Pro Pro Leu Asp
   355 360 365
   Lys Thr Ala Arg Arg Asp Pro Glu Glu Leu Gly Gly Tyr Ala Ala Ser
   370 375 380
   Trp Ala Val Pro Phe Ala Ala Arg Ile Tyr Val Glu Lys Met Arg Cys
   385 390 395 400
   Ser Gly Gly Gly Gly Gly Gly Gly Gly Gly Glu Gly Arg Gln Glu Lys
   405 410 415
   Asp Glu Glu Met Val Arg Val Leu Val Asn Asp Arg Val Met Thr Leu
   420 425 430
   Lys Gly Cys Gly Ala Asp Glu Arg Gly Met Cys Thr Leu Glu Arg Phe
   435 440 445
   Ile Glu Ser Met Ala Phe Ala Arg Gly Asn Gly Lys Trp Asp Leu Cys
   450 455 460
   Phe Ala
   465
<210> 14
   <211> 441
   <212> PRT
   <213> Artificial Sequence
<220>
   <223> Description of Artificial Sequence: consensus phytase
<400> 14
   Asn Ser His Ser Cys Asp Thr Val Asp Gly Gly Tyr Gln Cys Phe Pro
   1 5 10 15
   Glu Ile Ser His Leu Trp Gly Gln Tyr Ser Pro Tyr Phe Ser Leu Glu
   20 25 30
   Asp Glu Ser Ala Ile Ser Pro Asp Val Pro Asp Asp Cys Arg Val Thr
   35 40 45
   Phe Val Gln Val Leu Ser Arg His Gly Ala Arg Tyr Pro Thr Ser Ser
   50 55 60
   Lys Ser Lys Ala Tyr Ser Ala Leu Ile Glu Ala Ile Gln Lys Asn Ala
   65 70 75 80
   Thr Ala Phe Lys Gly Lys Tyr Ala Phe Leu Lys Thr Tyr Asn Tyr Thr
                   85                  90                  95
   Leu Gly Ala Asp Asp Leu Thr Pro Phe Gly Glu Asn Gln Met Val Asn
               100                 105                 110
   Ser Gly Ile Lys Phe Tyr Arg Arg Tyr Lys Ala Leu Ala Arg Lys Ile
           115                 120                 125
   Val Pro Phe Ile Arg Ala Ser Gly Ser Asp Arg Val Ile Ala Ser Ala
       130                 135                 140
   Glu Lys Phe Ile Glu Gly Phe Gln Ser Ala Lys Leu Ala Asp Pro Gly
   145                 150                 155                 160
   Ser Gln Pro His Gln Ala Ser Pro Val Ile Asp Val Ile Ile Pro Glu
                   165                 170                 175
   Gly Ser Gly Tyr Asn Asn Thr Leu Asp His Gly Thr Cys Thr Ala Phe
               180                 185                 190
   Glu Asp Ser Glu Leu Gly Asp Asp Val Glu Ala Asn Phe Thr Ala Leu
           195                 200                 205
   Phe Ala Pro Ala Ile Arg Ala Arg Leu Glu Ala Asp Leu Pro Gly Val
       210                 215                 220
   Thr Leu Thr Asp Glu Asp Val Val Tyr Leu Met Asp Met Cys Pro Phe
   225                 230                 235                 240
   Glu Thr Val Ala Arg Thr Ser Asp Ala Thr Glu Leu Ser Pro Phe Cys
                   245                 250                 255
   Ala Leu Phe Thr His Asp Glu Trp Arg Gln Tyr Asp Tyr Leu Gln Ser
               260                 265                 270
   Leu Gly Lys Tyr Tyr Gly Tyr Gly Ala Gly Asn Pro Leu Gly Pro Ala
           275                 280                 285
   Gln Gly Val Gly Phe Ala Asn Glu Leu Ile Ala Arg Leu Thr Arg Ser
       290                 295                 300
   Pro Val Gln Asp His Thr Ser Thr Asn His Thr Leu Asp Ser Asn Pro
   305                 310                 315                 320
   Ala Thr Phe Pro Leu Asn Ala Thr Leu Tyr Ala Asp Phe Ser His Asp
                   325                 330                 335
   Asn Ser Met Ile Ser Ile Phe Phe Ala Leu Gly Leu Tyr Asn Gly Thr
               340                 345                 350
   Ala Pro Leu Ser Thr Thr Ser Val Glu Ser Ile Glu Glu Thr Asp Gly
           355                 360                 365
   Tyr Ser Ala Ser Trp Thr Val Pro Phe Gly Ala Arg Ala Tyr Val Glu
       370                 375                 380
   Met Met Gln Cys Gln Ala Glu Lys Glu Pro Leu Val Arg Val Leu Val
   385                 390                 395                 400
   Asn Asp Arg Val Val Pro Leu His Gly Cys Ala Val Asp Lys Leu Gly
                   405                 410                 415
   Arg Cys Lys Arg Asp Asp Phe Val Glu Gly Leu Ser Phe Ala Arg Ser
               420                 425                 430
   Gly Gly Asn Trp Ala Glu Cys Phe Ala
           435                 440
<210> 15
   <211> 1426
   <212> DNA
   <213> Artificial Sequence
<220>
   <223> Description of Artificial Sequence: consensus phytase
<220>
   <221> CDS
   <222> (12)..(1412)
<220>
   <221> sig_peptide
   <222> (12)..(89)
<220>
   <221> mat_peptide
   <222> (90)..(1412)
<400> 15
   tatatgaatt c atg ggc gtg ttc gtc gtg cta ctg tcc att gcc acc ttg   50
                Met Gly Val Phe Val Val Leu Leu Ser Ile Ala Thr Leu
                    -25                 -20                 -15
   ttc ggt tcc aca tcc ggt acc gcc ttg ggt cct cgt ggt aat tct cac    98
   Phe Gly Ser Thr Ser Gly Thr Ala Leu Gly Pro Arg Gly Asn Ser His
               -10                  -5                  -1   1
   tct tgt gac act gtt gac ggt ggt tac caa tgt ttc cca gaa att tct   146
   Ser Cys Asp Thr Val Asp Gly Gly Tyr Gln Cys Phe Pro Glu Ile Ser
         5                  10                  15
   cac ttg tgg ggt caa tac tct cca tac ttc tct ttg gaa gac gaa tct   194
   His Leu Trp Gly Gln Tyr Ser Pro Tyr Phe Ser Leu Glu Asp Glu Ser
    20                  25                  30                  35
   gct att tct cca gac gtt cca gac gac tgt aga gtt act ttc gtt caa    242
   Ala Ile Ser Pro Asp Val Pro Asp Asp Cys Arg Val Thr Phe Val Gln
                   40                  45                  50
   gtt ttg tct aga cac ggt gct aga tac cca act tct tct aag tct aag    290
   Val Leu Ser Arg His Gly Ala Arg Tyr Pro Thr Ser Ser Lys Ser Lys
               55                  60                  65
   gct tac tct gct ttg att gaa gct att caa aag aac gct act gct ttc    338
   Ala Tyr Ser Ala Leu Ile Glu Ala Ile Gln Lys Asn Ala Thr Ala Phe
           70                  75                  80
   aag ggt aag tac gct ttc ttg aag act tac aac tac act ttg ggt gct    386
   Lys Gly Lys Tyr Ala Phe Leu Lys Thr Tyr Asn Tyr Thr Leu Gly Ala
       85                  90                  95
   gac gac ttg act cca ttc ggt gaa aac caa atg gtt aac tct ggt att    434
   Asp Asp Leu Thr Pro Phe Gly Glu Asn Gln Met Val Asn Ser Gly Ile
   100                 105                 110                 115
   aag ttc tac aga aga tac aag gct ttg gct aga aag att gtt cca ttc    482
   Lys Phe Tyr Arg Arg Tyr Lys Ala Leu Ala Arg Lys Ile Val Pro Phe
                   120                 125                 130
   att aga gct tct ggt tct gac aga gtt att gct tct gct gaa aag ttc    530
   Ile Arg Ala Ser Gly Ser Asp Arg Val Ile Ala Ser Ala Glu Lys Phe
               135                 140                 145
   att gaa ggt ttc caa tct gct aag ttg gct gac cca ggt tct caa cca    578
   Ile Glu Gly Phe Gln Ser Ala Lys Leu Ala Asp Pro Gly Ser Gln Pro
           150                 155                 160
   cac caa gct tct cca gtt att gac gtt att att cca gaa gga tcc ggt    626
   His Gln Ala Ser Pro Val Ile Asp Val Ile Ile Pro Glu Gly Ser Gly
       165                 170                 175
   tac aac aac act ttg gac cac ggt act tgt act gct ttc gaa gac tct    674
   Tyr Asn Asn Thr Leu Asp His Gly Thr Cys Thr Ala Phe Glu Asp Ser
   180                 185                 190                 195
   gaa ttg ggt gac gac gtt gaa gct aac ttc act gct ttg ttc gct cca    722
   Glu Leu Gly Asp Asp Val Glu Ala Asn Phe Thr Ala Leu Phe Ala Pro
                   200                 205                 210
   gct att aga gct aga ttg gaa gct gac ttg cca ggt gtt act ttg act    770
   Ala Ile Arg Ala Arg Leu Glu Ala Asp Leu Pro Gly Val Thr Leu Thr
               215                 220                 225
   gac gaa gac gtt gtt tac ttg atg gac atg tgt cca ttc gaa act gtt    818
   Asp Glu Asp Val Val Tyr Leu Met Asp Met Cys Pro Phe Glu Thr Val
           230                 235                 240
   gct aga act tct gac gct act gaa ttg tct cca ttc tgt gct ttg ttc    866
   Ala Arg Thr Ser Asp Ala Thr Glu Leu Ser Pro Phe Cys Ala Leu Phe
       245                 250                 255
   act cac gac gaa tgg aga caa tac gac tac ttg caa tct ttg ggt aag      914
   Thr His Asp Glu Trp Arg Gln Tyr Asp Tyr Leu Gln Ser Leu Gly Lys
   260                 265                 270                 275
   tac tac ggt tac ggt gct ggt aac cca ttg ggt cca gct caa ggt gtt      962
   Tyr Tyr Gly Tyr Gly Ala Gly Asn Pro Leu Gly Pro Ala Gln Gly Val
                   280                 285                 290
   ggt ttc gct aac gaa ttg att gct aga ttg act aga tct cca gtt caa      1010
   Gly Phe Ala Asn Glu Leu Ile Ala Arg Leu Thr Arg Ser Pro Val Gln
               295                 300                 305
   gac cac act tct act aac cac act ttg gac tct aac cca gct act ttc      1058
   Asp His Thr Ser Thr Asn His Thr Leu Asp Ser Asn Pro Ala Thr Phe
           310                 315                 320
   cca ttg aac gct act ttg tac gct gac ttc tct cac gac aac tct atg      1106
   Pro Leu Asn Ala Thr Leu Tyr Ala Asp Phe Ser His Asp Asn Ser Met
       325                 330                 335
   att tct att ttc ttc gct ttg ggt ttg tac aac ggt act gct cca ttg      1154
   Ile Ser Ile Phe Phe Ala Leu Gly Leu Tyr Asn Gly Thr Ala Pro Leu
   340                 345                 350                 355
   tct act act tct gtt gaa tct att gaa gaa act gac ggt tac tct gct      1202
   Ser Thr Thr Ser Val Glu Ser Ile Glu Glu Thr Asp Gly Tyr Ser Ala
                   360                 365                 370
   tct tgg act gtt cca ttc ggt gct aga gct tac gtt gaa atg atg caa      1250
   Ser Trp Thr Val Pro Phe Gly Ala Arg Ala Tyr Val Glu Met Met Gln
               375                 380                 385
   tgt caa gct gaa aag gaa cca ttg gtt aga gtt ttg gtt aac gac aga      1298
   Cys Gln Ala Glu Lys Glu Pro Leu Val Arg Val Leu Val Asn Asp Arg
           390                 395                 400
   gtt gtt cca ttg cac ggt tgt gct gtt gac aag ttg ggt aga tgt aag      1346
   Val Val Pro Leu His Gly Cys Ala Val Asp Lys Leu Gly Arg Cys Lys
       405                 410                 415
   aga gac gac ttc gtt gaa ggt ttg tct ttc gct aga tct ggt ggt aac      1394
   Arg Asp Asp Phe Val Glu Gly Leu Ser Phe Ala Arg Ser Gly Gly Asn
   420                 425                 430                 435
   tgg gct gaa tgt ttc gct taagaattca tata                              1426
   Trp Ala Glu Cys Phe Ala
                   440
<210> 16
   <211> 467
   <212> PRT
   <213> Artificial Sequence
   <223> Description of Artificial Sequence: consensus phytase
<400> 16
   Met Gly Val Phe Val Val Leu Leu Ser Ile Ala Thr Leu Phe Gly Ser
       -25                 -20                 -15
   Thr Ser Gly Thr Ala Leu Gly Pro Arg Gly Asn Ser His Ser Cys Asp
   -10                 -5                  -1   1               5
   Thr Val Asp Gly Gly Tyr Gln Cys Phe Pro Glu Ile Ser His Leu Trp
                   10                  15                  20
   Gly Gln Tyr Ser Pro Tyr Phe Ser Leu Glu Asp Glu Ser Ala Ile Ser
               25                  30                  35
   Pro Asp Val Pro Asp Asp Cys Arg Val Thr Phe Val Gln Val Leu Ser
        40                  45                  50
   Arg His Gly Ala Arg Tyr Pro Thr Ser Ser Lys Ser Lys Ala Tyr Ser
    55                  60                  65                  70
   Ala Leu Ile Glu Ala Ile Gln Lys Asn Ala Thr Ala Phe Lys Gly Lys
                       75                  80                  85
   Tyr Ala Phe Leu Lys Thr Tyr Asn Tyr Thr Leu Gly Ala Asp Asp Leu
                   90                  95                  100
   Thr Pro Phe Gly Glu Asn Gln Met Val Asn Ser Gly Ile Lys Phe Tyr
               105                 110                 115
   Arg Arg Tyr Lys Ala Leu Ala Arg Lys Ile Val Pro Phe Ile Arg Ala
       120                 125                 130
   Ser Gly Ser Asp Arg Val Ile Ala Ser Ala Glu Lys Phe Ile Glu Gly
   135                 140                 145                 150
   Phe Gln Ser Ala Lys Leu Ala Asp Pro Gly Ser Gln Pro His Gln Ala
                   155                 160                 165
   Ser Pro Val Ile Asp Val Ile Ile Pro Glu Gly Ser Gly Tyr Asn Asn
               170                 175                 180
   Thr Leu Asp His Gly Thr Cys Thr Ala Phe Glu Asp Ser Glu Leu Gly
           185                 190                 195
   Asp Asp Val Glu Ala Asn Phe Thr Ala Leu Phe Ala Pro Ala Ile Arg
       200                 205                 210
   Ala Arg Leu Glu Ala Asp Leu Pro Gly Val Thr Leu Thr Asp Glu Asp
   215                 220                 225                 230
   Val Val Tyr Leu Met Asp Met Cys Pro Phe Glu Thr Val Ala Arg Thr
                   235                 240                 245
   Ser Asp Ala Thr Glu Leu Ser Pro Phe Cys Ala Leu Phe Thr His Asp
               250                 255                 260
   Glu Trp Arg Gln Tyr Asp Tyr Leu Gln Ser Leu Gly Lys Tyr Tyr Gly
           265                 270                 275
   Tyr Gly Ala Gly Asn Pro Leu Gly Pro Ala Gln Gly Val Gly Phe Ala
       280                 285                 290
   Asn Glu Leu Ile Ala Arg Leu Thr Arg Ser Pro Val Gln Asp His Thr
   295                 300                 305                 310
   Ser Thr Asn His Thr Leu Asp Ser Asn Pro Ala Thr Phe Pro Leu Asn
                   315                 320                 325
   Ala Thr Leu Tyr Ala Asp Phe Ser His Asp Asn Ser Met Ile Ser Ile
               330                 335                 340
   Phe Phe Ala Leu Gly Leu Tyr Asn Gly Thr Ala Pro Leu Ser Thr Thr
           345                 350                 355
   Ser Val Glu Ser Ile Glu Glu Thr Asp Gly Tyr Ser Ala Ser Trp Thr
       360                 365                 370
   Val Pro Phe Gly Ala Arg Ala Tyr Val Glu Met Met Gln Cys Gln Ala
   375                 380                 385                 390
   Glu Lys Glu Pro Leu Val Arg Val Leu Val Asn Asp Arg Val Val Pro
                   395                 400                 405
   Leu His Gly Cys Ala Val Asp Lys Leu Gly Arg Cys Lys Arg Asp Asp
               410                 415                 420
   Phe Val Glu Gly Leu Ser Phe Ala Arg Ser Gly Gly Asn Trp Ala Glu
           425                 430                 435
   Cys Phe Ala
       440
<210> 17
   <211> 422
   <212> PRT
   <213> Paxillus involutus phyA1
<400> 17
   Ser Val Pro Lys Asn Thr Ala Pro Thr Phe Pro Ile Pro Glu Ser Glu
   1               5                   10                  15
   Gln Arg Asn Trp Ser Pro Tyr Ser Pro Tyr Phe Pro Leu Ala Glu Tyr
   20 25 30
   Lys Ala Pro Pro Ala Gly Cys Gln Ile Asn Gln Val Asn Ile Ile Gln
   35 40 45
   Arg His Gly Ala Arg Phe Pro Thr Ser Gly Ala Thr Thr Arg Ile Lys
   50 55 60
   Ala Gly Leu Thr Lys Leu Gln Gly Val Gln Asn Phe Thr Asp Ala Lys
   65 70 75 80
   Phe Asn Phe Ile Lys Ser Phe Lys Tyr Asp Leu Gly Asn Ser Asp Leu
   85 90 95
   Val Pro Phe Gly Ala Ala Gln Ser Phe Asp Ala Gly Gln Glu Ala Phe
   100 105 110
   Ala Arg Tyr Ser Lys Leu Val Ser Lys Asn Asn Leu Pro Phe Ile Arg
   115 120 125
   Ala Asp Gly Ser Asp Arg Val Val Asp Ser Ala Thr Asn Trp Thr Ala
   130 135 140
   Gly Phe Ala Ser Ala Ser His Asn Thr Val Gln Pro Lys Leu Asn Leu
   145 150 155 160
   Ile Leu Pro Gln Thr Gly Asn Asp Thr Leu Glu Asp Asn Met Cys Pro
   165 170 175
   Ala Ala Gly Asp Ser Asp Pro Gln Val Asn Ala Trp Leu Ala Val Ala
   180 185 190
   Phe Pro Ser Ile Thr Ala Arg Leu Asn Ala Ala Ala Pro Ser Val Asn
   195 200 205
   Leu Thr Asp Thr Asp Ala Phe Asn Leu Val Ser Leu Cys Ala Phe Leu
   210 215 220
   Thr Val Ser Lys Glu Lys Lys Ser Asp Phe Cys Thr Leu Phe Glu Gly
   225 230 235 240
   Ile Pro Gly Ser Phe Glu Ala Phe Ala Tyr Gly Gly Asp Leu Asp Lys
   245 250 255
   Phe Tyr Gly Thr Gly Tyr Gly Gln Glu Leu Gly Pro Val Gln Gly Val
   260 265 270
   Gly Tyr Val Asn Glu Leu Ile Ala Arg Leu Thr Asn Ser Ala Val Arg
   275 280 285
   Asp Asn Thr Gln Thr Asn Arg Thr Leu Asp Ala Ser Pro Val Thr Phe
   290 295 300
   Pro Leu Asn Lys Thr Phe Tyr Ala Asp Phe Ser His Asp Asn Leu Met
   305 310 315 320
   Val Ala Val Phe Ser Ala Met Gly Leu Phe Arg Gln Pro Ala Pro Leu
   325 330 335
   Ser Thr Ser Val Pro Asn Pro Trp Arg Thr Trp Arg Thr Ser Ser Leu
   340 345 350
   Val Pro Phe Ser Gly Arg Met Val Val Glu Arg Leu Ser Cys Phe Gly
   355 360 365
   Thr Thr Lys Val Arg Val Leu Val Gln Asp Gln Val Gln Pro Leu Glu
   370 375 380
   Phe Cys Gly Gly Asp Arg Asn Gly Leu Cys Thr Leu Ala Lys Phe Val
   385 390 395 400
   Glu Ser Gln Thr Phe Ala Arg Ser Asp Gly Ala Gly Asp Phe Glu Lys
   405 410 415
   Cys Phe Ala Thr Ser Ala
   420
<210> 18
   <211> 422
   <212> PRT
   <213> Paxillus involutus phyA2
<400> 18
   Ser Val Pro Arg Asn Ile Ala Pro Lys Phe Ser Ile Pro Glu Ser Glu
   1 5 10 15
   Gln Arg Asn Trp Ser Pro Tyr Ser Pro Tyr Phe Pro Leu Ala Glu Tyr
   20 25 30
   Lys Ala Pro Pro Ala Gly Cys Glu Ile Asn Gln Val Asn Ile Ile Gln
   35 40 45
   Arg His Gly Ala Arg Phe Pro Thr Ser Gly Ala Ala Thr Arg Ile Lys
   50 55 60
   Ala Gly Leu Ser Lys Leu Gln Ser Val Gln Asn Phe Thr Asp Pro Lys
   65 70 75 80
   Phe Asp Phe Ile Lys Ser Phe Thr Tyr Asp Leu Gly Thr Ser Asp Leu
   85 90 95
   Val Pro Phe Gly Ala Ala Gln Ser Phe Asp Ala Gly Leu Glu Val Phe
   100 105 110
   Ala Arg Tyr Ser Lys Leu Val Ser Ser Asp Asn Leu Pro Phe Ile Arg
   115 120 125
   Ser Asp Gly Ser Asp Arg Val Val Asp Thr Ala Thr Asn Trp Thr Ala
   130 135 140
   Gly Phe Ala Ser Ala Ser Arg Asn Ala Ile Gln Pro Lys Leu Asp Leu
   145 150 155 160
   Ile Leu Pro Gln Thr Gly Asn Asp Thr Leu Glu Asp Asn Met Cys Pro
   165 170 175
   Ala Ala Gly Glu Ser Asp Pro Gln Val Asp Ala Trp Leu Ala Ser Ala
   180 185 190
   Phe Pro Ser Val Thr Ala Gln Leu Asn Ala Ala Ala Pro Gly Ala Asn
   195 200 205
   Leu Thr Asp Ala Asp Ala Phe Asn Leu Val Ser Leu Cys Pro Phe Met
   210 215 220
   Thr Val Ser Lys Glu Gln Lys Ser Asp Phe Cys Thr Leu Phe Glu Gly
   225 230 235 240
   Ile Pro Gly Ser Phe Glu Ala Phe Ala Tyr Ala Gly Asp Leu Asp Lys
   245 250 255
   Phe Tyr Gly Thr Gly Tyr Gly Gln Ala Leu Gly Pro Val Gln Gly Val
   260 265 270
   Gly Tyr Ile Asn Glu Leu Leu Ala Arg Leu Thr Asn Ser Ala Val Asn
   275 280 285
   Asp Asn Thr Gln Thr Asn Arg Thr Leu Asp Ala Ala Pro Asp Thr Phe
   290 295 300
   Pro Leu Asn Lys Thr Met Tyr Ala Asp Phe Ser His Asp Asn Leu Met
   305 310 315 320
   Val Ala Val Phe Ser Ala Met Gly Leu Phe Arg Gln Ser Ala Pro Leu
   325 330 335
   Ser Thr Ser Thr Pro Asp Pro Asn Arg Thr Trp Leu Thr Ser Ser Val
   340 345 350
   Val Pro Phe Ser Ala Arg Met Ala Val Glu Arg Leu Ser Cys Ala Gly
   355 360 365
   Thr Thr Lys Val Arg Val Leu Val Gln Asp Gln Val Gln Pro Leu Glu
   370 375 380
   Phe Cys Gly Gly Asp Gln Asp Gly Leu Cys Ala Leu Asp Lys Phe Val
   385 390 395 400
   Glu Ser Gln Ala Tyr Ala Arg Ser Gly Gly Ala Gly Asp Phe Glu Lys
   405 410 415
   Cys Leu Ala Thr Thr Val
   420
<210> 19
   <211> 420
   <212> PRT
   <213> Trametes pubescens
<400> 19
   His Ile Pro Leu Arg Asp Thr Ser Ala Cys Leu Asp Val Thr Arg Asp
   1               5                   10                  15
   Val Gln Gln Ser Trp Ser Met Tyr Ser Pro Tyr Phe Pro Ala Ala Thr
               20                  25                  30
   Tyr Val Ala Pro Pro Ala Ser Cys Gln Ile Asn Gln Val His Ile Ile
           35                  40                  45
   Gln Arg His Gly Ala Arg Phe Pro Thr Ser Gly Ala Ala Lys Arg Ile
       50                  55                  60
   Gln Thr Ala Val Ala Lys Leu Lys Ala Ala Ser Asn Tyr Thr Asp Pro
   65                  70                  75                  80
   Leu Leu Ala Phe Val Thr Asn Tyr Thr Tyr Ser Leu Gly Gln Asp Ser
                   85                  90                  95
   Leu Val Glu Leu Gly Ala Thr Gln Ser Ser Glu Ala Gly Gln Glu Ala
               100                 105                 110
   Phe Thr Arg Tyr Ser Ser Leu Val Ser Ala Asp Glu Leu Pro Phe Val
           115                 120                 125
   Arg Ala Ser Gly Ser Asp Arg Val Val Ala Thr Ala Asn Asn Trp Thr
       130                 135                 140
   Ala Gly Phe Ala Leu Ala Ser Ser Asn Ser Ile Thr Pro Val Leu Ser
   145                 150                 155                 160
   Val Ile Ile Ser Glu Ala Gly Asn Asp Thr Leu Asp Asp Asn Met Cys
                   165                 170                 175
   Pro Ala Ala Gly Asp Ser Asp Pro Gln Val Asn Gln Trp Leu Ala Gln
               180                 185                 190
   Phe Ala Pro Pro Met Thr Ala Arg Leu Asn Ala Gly Ala Pro Gly Ala
           195                 200                 205
   Asn Leu Thr Asp Thr Asp Thr Tyr Asn Leu Leu Thr Leu Cys Pro Phe
       210                 215                 220
   Glu Thr Val Ala Thr Glu Arg Arg Ser Glu Phe Cys Asp Ile Tyr Glu
   225 230 235 240
   Glu Leu Gln Ala Glu Asp Ala Phe Ala Tyr Asn Ala Asp Leu Asp Lys
   245 250 255
   Phe Tyr Gly Thr Gly Tyr Gly Gln Pro Leu Gly Pro Val Gln Gly Val
   260 265 270
   Gly Tyr Ile Asn Glu Leu Ile Ala Arg Leu Thr Ala Gln Asn Val Ser
   275 280 285
   Asp His Thr Gln Thr Asn Ser Thr Leu Asp Ser Ser Pro Glu Thr Phe
   290 295 300
   Pro Leu Asn Arg Thr Leu Tyr Ala Asp Phe Ser His Asp Asn Gln Met
   305 310 315 320
   Val Ala Ile Phe Ser Ala Met Gly Leu Phe Asn Gln Ser Ala Pro Leu
   325 330 335
   Asp Pro Thr Thr Pro Asp Pro Ala Arg Thr Phe Leu Val Lys Lys Ile
   340 345 350
   Val Pro Phe Ser Ala Arg Met Val Val Glu Arg Leu Asp Cys Gly Gly
   355 360 365
   Ala Gln Ser Val Arg Leu Leu Val Asn Asp Ala Val Gln Pro Leu Ala
   370 375 380
   Phe Cys Gly Ala Asp Thr Ser Gly Val Cys Thr Leu Asp Ala Phe Val
   385 390 395 400
   Glu Ser Gln Ala Tyr Ala Arg Asn Asp Gly Glu Gly Asp Phe Glu Lys
   405 410 415
   Cys Phe Ala Thr
   420
<210> 20
   <211> 435
   <212> PRT
   <213> Agrocybe pediades
<400> 20
   Gly Gly Val Val Gln Ala Thr Phe Val Gln Pro Phe Phe Pro Pro Gln
   1 5 10 15
   Ile Gln Asp Ser Trp Ala Ala Tyr Thr Pro Tyr Tyr Pro Val Gln Ala
   20 25 30
   Tyr Thr Pro Pro Pro Lys Asp Cys Lys Ile Thr Gln Val Asn Ile Ile
   35 40 45
   Gln Arg His Gly Ala Arg Phe Pro Thr Ser Gly Ala Gly Thr Arg Ile
   50 55 60
   Gln Ala Ala Val Lys Lys Leu Gln Ser Ala Lys Thr Tyr Thr Asp Pro
   65 70 75 80
   Arg Leu Asp Phe Leu Thr Asn Tyr Thr Tyr Thr Leu Gly His Asp Asp
   85 90 95
   Leu Val Pro Phe Gly Ala Leu Gln Ser Ser Gln Ala Gly Glu Glu Thr
   100 105 110
   Phe Gln Arg Tyr Ser Phe Leu Val Ser Lys Glu Asn Leu Pro Phe Val
   115 120 125
   Arg Ala Ser Ser Ser Asn Arg Val Val Asp Ser Ala Thr Asn Trp Thr
   130 135 140
   Glu Gly Phe Ser Ala Ala Ser His His Val Leu Asn Pro Ile Leu Phe
   145 150 155 160
   Val Ile Leu Ser Glu Ser Leu Asn Asp Thr Leu Asp Asp Ala Met Cys
   165 170 175
   Pro Asn Ala Gly Ser Ser Asp Pro Gln Thr Gly Ile Trp Thr Ser Ile
   180 185 190
   Tyr Gly Thr Pro Ile Ala Asn Arg Leu Asn Gln Gln Ala Pro Gly Ala
   195 200 205
   Asn Ile Thr Ala Ala Asp Val Ser Asn Leu Ile Pro Leu Cys Ala Phe
   210 215 220
   Glu Thr Ile Val Lys Glu Thr Pro Ser Pro Phe Cys Asn Leu Phe Thr
   225 230 235 240
   Pro Glu Glu Phe Ala Gln Phe Glu Tyr Phe Gly Asp Leu Asp Lys Phe
   245 250 255
   Tyr Gly Thr Gly Tyr Gly Gln Pro Leu Gly Pro Val Gln Gly Val Gly
   260 265 270
   Tyr Ile Asn Glu Leu Leu Ala Arg Leu Thr Glu Met Pro Val Arg Asp
   275 280 285
   Asn Thr Gln Thr Asn Arg Thr Leu Asp Ser Ser Pro Leu Thr Phe Pro
   290 295 300
   Leu Asp Arg Ser Ile Tyr Ala Asp Leu Ser His Asp Asn Gln Met Ile
   305 310 315 320
   Ala Ile Phe Ser Ala Met Gly Leu Phe Asn Gln Ser Ser Pro Leu Asp
   325 330 335
   Pro Ser Phe Pro Asn Pro Lys Arg Thr Trp Val Thr Ser Arg Leu Thr
   340 345 350
   Pro Phe Ser Ala Arg Met Val Thr Glu Arg Leu Leu Cys Gln Arg Asp
   355 360 365
   Gly Thr Gly Ser Gly Gly Pro Ser Arg Ile Met Arg Asn Gly Asn Val
   370 375 380
   Gln Thr Phe Val Arg Ile Leu Val Asn Asp Ala Leu Gln Pro Leu Lys
   385 390 395 400
   Phe Cys Gly Gly Asp Met Asp Ser Leu Cys Thr Leu Glu Ala Phe Val
   405 410 415
   Glu Ser Gln Lys Tyr Ala Arg Glu Asp Gly Gln Gly Asp Phe Glu Lys
   420 425 430
   Cys Phe Asp
   435
<210> 21
   <211> 419
   <212> PRT
   <213> Peniophora lycii
<400> 21
   Ser Thr Gln Phe Ser Phe Val Ala Ala Gln Leu Pro Ile Pro Ala Gln
   1 5 10 15
   Asn Thr Ser Asn Trp Gly Pro Tyr Asp Pro Phe Phe Pro Val Glu Pro
   20 25 30
   Tyr Ala Ala Pro Pro Glu Gly Cys Thr Val Thr Gln Val Asn Leu Ile
   35 40 45
   Gln Arg His Gly Ala Arg Trp Pro Thr Ser Gly Ala Arg Ser Arg Gln
   50 55 60
   Val Ala Ala Val Ala Lys Ile Gln Met Ala Arg Pro Phe Thr Asp Pro
   65 70 75 80
   Lys Tyr Glu Phe Leu Asn Asp Phe Val Tyr Lys Phe Gly Val Ala Asp
   85 90 95
   Leu Leu Pro Phe Gly Ala Asn Gln Ser His Gln Thr Gly Thr Asp Met
   100 105 110
   Tyr Thr Arg Tyr Ser Thr Leu Phe Glu Gly Gly Asp Val Pro Phe Val
   115 120 125
   Arg Ala Ala Gly Asp Gln Arg Val Val Asp Ser Ser Thr Asn Trp Thr
       130                 135                 140
   Ala Gly Phe Gly Asp Ala Ser Gly Glu Thr Val Leu Pro Thr Leu Gln
   145                 150                 155                 160
   Val Val Leu Gln Glu Glu Gly Asn Cys Thr Leu Cys Asn Asn Met Cys
                   165                 170                 175
   Pro Asn Glu Val Asp Gly Asp Glu Ser Thr Thr Trp Leu Gly Val Phe
               180                 185                 190
   Ala Pro Asn Ile Thr Ala Arg Leu Asn Ala Ala Ala Pro Ser Ala Asn
           195                 200                 205
   Leu Ser Asp Ser Asp Ala Leu Thr Leu Met Asp Met Cys Pro Phe Asp
       210                 215                 220
   Thr Leu Ser Ser Gly Asn Ala Ser Pro Phe Cys Asp Leu Phe Thr Ala
   225                 230                 235                 240
   Glu Glu Tyr Val Ser Tyr Glu Tyr Tyr Tyr Asp Leu Asp Lys Tyr Tyr
                   245                 250                 255
   Gly Thr Gly Pro Gly Asn Ala Leu Gly Pro Val Gln Gly Val Gly Tyr
               260                 265                 270
   Val Asn Glu Leu Leu Ala Arg Leu Thr Gly Gln Ala Val Arg Asp Glu
           275                 280                 285
   Thr Gln Thr Asn Arg Thr Leu Asp Ser Asp Pro Ala Thr Phe Pro Leu
       290                 295                 300
   Asn Arg Thr Phe Tyr Ala Asp Phe Ser His Asp Asn Thr Met Val Pro
   305                 310                 315                 320
   Ile Phe Ala Ala Leu Gly Leu Phe Asn Ala Thr Ala Leu Asp Pro Leu
                   325                 330                 335
   Lys Pro Asp Glu Asn Arg Leu Trp Val Asp Ser Lys Leu Val Pro Phe
               340                 345                 350
   Ser Gly His Met Thr Val Glu Lys Leu Ala Cys Ser Gly Lys Glu Ala
           355                 360                 365
   Val Arg Val Leu Val Asn Asp Ala Val Gln Pro Leu Glu Phe Cys Gly
       370                 375                 380
   Gly Val Asp Gly Val Cys Glu Leu Ser Ala Phe Val Glu Ser Gln Thr
   385                 390                 395                 400
   Tyr Ala Arg Glu Asn Gly Gln Gly Asp Phe Ala Lys Cys Gly Phe Val
                   405                 410                 415
   Pro Ser Glu
<210> 22
   <211> 369
   <212> PRT
   <213> Artificial Sequence
<220>
   <223> Description of Artificial Sequence:basidio consensus
<400> 22
   Ser Pro Arg Thr Ala Ala Gln Leu Pro Ile Pro Gln Gln Trp Ser Pro
    1               5                  10                  15
   Tyr Ser Pro Tyr Phe Pro Val Ala Tyr Ala Pro Pro Ala Gly Cys Gln
               20                  25                  30
   Ile Gln Val Asn Ile Ile Gln Arg His Gly Ala Arg Phe Pro Thr Ser
           35                  40                  45
   Gly Ala Ala Thr Arg Ile Gln Ala Ala Val Ala Lys Leu Gln Ser Ala
       50                  55                  60
   Thr Asp Pro Lys Leu Asp Phe Leu Asn Thr Tyr Leu Gly Asp Asp Leu
   65                  70                  75                  80
   Val Pro Phe Gly Ala Gln Ser Ser Gln Ala Gly Gln Glu Ala Phe Thr
                   85                  90                  95
   Arg Tyr Ser Leu Val Ser Asp Asn Leu Pro Phe Val Arg Ala Ser Gly
               100                 105                 110
   Ser Asp Arg Val Val Asp Ser Ala Thr Asn Trp Thr Ala Gly Phe Ala
           115                 120                 125
   Ala Ser Asn Thr Pro Leu Val Ile Leu Ser Glu Gly Asn Asp Thr Leu
       130                 135                 140
   Asp Asp Asn Met Cys Pro Ala Gly Asp Ser Asp Pro Gln Asn Trp Leu
   145                 150                 155                 160
   Ala Val Phe Ala Pro Pro Ile Thr Ala Arg Leu Asn Ala Ala Ala Pro
                   165                 170                 175
   Gly Ala Asn Leu Thr Asp Asp Ala Asn Leu Leu Cys Pro Phe Glu Thr
               180                 185                 190
   Val Ser Glu Ser Phe Cys Asp Leu Phe Glu Pro Glu Glu Phe Ala Phe
           195                 200                 205
   Tyr Gly Asp Leu Asp Lys Phe Tyr Gly Thr Gly Tyr Gly Gln Pro Leu
   210 215 220
   Gly Pro Val Gln Gly Val Gly Tyr Ile Asn Glu Leu Leu Ala Arg Leu
   225 230 235 240
   Thr Gln Ala Val Arg Asp Asn Thr Gln Thr Asn Arg Thr Leu Asp Ser
   245 250 255
   Ser Pro Thr Phe Pro Leu Asn Arg Thr Phe Tyr Ala Asp Phe Ser His
   260 265 270
   Asp Asn Gln Met Val Ala Ile Phe Ser Ala Met Gly Leu Phe Asn Gln
   275 280 285
   Ser Ala Pro Leu Asp Pro Ser Pro Asp Pro Asn Arg Thr Trp Val Thr
   290 295 300
   Ser Lys Leu Val Pro Phe Ser Ala Arg Met Val Val Glu Arg Leu Cys
   305 310 315 320
   Gly Thr Val Arg Val Leu Val Asn Asp Ala Val Gln Pro Leu Glu Phe
   325 330 335
   Cys Gly Gly Asp Asp Gly Cys Thr Leu Asp Ala Phe Val Glu Ser Gln
   340 345 350
   Tyr Ala Arg Glu Asp Gly Gln Gly Asp Phe Glu Lys Cys Phe Ala Thr
   355 360 365
   Pro
<210> 23
   <211> 440
   <212> PRT
   <213> Thermomyces lanuginosus
<400> 23
   Asn Val Asp Ile Ala Arg His Trp Gly Gln Tyr Ser Pro Phe Phe Ser
   1 5 10 15
   Leu Ala Glu Val Ser Glu Ile Ser Pro Ala Val Pro Lys Gly Cys Arg
   20 25 30
   Val Glu Phe Val Gln Val Leu Ser Arg His Gly Ala Arg Tyr Pro Thr
   35 40 45
   Ala His Lys Ser Glu Val Tyr Ala Glu Leu Leu Gln Arg Ile Gln Asp
   50 55 60
   Thr Ala Thr Glu Phe Lys Gly Asp Phe Ala Phe Leu Arg Asp Tyr Ala
   65 70 75 80
   Tyr His Leu Gly Ala Asp Asn Leu Thr Arg Phe Gly Glu Glu Gln Met
                   85                  90                  95
   Met Glu Ser Gly Arg Gln Phe Tyr His Arg Tyr Arg Glu Gln Ala Arg
               100                 105                 110
   Glu Ile Val Pro Phe Val Arg Ala Ala Gly Ser Ala Arg Val Ile Ala
           115                 120                 125
   Ser Ala Glu Phe Phe Asn Arg Gly Phe Gln Asp Ala Lys Asp Arg Asp
       130                 135                 140
   Pro Arg Ser Asn Lys Asp Gln Ala Glu Pro Val Ile Asn Val Ile Ile
   145                 150                 155                 160
   Ser Glu Glu Thr Gly Ser Asn Asn Thr Leu Asp Gly Leu Thr Cys Pro
                   165                 170                 175
   Ala Ala Glu Glu Ala Pro Asp Pro Thr Gln Pro Ala Glu Phe Leu Gln
               180                 185                 190
   Val Phe Gly Pro Arg Val Leu Lys Lys Ile Thr Lys His Met Pro Gly
           195                 200                 205
   Val Asn Leu Thr Leu Glu Asp Val Pro Leu Phe Met Asp Leu Cys Pro
       210                 215                 220
   Phe Asp Thr Val Gly Ser Asp Pro Val Leu Phe Pro Arg Gln Leu Ser
   225                 230                 235                 240
   Pro Phe Cys His Leu Phe Thr Ala Asp Asp Trp Met Ala Tyr Asp Tyr
                   245                 250                 255
   Tyr Tyr Thr Leu Asp Lys Tyr Tyr Ser His Gly Gly Gly Ser Ala Phe
               260                 265                 270
   Gly Pro Ser Arg Gly Val Gly Phe Val Asn Glu Leu Ile Ala Arg Met
           275                 280                 285
   Thr Gly Asn Leu Pro Val Lys Asp His Thr Thr Val Asn His Thr Leu
       290                 295                 300
   Asp Asp Asn Pro Glu Thr Phe Pro Leu Asp Ala Val Leu Tyr Ala Asp
   305                 310                 315                 320
   Phe Ser His Asp Asn Thr Met Thr Gly Ile Phe Ser Ala Met Gly Leu
                   325                 330                 335
   Tyr Asn Gly Thr Lys Pro Leu Ser Thr Ser Lys Ile Gln Pro Pro Thr
               340                 345                 350
   Gly Ala Ala Ala Asp Gly Tyr Ala Ala Ser Trp Thr Val Pro Phe Ala
           355                 360                 365
   Ala Arg Ala Tyr Val Glu Leu Leu Arg Cys Glu Thr Glu Thr Ser Ser
       370                 375                 380
   Glu Glu Glu Glu Glu Gly Glu Asp Glu Pro Phe Val Arg Val Leu Val
   385                 390                 395                 400
   Asn Asp Arg Val Val Pro Leu His Gly Cys Arg Val Asp Arg Trp Gly
                   405                 410                 415
   Arg Cys Arg Arg Asp Glu Trp Ile Lys Gly Leu Thr Phe Ala Arg Gln
               420                 425                 430
   Gly Gly His Trp Asp Arg Cys Phe
           435                 440
<210> 24
   <211> 441
   <212> PRT
   <213> Artificial Sequence
<220>
   <223> Description of Artificial Sequence: consensus 10
<400> 24
   Asn Ser His Ser Cys Asp Thr Val Asp Gly Gly Tyr Gln Cys Phe Pro
     1               5                  10                  15
   Glu Ile Ser His Leu Trp Gly Gln Tyr Ser Pro Phe Phe Ser Leu Ala
                20                  25                  30
   Asp Glu Ser Ala Ile Ser Pro Asp Val Pro Lys Gly Cys Arg Val Thr
            35                  40                  45
   Phe Val Gln Val Leu Ser Arg His Gly Ala Arg Tyr Pro Thr Ser Ser
        50                  55                  60
   Lys Ser Lys Lys Tyr Ser Ala Leu Ile Glu Ala Ile Gln Lys Asn Ala
    65                  70                  75                  80
   Thr Ala Phe Lys Gly Lys Tyr Ala Phe Leu Lys Thr Tyr Asn Tyr Thr
                    85                  90                  95
   Leu Gly Ala Asp Asp Leu Thr Pro Phe Gly Glu Gln Gln Met Val Asn
               100                 105                 110
   Ser Gly Ile Lys Phe Tyr Arg Arg Tyr Lys Ala Leu Ala Arg Lys Ile
           115                 120                 125
   Val Pro Phe Val Arg Ala Ser Gly Ser Asp Arg Val Ile Ala Ser Ala
       130                 135                 140
   Glu Lys Phe Ile Glu Gly Phe Gln Ser Ala Lys Leu Ala Asp Pro Gly
   145                 150                 155                 160
   Ala Asn Pro His Gln Ala Ser Pro Val Ile Asn Val Ile Ile Pro Glu
                   165                 170                 175
   Gly Ala Gly Tyr Asn Asn Thr Leu Asp His Gly Leu Cys Thr Ala Phe
               180                 185                 190
   Glu Glu Ser Glu Leu Gly Asp Asp Val Glu Ala Asn Phe Thr Ala Val
           195                 200                 205
   Phe Ala Pro Pro Ile Arg Ala Arg Leu Glu Ala His Leu Pro Gly Val
       210                 215                 220
   Asn Leu Thr Asp Glu Asp Val Val Asn Leu Met Asp Met Cys Pro Phe
   225                 230                 235                 240
   Asp Thr Val Ala Arg Thr Ser Asp Ala Thr Gln Leu Ser Pro Phe Cys
                   245                 250                 255
   Asp Leu Phe Thr His Asp Glu Trp Ile Gln Tyr Asp Tyr Leu Gln Ser
               260                 265                 270
   Leu Gly Lys Tyr Tyr Gly Tyr Gly Ala Gly Asn Pro Leu Gly Pro Ala
           275                 280                 285
   Gln Gly Val Gly Phe Val Asn Glu Leu Ile Ala Arg Leu Thr His Ser
       290                 295                 300
   Pro Val Gln Asp His Thr Ser Thr Asn His Thr Leu Asp Ser Asn Pro
   305                 310                 315                 320
   Ala Thr Phe Pro Leu Asn Ala Thr Leu Tyr Ala Asp Phe Ser His Asp
                   325                 330                 335
   Asn Thr Met Val Ser Ile Phe Phe Ala Leu Gly Leu Tyr Asn Gly Thr
               340                 345                 350
   Lys Pro Leu Ser Thr Thr Ser Val Glu Ser Ile Glu Glu Thr Asp Gly
           355                 360                 365
   Tyr Ala Ala Ser Trp Thr Val Pro Phe Ala Ala Arg Ala Tyr Val Glu
       370                 375                 380
   Met Met Gln Cys Glu Ala Glu Lys Glu Pro Leu Val Arg Val Leu Val
   385                 390                 395                 400
   Asn Asp Arg Val Val Pro Leu His Gly Cys Gly Val Asp Lys Leu Gly
                   405                 410                 415
   Arg Cys Lys Arg Asp Asp Phe Val Glu Gly Leu Ser Phe Ala Arg Ser
               420                 425                 430
   Gly Gly Asn Trp Glu Glu Cys Phe Ala
               435                 440
<210> 25
   <211> 1426
   <212> DNA
   <213> Artificial Sequence
<220>
   <223> Description of Artificial Sequence: consensus phytase 10
<220>
   <221> CDS
   <222> (12)..(1412)
<220>
   <221> mat_peptide
   <222> (90)..(1412)
<220>
   <221> sig_peptide
   <222> (12)..(89)
<400> 25
   tatatgaatt c atg ggc gtg ttc gtc gtg cta ctg tcc att gcc acc ttg  50
                Met Gly Val Phe Val Val Leu Leu Ser Ile Ala Thr Leu
                    -25                 -20                 -15
   ttc ggt tcc aca tcc ggt acc gcc ttg ggt cct cgt ggt aat tct cac   98
   Phe Gly Ser Thr Ser Gly Thr Ala Leu Gly Pro Arg Gly Asn Ser His
               -10                  -5              -1   1
   tct tgt gac act gtt gac ggt ggt tac caa tgt ttc cca gaa att tct   146
   Ser Cys Asp Thr Val Asp Gly Gly Tyr Gln Cys Phe Pro Glu Ile Ser
           5                  10                  15
   cac ttg tgg ggt caa tac tct cca ttc ttc tct ttg gct gac gaa tct   194
   His Leu Trp Gly Gln Tyr Ser Pro Phe Phe Ser Leu Ala Asp Glu Ser
    20                  25                  30                  35
   gct att tct cca gac gtt cca aag ggt tgt aga gtt act ttc gtt caa   242
   Ala Ile Ser Pro Asp Val Pro Lys Gly Cys Arg Val Thr Phe Val Gln
                    40                  45                  50
   gtt ttg tct aga cac ggt gct aga tac cca act tct tct aag tct aag   290
   Val Leu Ser Arg His Gly Ala Arg Tyr Pro Thr Ser Ser Lys Ser Lys
               55                  60                  65
   aag tac tct gct ttg att gaa gct att caa aag aac gct act gct ttc   338
   Lys Tyr Ser Ala Leu Ile Glu Ala Ile Gln Lys Asn Ala Thr Ala Phe
           70                  75                  80
   aag ggt aag tac gct ttc ttg aag act tac aac tac act ttg ggt gct    386
   Lys Gly Lys Tyr Ala Phe Leu Lys Thr Tyr Asn Tyr Thr Leu Gly Ala
        85                  90                  95
   gac gac ttg act cca ttc ggt gaa caa caa atg gtt aac tct ggt att    434
   Asp Asp Leu Thr Pro Phe Gly Glu Gln Gln Met Val Asn Ser Gly Ile
   100                 105                 110                 115
   aag ttc tac aga aga tac aag gct ttg gct aga aag att gtt cca ttc    482
   Lys Phe Tyr Arg Arg Tyr Lys Ala Leu Ala Arg Lys Ile Val Pro Phe
                   120                 125                 130
   gtt aga gct tct ggt tct gac aga gtt att gct tct gct gaa aag ttc    530
   Val Arg Ala Ser Gly Ser Asp Arg Val Ile Ala Ser Ala Glu Lys Phe
               135                 140                 145
   att gaa ggt ttc caa tct gct aag ttg gct gac cca ggt gct aac cca    578
   Ile Glu Gly Phe Gln Ser Ala Lys Leu Ala Asp Pro Gly Ala Asn Pro
           150                 155                 160
   cac caa gct tct cca gtt att aac gtt att att cca gaa ggt gct ggt    626
   His Gln Ala Ser Pro Val Ile Asn Val Ile Ile Pro Glu Gly Ala Gly
       165                 170                 175
   tac aac aac act ttg gac cac ggt ttg tgt act gct ttc gaa gaa tct    674
   Tyr Asn Asn Thr Leu Asp His Gly Leu Cys Thr Ala Phe Glu Glu Ser
   180                 185                 190                 195
   gaa ttg ggt gac gac gtt gaa gct aac ttc act gct gtt ttc gct cca    722
   Glu Leu Gly Asp Asp Val Glu Ala Asn Phe Thr Ala Val Phe Ala Pro
                   200                 205                 210
   cct att aga gct aga ttg gaa gct cac ttg cca ggt gtt aac ttg act    770
   Pro Ile Arg Ala Arg Leu Glu Ala His Leu Pro Gly Val Asn Leu Thr
               215                 220                 225
   gac gaa gac gtt gtt aac ttg atg gac atg tgt cca ttc gac act gtt    818
   Asp Glu Asp Val Val Asn Leu Met Asp Met Cys Pro Phe Asp Thr Val
           230                 235                 240
   gct aga act tct gac gct act caa ttg tct cca ttc tgt gac ttg ttc    866
   Ala Arg Thr Ser Asp Ala Thr Gln Leu Ser Pro Phe Cys Asp Leu Phe
       245                 250                 255
   act cac gac gaa tgg att caa tac gac tac ttg caa tct ttg ggt aag    914
   Thr His Asp Glu Trp Ile Gln Tyr Asp Tyr Leu Gln Ser Leu Gly Lys
   260                 265                 270                 275
   tac tac ggt tac ggt gct ggt aac cca ttg ggt cca gct caa ggt gtt    962
   Tyr Tyr Gly Tyr Gly Ala Gly Asn Pro Leu Gly Pro Ala Gln Gly Val
                   280                 285                 290
   ggt ttc gtt aac gaa ttg att gct aga ttg act cac tct cca gtt caa      1010
   Gly Phe Val Asn Glu Leu Ile Ala Arg Leu Thr His Ser Pro Val Gln
               295                 300                 305
   gac cac act tct act aac cac act ttg gac tct aac cca gct act ttc      1058
   Asp His Thr Ser Thr Asn His Thr Leu Asp Ser Asn Pro Ala Thr Phe
           310                 315                 320
   cca ttg aac gct act ttg tac gct gac ttc tct cac gac aac act atg      1106
   Pro Leu Asn Ala Thr Leu Tyr Ala Asp Phe Ser His Asp Asn Thr Met
       325                 330                 335
   gtt tct att ttc ttc gct ttg ggt ttg tac aac ggt act aag cca ttg      1154
   Val Ser Ile Phe Phe Ala Leu Gly Leu Tyr Asn Gly Thr Lys Pro Leu
   340                 345                 350                 355
   tct act act tct gtt gaa tct att gaa gaa act gac ggt tac gct gct      1202
   Ser Thr Thr Ser Val Glu Ser Ile Glu Glu Thr Asp Gly Tyr Ala Ala
                   360                 365                 370
   tct tgg act gtt cca ttc gct gct aga gct tac gtt gaa atg atg caa      1250
   Ser Trp Thr Val Pro Phe Ala Ala Arg Ala Tyr Val Glu Met Met Gln
               375                 380                 385
   tgt gaa gct gaa aag gaa cca ttg gtt aga gtt ttg gtt aac gac aga      1298
   Cys Glu Ala Glu Lys Glu Pro Leu Val Arg Val Leu Val Asn Asp Arg
           390                 395                 400
   gtt gtt cca ttg cac ggt tgt ggt gtt gac aag ttg ggt aga tgt aag      1346
   Val Val Pro Leu His Gly Cys Gly Val Asp Lys Leu Gly Arg Cys Lys
       405                 410                 415
   aga gac gac ttc gtt gaa ggt ttg tct ttc gct aga tct ggt ggt aac      1394
   Arg Asp Asp Phe Val Glu Gly Leu Ser Phe Ala Arg Ser Gly Gly Asn
   420                 425                 430                 435
   tgg gaa gaa tgt ttc gct taagaattca tata                              1426
   Trp Glu Glu Cys Phe Ala
                   440
<210> 26
   <211> 467
   <212> PRT
   <213> Artificial Sequence
   <223> Description of Artificial Sequence: consensus phytase 10
<400> 26
   Met Gly Val Phe Val Val Leu Leu Ser Ile Ala Thr Leu Phe Gly Ser
       -25                 -20                 -15
   Thr Ser Gly Thr Ala Leu Gly Pro Arg Gly Asn Ser His Ser Cys Asp
   -10                 -5                  -1  1               5
   Thr Val Asp Gly Gly Tyr Gln Cys Phe Pro Glu Ile Ser His Leu Trp
               10                  15                  20
   Gly Gln Tyr Ser Pro Phe Phe Ser Leu Ala Asp Glu Ser Ala Ile Ser
           25                  30                  35
   Pro Asp Val Pro Lys Gly Cys Arg Val Thr Phe Val Gln Val Leu Ser
       40                  45                  50
   Arg His Gly Ala Arg Tyr Pro Thr Ser Ser Lys Ser Lys Lys Tyr Ser
   55                  60                  65                  70
   Ala Leu Ile Glu Ala Ile Gln Lys Asn Ala Thr Ala Phe Lys Gly Lys
                   75                  80                  85
   Tyr Ala Phe Leu Lys Thr Tyr Asn Tyr Thr Leu Gly Ala Asp Asp Leu
               90                  95                  100
   Thr Pro Phe Gly Glu Gln Gln Met Val Asn Ser Gly Ile Lys Phe Tyr
           105                 110                 115
   Arg Arg Tyr Lys Ala Leu Ala Arg Lys Ile Val Pro Phe Val Arg Ala
       120                 125                 130
   Ser Gly Ser Asp Arg Val Ile Ala Ser Ala Glu Lys Phe Ile Glu Gly
   135                 140                 145                 150
   Phe Gln Ser Ala Lys Leu Ala Asp Pro Gly Ala Asn Pro His Gln Ala
                   155                 160                 165
   Ser Pro Val Ile Asn Val Ile Ile Pro Glu Gly Ala Gly Tyr Asn Asn
               170                 175                 180
   Thr Leu Asp His Gly Leu Cys Thr Ala Phe Glu Glu Ser Glu Leu Gly
           185                 190                 195
   Asp Asp Val Glu Ala Asn Phe Thr Ala Val Phe Ala Pro Pro Ile Arg
       200                 205                 210
   Ala Arg Leu Glu Ala His Leu Pro Gly Val Asn Leu Thr Asp Glu Asp
   215                 220                 225                 230
   Val Val Asn Leu Met Asp Met Cys Pro Phe Asp Thr Val Ala Arg Thr
                   235                 240                 245
   Ser Asp Ala Thr Gln Leu Ser Pro Phe Cys Asp Leu Phe Thr His Asp
               250                 255                 260
   Glu Trp Ile Gln Tyr Asp Tyr Leu Gln Ser Leu Gly Lys Tyr Tyr Gly
           265                 270                 275
   Tyr Gly Ala Gly Asn Pro Leu Gly Pro Ala Gln Gly Val Gly Phe Val
       280                 285                 290
   Asn Glu Leu Ile Ala Arg Leu Thr His Ser Pro Val Gln Asp His Thr
   295                 300                 305                 310
   Ser Thr Asn His Thr Leu Asp Ser Asn Pro Ala Thr Phe Pro Leu Asn
                   315                 320                 325
   Ala Thr Leu Tyr Ala Asp Phe Ser His Asp Asn Thr Met Val Ser Ile
               330                 335                 340
   Phe Phe Ala Leu Gly Leu Tyr Asn Gly Thr Lys Pro Leu Ser Thr Thr
           345                 350                 355
   Ser Val Glu Ser Ile Glu Glu Thr Asp Gly Tyr Ala Ala Ser Trp Thr
       360                 365                 370
   Val Pro Phe Ala Ala Arg Ala Tyr Val Glu Met Met Gln Cys Glu Ala
   375                 380                 385                 390
   Glu Lys Glu Pro Leu Val Arg Val Leu Val Asn Asp Arg Val Val Pro
                   395                 400                 405
   Leu His Gly Cys Gly Val Asp Lys Leu Gly Arg Cys Lys Arg Asp Asp
               410                 415                 420
   Phe Val Glu Gly Leu Ser Phe Ala Arg Ser Gly Gly Asn Trp Glu Glu
           425                 430                 435
   Cys Phe Ala
       440
<210> 27
   <211> 437
   <212> PRT
   <213> Artificial Sequence
<220>
   <223> Description of Artificial Sequence: consensus phytase 11
<400> 27
   Asn Ser His Ser Cys Asp Thr Val Asp Gly Tyr Gln Cys Pro Glu Ile
    1               5                  10                  15
   Ser His Leu Trp Gly Gln Tyr Ser Pro Phe Phe Ser Leu Ala Asp Glu
               20                  25                  30
   Ser Ala Ile Ser Pro Asp Val Pro Lys Gly Cys Arg Val Thr Phe Val
           35                  40                  45
   Gln Val Leu Ser Arg His Gly Ala Arg Tyr Pro Thr Ser Ser Lys Ser
       50                  55                  60
   Lys Lys Tyr Ser Ala Leu Ile Glu Arg Ile Gln Lys Asn Ala Thr Phe
    65                 70                  75                  80
   Lys Gly Lys Tyr Ala Phe Leu Lys Thr Tyr Asn Tyr Thr Leu Gly Ala
                   85                  90                  95
   Asp Asp Leu Thr Pro Phe Gly Glu Asn Gln Met Val Asn Ser Gly Ile
               100                 105                 110
   Lys Phe Tyr Arg Arg Tyr Lys Ala Leu Ala Arg Asn Ile Val Pro Phe
           115                 120                 125
   Val Arg Ala Ser Gly Ser Asp Arg Val Ile Ala Ser Ala Glu Lys Phe
       130                 135                 140
   Ile Glu Gly Phe Gln Ser Ala Lys Leu Ala Asp Pro Ala His Gln Ala
   145                 150                 155                 160
   Ser Pro Val Ile Asn Val Ile Ile Pro Glu Gly Ser Gly Tyr Asn Asn
                   165                 170                 175
   Thr Leu Asp His Gly Leu Cys Thr Ala Phe Glu Asp Ser Thr Leu Gly
               180                 185                 190
   Asp Asp Ala Glu Ala Asn Phe Thr Ala Val Phe Ala Pro Pro Ile Arg
           195                 200                 205
   Ala Arg Leu Glu Ala Leu Pro Gly Val Asn Leu Thr Asp Glu Asp Val
       210                 215                 220
   Val Asn Leu Met Asp Met Cys Pro Phe Asp Thr Val Ala Arg Thr Ser
   225                 230                 235                 240
   Asp Ala Thr Gln Leu Ser Pro Phe Cys Asp Leu Phe Thr Ala Asp Glu
                   245                 250                 255
   Trp Gln Tyr Asp Tyr Leu Gln Ser Leu Lys Tyr Tyr Gly Tyr Gly Ala
               260                 265                 270
   Gly Asn Pro Leu Gly Pro Ala Gln Gly Val Gly Phe Asn Glu Leu Ile
           275                 280                 285
   Ala Arg Leu Thr His Ser Pro Val Gln Asp His Thr Ser Thr Asn His
       290                 295                 300
   Thr Leu Asp Ser Asn Pro Ala Thr Phe Pro Leu Asn Ala Thr Leu Tyr
   305                 310                 315                 320
   Ala Asp Phe Ser His Asp Asn Thr Met Val Ser Ile Phe Phe Ala Leu
                   325                 330                 335
   Gly Leu Tyr Asn Gly Thr Lys Pro Leu Ser Thr Thr Ser Val Glu Ser
               340                 345                 350
   Ile Glu Thr Asp Gly Tyr Ala Ala Ser Trp Thr Val Pro Phe Ala Ala
           355                 360                 365
   Arg Ala Tyr Val Glu Met Met Gln Cys Glu Ala Gly Gly Gly Gly Gly
       370                 375                 380
   Glu Gly Glu Lys Glu Pro Leu Val Arg Val Leu Val Asn Asp Arg Val
   385                 390                 395                 400
   Val Pro Leu His Gly Cys Gly Val Asp Lys Leu Gly Arg Cys Lys Leu
                   405                 410                 415
   Asp Asp Phe Val Glu Gly Leu Ser Phe Ala Arg Ser Gly Gly Asn Trp
               420                 425                 430
   Ala Glu Cys Phe Ala
           435
<210> 28
   <211> 1404
   <212> DNA
   <213> Artificial Sequence
<220>
   <223> Description of Artificial Sequence: consensus phytase 1 thermo 8 q50t, k91a
<220>
   <221> CDS
   <222> (1)..(1401)
<220>
   <221> mat_peptide
   <222> (79)..(1401)
<220>
   <221> sig_peptide
   <222> (1)..(78)
<400> 28
   atg ggc gtg ttc gtc gtg cta ctg tcc att gcc acc ttg ttc ggt tcc    48
   Met Gly Val Phe Val Val Leu Leu Ser Ile Ala Thr Leu Phe Gly Ser
       -25                 -20                 -15
   aca tcc ggt acc gcc ttg ggt cct cgt ggt aat tct cac tct tgt gac    96
   Thr Ser Gly Thr Ala Leu Gly Pro Arg Gly Asn Ser His Ser Cys Asp
   -10                 -5                  -1   1               5
   act gtt gac ggt ggt tac caa tgt ttc cca gaa att tct cac ttg tgg   144
   Thr Val Asp Gly Gly Tyr Gln Cys Phe Pro Glu Ile Ser His Leu Trp
                   10                  15                  20
   ggt acc tac tct cca tac ttc tct ttg gca gac gaa tct gct att tct     192
   Gly Thr Tyr Ser Pro Tyr Phe Ser Leu Ala Asp Glu Ser Ala Ile Ser
           25                  30                  35
   cca gac gtt cca gac gac tgt aga gtt act ttc gtt caa gtt ttg tct     240
   Pro Asp Val Pro Asp Asp Cys Arg Val Thr Phe Val Gln Val Leu Ser
       40                  45                  50
   aga cac ggt gct aga tac cca act tct tct gcg tct aag gct tac tct     288
   Arg His Gly Ala Arg Tyr Pro Thr Ser Ser Ala Ser Lys Ala Tyr Ser
   55                  60                  65                  70
   gct ttg att gaa gct att caa aag aac gct act gct ttc aag ggt aag     336
   Ala Leu Ile Glu Ala Ile Gln Lys Asn Ala Thr Ala Phe Lys Gly Lys
                   75                  80                  85
   tac gct ttc ttg aag act tac aac tac act ttg ggt gct gac gac ttg     384
   Tyr Ala Phe Leu Lys Thr Tyr Asn Tyr Thr Leu Gly Ala Asp Asp Leu
               90                  95                  100
   act cca ttc ggt gaa aac caa atg gtt aac tct ggt att aag ttc tac     432
   Thr Pro Phe Gly Glu Asn Gln Met Val Asn Ser Gly Ile Lys Phe Tyr
           105                 110                 115
   aga aga tac aag gct ttg gct aga aag att gtt cca ttc att aga gct     480
   Arg Arg Tyr Lys Ala Leu Ala Arg Lys Ile Val Pro Phe Ile Arg Ala
       120                 125                 130
   tct ggt tct gac aga gtt att gct tct gct gaa aag ttc att gaa ggt     528
   Ser Gly Ser Asp Arg Val Ile Ala Ser Ala Glu Lys Phe Ile Glu Gly
   135                 140                 145                 150
   ttc caa tct gct aag ttg gct gac cca ggt tct caa cca cac caa gct     576
   Phe Gln Ser Ala Lys Leu Ala Asp Pro Gly Ser Gln Pro His Gln Ala
                   155                 160                 165
   tct cca gtt att aac gtg atc att cca gaa gga tcc ggt tac aac aac     624
   Ser Pro Val Ile Asn Val Ile Ile Pro Glu Gly Ser Gly Tyr Asn Asn
               170                 175                 180
   act ttg gac cac ggt act tgt act gct ttc gaa gac tct gaa tta ggt     672
   Thr Leu Asp His Gly Thr Cys Thr Ala Phe Glu Asp Ser Glu Leu Gly
           185                 190                 195
   gac gac gtt gaa gct aac ttc act gct ttg ttc gct cca gct att aga     720
   Asp Asp Val Glu Ala Asn Phe Thr Ala Leu Phe Ala Pro Ala Ile Arg
       200                 205                 210
   gct aga ttg gaa gct gac ttg cca ggt gtt act ttg act gac gaa gac     768
   Ala Arg Leu Glu Ala Asp Leu Pro Gly Val Thr Leu Thr Asp Glu Asp
   215                 220                 225                 230
   gtt gtt tac ttg atg gac atg tgt cca ttc gac act gtc gct aga act    816
   Val Val Tyr Leu Met Asp Met Cys Pro Phe Asp Thr Val Ala Arg Thr
                   235                 240                 245
   tct gac gct act gaa ttg tct cca ttc tgt gct ttg ttc act cac gac    864
   Ser Asp Ala Thr Glu Leu Ser Pro Phe Cys Ala Leu Phe Thr His Asp
               250                 255                 260
   gaa tgg atc caa tac gac tac ttg caa agc ttg ggt aag tac tac ggt    912
   Glu Trp Ile Gln Tyr Asp Tyr Leu Gln Ser Leu Gly Lys Tyr Tyr Gly
           265                 270                 275
   tac ggt gct ggt aac cca ttg ggt cca gct caa ggt gtt ggt ttc gct    960
   Tyr Gly Ala Gly Asn Pro Leu Gly Pro Ala Gln Gly Val Gly Phe Ala
       280                 285                 290
   aac gaa ttg att gct aga ttg act cac tct cca gtt caa gac cac act    1008
   Asn Glu Leu Ile Ala Arg Leu Thr His Ser Pro Val Gln Asp His Thr
   295                 300                 305                 310
   tct act aac cac act ttg gac tct aac cca gct act ttc cca ttg aac    1056
   Ser Thr Asn His Thr Leu Asp Ser Asn Pro Ala Thr Phe Pro Leu Asn
                   315                 320                 325
   gct act ttg tac gct gac ttc tct cac gac aac act atg ata tct att    1104
   Ala Thr Leu Tyr Ala Asp Phe Ser His Asp Asn Thr Met Ile Ser Ile
               330                 335                 340
   ttc ttc gct ttg ggt ttg tac aac ggt acc aag cca ttg tct act act    1152
   Phe Phe Ala Leu Gly Leu Tyr Asn Gly Thr Lys Pro Leu Ser Thr Thr
           345                 350                 355
   tct gtt gaa tct att gaa gaa act gac ggt tac tct gct tct tgg act    1200
   Ser Val Glu Ser Ile Glu Glu Thr Asp Gly Tyr Ser Ala Ser Trp Thr
       360                 365                 370
   gtt cca ttc gct gct aga gct tac gtt gaa atg atg caa tgt caa gct    1248
   Val Pro Phe Ala Ala Arg Ala Tyr Val Glu Met Met Gln Cys Gln Ala
   375                 380                 385                 390
   gaa aag gaa cca ttg gtt aga gtt ttg gtt aac gac aga gtt gtt cca    1296
   Glu Lys Glu Pro Leu Val Arg Val Leu Val Asn Asp Arg Val Val Pro
                   395                 400                 405
   ttg cac ggt tgt gct gtt gac aag ttg ggt aga tgt aag aga gac gac    1344
   Leu His Gly Cys Ala Val Asp Lys Leu Gly Arg Cys Lys Arg Asp Asp
               410                 415                 420
   ttc gtt gaa ggt ttg tct ttc gct aga tct ggt ggt aac tgg gct gaa    1392
   Phe Val Glu Gly Leu Ser Phe Ala Arg Ser Gly Gly Asn Trp Ala Glu
           425                 430                 435
   tgt ttc gct taa                                               1404
   Cys Phe Ala
       440
<210> 29
   <211> 467
   <212> PRT
   <213> Artificial Sequence
   <223> Description of Artificial Sequence: consensus phytase 1 thermo 8 q50t, k91a
<400> 29
   Met Gly Val Phe Val Val Leu Leu Ser Ile Ala Thr Leu Phe Gly Ser
       -25                 -20                 -15
   Thr Ser Gly Thr Ala Leu Gly Pro Arg Gly Asn Ser His Ser Cys Asp
   -10                 -5                  -1   1               5
   Thr Val Asp Gly Gly Tyr Gln Cys Phe Pro Glu Ile Ser His Leu Trp
                   10                  15                  20
   Gly Thr Tyr Ser Pro Tyr Phe Ser Leu Ala Asp Glu Ser Ala Ile Ser
               25                  30                  35
   Pro Asp Val Pro Asp Asp Cys Arg Val Thr Phe Val Gln Val Leu Ser
       40                  45                  50
   Arg His Gly Ala Arg Tyr Pro Thr Ser Ser Ala Ser Lys Ala Tyr Ser
    55                  60                  65                  70
   Ala Leu Ile Glu Ala Ile Gln Lys Asn Ala Thr Ala Phe Lys Gly Lys
                       75                  80                  85
   Tyr Ala Phe Leu Lys Thr Tyr Asn Tyr Thr Leu Gly Ala Asp Asp Leu
                   90                  95                  100
   Thr Pro Phe Gly Glu Asn Gln Met Val Asn Ser Gly Ile Lys Phe Tyr
               105                 110                 115
   Arg Arg Tyr Lys Ala Leu Ala Arg Lys Ile Val Pro Phe Ile Arg Ala
       120                 125                 130
   Ser Gly Ser Asp Arg Val Ile Ala Ser Ala Glu Lys Phe Ile Glu Gly
   135                 140                 145                 150
   Phe Gln Ser Ala Lys Leu Ala Asp Pro Gly Ser Gln Pro His Gln Ala
                   155                 160                 165
   Ser Pro Val Ile Asn Val Ile Ile Pro Glu Gly Ser Gly Tyr Asn Asn
               170                 175                 180
   Thr Leu Asp His Gly Thr Cys Thr Ala Phe Glu Asp Ser Glu Leu Gly
           185                 190                 195
   Asp Asp Val Glu Ala Asn Phe Thr Ala Leu Phe Ala Pro Ala Ile Arg
   200 205 210
   Ala Arg Leu Glu Ala Asp Leu Pro Gly Val Thr Leu Thr Asp Glu Asp
   215 220 225 230
   Val Val Tyr Leu Met Asp Met Cys Pro Phe Asp Thr Val Ala Arg Thr
   235 240 245
   Ser Asp Ala Thr Glu Leu Ser Pro Phe Cys Ala Leu Phe Thr His Asp
   250 255 260
   Glu Trp Ile Gln Tyr Asp Tyr Leu Gln Ser Leu Gly Lys Tyr Tyr Gly
   265 270 275
   Tyr Gly Ala Gly Asn Pro Leu Gly Pro Ala Gln Gly Val Gly Phe Ala
   280 285 290
   Asn Glu Leu Ile Ala Arg Leu Thr His Ser Pro Val Gln Asp His Thr
   295 300 305 310
   Ser Thr Asn His Thr Leu Asp Ser Asn Pro Ala Thr Phe Pro Leu Asn
   315 320 325
   Ala Thr Leu Tyr Ala Asp Phe Ser His Asp Asn Thr Met Ile Ser Ile
   330 335 340
   Phe Phe Ala Leu Gly Leu Tyr Asn Gly Thr Lys Pro Leu Ser Thr Thr
   345 350 355
   Ser Val Glu Ser Ile Glu Glu Thr Asp Gly Tyr Ser Ala Ser Trp Thr
   360 365 370
   Val Pro Phe Ala Ala Arg Ala Tyr Val Glu Met Met Gln Cys Gln Ala
   375 380 385 390
   Glu Lys Glu Pro Leu Val Arg Val Leu Val Asn Asp Arg Val Val Pro
   395 400 405
   Leu His Gly Cys Ala Val Asp Lys Leu Gly Arg Cys Lys Arg Asp Asp
   410 415 420
   Phe Val Glu Gly Leu Ser Phe Ala Arg Ser Gly Gly Asn Trp Ala Glu
   425 430 435
   Cys Phe Ala
   440
<210> 30
   <211> 1404
   <212> DNA
   <213> Artificial Sequence
<220>
   <223> Description of Artificial Sequence: consensus phytase 10 thermo 3 q50t, k91a
<220>
   <221> CDS
   <222> (1)..(1401)
<220>
   <221> mat_peptide
   <222> (79)..(1401)
<220>
   <221> sig_peptide
   <222> (1)..(78)
<400> 30
   atg ggc gtg ttc gtc gtg cta ctg tcc att gcc acc ttg ttc ggt tcc     48
   Met Gly Val Phe Val Val Leu Leu Ser Ile Ala Thr Leu Phe Gly Ser
       -25                 -20                 -15
   aca tcc ggt acc gcc ttg ggt cct cgt ggt aac tct cac tct tgt gac     96
   Thr Ser Gly Thr Ala Leu Gly Pro Arg Gly Asn Ser His Ser Cys Asp
   -10                 -5                  -1  1               5
   act gtt gac ggt ggt tac caa tgt ttc cca gaa att tct cac ttg tgg    144
   Thr Val Asp Gly Gly Tyr Gln Cys Phe Pro Glu Ile Ser His Leu Trp
               10                  15                  20
   ggt aca tac tct cca ttc ttc tct ttg gct gac gaa tct gct att tct    192
   Gly Thr Tyr Ser Pro Phe Phe Ser Leu Ala Asp Glu Ser Ala Ile Ser
           25                  30                  35
   cca gac gtt cca aag ggt tgt aga gtt act ttc gtt caa gtt ttg tct    240
   Pro Asp Val Pro Lys Gly Cys Arg Val Thr Phe Val Gln Val Leu Ser
       40                  45                  50
   aga cac ggt gct aga tac cca act tct tct gcg tct aag gcg tac tct    288
   Arg His Gly Ala Arg Tyr Pro Thr Ser Ser Ala Ser Lys Ala Tyr Ser
    55                  60                  65                  70
   gct ttg att gaa gct att caa aag aac gct act gct ttc aag ggt aag    336
   Ala Leu Ile Glu Ala Ile Gln Lys Asn Ala Thr Ala Phe Lys Gly Lys
                   75                  80                  85
   tac gct ttc ttg aag act tac aac tac act ttg ggt gct gac gac ttg    384
   Tyr Ala Phe Leu Lys Thr Tyr Asn Tyr Thr Leu Gly Ala Asp Asp Leu
               90                  95                  100
   act cca ttc ggt gaa caa caa atg gtt aac tct ggt att aag ttc tac    432
   Thr Pro Phe Gly Glu Gln Gln Met Val Asn Ser Gly Ile Lys Phe Tyr
           105                 110                 115
   aga aga tac aag gct ttg gct aga aag att gtt cca ttc att aga gct     480
   Arg Arg Tyr Lys Ala Leu Ala Arg Lys Ile Val Pro Phe Ile Arg Ala
       120                 125                 130
   tct ggt tct gac aga gtt att gct tct gct gaa aag ttc att gaa ggt     528
   Ser Gly Ser Asp Arg Val Ile Ala Ser Ala Glu Lys Phe Ile Glu Gly
   135                 140                 145                 150
   ttc caa tct gct aag ttg gct gac cca ggt gct aac cca cac caa gct     576
   Phe Gln Ser Ala Lys Leu Ala Asp Pro Gly Ala Asn Pro His Gln Ala
                   155                 160                 165
   tct cca gtt att aac gtt att att cca gaa ggt gct ggt tac aac aac     624
   Ser Pro Val Ile Asn Val Ile Ile Pro Glu Gly Ala Gly Tyr Asn Asn
               170                 175                 180
   act ttg gac cac ggt ttg tgt act gct ttc gaa gaa tct gaa ttg ggt     672
   Thr Leu Asp His Gly Leu Cys Thr Ala Phe Glu Glu Ser Glu Leu Gly
           185                 190                 195
   gac gac gtt gaa gct aac ttc act gct gtt ttc gct cca cca att aga     720
   Asp Asp Val Glu Ala Asn Phe Thr Ala Val Phe Ala Pro Pro Ile Arg
       200                 205                 210
   gct aga ttg gaa gct cac ttg cca ggt gtt aac ttg act gac gaa gac     768
   Ala Arg Leu Glu Ala His Leu Pro Gly Val Asn Leu Thr Asp Glu Asp
   215                 220                 225                 230
   gtt gtt aac ttg atg gac atg tgt cca ttc gac act gtt gct aga act     816
   Val Val Asn Leu Met Asp Met Cys Pro Phe Asp Thr Val Ala Arg Thr
                   235                 240                 245
   tct gac gct act caa ttg tct cca ttc tgt gac ttg ttc act cac gac     864
   Ser Asp Ala Thr Gln Leu Ser Pro Phe Cys Asp Leu Phe Thr His Asp
               250                 255                 260
   gaa tgg att caa tac gac tac ttg caa tct ttg ggt aag tac tac ggt     912
   Glu Trp Ile Gln Tyr Asp Tyr Leu Gln Ser Leu Gly Lys Tyr Tyr Gly
           265                 270                 275
   tac ggt gct ggt aac cca ttg ggt cca gct caa ggt gtt ggt ttc gtt     960
   Tyr Gly Ala Gly Asn Pro Leu Gly Pro Ala Gln Gly Val Gly Phe Val
       280                 285                 290
   aac gaa ttg att gct aga ttg act cac tct cca gtt caa gac cac act     1008
   Asn Glu Leu Ile Ala Arg Leu Thr His Ser Pro Val Gln Asp His Thr
   295                 300                 305                 310
   tct act aac cac act ttg gac tct aac cca gct act ttc cca ttg aac     1056
   Ser Thr Asn His Thr Leu Asp Ser Asn Pro Ala Thr Phe Pro Leu Asn
                   315                 320                 325
   gct act ttg tac gct gac ttc tct cac gac aac act atg gtt tct att    1104
   Ala Thr Leu Tyr Ala Asp Phe Ser His Asp Asn Thr Met Val Ser Ile
               330                 335                 340
   ttc ttc gct ttg ggt ttg tac aac ggt act aag cca ttg tct act act    1152
   Phe Phe Ala Leu Gly Leu Tyr Asn Gly Thr Lys Pro Leu Ser Thr Thr
           345                 350                 355
   tct gtt gaa tct att gaa gaa act gac ggt tac tct gct tct tgg act    1200
   Ser Val Glu Ser Ile Glu Glu Thr Asp Gly Tyr Ser Ala Ser Trp Thr
       360                 365                 370
   gtt cca ttc gct gct aga gct tac gtt gaa atg atg caa tgt gaa gct    1248
   Val Pro Phe Ala Ala Arg Ala Tyr Val Glu Met Met Gln Cys Glu Ala
   375                 380                 385                 390
   gaa aag gaa cca ttg gtt aga gtt ttg gtt aac gac aga gtt gtt cca    1296
   Glu Lys Glu Pro Leu Val Arg Val Leu Val Asn Asp Arg Val Val Pro
                   395                 400                 405
   ttg cac ggt tgt ggt gtt gac aag ttg ggt aga tgt aag aga gac gac    1344
   Leu His Gly Cys Gly Val Asp Lys Leu Gly Arg Cys Lys Arg Asp Asp
               410                 415                 420
   ttc gtt gaa ggt ttg tct ttc gct aga tct ggt ggt aac tgg gaa gaa    1392
   Phe Val Glu Gly Leu Ser Phe Ala Arg Ser Gly Gly Asn Trp Glu Glu
           425                 430                 435
   tgt ttc gct taa                                                    1404
   Cys Phe Ala
       440
<210> 31
   <211> 467
   <212> PRT
   <213> Artificial Sequence
   <223> Description of Artificial Sequence: consensus phytase 10 thermo 3 q50t, k91a
<400> 31
   Met Gly Val Phe Val Val Leu Leu Ser Ile Ala Thr Leu Phe Gly Ser
       -25                 -20                 -15
   Thr Ser Gly Thr Ala Leu Gly Pro Arg Gly Asn Ser His Ser Cys Asp
   -10                 -5                  -1  1               5
   Thr Val Asp Gly Gly Tyr Gln Cys Phe Pro Glu Ile Ser His Leu Trp
               10                  15                  20
   Gly Thr Tyr Ser Pro Phe Phe Ser Leu Ala Asp Glu Ser Ala Ile Ser
           25                  30                  35
   Pro Asp Val Pro Lys Gly Cys Arg Val Thr Phe Val Gln Val Leu Ser
       40                  45                  50
   Arg His Gly Ala Arg Tyr Pro Thr Ser Ser Ala Ser Lys Ala Tyr Ser
    55                 60                  65                  70
   Ala Leu Ile Glu Ala Ile Gln Lys Asn Ala Thr Ala Phe Lys Gly Lys
                   75                  80                  85
   Tyr Ala Phe Leu Lys Thr Tyr Asn Tyr Thr Leu Gly Ala Asp Asp Leu
               90                  95                  100
   Thr Pro Phe Gly Glu Gln Gln Met Val Asn Ser Gly Ile Lys Phe Tyr
           105                 110                 115
   Arg Arg Tyr Lys Ala Leu Ala Arg Lys Ile Val Pro Phe Ile Arg Ala
       120                 125                 130
   Ser Gly Ser Asp Arg Val Ile Ala Ser Ala Glu Lys Phe Ile Glu Gly
   135                 140                 145                 150
   Phe Gln Ser Ala Lys Leu Ala Asp Pro Gly Ala Asn Pro His Gln Ala
                   155                 160                 165
   Ser Pro Val Ile Asn Val Ile Ile Pro Glu Gly Ala Gly Tyr Asn Asn
               170                 175                 180
   Thr Leu Asp His Gly Leu Cys Thr Ala Phe Glu Glu Ser Glu Leu Gly
           185                 190                 195
   Asp Asp Val Glu Ala Asn Phe Thr Ala Val Phe Ala Pro Pro Ile Arg
       200                 205                 210
   Ala Arg Leu Glu Ala His Leu Pro Gly Val Asn Leu Thr Asp Glu Asp
   215                 220                 225                 230
   Val Val Asn Leu Met Asp Met Cys Pro Phe Asp Thr Val Ala Arg Thr
                   235                 240                 245
   Ser Asp Ala Thr Gln Leu Ser Pro Phe Cys Asp Leu Phe Thr His Asp
               250                 255                 260
   Glu Trp Ile Gln Tyr Asp Tyr Leu Gln Ser Leu Gly Lys Tyr Tyr Gly
           265                 270                 275
   Tyr Gly Ala Gly Asn Pro Leu Gly Pro Ala Gln Gly Val Gly Phe Val
       280                 285                 290
   Asn Glu Leu Ile Ala Arg Leu Thr His Ser Pro Val Gln Asp His Thr
   295                 300                 305                 310
   Ser Thr Asn His Thr Leu Asp Ser Asn Pro Ala Thr Phe Pro Leu Asn
                   315                 320                 325
   Ala Thr Leu Tyr Ala Asp Phe Ser His Asp Asn Thr Met Val Ser Ile
               330                 335                 340
   Phe Phe Ala Leu Gly Leu Tyr Asn Gly Thr Lys Pro Leu Ser Thr Thr
           345                 350                 355
   Ser Val Glu Ser Ile Glu Glu Thr Asp Gly Tyr Ser Ala Ser Trp Thr
       360                 365                 370
   Val Pro Phe Ala Ala Arg Ala Tyr Val Glu Met Met Gln Cys Glu Ala
   375                 380                 385                 390
   Glu Lys Glu Pro Leu Val Arg Val Leu Val Asn Asp Arg Val Val Pro
                   395                 400                 405
   Leu His Gly Cys Gly Val Asp Lys Leu Gly Arg Cys Lys Arg Asp Asp
               410                 415                 420
   Phe Val Glu Gly Leu Ser Phe Ala Arg Ser Gly Gly Asn Trp Glu Glu
           425                 430                 435
   Cys Phe Ala
       440
<210> 32
   <211> 1404
   <212> DNA
   <213> Artificial Sequence
<220>
   <223> Description of Artificial Sequence: Aspergillus fumigatus alpha-mutant
<220>
   <221> CDS
   <222> (1)..(1401)
<220>
   <221> mat_peptide
   <222> (79)..(1401)
<220>
   <221> sig_peptide
   <222> (1)..(78)
<400> 32
   atg ggg gtt ttc gtc gtt cta tta tct atc gcg act ctg ttc ggc agc     48
   Met Gly Val Phe Val Val Leu Leu Ser Ile Ala Thr Leu Phe Gly Ser
       -25                 -20                 -15
   aca tcg ggc act gcg ctg ggc ccc cgt gga aat cac tcc aag tcc tgc    96
   Thr Ser Gly Thr Ala Leu Gly Pro Arg Gly Asn His Ser Lys Ser Cys
   -10                 -5                  -1   1               5
   gat acg gta gac cta ggg tac cag tgc tcc cct gcg act tct cat cta   144
   Asp Thr Val Asp Leu Gly Tyr Gln Cys Ser Pro Ala Thr Ser His Leu
                10                  15                  20
   tgg ggc acg tac tcg cca tac ttt tcg ctc gag gac gag ctg tcc gtg   192
   Trp Gly Thr Tyr Ser Pro Tyr Phe Ser Leu Glu Asp Glu Leu Ser Val
            25                  30                  35
   tcg agt aag ctt ccc aag gat tgc cgg atc acc ttg gta cag gtg cta   240
   Ser Ser Lys Leu Pro Lys Asp Cys Arg Ile Thr Leu Val Gln Val Leu
        40                  45                  50
   tcg cgc cat gga gcg cgg tac cca acc agc tcc aag agc aaa aag tat   288
   Ser Arg His Gly Ala Arg Tyr Pro Thr Ser Ser Lys Ser Lys Lys Tyr
    55                  60                  65                  70
   aag aag ctt att acg gcg atc cag gcc aat gcc acc gac ttc aag ggc   336
   Lys Lys Leu Ile Thr Ala Ile Gln Ala Asn Ala Thr Asp Phe Lys Gly
                    75                  80                  85
   aag tac gcc ttt ttg aag acg tac aac tat act ctg ggt gcg gat gac   384
   Lys Tyr Ala Phe Leu Lys Thr Tyr Asn Tyr Thr Leu Gly Ala Asp Asp
                90                  95                 100
   ctc act ccc ttt ggg gag cag cag ctg gtg aac tcg ggc atc aag ttc   432
   Leu Thr Pro Phe Gly Glu Gln Gln Leu Val Asn Ser Gly Ile Lys Phe
           105                 110                 115
   tac cag agg tac aag gct ctg gcg cgc agt gtg gtg ccg ttt att cgc   480
   Tyr Gln Arg Tyr Lys Ala Leu Ala Arg Ser Val Val Pro Phe Ile Arg
       120                 125                 130
   gcc tca ggc tcg gac cgg gtt att gct tcg gga gag aag ttc atc gag   528
   Ala Ser Gly Ser Asp Arg Val Ile Ala Ser Gly Glu Lys Phe Ile Glu
   135                 140                 145                 150
   ggg ttc cag cag gcg aag ctg gct gat cct ggc gcg acg aac cgc gcc   576
   Gly Phe Gln Gln Ala Lys Leu Ala Asp Pro Gly Ala Thr Asn Arg Ala
                   155                 160                 165
   gct ccg gcg att agt gtg att att ccg gag agc gag acg ttc aac aat   624
   Ala Pro Ala Ile Ser Val Ile Ile Pro Glu Ser Glu Thr Phe Asn Asn
               170                 175                 180
   acg ctg gac cac ggt gtg tgc acg aag ttt gag gcg agt cag ctg gga   672
   Thr Leu Asp His Gly Val Cys Thr Lys Phe Glu Ala Ser Gln Leu Gly
           185                 190                 195
   gat gag gtt gcg gcc aat ttc act gcg ctc ttt gca ccc gac atc cga    720
   Asp Glu Val Ala Ala Asn Phe Thr Ala Leu Phe Ala Pro Asp Ile Arg
       200                 205                 210
   gct cgc ctc gag aag cat ctt cct ggc gtg acg ctg aca gac gag gac    768
   Ala Arg Leu Glu Lys His Leu Pro Gly Val Thr Leu Thr Asp Glu Asp
   215                 220                 225                 230
   gtt gtc agt cta atg gac atg tgt ccg ttt gat acg gta gcg cgc acc    816
   Val Val Ser Leu Met Asp Met Cys Pro Phe Asp Thr Val Ala Arg Thr
                   235                 240                 245
   agc gac gca agt cag ctg tca ccg ttc tgt caa ctc ttc act cac aat    864
   Ser Asp Ala Ser Gln Leu Ser Pro Phe Cys Gln Leu Phe Thr His Asn
               250                 255                 260
   gag tgg aag aag tac gac tac ctt cag tcc ttg ggc aag tac tac ggc    912
   Glu Trp Lys Lys Tyr Asp Tyr Leu Gln Ser Leu Gly Lys Tyr Tyr Gly
           265                 270                 275
   tac ggc gca ggc aac cct ctg gga ccg gct cag ggg ata ggg ttc acc    960
   Tyr Gly Ala Gly Asn Pro Leu Gly Pro Ala Gln Gly Ile Gly Phe Thr
       280                 285                 290
   aac gag ctg att gcc cgg ttg acg cgt tcg cca gtg cag gac cac acc    1008
   Asn Glu Leu Ile Ala Arg Leu Thr Arg Ser Pro Val Gln Asp His Thr
   295                 300                 305                 310
   agc act aac tcg act cta gtc tcc aac ccg gcc acc ttc ccg ttg aac    1056
   Ser Thr Asn Ser Thr Leu Val Ser Asn Pro Ala Thr Phe Pro Leu Asn
                   315                 320                 325
   gct acc atg tac gtc gac ttt tca cac gac aac agc atg gtt tcc atc    1104
   Ala Thr Met Tyr Val Asp Phe Ser His Asp Asn Ser Met Val Ser Ile
               330                 335                 340
   ttc ttt gca ttg ggc ctg tac aac ggc act gaa ccc ttg tcc cgg acc    1152
   Phe Phe Ala Leu Gly Leu Tyr Asn Gly Thr Glu Pro Leu Ser Arg Thr
           345                 350                 355
   tcg gtg gaa agc gcc aag gaa ttg gat ggg tat tct gca tcc tgg gtg    1200
   Ser Val Glu Ser Ala Lys Glu Leu Asp Gly Tyr Ser Ala Ser Trp Val
       360                 365                 370
   gtg cct ttc ggc gcg cga gcc tac ttc gag acg atg caa tgc aag tcg    1248
   Val Pro Phe Gly Ala Arg Ala Tyr Phe Glu Thr Met Gln Cys Lys Ser
   375                 380                 385                 390
   gaa aag gag cct ctt gtt cgc gct ttg att aat gac cgg gtt gtg cca    1296
   Glu Lys Glu Pro Leu Val Arg Ala Leu Ile Asn Asp Arg Val Val Pro
                   395                 400                 405
   ctg cat ggc tgc gat gtg gac aag ctg ggg cga tgc aag ctg aat gac   1344
   Leu His Gly Cys Asp Val Asp Lys Leu Gly Arg Cys Lys Leu Asn Asp
               410                 415                 420
   ttt gtc aag gga ttg agt tgg gcc aga tct ggg ggc aac tgg gga gag   1392
   Phe Val Lys Gly Leu Ser Trp Ala Arg Ser Gly Gly Asn Trp Gly Glu
           425                 430                 435
   tgc ttt agt tga                                                   1404
   Cys Phe Ser
       440
<210> 33
   <211> 467
   <212> PRT
   <213> Artificial Sequence
   <223> Description of Artificial Sequence: Aspergillus fumigatus alpha-mutant
<400> 33
   Met Gly Val Phe Val Val Leu Leu Ser Ile Ala Thr Leu Phe Gly Ser
       -25                 -20                 -15
   Thr Ser Gly Thr Ala Leu Gly Pro Arg Gly Asn His Ser Lys Ser Cys
   -10                 -5              -1   1               5
   Asp Thr Val Asp Leu Gly Tyr Gln Cys Ser Pro Ala Thr Ser His Leu
               10                  15                  20
   Trp Gly Thr Tyr Ser Pro Tyr Phe Ser Leu Glu Asp Glu Leu Ser Val
           25                  30                  35
   Ser Ser Lys Leu Pro Lys Asp Cys Arg Ile Thr Leu Val Gln Val Leu
       40                  45                  50
   Ser Arg His Gly Ala Arg Tyr Pro Thr Ser Ser Lys Ser Lys Lys Tyr
   55                  60                  65                  70
   Lys Lys Leu Ile Thr Ala Ile Gln Ala Asn Ala Thr Asp Phe Lys Gly
                   75                  80                  85
   Lys Tyr Ala Phe Leu Lys Thr Tyr Asn Tyr Thr Leu Gly Ala Asp Asp
               90                  95                  100
   Leu Thr Pro Phe Gly Glu Gln Gln Leu Val Asn Ser Gly Ile Lys Phe
           105                 110                 115
   Tyr Gln Arg Tyr Lys Ala Leu Ala Arg Ser Val Val Pro Phe Ile Arg
       120                 125                 130
   Ala Ser Gly Ser Asp Arg Val Ile Ala Ser Gly Glu Lys Phe Ile Glu
   135                 140                 145                 150
   Gly Phe Gln Gln Ala Lys Leu Ala Asp Pro Gly Ala Thr Asn Arg Ala
                   155                 160                 165
   Ala Pro Ala Ile Ser Val Ile Ile Pro Glu Ser Glu Thr Phe Asn Asn
               170                 175                 180
   Thr Leu Asp His Gly Val Cys Thr Lys Phe Glu Ala Ser Gln Leu Gly
           185                 190                 195
   Asp Glu Val Ala Ala Asn Phe Thr Ala Leu Phe Ala Pro Asp Ile Arg
       200                 205                 210
   Ala Arg Leu Glu Lys His Leu Pro Gly Val Thr Leu Thr Asp Glu Asp
   215                 220                 225                 230
   Val Val Ser Leu Met Asp Met Cys Pro Phe Asp Thr Val Ala Arg Thr
                   235                 240                 245
   Ser Asp Ala Ser Gln Leu Ser Pro Phe Cys Gln Leu Phe Thr His Asn
               250                 255                 260
   Glu Trp Lys Lys Tyr Asp Tyr Leu Gln Ser Leu Gly Lys Tyr Tyr Gly
           265                 270                 275
   Tyr Gly Ala Gly Asn Pro Leu Gly Pro Ala Gln Gly Ile Gly Phe Thr
       280                 285                 290
   Asn Glu Leu Ile Ala Arg Leu Thr Arg Ser Pro Val Gln Asp His Thr
   295                 300                 305                 310
   Ser Thr Asn Ser Thr Leu Val Ser Asn Pro Ala Thr Phe Pro Leu Asn
                   315                 320                 325
   Ala Thr Met Tyr Val Asp Phe Ser His Asp Asn Ser Met Val Ser Ile
               330                 335                 340
   Phe Phe Ala Leu Gly Leu Tyr Asn Gly Thr Glu Pro Leu Ser Arg Thr
           345                 350                 355
   Ser Val Glu Ser Ala Lys Glu Leu Asp Gly Tyr Ser Ala Ser Trp Val
       360                 365                 370
   Val Pro Phe Gly Ala Arg Ala Tyr Phe Glu Thr Met Gln Cys Lys Ser
   375                 380                 385                 390
   Glu Lys Glu Pro Leu Val Arg Ala Leu Ile Asn Asp Arg Val Val Pro
                   395                 400                 405
   Leu His Gly Cys Asp Val Asp Lys Leu Gly Arg Cys Lys Leu Asn Asp
               410                 415                 420
   Phe Val Lys Gly Leu Ser Trp Ala Arg Ser Gly Gly Asn Trp Gly Glu
           425                 430                 435
   Cys Phe Ser
   440
<210> 34
   <211> 1426
   <212> DNA
   <213> Artificial Sequence
<220>
   <223> Description of Artificial Sequence: consensus phytase 7
<220>
   <221> CDS
   <222> (12)..(1412)
<220>
   <221> mat_peptide
   <222> (90)..(1412)
<220>
   <221> sig_peptide
   <222> (12)..(89)
<400> 34
   tatatgaatt c atg ggc gtg ttc gtc gtg cta ctg tcc att gcc acc ttg 50
   Met Gly Val Phe Val Val Leu Leu Ser Ile Ala Thr Leu
   -25 -20 -15
   ttc ggt tcc aca tcc ggt acc gcc ttg ggt cct cgt ggt aat tct cac 98
   Phe Gly Ser Thr Ser Gly Thr Ala Leu Gly Pro Arg Gly Asn Ser His
   -10 -5 -1 1
   tct tgt gac act gtt gac ggt ggt tac caa tgt ttc cca gaa att tct 146
   Ser Cys Asp Thr Val Asp Gly Gly Tyr Gln Cys Phe Pro Glu Ile Ser
   5 10 15
   cac ttg tgg ggt caa tac tct cca tac ttc tct ttg gaa gac gaa tct 194
   His Leu Trp Gly Gln Tyr Ser Pro Tyr Phe Ser Leu Glu Asp Glu Ser
   20 25 30 35
   gct att tct cca gac gtt cca gac gac tgt aga gtt act ttc gtt caa 242
   Ala Ile Ser Pro Asp Val Pro Asp Asp Cys Arg Val Thr Phe Val Gln
   40 45 50
   gtt ttg tct aga cac ggt gct aga tac cca act gac tct aag ggt aag 290
   Val Leu Ser Arg His Gly Ala Arg Tyr Pro Thr Asp Ser Lys Gly Lys
   55 60 65
   aag tac tct gct ttg att gaa gct att caa aag aac gct act gct ttc 338
   Lys Tyr Ser Ala Leu Ile Glu Ala Ile Gln Lys Asn Ala Thr Ala Phe
   70 75 80
   aag ggt aag tac gct ttc ttg aag act tac aac tac act ttg ggt gct     386
   Lys Gly Lys Tyr Ala Phe Leu Lys Thr Tyr Asn Tyr Thr Leu Gly Ala
        85                  90                  95
   gac gac ttg act cca ttc ggt gaa aac caa atg gtt aac tct ggt att     434
   Asp Asp Leu Thr Pro Phe Gly Glu Asn Gln Met Val Asn Ser Gly Ile
   100                 105                 110                 115
   aag ttc tac aga aga tac aag gct ttg gct aga aag att gtt cca ttc     482
   Lys Phe Tyr Arg Arg Tyr Lys Ala Leu Ala Arg Lys Ile Val Pro Phe
                   120                 125                 130
   att aga gct tct ggt tct tct aga gtt att gct tct gct gaa aag ttc     530
   Ile Arg Ala Ser Gly Ser Ser Arg Val Ile Ala Ser Ala Glu Lys Phe
               135                 140                 145
   att gaa ggt ttc caa tct gct aag ttg gct gac cca ggt tct caa cca     578
   Ile Glu Gly Phe Gln Ser Ala Lys Leu Ala Asp Pro Gly Ser Gln Pro
           150                 155                 160
   cac caa gct tct cca gtt att gac gtt att att tct gac gct tct tct     626
   His Gln Ala Ser Pro Val Ile Asp Val Ile Ile Ser Asp Ala Ser Ser
       165                 170                 175
   tac aac aac act ttg gac cca ggt act tgt act gct ttc gaa gac tct     674
   Tyr Asn Asn Thr Leu Asp Pro Gly Thr Cys Thr Ala Phe Glu Asp Ser
   180                 185                 190                 195
   gaa ttg gct gac act gtt gaa gct aac ttc act gct ttg ttc gct cca     722
   Glu Leu Ala Asp Thr Val Glu Ala Asn Phe Thr Ala Leu Phe Ala Pro
                   200                 205                 210
   gct att aga gct aga ttg gaa gct gac ttg cca ggt gtt act ttg act     770
   Ala Ile Arg Ala Arg Leu Glu Ala Asp Leu Pro Gly Val Thr Leu Thr
               215                 220                 225
   gac act gaa gtt act tac ttg atg gac atg tgt tct ttc gaa act gtt     818
   Asp Thr Glu Val Thr Tyr Leu Met Asp Met Cys Ser Phe Glu Thr Val
           230                 235                 240
   gct aga act tct gac gct act gaa ttg tct cca ttc tgt gct ttg ttc     866
   Ala Arg Thr Ser Asp Ala Thr Glu Leu Ser Pro Phe Cys Ala Leu Phe
       245                 250                 255
   act cac gac gaa tgg aga cac tac gac tac ttg caa tct ttg aag aag     914
   Thr His Asp Glu Trp Arg His Tyr Asp Tyr Leu Gln Ser Leu Lys Lys
   260                 265                 270                 275
   tac tac ggt cac ggt gct ggt aac cca ttg ggt cca act caa ggt gtt     962
   Tyr Tyr Gly His Gly Ala Gly Asn Pro Leu Gly Pro Thr Gln Gly Val
                   280                 285                 290
   ggt ttc gct aac gaa ttg att gct aga ttg act aga tct cca gtt caa    1010
   Gly Phe Ala Asn Glu Leu Ile Ala Arg Leu Thr Arg Ser Pro Val Gln
               295                 300                 305
   gac cac act tct act aac cac act ttg gac tct aac cca gct act ttc    1058
   Asp His Thr Ser Thr Asn His Thr Leu Asp Ser Asn Pro Ala Thr Phe
           310                 315                 320
   cca ttg aac gct act ttg tac gct gac ttc tct cac gac aac ggt att    1106
   Pro Leu Asn Ala Thr Leu Tyr Ala Asp Phe Ser His Asp Asn Gly Ile
       325                 330                 335
   att tct att ttc ttc gct ttg ggt ttg tac aac ggt act gct cca ttg    1154
   Ile Ser Ile Phe Phe Ala Leu Gly Leu Tyr Asn Gly Thr Ala Pro Leu
   340                 345                 350                 355
   tct act act tct gtt gaa tct att gaa gaa act gac ggt tac tct tct    1202
   Ser Thr Thr Ser Val Glu Ser Ile Glu Glu Thr Asp Gly Tyr Ser Ser
                   360                 365                 370
   gct tgg act gtt cca ttc gct tct aga gct tac gtt gaa atg atg caa    1250
   Ala Trp Thr Val Pro Phe Ala Ser Arg Ala Tyr Val Glu Met Met Gln
               375                 380                 385
   tgt caa gct gaa aag gaa cca ttg gtt aga gtt ttg gtt aac gac aga    1298
   Cys Gln Ala Glu Lys Glu Pro Leu Val Arg Val Leu Val Asn Asp Arg
           390                 395                 400
   gtt gtt cca ttg cac ggt tgt gct gtt gac aag ttg ggt aga tgt aag    1346
   Val Val Pro Leu His Gly Cys Ala Val Asp Lys Leu Gly Arg Cys Lys
       405                 410                 415
   aga gac gac ttc gtt gaa ggt ttg tct ttc gct aga tct ggt ggt aac    1394
   Arg Asp Asp Phe Val Glu Gly Leu Ser Phe Ala Arg Ser Gly Gly Asn
   420                 425                 430                 435
   tgg gct gaa tgt ttc gct taagaattca tata                            1426
   Trp Ala Glu Cys Phe Ala
                   440
<210> 35
   <211> 467
   <212> PRT
   <213> Artificial Sequence
   <223> Description of Artificial Sequence: consensus phytase 7
<400> 35
   Met Gly Val Phe Val Val Leu Leu Ser Ile Ala Thr Leu Phe Gly Ser
       -25                 -20                 -15
   Thr Ser Gly Thr Ala Leu Gly Pro Arg Gly Asn Ser His Ser Cys Asp
   -10                 -5                  -1  1               5
   Thr Val Asp Gly Gly Tyr Gln Cys Phe Pro Glu Ile Ser His Leu Trp
               10                  15                  20
   Gly Gln Tyr Ser Pro Tyr Phe Ser Leu Glu Asp Glu Ser Ala Ile Ser
           25                  30                  35
   Pro Asp Val Pro Asp Asp Cys Arg Val Thr Phe Val Gln Val Leu Ser
       40                  45                  50
   Arg His Gly Ala Arg Tyr Pro Thr Asp Ser Lys Gly Lys Lys Tyr Ser
   55                  60                  65                  70
   Ala Leu Ile Glu Ala Ile Gln Lys Asn Ala Thr Ala Phe Lys Gly Lys
                   75                  80                  85
   Tyr Ala Phe Leu Lys Thr Tyr Asn Tyr Thr Leu Gly Ala Asp Asp Leu
               90                  95                  100
   Thr Pro Phe Gly Glu Asn Gln Met Val Asn Ser Gly Ile Lys Phe Tyr
           105                 110                 115
   Arg Arg Tyr Lys Ala Leu Ala Arg Lys Ile Val Pro Phe Ile Arg Ala
       120                 125                 130
   Ser Gly Ser Ser Arg Val Ile Ala Ser Ala Glu Lys Phe Ile Glu Gly
   135                 140                 145                 150
   Phe Gln Ser Ala Lys Leu Ala Asp Pro Gly Ser Gln Pro His Gln Ala
                   155                 160                 165
   Ser Pro Val Ile Asp Val Ile Ile Ser Asp Ala Ser Ser Tyr Asn Asn
               170                 175                 180
   Thr Leu Asp Pro Gly Thr Cys Thr Ala Phe Glu Asp Ser Glu Leu Ala
           185                 190                 195
   Asp Thr Val Glu Ala Asn Phe Thr Ala Leu Phe Ala Pro Ala Ile Arg
       200                 205                 210
   Ala Arg Leu Glu Ala Asp Leu Pro Gly Val Thr Leu Thr Asp Thr Glu
   215                 220                 225                 230
   Val Thr Tyr Leu Met Asp Met Cys Ser Phe Glu Thr Val Ala Arg Thr
                   235                 240                 245
   Ser Asp Ala Thr Glu Leu Ser Pro Phe Cys Ala Leu Phe Thr His Asp
               250                 255                 260
   Glu Trp Arg His Tyr Asp Tyr Leu Gln Ser Leu Lys Lys Tyr Tyr Gly
           265                 270                 275
   His Gly Ala Gly Asn Pro Leu Gly Pro Thr Gln Gly Val Gly Phe Ala
       280                 285                 290
   Asn Glu Leu Ile Ala Arg Leu Thr Arg Ser Pro Val Gln Asp His Thr
   295 300 305 310
   Ser Thr Asn His Thr Leu Asp Ser Asn Pro Ala Thr Phe Pro Leu Asn
   315 320 325
   Ala Thr Leu Tyr Ala Asp Phe Ser His Asp Asn Gly Ile Ile Ser Ile
   330 335 340
   Phe Phe Ala Leu Gly Leu Tyr Asn Gly Thr Ala Pro Leu Ser Thr Thr
   345 350 355
   Ser Val Glu Ser Ile Glu Glu Thr Asp Gly Tyr Ser Ser Ala Trp Thr
   360 365 370
   Val Pro Phe Ala Ser Arg Ala Tyr Val Glu Met Met Gln Cys Gln Ala
   375 380 385 390
   Glu Lys Glu Pro Leu Val Arg Val Leu Val Asn Asp Arg Val Val Pro
   395 400 405
   Leu His Gly Cys Ala Val Asp Lys Leu Gly Arg Cys Lys Arg Asp Asp
   410 415 420
   Phe Val Glu Gly Leu Ser Phe Ala Arg Ser Gly Gly Asn Trp Ala Glu
   425 430 435
   Cys Phe Ala
   440
<210> 36
   <211> 467
   <212> PRT
   <213> Artificial Sequence
<220>
   <223> Description of Artificial Sequence: consensus phytase 12
<400> 36
   Met Gly Val Phe Val Val Leu Leu Ser Ile Ala Thr Leu Phe Gly Ser
   1 5 10 15
   Thr Ser Gly Thr Ala Leu Gly Pro Arg Gly Asn Ser His Ser Cys Asp
   20 25 30
   Thr Val Asp Gly Gly Tyr Gln Cys Phe Pro Glu Ile Ser Ser Asn Trp
   35 40 45
   Ser Pro Tyr Ser Pro Tyr Phe Ser Leu Ala Asp Glu Ser Ala Ile Ser
   50 55 60
   Pro Asp Val Pro Lys Gly Cys Arg Val Thr Phe Val Gln Val Leu Gln
    65                  70                  75                  80
   Arg His Gly Ala Arg Phe Pro Thr Ser Gly Ala Ala Thr Arg Ile Ser
                    85                  90                  95
   Ala Leu Ile Glu Ala Ile Gln Lys Asn Ala Thr Ala Phe Lys Gly Lys
               100                 105                 110
   Tyr Ala Phe Leu Lys Thr Tyr Asn Tyr Thr Leu Gly Ala Asp Asp Leu
           115                 120                 125
   Val Pro Phe Gly Ala Asn Gln Ser Ser Gln Ala Gly Ile Lys Phe Tyr
       130                 135                 140
   Arg Arg Tyr Lys Ala Leu Ala Arg Lys Ile Val Pro Phe Ile Arg Ala
   145                 150                 155                 160
   Ser Gly Ser Asp Arg Val Ile Asp Ser Ala Thr Asn Trp Ile Glu Gly
                   165                 170                 175
   Phe Gln Ser Ala Lys Leu Ala Asp Pro Gly Ala Asn Pro His Gln Ala
               180                 185                 190
   Ser Pro Val Ile Asn Val Ile Ile Pro Glu Gly Ala Gly Tyr Asn Asn
           195                 200                 205
   Thr Leu Asp His Gly Leu Cys Thr Ala Phe Glu Glu Ser Glu Leu Gly
       210                 215                 220
   Asp Asp Val Glu Ala Asn Phe Thr Ala Val Phe Ala Pro Pro Ile Arg
   225                 230                 235                 240
   Ala Arg Leu Glu Ala His Leu Pro Gly Val Asn Leu Thr Asp Glu Asp
                   245                 250                 255
   Val Val Asn Leu Met Asp Met Cys Pro Phe Asp Thr Val Ala Arg Thr
               260                 265                 270
   Ser Asp Ala Thr Glu Leu Ser Pro Phe Cys Asp Leu Phe Thr His Asp
           275                 280                 285
   Glu Trp Ile Gln Tyr Asp Tyr Leu Gly Asp Leu Asp Lys Tyr Tyr Gly
       290                 295                 300
   Thr Gly Ala Gly Asn Pro Leu Gly Pro Ala Gln Gly Val Gly Phe Val
   305                 310                 315                 320
   Asn Glu Leu Ile Ala Arg Leu Thr His Ser Pro Val Gln Asp His Thr
                   325                 330                 335
   Ser Thr Asn His Thr Leu Asp Ser Asn Pro Ala Thr Phe Pro Leu Asn
               340                 345                 350
   Ala Thr Leu Tyr Ala Asp Phe Ser His Asp Asn Thr Met Val Ala Ile
           355                 360                 365
   Phe Phe Ala Leu Gly Leu Tyr Asn Gly Thr Lys Pro Leu Ser Thr Thr
       370                 375                 380
   Ser Val Glu Ser Ile Glu Glu Thr Asp Gly Tyr Ser Ala Ser Trp Leu
   385                 390                 395                 400
   Val Pro Phe Ser Ala Arg Met Tyr Val Glu Met Met Gln Cys Glu Ala
                   405                 410                 415
   Glu Lys Glu Pro Leu Val Arg Val Leu Val Asn Asp Arg Val Val Pro
               420                 425                 430
   Leu His Gly Cys Gly Val Asp Lys Leu Gly Arg Cys Lys Arg Asp Asp
           435                 440                 445
   Phe Val Glu Gly Leu Ser Phe Ala Arg Ser Gly Gly Asn Trp Glu Glu
       450                 455                 460
   Cys Phe Ala
   465
<210> 37
   <211> 26
   <212> DNA
   <213> Artificial Sequence
<220>
   <223> Description of Artificial Sequence: primer
<400> 37
   tatatgaatt catgggcgtg ttcgtc 26
<210> 38
   <211> 22
   <212> DNA
   <213> Artificial Sequence
<220>
   <223> Description of Artificial Sequence: primer
<400> 38
   tgaaaagttc attgaaggtt tc 22
<210> 39
   <211> 22
   <212> DNA
   <213> Artificial Sequence
<220>
   <223> Description of Artificial Sequence: primer
<400> 39
   tcttcgaaag cagtacacaa ac 22
<210> 40
   <211> 22
   <212> DNA
   <213> Artificial Sequence
<220>
   <223> Description of Artificial Sequence:primer
<400> 40
   tatatgaatt cttaagcgaa ac 22
<210> 41
   <211> 32
   <212> DNA
   <213> Artificial Sequence
<220>
   <223> Description of Artificial Sequence: primer
<400> 41
   cacttgtggg gtacctactc tccatacttc tc 32
<210> 42
   <211> 31
   <212> DNA
   <213> Artificial Sequence
<220>
   <223> Description of Artificial Sequence: primer
<400> 42
   ggtcaatact ctccattctt ctctttggaa g 31
<210> 43
   <211> 28
   <212> DNA
   <213> Artificial Sequence
<220>
   <223> Description of Artificial Sequence: primer
<400> 43
   catacttctc tttggcagac gaatctgc 28
<210> 44
   <211> 31
   <212> DNA
   <213> Artificial Sequence
<220>
   <223> Description of Artificial Sequence: primer
<400> 44
   ctccagacgt cccaaaggac tgtagagtta c 31
<210> 45
   <211> 31
   <212> DNA
   <213> Artificial Sequence
<220>
   <223> Description of Artificial Sequence: primer
<400> 45
   ctccagacgt cccagacggc tgtagagtta c 31
<210> 46
   <211> 36
   <212> DNA
   <213> Artificial Sequence
<220>
   <223> Description of Artificial Sequence: primer
<400> 46
   gatacccaac ttcttctgcg tctaaggctt actctg 36
<210> 47
   <211> 29
   <212> DNA
   <213> Artificial Sequence
<220>
   <223> Description of Artificial Sequence: primer
<400> 47
   cttctaagtc taagaagtac tctgctttg 29
<210> 48
   <211> 41
   <212> DNA
   <213> Artificial Sequence
<220>
   <223> Description of Artificial Sequence: primer
<400> 48
   gcttactctg ctttgattga acggattcaa aagaacgcta c 41
<210> 49
   <211> 29
   <212> DNA
   <213> Artificial Sequence
<220>
   <223> Description of Artificial Sequence: primer
<400> 49
   ccattcggtg aacagcaaat ggttaactc 29
<210> 50
   <211> 30
   <212> DNA
   <213> Artificial Sequence
<220>
   <223> Description of Artificial Sequence: primer
<400> 50
   gatacaaggc tctcgcgaga aacattgttc 30
<210> 51
   <211> 30
   <212> DNA
   <213> Artificial Sequence
<220>
   <223> Description of Artificial Sequence: primer
<400> 51
   gattgttcca ttcgtgcgcg cttctggttc 30
<210> 52
   <211> 31
   <212> DNA
   <213> Artificial Sequence
<220>
   <223> Description of Artificial Sequence: primer
<400> 52
   ctccagttat taacgtgatc attccagaag g 31
<210> 53
   <211> 30
   <212> DNA
   <213> Artificial Sequence
<220>
   <223> Description of Artificial Sequence: primer
<400> 53
   ggctgaccca ggggcccaac cacaccaagc 30
<210> 54
   <211> 32
   <212> DNA
   <213> Artificial Sequence
<220>
   <223> Description of Artificial Sequence: primer
<400> 54
   cactttggac catggtcttt gtactgcttt cg 32
<210> 55
   <211> 34
   <212> DNA
   <213> Artificial Sequence
<220>
   <223> Description of Artificial Sequence: primer
<400> 55
   gctttcgaag actctaccct aggtgacgac gttg 34
<210> 56
   <211> 26
   <212> DNA
   <213> Artificial Sequence
<220>
   <223> Description of Artificial Sequence: primer
<400> 56
   ggtgacgacg ctgaagctaa cttcac 26
<210> 57
   <211> 27
   <212> DNA
   <213> Artificial Sequence
<220>
   <223> Description of Artificial Sequence: primer
<400> 57
   ctaacttcac cgcggtgttc gctccag 27
<210> 58
   <211> 34
   <212> DNA
   <213> Artificial Sequence
<220>
   <223> Description of Artificial Sequence: primer
<400> 58
   gctttgttcg ctccacctat tagagctaga ttgg 34
<210> 59
   <211> 26
   <212> DNA
   <213> Artificial Sequence
<220>
   <223> Description of Artificial Sequence: primer
<400> 59
   gccaggtgtt aacttgactg acgaag 26
<210> 60
   <211> 27
   <212> DNA
   <213> Artificial Sequence
<220>
   <223> Description of Artificial Sequence: primer
<400> 60
   gacgaagacg tcgttaactt gatggac 27
<210> 61
   <211> 28
   <212> DNA
   <213> Artificial Sequence
<220>
   <223> Description of Artificial Sequence: primer
<400> 61
   gtccattcga cactgtcgct agaacttc 28
<210> 62
   <211> 26
   <212> DNA
   <213> Artificial Sequence
<220>
   <223> Description of Artificial Sequence: primer
<400> 62
   ctgacgctac tcagctgtct ccattc 26
<210> 63
   <211> 28
   <212> DNA
   <213> Artificial Sequence
<220>
   <223> Description of Artificial Sequence: primer
<400> 63
   gtctccattc tgtgatttgt tcactcac 28
<210> 64
   <211> 26
   <212> DNA
   <213> Artificial Sequence
<220>
   <223> Description of Artificial Sequence: primer
<400> 64
   gctttgttca ccgcggacga atggag 26
<210> 65
   <211> 27
   <212> DNA
   <213> Artificial Sequence
<220>
   <223> Description of Artificial Sequence: primer
<400> 65
   cacgacgaat ggatccaata cgactac 27
<210> 66
   <211> 27
   <212> DNA
   <213> Artificial Sequence
<220>
   <223> Description of Artificial Sequence: primer
<400> 66
   gacgaatgga gagcgtacga ctacttg 27
<210> 67
   <211> 29
   <212> DNA
   <213> Artificial Sequence
<220>
   <223> Description of Artificial Sequence: primer
<400> 67
   ggtgttggtt tcgttaacga attgattgc 29
<210> 68
   <211> 28
   <212> DNA
   <213> Artificial Sequence
<220>
   <223> Description of Artificial Sequence: primer
<400> 68
   gctagattga ctcactctcc agttcaag 28
<210> 69
   <211> 32
   <212> DNA
   <213> Artificial Sequence
<220>
   <223> Description of Artificial Sequence: primer
<400> 69
   ctcacgacaa cactatgata tctattttct tc 32
<210> 70
   <211> 30
   <212> DNA
   <213> Artificial Sequence
<220>
   <223> Description of Artificial Sequence: primer
<400> 70
   cgacaactcc atggtttcta ttttcttcgc 30
<210> 71
   <211> 27
   <212> DNA
   <213> Artificial Sequence
<220>
   <223> Description of Artificial Sequence: primer
<400> 71
   gtacaacggt accaagccat tgtctac 27
<210> 72
   <211> 25
   <212> DNA
   <213> Artificial Sequence
<220>
   <223> Description of Artificial Sequence: primer
<400> 72
   ctgacggtta cgctgcttct tggac 25
<210> 73
   <211> 26
   <212> DNA
   <213> Artificial Sequence
<220>
   <223> Description of Artificial Sequence: primer
<400> 73
   ctgttccatt cgctgctaga gcttac 26
<210> 74
   <211> 27
   <212> DNA
   <213> Artificial Sequence
<220>
   <223> Description of Artificial Sequence: primer
<400> 74
   gatgcaatgt gaagctgaaa aggaacc 27
<210> 75
   <211> 26
   <212> DNA
   <213> Artificial Sequence
<220>
   <223> Description of Artificial Sequence: primer
<400> 75
   cacggttgtg gtgtcgacaa gttggg 26
<210> 76
   <211> 30
   <212> DNA
   <213> Artificial Sequence
<220>
   <223> Description of Artificial Sequence: primer
<400> 76
   gatctggtgg caattgggag gaatgtttcg 30
<210> 77
   <211> 28
   <212> DNA
   <213> Artificial Sequence
<220>
   <223> Description of Artificial Sequence: primer
<400> 77
   cacgtactcg ccatactttt cgctcgag 28
<210> 78
   <211> 33
   <212> DNA
   <213> Artificial Sequence
<220>
   <223> Description of Artificial Sequence: primer
<400> 78
   ccatactttt cgctcgcgga cgagctgtcc gtg 33
<210> 79
   <211> 31
   <212> DNA
   <213> Artificial Sequence
<220>
   <223> Description of Artificial Sequence: primer
<400> 79
   gtataagaag cttattacgg cgatccaggc c 31
<210> 80
   <211> 31
   <212> DNA
   <213> Artificial Sequence
<220>
   <223> Description of Artificial Sequence: primer
<400> 80
   cttcaagggc aagtacgcct ttttgaagac g 31
<210> 81
   <211> 29
   <212> DNA
   <213> Artificial Sequence
<220>
   <223> Description of Artificial Sequence: primer
<400> 81
   catccgagct cgcctcgaga agcatcttc 29
<210> 82
   <211> 29
   <212> DNA
   <213> Artificial Sequence
<220>
   <223> Description of Artificial Sequence: primer
<400> 82
   ctaatggatg tgtccgtttg atacggtag 29
<210> 83
   <211> 27
   <212> DNA
   <213> Artificial Sequence
<220>
   <223> Description of Artificial Sequence: primer
<400> 83
   gtggaagaag tacgactacc ttcagtc 27
<210> 84
   <211> 28
   <212> DNA
   <213> Artificial Sequence
<220>
   <223> Description of Artificial Sequence: primer
<400> 84
   gcccggttga cgcattcgcc agtgcagg 28
<210> 85
   <211> 29
   <212> DNA
   <213> Artificial Sequence
<220>
   <223> Description of Artificial Sequence: primer
<400> 85
   cacacgacaa caccatggtt tccatcttc 29
<210> 86
   <211> 30
   <212> DNA
   <213> Artificial Sequence
<220>
   <223> Description of Artificial Sequence: primer
<400> 86
   gtggtgcctt tcgccgcgcg agcctacttc 30
<210> 87
   <211> 33
   <212> DNA
   <213> Artificial Sequence
<220>
   <223> Description of Artificial Sequence: primer
<400> 87
   tatatcatga gcgtgttcgt cgtgctactg ttc 33
<210> 88
   <211> 33
   <212> DNA
   <213> Artificial Sequence
<220>
   <223> Description of Artificial Sequence: primer
<400> 88
   acccgactta caaagcgaat tctatagata tat 33
<210> 89
   <211> 33
   <212> DNA
   <213> Artificial Sequence
<220>
   <223> Description of Artificial Sequence: primer
<400> 89
   acccttctta caaagcgaat tctatagata tat 33
<210> 90
   <211> 1404
   <212> DNA
   <213> Artificial Sequence
<220>
   <223> Description of Artificial Sequence: Consensus-phytase-3-thermo-11-Q50T
<220>
   <221> CDS
   <222> (1)..(1401)
<220>
   <221> sig_peptide
   <222> (1)..(69)
<220>
   <221> mat_peptide
   <222> (70)..(1401)
<400> 90
   atg ggc gtg ttc gtc gtg cta ctg tcc att gcc acc ttg ttc ggt tcc     48
   Met Gly Val Phe Val Val Leu Leu Ser Ile Ala Thr Leu Phe Gly Ser
               -20                     -15                 -10
   aca tcc ggt acc gcc ttg ggt cct cgt ggt aat tct cac tct tgt gac     96
   Thr Ser Gly Thr Ala Leu Gly Pro Arg Gly Asn Ser His Ser Cys Asp
            -5                 -1   1               5
   act gtt gac ggt ggt tac caa tgt ttc cca gaa att tct cac ttg tgg    144
   Thr Val Asp Gly Gly Tyr Gln Cys Phe Pro Glu Ile Ser His Leu Trp
    10                  15                  20                  25
   ggt acc tac tct cca tac ttc tct ttg gca gac gaa tct gct att tct    192
   Gly Thr Tyr Ser Pro Tyr Phe Ser Leu Ala Asp Glu Ser Ala Ile Ser
                    30                  35                  40
   cca gac gtc cca aag gac tgt aga gtt act ttc gtt caa gtt ttg tct    240
   Pro Asp Val Pro Lys Asp Cys Arg Val Thr Phe Val Gln Val Leu Ser
                45                  50                  55
   aga cac ggt gct aga tac cca act tct tct aag tct aag gct tac tct     288
   Arg His Gly Ala Arg Tyr Pro Thr Ser Ser Lys Ser Lys Ala Tyr Ser
           60                  65                  70
   gct ttg att gaa gct att caa aag aac gct act gct ttc aag ggt aag     336
   Ala Leu Ile Glu Ala Ile Gln Lys Asn Ala Thr Ala Phe Lys Gly Lys
       75                  80                  85
   tac gct ttc ttg aag act tac aac tac act ttg ggt gct gac gac ttg     384
   Tyr Ala Phe Leu Lys Thr Tyr Asn Tyr Thr Leu Gly Ala Asp Asp Leu
   90                  95                  100                 105
   act cca ttc ggt gaa aac caa atg gtt aac tct ggt att aag ttc tac     432
   Thr Pro Phe Gly Glu Asn Gln Met Val Asn Ser Gly Ile Lys Phe Tyr
                   110                 115                 120
   aga aga tac aag gct ttg gct aga aag att gtt cca ttc att aga gct     480
   Arg Arg Tyr Lys Ala Leu Ala Arg Lys Ile Val Pro Phe Ile Arg Ala
               125                 130                 135
   tct ggt tct gac aga gtt att gct tct gct gaa aag ttc att gaa ggt     528
   Ser Gly Ser Asp Arg Val Ile Ala Ser Ala Glu Lys Phe Ile Glu Gly
           140                 145                 150
   ttc caa tct gct aag ttg gct gac cca ggt tct caa cca cac caa gct     576
   Phe Gln Ser Ala Lys Leu Ala Asp Pro Gly Ser Gln Pro His Gln Ala
       155                 160                 165
   tct cca gtt att aac gtg atc att cca gaa gga tcc ggt tac aac aac     624
   Ser Pro Val Ile Asn Val Ile Ile Pro Glu Gly Ser Gly Tyr Asn Asn
   170                 175                 180                 185
   act ttg gac cat ggt ctt tgt act gct ttc gaa gac tct acc cta ggt     672
   Thr Leu Asp His Gly Leu Cys Thr Ala Phe Glu Asp Ser Thr Leu Gly
                   190                 195                 200
   gac gac gtt gaa gct aac ttc act gct ttg ttc gct cca gct att aga     720
   Asp Asp Val Glu Ala Asn Phe Thr Ala Leu Phe Ala Pro Ala Ile Arg
               205                 210                 215
   gct aga ttg gaa gct gac ttg cca ggt gtt act ttg act gac gaa gac     768
   Ala Arg Leu Glu Ala Asp Leu Pro Gly Val Thr Leu Thr Asp Glu Asp
           220                 225                 230
   gtt gtt tac ttg atg gac atg tgt cca ttc gac act gtc gct aga act     816
   Val Val Tyr Leu Met Asp Met Cys Pro Phe Asp Thr Val Ala Arg Thr
       235                 240                 245
   tct gac gct act gaa ttg tct cca ttc tgt gct ttg ttc act cac gac     864
   Ser Asp Ala Thr Glu Leu Ser Pro Phe Cys Ala Leu Phe Thr His Asp
   250                 255                 260                 265
   gaa tgg atc caa tac gac tac ttg caa agc ttg ggt aag tac tac ggt    912
   Glu Trp Ile Gln Tyr Asp Tyr Leu Gln Ser Leu Gly Lys Tyr Tyr Gly
                   270                 275                 280
   tac ggt gct ggt aac cca ttg ggt cca gct caa ggt gtt ggt ttc gct    960
   Tyr Gly Ala Gly Asn Pro Leu Gly Pro Ala Gln Gly Val Gly Phe Ala
               285                 290                 295
   aac gaa ttg att gct aga ttg act cac tct cca gtt caa gac cac act    1008
   Asn Glu Leu Ile Ala Arg Leu Thr His Ser Pro Val Gln Asp His Thr
           300                 305                 310
   tct act aac cac act ttg gac tct aac cca gct act ttc cca ttg aac    1056
   Ser Thr Asn His Thr Leu Asp Ser Asn Pro Ala Thr Phe Pro Leu Asn
       315                 320                 325
   gct act ttg tac gct gac ttc tct cac gac aac act atg ata tct att    1104
   Ala Thr Leu Tyr Ala Asp Phe Ser His Asp Asn Thr Met Ile Ser Ile
   330                 335                 340                 345
   ttc ttc gct ttg ggt ttg tac aac ggt acc aag cca ttg tct act act    1152
   Phe Phe Ala Leu Gly Leu Tyr Asn Gly Thr Lys Pro Leu Ser Thr Thr
                   350                 355                 360
   tct gtt gaa tct att gaa gaa act gac ggt tac tct gct tct tgg act    1200
   Ser Val Glu Ser Ile Glu Glu Thr Asp Gly Tyr Ser Ala Ser Trp Thr
               365                 370                 375
   gtt cca ttc gct gct aga gct tac gtt gaa atg atg caa tgt caa gct    1248
   Val Pro Phe Ala Ala Arg Ala Tyr Val Glu Met Met Gln Cys Gln Ala
           380                 385                 390
   gaa aag gaa cca ttg gtt aga gtt ttg gtt aac gac aga gtt gtt cca    1296
   Glu Lys Glu Pro Leu Val Arg Val Leu Val Asn Asp Arg Val Val Pro
       395                 400                 405
   ttg cac ggt tgt gct gtt gac aag ttg ggt aga tgt aag aga gac gac    1344
   Leu His Gly Cys Ala Val Asp Lys Leu Gly Arg Cys Lys Arg Asp Asp
   410                 415                 420                 425
   ttc gtt gaa ggt ttg tct ttc gct aga tct ggt ggt aac tgg gct gaa    1392
   Phe Val Glu Gly Leu Ser Phe Ala Arg Ser Gly Gly Asn Trp Ala Glu
                   430                 435                 440
   tgt ttc gct taa                                                    1404
   Cys Phe Ala
<210> 91
   <211> 467
   <212> PRT
   <213> Artificial Sequence
   <223> Description of Artificial Sequence: Consensus-phytase-3-thermo-11-Q50T
<400> 91
   Met Gly Val Phe Val Val Leu Leu Ser Ile Ala Thr Leu Phe Gly Ser
               -20                 -15                 -10
   Thr Ser Gly Thr Ala Leu Gly Pro Arg Gly Asn Ser His Ser Cys Asp
           -5              -1   1               5
   Thr Val Asp Gly Gly Tyr Gln Cys Phe Pro Glu Ile Ser His Leu Trp
    10                  15                  20                  25
   Gly Thr Tyr Ser Pro Tyr Phe Ser Leu Ala Asp Glu Ser Ala Ile Ser
                   30                  35                  40
   Pro Asp Val Pro Lys Asp Cys Arg Val Thr Phe Val Gln Val Leu Ser
               45                  50                  55
   Arg His Gly Ala Arg Tyr Pro Thr Ser Ser Lys Ser Lys Ala Tyr Ser
           60                  65                  70
   Ala Leu Ile Glu Ala Ile Gln Lys Asn Ala Thr Ala Phe Lys Gly Lys
       75                  80                  85
   Tyr Ala Phe Leu Lys Thr Tyr Asn Tyr Thr Leu Gly Ala Asp Asp Leu
    90                  95                 100                 105
   Thr Pro Phe Gly Glu Asn Gln Met Val Asn Ser Gly Ile Lys Phe Tyr
                   110                 115                 120
   Arg Arg Tyr Lys Ala Leu Ala Arg Lys Ile Val Pro Phe Ile Arg Ala
               125                 130                 135
   Ser Gly Ser Asp Arg Val Ile Ala Ser Ala Glu Lys Phe Ile Glu Gly
           140                 145                 150
   Phe Gln Ser Ala Lys Leu Ala Asp Pro Gly Ser Gln Pro His Gln Ala
       155                 160                 165
   Ser Pro Val Ile Asn Val Ile Ile Pro Glu Gly Ser Gly Tyr Asn Asn
   170                 175                 180                 185
   Thr Leu Asp His Gly Leu Cys Thr Ala Phe Glu Asp Ser Thr Leu Gly
                   190                 195                 200
   Asp Asp Val Glu Ala Asn Phe Thr Ala Leu Phe Ala Pro Ala Ile Arg
               205                 210                 215
   Ala Arg Leu Glu Ala Asp Leu Pro Gly Val Thr Leu Thr Asp Glu Asp
           220                 225                 230
   Val Val Tyr Leu Met Asp Met Cys Pro Phe Asp Thr Val Ala Arg Thr
       235                 240                 245
   Ser Asp Ala Thr Glu Leu Ser Pro Phe Cys Ala Leu Phe Thr His Asp
   250                 255                 260                 265
   Glu Trp Ile Gln Tyr Asp Tyr Leu Gln Ser Leu Gly Lys Tyr Tyr Gly
                   270                 275                 280
   Tyr Gly Ala Gly Asn Pro Leu Gly Pro Ala Gln Gly Val Gly Phe Ala
               285                 290                 295
   Asn Glu Leu Ile Ala Arg Leu Thr His Ser Pro Val Gln Asp His Thr
           300                 305                 310
   Ser Thr Asn His Thr Leu Asp Ser Asn Pro Ala Thr Phe Pro Leu Asn
       315                 320                 325
   Ala Thr Leu Tyr Ala Asp Phe Ser His Asp Asn Thr Met Ile Ser Ile
   330                 335                 340                 345
   Phe Phe Ala Leu Gly Leu Tyr Asn Gly Thr Lys Pro Leu Ser Thr Thr
                   350                 355                 360
   Ser Val Glu Ser Ile Glu Glu Thr Asp Gly Tyr Ser Ala Ser Trp Thr
               365                 370                 375
   Val Pro Phe Ala Ala Arg Ala Tyr Val Glu Met Met Gln Cys Gln Ala
           380                 385                 390
   Glu Lys Glu Pro Leu Val Arg Val Leu Val Asn Asp Arg Val Val Pro
       395                 400                 405
   Leu His Gly Cys Ala Val Asp Lys Leu Gly Arg Cys Lys Arg Asp Asp
   410                 415                 420                 425
   Phe Val Glu Gly Leu Ser Phe Ala Arg Ser Gly Gly Asn Trp Ala Glu
                   430                 435                 440
   Cys Phe Ala
<210> 92
   <211> 1404
   <212> DNA
   <213> Artificial Sequence
<220>
   <223> Description of Artificial Sequence: Consensus phytase-3-thermo-11-Q50T-K91A
<220>
   <221> sig_peptide
   <222> (1)..(69)
<220>
   <221> CDS
   <222> (1)..(1401)
<220>
   <221> mat_peptide
   <222> (70)..(1401)
<400> 92
   atg ggc gtg ttc gtc gtg cta ctg tcc att gcc acc ttg ttc ggt tcc    48
   Met Gly Val Phe Val Val Leu Leu Ser Ile Ala Thr Leu Phe Gly Ser
               -20                 -15                 -10
   aca tcc ggt acc gcc ttg ggt cct cgt ggt aat tct cac tct tgt gac    96
   Thr Ser Gly Thr Ala Leu Gly Pro Arg Gly Asn Ser His Ser Cys Asp
           -5                  -1   1               5
   act gtt gac ggt ggt tac caa tgt ttc cca gaa att tct cac ttg tgg    144
   Thr Val Asp Gly Gly Tyr Gln Cys Phe Pro Glu Ile Ser His Leu Trp
    10                  15                  20                  25
   ggt acc tac tct cca tac ttc tct ttg gca gac gaa tct gct att tct    192
   Gly Thr Tyr Ser Pro Tyr Phe Ser Leu Ala Asp Glu Ser Ala Ile Ser
                    30                  35                  40
   cca gac gtc cca aag gac tgt aga gtt act ttc gtt caa gtt ttg tct    240
   Pro Asp Val Pro Lys Asp Cys Arg Val Thr Phe Val Gln Val Leu Ser
                45                  50                  55
   aga cac ggt gct aga tac cca act tct tct gcg tct aag gct tac tct    288
   Arg His Gly Ala Arg Tyr Pro Thr Ser Ser Ala Ser Lys Ala Tyr Ser
            60                  65                  70
   gct ttg att gaa gct att caa aag aac gct act gct ttc aag ggt aag    336
   Ala Leu Ile Glu Ala Ile Gln Lys Asn Ala Thr Ala Phe Lys Gly Lys
        75                  80                  85
   tac gct ttc ttg aag act tac aac tac act ttg ggt gct gac gac ttg    384
   Tyr Ala Phe Leu Lys Thr Tyr Asn Tyr Thr Leu Gly Ala Asp Asp Leu
    90                  95                 100                 105
   act cca ttc ggt gaa aac caa atg gtt aac tct ggt att aag ttc tac    432
   Thr Pro Phe Gly Glu Asn Gln Met Val Asn Ser Gly Ile Lys Phe Tyr
                   110                 115                 120
   aga aga tac aag gct ttg gct aga aag att gtt cca ttc att aga gct    480
   Arg Arg Tyr Lys Ala Leu Ala Arg Lys Ile Val Pro Phe Ile Arg Ala
               125                 130                 135
   tct ggt tct gac aga gtt att gct tct gct gaa aag ttc att gaa ggt    528
   Ser Gly Ser Asp Arg Val Ile Ala Ser Ala Glu Lys Phe Ile Glu Gly
           140                 145                 150
   ttc caa tct gct aag ttg gct gac cca ggt tct caa cca cac caa gct    576
   Phe Gln Ser Ala Lys Leu Ala Asp Pro Gly Ser Gln Pro His Gln Ala
       155                 160                 165
   tct cca gtt att aac gtg atc att cca gaa gga tcc ggt tac aac aac    624
   Ser Pro Val Ile Asn Val Ile Ile Pro Glu Gly Ser Gly Tyr Asn Asn
   170                 175                 180                 185
   act ttg gac cat ggt ctt tgt act gct ttc gaa gac tct acc cta ggt    672
   Thr Leu Asp His Gly Leu Cys Thr Ala Phe Glu Asp Ser Thr Leu Gly
                   190                 195                 200
   gac gac gtt gaa gct aac ttc act gct ttg ttc gct cca gct att aga    720
   Asp Asp Val Glu Ala Asn Phe Thr Ala Leu Phe Ala Pro Ala Ile Arg
               205                 210                 215
   gct aga ttg gaa gct gac ttg cca ggt gtt act ttg act gac gaa gac    768
   Ala Arg Leu Glu Ala Asp Leu Pro Gly Val Thr Leu Thr Asp Glu Asp
           220                 225                 230
   gtt gtt tac ttg atg gac atg tgt cca ttc gac act gtc gct aga act    816
   Val Val Tyr Leu Met Asp Met Cys Pro Phe Asp Thr Val Ala Arg Thr
       235                 240                 245
   tct gac gct act gaa ttg tct cca ttc tgt gct ttg ttc act cac gac    864
   Ser Asp Ala Thr Glu Leu Ser Pro Phe Cys Ala Leu Phe Thr His Asp
   250                 255                 260                 265
   gaa tgg atc caa tac gac tac ttg caa agc ttg ggt aag tac tac ggt    912
   Glu Trp Ile Gln Tyr Asp Tyr Leu Gln Ser Leu Gly Lys Tyr Tyr Gly
                   270                 275                 280
   tac ggt gct ggt aac cca ttg ggt cca gct caa ggt gtt ggt ttc gct    960
   Tyr Gly Ala Gly Asn Pro Leu Gly Pro Ala Gln Gly Val Gly Phe Ala
               285                 290                 295
   aac gaa ttg att gct aga ttg act cac tct cca gtt caa gac cac act    1008
   Asn Glu Leu Ile Ala Arg Leu Thr His Ser Pro Val Gln Asp His Thr
           300                 305                 310
   tct act aac cac act ttg gac tct aac cca gct act ttc cca ttg aac    1056
   Ser Thr Asn His Thr Leu Asp Ser Asn Pro Ala Thr Phe Pro Leu Asn
       315                 320                 325
   gct act ttg tac gct gac ttc tct cac gac aac act atg ata tct att    1104
   Ala Thr Leu Tyr Ala Asp Phe Ser His Asp Asn Thr Met Ile Ser Ile
   330                 335                 340                 345
   ttc ttc gct ttg ggt ttg tac aac ggt acc aag cca ttg tct act act    1152
   Phe Phe Ala Leu Gly Leu Tyr Asn Gly Thr Lys Pro Leu Ser Thr Thr
                   350                 355                 360
   tct gtt gaa tct att gaa gaa act gac ggt tac tct gct tct tgg act    1200
   Ser Val Glu Ser Ile Glu Glu Thr Asp Gly Tyr Ser Ala Ser Trp Thr
               365                 370                 375
   gtt cca ttc gct gct aga gct tac gtt gaa atg atg caa tgt caa gct    1248
   Val Pro Phe Ala Ala Arg Ala Tyr Val Glu Met Met Gln Cys Gln Ala
           380                 385                 390
   gaa aag gaa cca ttg gtt aga gtt ttg gtt aac gac aga gtt gtt cca    1296
   Glu Lys Glu Pro Leu Val Arg Val Leu Val Asn Asp Arg Val Val Pro
       395                 400                 405
   ttg cac ggt tgt gct gtt gac aag ttg ggt aga tgt aag aga gac gac    1344
   Leu His Gly Cys Ala Val Asp Lys Leu Gly Arg Cys Lys Arg Asp Asp
   410                 415                 420                     425
   ttc gtt gaa ggt ttg tct ttc gct aga tct ggt ggt aac tgg gct gaa    1392
   Phe Val Glu Gly Leu Ser Phe Ala Arg Ser Gly Gly Asn Trp Ala Glu
                   430                 435                 440
   tgt ttc gct taa                                                    1404
   Cys Phe Ala
<210> 93
   <211> 467
   <212> PRT
   <213> Artificial Sequence
   <223> Description of Artificial Sequence: Consensus phytase-3-thermo-11-Q50T-K91A
<400> 93
   Met Gly Val Phe Val Val Leu Leu Ser Ile Ala Thr Leu Phe Gly Ser
               -20                 -15                 -10
   Thr Ser Gly Thr Ala Leu Gly Pro Arg Gly Asn Ser His Ser Cys Asp
           -5                  -1  1               5
   Thr Val Asp Gly Gly Tyr Gln Cys Phe Pro Glu Ile Ser His Leu Trp
    10                  15                  20                  25
   Gly Thr Tyr Ser Pro Tyr Phe Ser Leu Ala Asp Glu Ser Ala Ile Ser
                   30                  35                  40
   Pro Asp Val Pro Lys Asp Cys Arg Val Thr Phe Val Gln Val Leu Ser
               45                  50                  55
   Arg His Gly Ala Arg Tyr Pro Thr Ser Ser Ala Ser Lys Ala Tyr Ser
           60                  65                  70
   Ala Leu Ile Glu Ala Ile Gln Lys Asn Ala Thr Ala Phe Lys Gly Lys
       75                  80                  85
   Tyr Ala Phe Leu Lys Thr Tyr Asn Tyr Thr Leu Gly Ala Asp Asp Leu
   90 95 100 105
   Thr Pro Phe Gly Glu Asn Gln Met Val Asn Ser Gly Ile Lys Phe Tyr
   110 115 120
   Arg Arg Tyr Lys Ala Leu Ala Arg Lys Ile Val Pro Phe Ile Arg Ala
   125 130 135
   Ser Gly Ser Asp Arg Val Ile Ala Ser Ala Glu Lys Phe Ile Glu Gly
   140 145 150
   Phe Gln Ser Ala Lys Leu Ala Asp Pro Gly Ser Gln Pro His Gln Ala
   155 160 165
   Ser Pro Val Ile Asn Val Ile Ile Pro Glu Gly Ser Gly Tyr Asn Asn
   170 175 180 185
   Thr Leu Asp His Gly Leu Cys Thr Ala Phe Glu Asp Ser Thr Leu Gly
   190 195 200
   Asp Asp Val Glu Ala Asn Phe Thr Ala Leu Phe Ala Pro Ala Ile Arg
   205 210 215
   Ala Arg Leu Glu Ala Asp Leu Pro Gly Val Thr Leu Thr Asp Glu Asp
   220 225 230
   Val Val Tyr Leu Met Asp Met Cys Pro Phe Asp Thr Val Ala Arg Thr
   235 240 245
   Ser Asp Ala Thr Glu Leu Ser Pro Phe Cys Ala Leu Phe Thr His Asp
   250 255 260 265
   Glu Trp Ile Gln Tyr Asp Tyr Leu Gln Ser Leu Gly Lys Tyr Tyr Gly
   270 275 280
   Tyr Gly Ala Gly Asn Pro Leu Gly Pro Ala Gln Gly Val Gly Phe Ala
   285 290 295
   Asn Glu Leu Ile Ala Arg Leu Thr His Ser Pro Val Gln Asp His Thr
   300 305 310
   Ser Thr Asn His Thr Leu Asp Ser Asn Pro Ala Thr Phe Pro Leu Asn
   315 320 325
   Ala Thr Leu Tyr Ala Asp Phe Ser His Asp Asn Thr Met Ile Ser Ile
   330 335 340 345
   Phe Phe Ala Leu Gly Leu Tyr Asn Gly Thr Lys Pro Leu Ser Thr Thr
   350 355 360
   Ser Val Glu Ser Ile Glu Glu Thr Asp Gly Tyr Ser Ala Ser Trp Thr
   365 370 375
   Val Pro Phe Ala Ala Arg Ala Tyr Val Glu Met Met Gln Cys Gln Ala
   380 385 390
   Glu Lys Glu Pro Leu Val Arg Val Leu Val Asn Asp Arg Val Val Pro
   395 400 405
   Leu His Gly Cys Ala Val Asp Lys Leu Gly Arg Cys Lys Arg Asp Asp
   410 415 420 425
   Phe Val Glu Gly Leu Ser Phe Ala Arg Ser Gly Gly Asn Trp Ala Glu
   430 435 440
   Cys Phe Ala
<210> 94
   <211> 1404
   <212> DNA
   <213> Artificial Sequence
<220>
   <223> Description of Artificial Sequence: Consensus phytase-10-thermo-5-Q50T
<220>
   <221> sig_peptide
   <222> (1)..(69)
<220>
   <221> CDS
   <222> (1)..(1401)
<220>
   <221> mat_peptide
   <222> (70)..(1401)
<400> 94
   atg ggc gtg ttc gtc gtg cta ctg tcc att gcc acc ttg ttc ggt tcc 48
   Met Gly Val Phe Val Val Leu Leu Ser Ile Ala Thr Leu Phe Gly Ser
   -20 -15 -10
   aca tcc ggt acc gcc ttg ggt cct cgt ggt aat tct cac tct tgt gac 96
   Thr Ser Gly Thr Ala Leu Gly Pro Arg Gly Asn Ser His Ser Cys Asp
   -5 -1 1 5
   act gtt gac ggt ggt tac caa tgt ttc cca gaa att tct cac ttg tgg 144
   Thr Val Asp Gly Gly Tyr Gln Cys Phe Pro Glu Ile Ser His Leu Trp
   10 15 20 25
   ggt aca tac tct cca ttc ttc tct ttg gct gac gaa tct gct att tct 192
   Gly Thr Tyr Ser Pro Phe Phe Ser Leu Ala Asp Glu Ser Ala Ile Ser
   30 35 40
   cca gac gtt cca aag ggt tgt aga gtt act ttc gtt caa gtt ttg tct 240
   Pro Asp Val Pro Lys Gly Cys Arg Val Thr Phe Val Gln Val Leu Ser
   45 50 55
   aga cac ggt gct aga tac cca act tct tct aag tct aag gct tac tct 288
   Arg His Gly Ala Arg Tyr Pro Thr Ser Ser Lys Ser Lys Ala Tyr Ser
   60 65 70
   gct ttg att gaa gct att caa aag aac gct act gct ttc aag ggt aag 336
   Ala Leu Ile Glu Ala Ile Gln Lys Asn Ala Thr Ala Phe Lys Gly Lys
   75 80 85
   tac gct ttc ttg aag act tac aat tac act ttg ggt gct gac gac ttg 384
   Tyr Ala Phe Leu Lys Thr Tyr Asn Tyr Thr Leu Gly Ala Asp Asp Leu
   90 95 100 105
   act cca ttc ggt gaa caa caa atg gtt aac tct ggt att aag ttc tac 432
   Thr Pro Phe Gly Glu Gln Gln Met Val Asn Ser Gly Ile Lys Phe Tyr
   110 115 120
   aga aga tac aag gct ttg gct aga aag att gtt cca ttc att aga gct 480
   Arg Arg Tyr Lys Ala Leu Ala Arg Lys Ile Val Pro Phe Ile Arg Ala
   125 130 135
   tct ggt tct gac aga gtt att gct tct gcc gaa aag ttc att gaa ggt 528
   Ser Gly Ser Asp Arg Val Ile Ala Ser Ala Glu Lys Phe Ile Glu Gly
   140 145 150
   ttc caa tct gct aag ttg gct gac cca ggt gct aac cca cac caa gct 576
   Phe Gln Ser Ala Lys Leu Ala Asp Pro Gly Ala Asn Pro His Gln Ala
   155 160 165
   tct cca gtt att aac gtt att att cca gaa ggt gct ggt tac aac aac 624
   Ser Pro Val Ile Asn Val Ile Ile Pro Glu Gly Ala Gly Tyr Asn Asn
   170 175 180 185
   act ttg gac cac ggt ttg tgt act gct ttc gaa gaa tct acc cta ggt 672
   Thr Leu Asp His Gly Leu Cys Thr Ala Phe Glu Glu Ser Thr Leu Gly
   190 195 200
   gac gac gtt gaa gct aac ttc act gct gtt ttc gct cca cca att aga 720
   Asp Asp Val Glu Ala Asn Phe Thr Ala Val Phe Ala Pro Pro Ile Arg
   205 210 215
   gct aga ttg gaa gct cac ttg cca ggt gtt aac ttg act gac gaa gac 768
   Ala Arg Leu Glu Ala His Leu Pro Gly Val Asn Leu Thr Asp Glu Asp
   220 225 230
   gtt gtt aac ttg atg gac atg tgt cca ttc gac act gtt gct aga act 816
   Val Val Asn Leu Met Asp Met Cys Pro Phe Asp Thr Val Ala Arg Thr
   235 240 245
   tct gac gct act caa ttg tct cca ttc tgt gac ttg ttc act cac gac    864
   Ser Asp Ala Thr Gln Leu Ser Pro Phe Cys Asp Leu Phe Thr His Asp
   250                 255                 260                 265
   gaa tgg att caa tac gac tac ttg caa tct ttg ggt aag tac tac ggt    912
   Glu Trp Ile Gln Tyr Asp Tyr Leu Gln Ser Leu Gly Lys Tyr Tyr Gly
                   270                 275                 280
   tac ggt gct ggt aac cca ttg ggt cca gct caa ggt gtt ggt ttc gtt    960
   Tyr Gly Ala Gly Asn Pro Leu Gly Pro Ala Gln Gly Val Gly Phe Val
               285                 290                 295
   aac gaa ttg att gct aga ttg act cac tct cca gtt caa gac cac act   1008
   Asn Glu Leu Ile Ala Arg Leu Thr His Ser Pro Val Gln Asp His Thr
           300                 305                 310
   tct act aac cac act ttg gac tct aac cca gct act ttc cca ttg aac   1056
   Ser Thr Asn His Thr Leu Asp Ser Asn Pro Ala Thr Phe Pro Leu Asn
       315                 320                 325
   gct act ttg tac gct gac ttc tct cac gac aac act atg gtt tct att   1104
   Ala Thr Leu Tyr Ala Asp Phe Ser His Asp Asn Thr Met Val Ser Ile
   330                 335                 340                 345
   ttc ttc gct ttg ggt ttg tac aac ggt act aag cca ttg tct act act   1152
   Phe Phe Ala Leu Gly Leu Tyr Asn Gly Thr Lys Pro Leu Ser Thr Thr
                   350                 355                 360
   tct gtt gaa tct att gaa gaa act gac ggt tac tct gct tct tgg act   1200
   Ser Val Glu Ser Ile Glu Glu Thr Asp Gly Tyr Ser Ala Ser Trp Thr
               365                 370                 375
   gtt cca ttc gct gct aga gct tac gtt gaa atg atg caa tgt gaa gct   1248
   Val Pro Phe Ala Ala Arg Ala Tyr Val Glu Met Met Gln Cys Glu Ala
           380                 385                 390
   gaa aag gaa cca ttg gtt aga gtt ttg gtt aac gac aga gtt gtt cca   1296
   Glu Lys Glu Pro Leu Val Arg Val Leu Val Asn Asp Arg Val Val Pro
       395                 400                 405
   ttg cac ggt tgt gct gtt gac aag ttg ggt aga tgt aag aga gac gac   1344
   Leu His Gly Cys Ala Val Asp Lys Leu Gly Arg Cys Lys Arg Asp Asp
   410                 415                 420                 425
   ttc gtt gaa ggt ttg tct ttc gct aga tct ggt ggt aac tgg gaa gaa   1392
   Phe Val Glu Gly Leu Ser Phe Ala Arg Ser Gly Gly Asn Trp Glu Glu
                   430                 435                 440
   tgt ttc gct taa                                                   1404
   Cys Phe Ala
<210> 95
   <211> 467
   <212> PRT
   <213> Artificial Sequence
   <223> Description of Artificial Sequence: Consensus phytase-10-thermo-5-Q50T
<400> 95
   Met Gly Val Phe Val Val Leu Leu Ser Ile Ala Thr Leu Phe Gly Ser
               -20                 -15                 -10
   Thr Ser Gly Thr Ala Leu Gly Pro Arg Gly Asn Ser His Ser Cys Asp
           -5              -1   1               5
   Thr Val Asp Gly Gly Tyr Gln Cys Phe Pro Glu Ile Ser His Leu Trp
    10                  15                  20                  25
   Gly Thr Tyr Ser Pro Phe Phe Ser Leu Ala Asp Glu Ser Ala Ile Ser
                    30                  35                  40
   Pro Asp Val Pro Lys Gly Cys Arg Val Thr Phe Val Gln Val Leu Ser
                45                  50                  55
   Arg His Gly Ala Arg Tyr Pro Thr Ser Ser Lys Ser Lys Ala Tyr Ser
            60                  65                  70
   Ala Leu Ile Glu Ala Ile Gln Lys Asn Ala Thr Ala Phe Lys Gly Lys
        75                  80                  85
   Tyr Ala Phe Leu Lys Thr Tyr Asn Tyr Thr Leu Gly Ala Asp Asp Leu
    90                  95                 100                 105
   Thr Pro Phe Gly Glu Gln Gln Met Val Asn Ser Gly Ile Lys Phe Tyr
                   110                 115                 120
   Arg Arg Tyr Lys Ala Leu Ala Arg Lys Ile Val Pro Phe Ile Arg Ala
               125                 130                 135
   Ser Gly Ser Asp Arg Val Ile Ala Ser Ala Glu Lys Phe Ile Glu Gly
           140                 145                 150
   Phe Gln Ser Ala Lys Leu Ala Asp Pro Gly Ala Asn Pro His Gln Ala
       155                 160                 165
   Ser Pro Val Ile Asn Val Ile Ile Pro Glu Gly Ala Gly Tyr Asn Asn
   170                 175                 180                 185
   Thr Leu Asp His Gly Leu Cys Thr Ala Phe Glu Glu Ser Thr Leu Gly
                   190                 195                 200
   Asp Asp Val Glu Ala Asn Phe Thr Ala Val Phe Ala Pro Pro Ile Arg
               205                 210                 215
   Ala Arg Leu Glu Ala His Leu Pro Gly Val Asn Leu Thr Asp Glu Asp
           220                 225                 230
   Val Val Asn Leu Met Asp Met Cys Pro Phe Asp Thr Val Ala Arg Thr
   235 240 245
   Ser Asp Ala Thr Gln Leu Ser Pro Phe Cys Asp Leu Phe Thr His Asp
   250 255 260 265
   Glu Trp Ile Gln Tyr Asp Tyr Leu Gln Ser Leu Gly Lys Tyr Tyr Gly
   270 275 280
   Tyr Gly Ala Gly Asn Pro Leu Gly Pro Ala Gln Gly Val Gly Phe Val
   285 290 295
   Asn Glu Leu Ile Ala Arg Leu Thr His Ser Pro Val Gln Asp His Thr
   300 305 310
   Ser Thr Asn His Thr Leu Asp Ser Asn Pro Ala Thr Phe Pro Leu Asn
   315 320 325
   Ala Thr Leu Tyr Ala Asp Phe Ser His Asp Asn Thr Met Val Ser Ile
   330 335 340 345
   Phe Phe Ala Leu Gly Leu Tyr Asn Gly Thr Lys Pro Leu Ser Thr Thr
   350 355 360
   Ser Val Glu Ser Ile Glu Glu Thr Asp Gly Tyr Ser Ala Ser Trp Thr
   365 370 375
   Val Pro Phe Ala Ala Arg Ala Tyr Val Glu Met Met Gln Cys Glu Ala
   380 385 390
   Glu Lys Glu Pro Leu Val Arg Val Leu Val Asn Asp Arg Val Val Pro
   395 400 405
   Leu His Gly Cys Ala Val Asp Lys Leu Gly Arg Cys Lys Arg Asp Asp
   410 415 420 425
   Phe Val Glu Gly Leu Ser Phe Ala Arg Ser Gly Gly Asn Trp Glu Glu
   430 435 440
   Cys Phe Ala
<210> 96
   <211> 1404
   <212> DNA
   <213> Artificial Sequence
<220>
   <223> Description of Artificial Sequence: Consensus phytase-10-thermo-5-Q50T-K91A
<220>
   <221> sig_peptide
   <222> (1)..(69)
<220>
   <221> CDS
   <222> (1)..(1401)
<220>
   <221> mat_peptide
   <222> (70)..(1401)
<400> 96
   atg ggc gtg ttc gtc gtg cta ctg tcc att gcc acc ttg ttc ggt tcc      48
   Met Gly Val Phe Val Val Leu Leu Ser Ile Ala Thr Leu Phe Gly Ser
               -20                 -15                 -10
   aca tcc ggt acc gcc ttg ggt cct cgt ggt aat tct cac tct tgt gac      96
   Thr Ser Gly Thr Ala Leu Gly Pro Arg Gly Asn Ser His Ser Cys Asp
           -5                  -1   1               5
   act gtt gac ggt ggt tac caa tgt ttc cca gaa att tct cac ttg tgg     144
   Thr Val Asp Gly Gly Tyr Gln Cys Phe Pro Glu Ile Ser His Leu Trp
    10                  15                  20                  25
   ggt aca tac tct cca ttc ttc tct ttg gct gac gaa tct gct att tct     192
   Gly Thr Tyr Ser Pro Phe Phe Ser Leu Ala Asp Glu Ser Ala Ile Ser
                    30                  35                  40
   cca gac gtt cca aag ggt tgt aga gtt act ttc gtt caa gtt ttg tct     240
   Pro Asp Val Pro Lys Gly Cys Arg Val Thr Phe Val Gln Val Leu Ser
                45                  50                  55
   aga cac ggt gct aga tac cca act tct tct gcg tct aag gct tac tct     288
   Arg His Gly Ala Arg Tyr Pro Thr Ser Ser Ala Ser Lys Ala Tyr Ser
           60                  65                  70
   gct ttg att gaa gct att caa aag aac gct act gct ttc aag ggt aag     336
   Ala Leu Ile Glu Ala Ile Gln Lys Asn Ala Thr Ala Phe Lys Gly Lys
       75                  80                  85
   tac gct ttc ttg aag act tac aat tac act ttg ggt gct gac gac ttg     384
   Tyr Ala Phe Leu Lys Thr Tyr Asn Tyr Thr Leu Gly Ala Asp Asp Leu
    90                  95                 100                 105
   act cca ttc ggt gaa caa caa atg gtt aac tct ggt att aag ttc tac     432
   Thr Pro Phe Gly Glu Gln Gln Met Val Asn Ser Gly Ile Lys Phe Tyr
                   110                 115                 120
   aga aga tac aag gct ttg gct aga aag att gtt cca ttc att aga gct     480
   Arg Arg Tyr Lys Ala Leu Ala Arg Lys Ile Val Pro Phe Ile Arg Ala
               125                 130                 135
   tct ggt tct gac aga gtt att gct tct gcc gaa aag ttc att gaa ggt    528
   Ser Gly Ser Asp Arg Val Ile Ala Ser Ala Glu Lys Phe Ile Glu Gly
           140                 145                 150
   ttc caa tct gct aag ttg gct gac cca ggt gct aac cca cac caa gct    576
   Phe Gln Ser Ala Lys Leu Ala Asp Pro Gly Ala Asn Pro His Gln Ala
       155                 160                 165
   tct cca gtt att aac gtt att att cca gaa ggt gct ggt tac aac aac    624
   Ser Pro Val Ile Asn Val Ile Ile Pro Glu Gly Ala Gly Tyr Asn Asn
   170                 175                 180                 185
   act ttg gac cac ggt ttg tgt act gct ttc gaa gaa tct acc cta ggt    672
   Thr Leu Asp His Gly Leu Cys Thr Ala Phe Glu Glu Ser Thr Leu Gly
                   190                 195                 200
   gac gac gtt gaa gct aac ttc act gct gtt ttc gct cca cca att aga    720
   Asp Asp Val Glu Ala Asn Phe Thr Ala Val Phe Ala Pro Pro Ile Arg
               205                 210                 215
   gct aga ttg gaa gct cac ttg cca ggt gtt aac ttg act gac gaa gac    768
   Ala Arg Leu Glu Ala His Leu Pro Gly Val Asn Leu Thr Asp Glu Asp
           220                 225                 230
   gtt gtt aac ttg atg gac atg tgt cca ttc gac act gtt gct aga act    816
   Val Val Asn Leu Met Asp Met Cys Pro Phe Asp Thr Val Ala Arg Thr
       235                 240                 245
   tct gac gct act caa ttg tct cca ttc tgt gac ttg ttc act cac gac    864
   Ser Asp Ala Thr Gln Leu Ser Pro Phe Cys Asp Leu Phe Thr His Asp
   250                 255                 260                 265
   gaa tgg att caa tac gac tac ttg caa tct ttg ggt aag tac tac ggt    912
   Glu Trp Ile Gln Tyr Asp Tyr Leu Gln Ser Leu Gly Lys Tyr Tyr Gly
                   270                 275                 280
   tac ggt gct ggt aac cca ttg ggt cca gct caa ggt gtt ggt ttc gtt    960
   Tyr Gly Ala Gly Asn Pro Leu Gly Pro Ala Gln Gly Val Gly Phe Val
               285                 290                 295
   aac gaa ttg att gct aga ttg act cac tct cca gtt caa gac cac act    1008
   Asn Glu Leu Ile Ala Arg Leu Thr His Ser Pro Val Gln Asp His Thr
           300                 305                 310
   tct act aac cac act ttg gac tct aac cca gct act ttc cca ttg aac    1056
   Ser Thr Asn His Thr Leu Asp Ser Asn Pro Ala Thr Phe Pro Leu Asn
       315                 320                 325
   gct act ttg tac gct gac ttc tct cac gac aac act atg gtt tct att    1104
   Ala Thr Leu Tyr Ala Asp Phe Ser His Asp Asn Thr Met Val Ser Ile
   330                 335                 340                 345
   ttc ttc gct ttg ggt ttg tac aac ggt act aag cca ttg tct act act    1152
   Phe Phe Ala Leu Gly Leu Tyr Asn Gly Thr Lys Pro Leu Ser Thr Thr
                   350                 355                 360
   tct gtt gaa tct att gaa gaa act gac ggt tac tct gct tct tgg act    1200
   Ser Val Glu Ser Ile Glu Glu Thr Asp Gly Tyr Ser Ala Ser Trp Thr
               365                 370                 375
   gtt cca ttc gct gct aga gct tac gtt gaa atg atg caa tgt gaa gct    1248
   Val Pro Phe Ala Ala Arg Ala Tyr Val Glu Met Met Gln Cys Glu Ala
           380                 385                 390
   gaa aag gaa cca ttg gtt aga gtt ttg gtt aac gac aga gtt gtt cca    1296
   Glu Lys Glu Pro Leu Val Arg Val Leu Val Asn Asp Arg Val Val Pro
       395                 400                 405
   ttg cac ggt tgt gct gtt gac aag ttg ggt aga tgt aag aga gac gac    1344
   Leu His Gly Cys Ala Val Asp Lys Leu Gly Arg Cys Lys Arg Asp Asp
   410                 415                 420                 425
   ttc gtt gaa ggt ttg tct ttc gct aga tct ggt ggt aac tgg gaa gaa    1392
   Phe Val Glu Gly Leu Ser Phe Ala Arg Ser Gly Gly Asn Trp Glu Glu
                   430                 435                 440
   tgt ttc gct taa                                                    1404
   Cys Phe Ala
<210> 97
   <211> 467
   <212> PRT
   <213> Artificial Sequence
   <223> Description of Artificial Sequence: Consensus phytase-10-thermo-5-Q50T-K91A
<400> 97
   Met Gly Val Phe Val Val Leu Leu Ser Ile Ala Thr Leu Phe Gly Ser
               -20                 -15                 -10
   Thr Ser Gly Thr Ala Leu Gly Pro Arg Gly Asn Ser His Ser Cys Asp
           -5                  -1   1               5
   Thr Val Asp Gly Gly Tyr Gln Cys Phe Pro Glu Ile Ser His Leu Trp
    10                  15                  20                  25
   Gly Thr Tyr Ser Pro Phe Phe Ser Leu Ala Asp Glu Ser Ala Ile Ser
                   30                  35                  40
   Pro Asp Val Pro Lys Gly Cys Arg Val Thr Phe Val Gln Val Leu Ser
               45                  50                  55
   Arg His Gly Ala Arg Tyr Pro Thr Ser Ser Ala Ser Lys Ala Tyr Ser
           60                  65                  70
   Ala Leu Ile Glu Ala Ile Gln Lys Asn Ala Thr Ala Phe Lys Gly Lys
   75 80 85
   Tyr Ala Phe Leu Lys Thr Tyr Asn Tyr Thr Leu Gly Ala Asp Asp Leu
   90 95 100 105
   Thr Pro Phe Gly Glu Gln Gln Met Val Asn Ser Gly Ile Lys Phe Tyr
   110 115 120
   Arg Arg Tyr Lys Ala Leu Ala Arg Lys Ile Val Pro Phe Ile Arg Ala
   125 130 135
   Ser Gly Ser Asp Arg Val Ile Ala Ser Ala Glu Lys Phe Ile Glu Gly
   140 145 150
   Phe Gln Ser Ala Lys Leu Ala Asp Pro Gly Ala Asn Pro His Gln Ala
   155 160 165
   Ser Pro Val Ile Asn Val Ile Ile Pro Glu Gly Ala Gly Tyr Asn Asn
   170 175 180 185
   Thr Leu Asp His Gly Leu Cys Thr Ala Phe Glu Glu Ser Thr Leu Gly
   190 195 200
   Asp Asp Val Glu Ala Asn Phe Thr Ala Val Phe Ala Pro Pro Ile Arg
   205 210 215
   Ala Arg Leu Glu Ala His Leu Pro Gly Val Asn Leu Thr Asp Glu Asp
   220 225 230
   Val Val Asn Leu Met Asp Met Cys Pro Phe Asp Thr Val Ala Arg Thr
   235 240 245
   Ser Asp Ala Thr Gln Leu Ser Pro Phe Cys Asp Leu Phe Thr His Asp
   250 255 260 265
   Glu Trp Ile Gln Tyr Asp Tyr Leu Gln Ser Leu Gly Lys Tyr Tyr Gly
   270 275 280
   Tyr Gly Ala Gly Asn Pro Leu Gly Pro Ala Gln Gly Val Gly Phe Val
   285 290 295
   Asn Glu Leu Ile Ala Arg Leu Thr His Ser Pro Val Gln Asp His Thr
   300 305 310
   Ser Thr Asn His Thr Leu Asp Ser Asn Pro Ala Thr Phe Pro Leu Asn
   315 320 325
   Ala Thr Leu Tyr Ala Asp Phe Ser His Asp Asn Thr Met Val Ser Ile
   330 335 340 345
   Phe Phe Ala Leu Gly Leu Tyr Asn Gly Thr Lys Pro Leu Ser Thr Thr
   350 355 360
   Ser Val Glu Ser Ile Glu Glu Thr Asp Gly Tyr Ser Ala Ser Trp Thr
               365                 370                 375
   Val Pro Phe Ala Ala Arg Ala Tyr Val Glu Met Met Gln Cys Glu Ala
           380                 385                 390
   Glu Lys Glu Pro Leu Val Arg Val Leu Val Asn Asp Arg Val Val Pro
       395                 400                 405
   Leu His Gly Cys Ala Val Asp Lys Leu Gly Arg Cys Lys Arg Asp Asp
   410                 415                 420                     425
   Phe Val Glu Gly Leu Ser Phe Ala Arg Ser Gly Gly Asn Trp Glu Glu
                   430                 435                 440
   Cys Phe Ala

## Description

Phytases are enzymes that hydrolyze phytate (myo-inositol hexakisphosphate) to myo-inositol and inorganic phosphate. They are known to be valuable feed additives.

The present invention relates to improved phytases, viz. phytases of amended characteristics, e.g. amended activity characteristics, reference being made to e.g. the phytase(s) it has been derived from, or to known phytases. Amended activity characteristics means amended in at least one phytase activity related respect, such as (non-exclusive list): pH stability, temperature stability, pH profile, temperature profile, specific activity (in particular in relation to pH and temperature), substrate specificity, substrate cleavage pattern, substrate binding, position specificity, the velocity and level of release of phosphate from corn, reaction rate, phytate degradation rate), end level of released phosphate reached.

Examples of amended activity characteristics are amended specific activity (e.g. increased, e.g. increased at a pH of 3, 4, 5, or 6); amended pH or temperature profile; and/or amended (e.g. increased) thermostability, e.g. of an increased melting temperature as measured using Differential Scanning Calorimetry (DSC).

The present invention also discloses a process for the preparation of a modified protein, wherein in a first step a consensus sequence is determined from a number of highly homologous sequences according to steps a), b) and c) below:
a) at least three, preferably at least four amino acid sequences are aligned by any standard alignment program known in the art;
b) at every position of the amino acid sequence alignment, the amino acids are evaluated for their evolutionary similarity and a consensus residue is chosen by any standard program known in the art, whereby the minimal requirements for calculation of a consensus residue are set in such a way that the program is already able to determine a consensus residue if a given residue occurs in only two of the aligned sequences. However, if there is a subgroup of sequences among the compared amino acid sequences that shows a much higher degree of similarity with each other than with the remaining sequences of the alignment, the subgroup may be represented in the calculation only with its consensus sequence determined in the same way as outlined in EP 897985, or alternatively, to each sequence of the subgroup, a vote weight of 1 divided by the number of sequences in the subgroup will be assigned;
c) in case no consensus amino acid at a defined position is identified by the program, any of the amino acids, preferably the most frequently occurring amino acid at this position is selected.

In a second aspect, a homologous sequence is compared with the consensus sequence, and one or more non-consensus residues in this homologous sequence are replaced by the corresponding consensus residues.

Preferably, only such amino acid residues are replaced in the homologous amino acid sequence where a consensus residue can clearly be defined by the program under moderately stringent conditions whereas at all positions of the alignment where no preferred consensus amino acid can be determined under moderately stringent conditions, the amino acids of the homologous protein remain unchanged.

In a third aspect, the active center of the protein of interest is determined, comprising all amino acid residues that are involved in forming the active center, both in the consensus sequence, and in the sequence of a homologous protein; subsequently, some or all of the divergent amino acid residues of the homologous protein are inserted in the backbone of the consensus sequence.

In one embodiment of this process, the program used for the comparison of amino acids at a defined position regarding their evolutionary similarity is the program "PRETTY".

The active center of the protein can be determined by using an analysis of the three-dimensional structure of the protein.

An example of a homologous protein is an enzyme family, an example of a defined protein family is the family of phytases, e.g. of fungal origin.

For example, the amino acid sequence of the phytase can be changed by the introduction of at least one mutation or substitution chosen from

| | |
|---|---|
| E58A | F54Y |
| D69K | I73V |
| D197N | K94A |
| T214L | R101A |
| E222T | N153K |
| E267D | V158I |
| R291I | A203G |
| R329H | S205G |
| S364T | V217A |
| A379K | A227V |
| G404A | V234L |
| | P238A |
| Q277E | |
| A287H | |
| A292Q | |
| V366I | |
| A396S | |
| E415Q | |
| G437A | |
| R451E | |

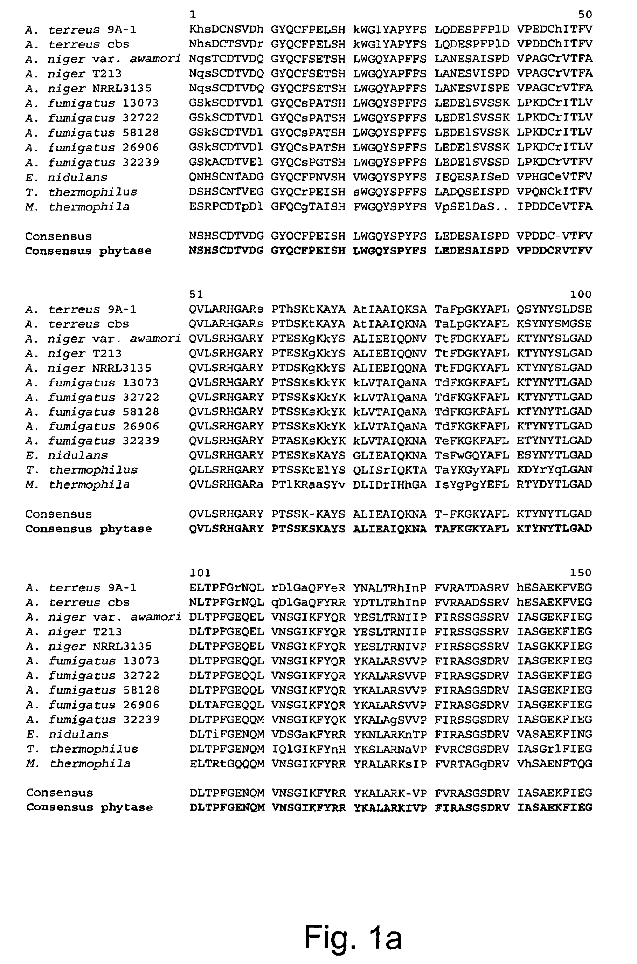

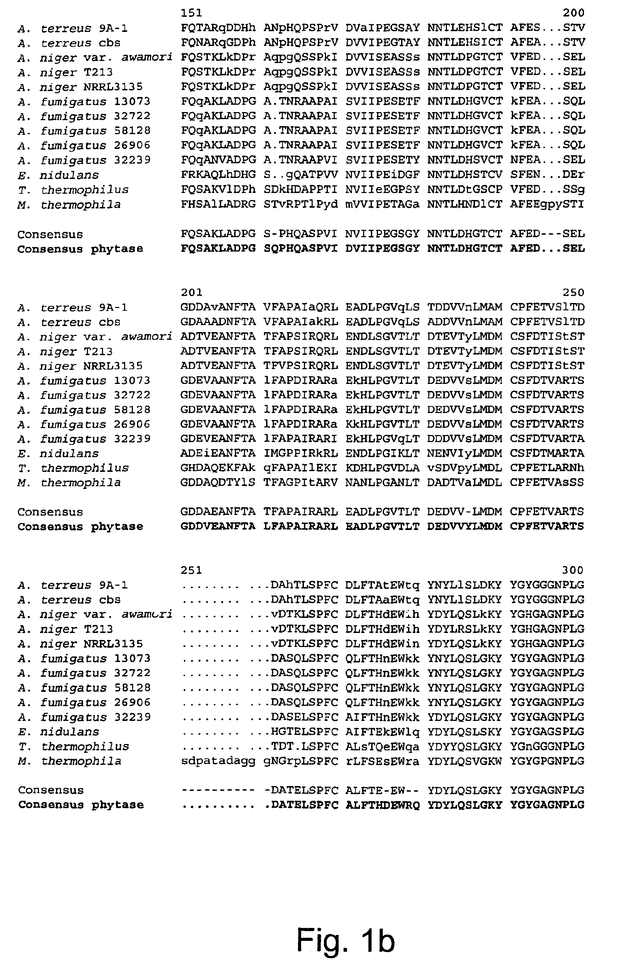

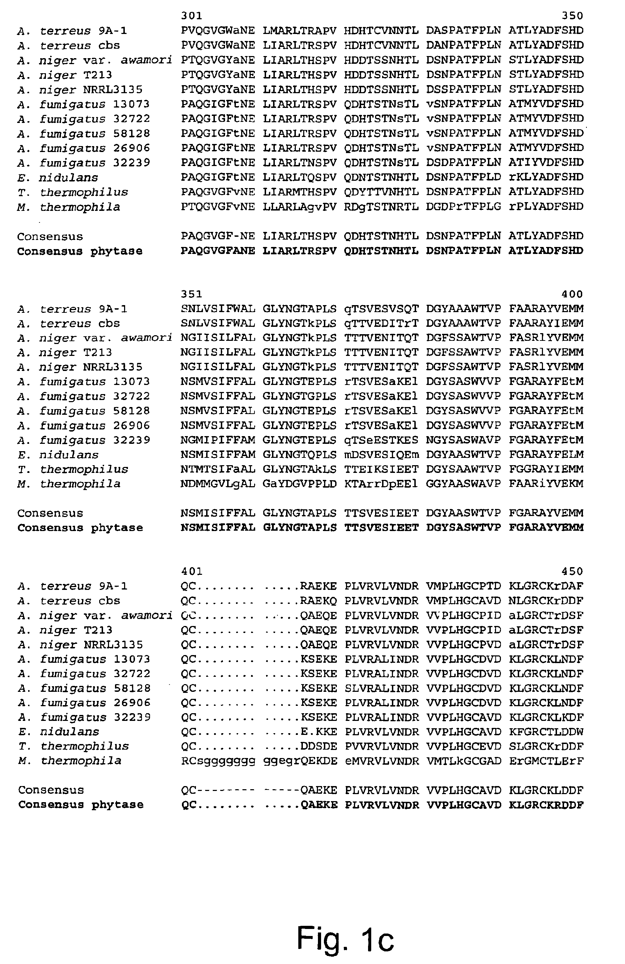

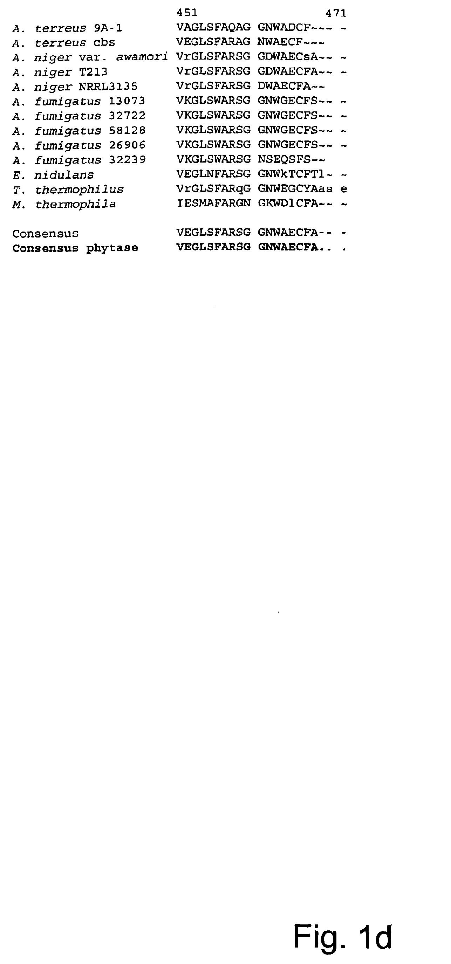

For interpreting these abbreviations, as an example, the mutation E58A is to be interpreted as follows: When subtracting 26 from the number, you get the position or residue number in the consensus phytase sequence or another phytase sequence aligned as shown in Fig. 1 (corresponding to the addition of a 26 amino acid signal sequence to the sequences shown in Fig. 1). For example, in E58A, number 58 means position number 32 (58-26=32). And the letter before the number, i.e. E, represents the amino acid in the phytase to be modified which is replaced by the amino acid behind the number, i.e. A.

The above-mentioned amino acid replacements, alone and/or in combination, have a positive effect on the protein stability.

The following sub-groups of mutations are also interesting (i.e. phytases comprising at least one mutation selected from either one of the groups of):
E58A, D69K, D197N, T214L, E222T, E267D, R291I, R329H, S364T, A379K, G404A;
F54Y, I73V, K94A, R101A, N153K, V158I, A203G, S205G, V217A, A227V, V234L, P238A, Q277E, A287H, A292Q, V366I, A396S, E415Q, G437A, R451E;
E58A, D69K, D197N, F54Y, I73V, K94A;
T214L, E222T, E267D, R101A, N153K, V158I;
R291I, R329H, S364T, A203G, S205G, V217A;
A379K, G404A, A227V, V234L, P238A, Q277E;
A287H, A292Q, V366I, A396S, E415Q, G437A, R451E;
T214L, E222T, S364T, V158I, A203G, G404A, A227V, P238A, A396S, G437A, R451E.

Examples of host cells are plant cells, animal cells, and microbial cells, e.g. prokaryotic or eukaryotic cells, such as bacterial, fungal or yeast cells. An example of a fungal host is a strain of the genus Aspergillus, and examples of yeast hosts are strains of Saccharomyces, and strains of Hansenula.

The invention also discloses a modified protein obtainable or obtained by any of the processes described above.

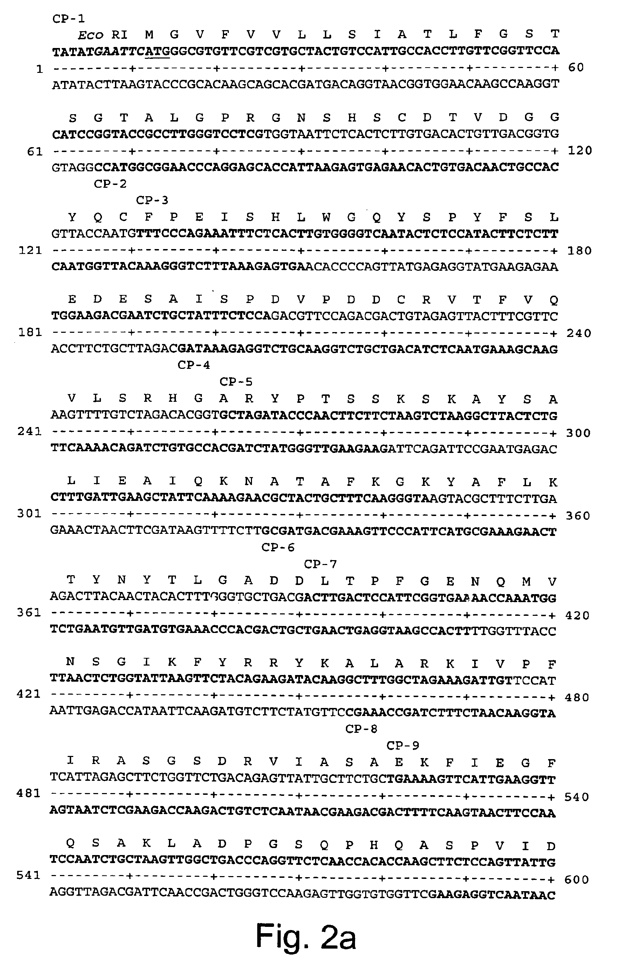

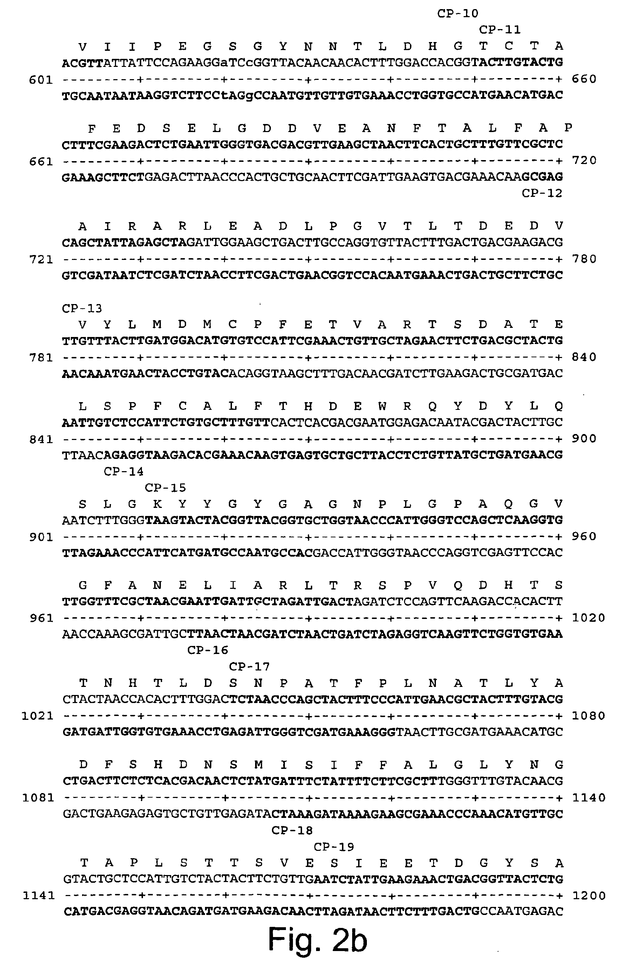

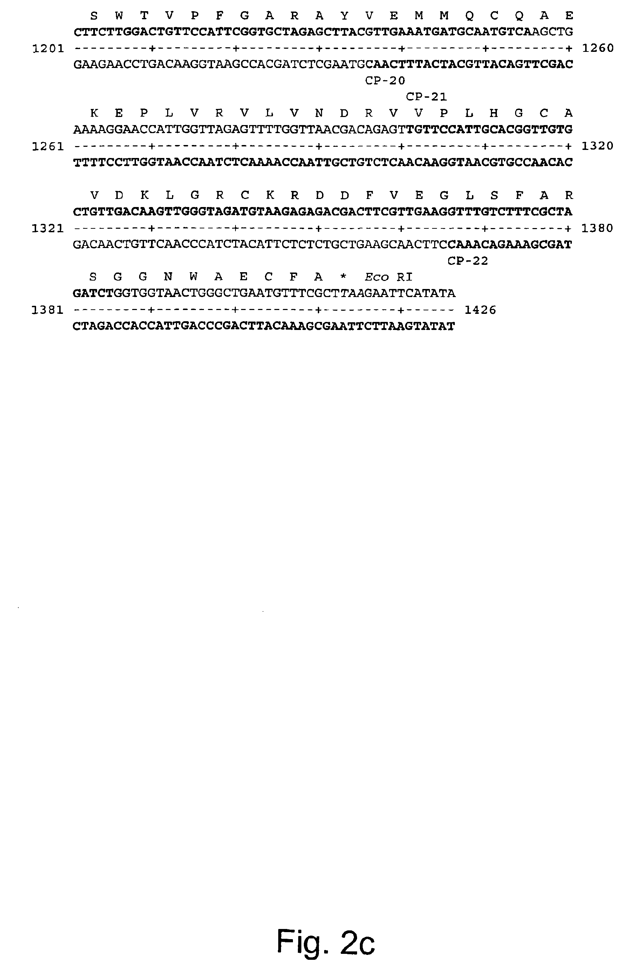

The invention also relates to a variant or mutein of a phytase such as (but not limited to) the consensus phytase-1, wherein, in the amino acid sequence in Figure 2, at least one of the following replacements have been effected: Q50L, Q50T, Q50G, Q50T-Y51N, Q50L-Y51N or Q50T-K91A.

In the third aspect mentioned above, a consensus sequence is determined from homologous sequences as described above; in a second step the active center of the protein comprising all amino acid residues that are involved in forming the active center is determined in the consensus sequence and in the sequence of a single homologous protein as well. The single homologous protein may have preferred properties like high specific activity or different pH dependency of enzymatic activity. In a third step some or all amino acid residues that are involved in forming the active center of the homologous protein are inserted into the backbone of the consensus sequence. The result thereof is a chimeric protein having the active center derived from a single protein and the backbone of the consensus sequence.

The active center of the protein can be determined e.g. by using any analysis of the three-dimensional structure of the protein, e.g. by homology modelling on the basis of a known 3D-structure of a known protein.

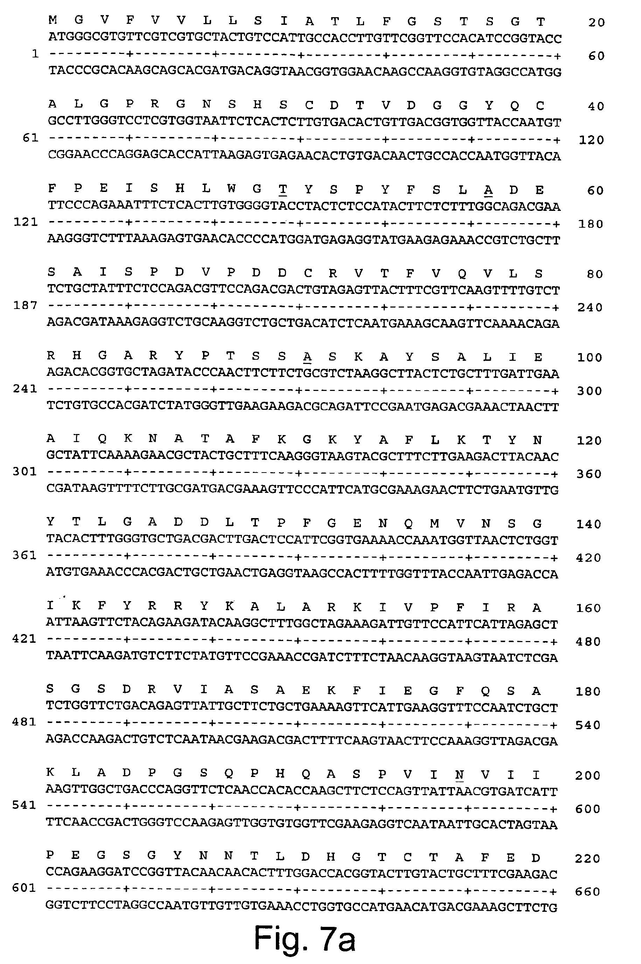

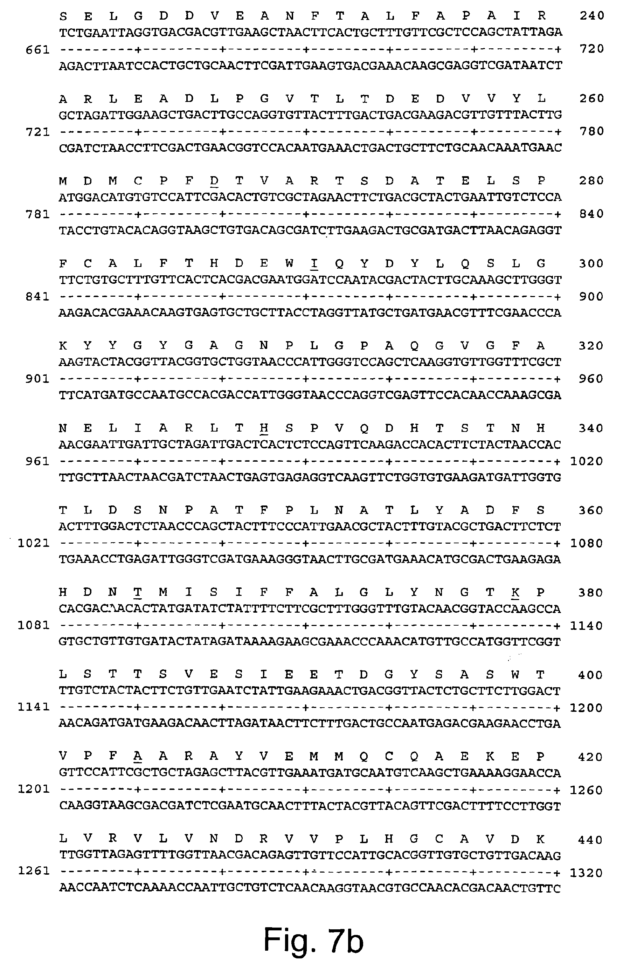

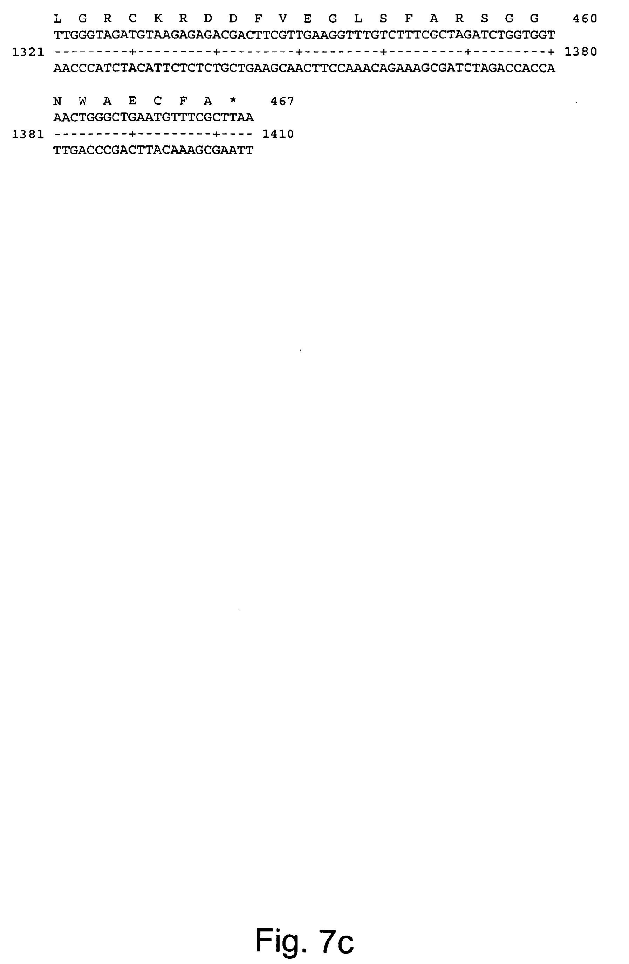

The present invention also provides consensus proteins obtainable or obtained by such processes, in particular proteins comprising at least one of the amino acid sequences shown in Figures 2-6, 10 or 21, or variants or muteins thereof. Examples of such variants are shown in Figs. 7-9.

Such variants or muteins can be defined and prepared on the basis of the teachings given in European Patent Application number 0897010, e.g. Q50L, Q50T, Q50G, Q50L-Y51N, or Q50T-Y51N. These mutations are defined as above, or, alternatively, by reference to Fig. 2. When referring to Fig. 2, no subtraction of the 26 amino acid signal peptide is required (e.g. in "Q50L," at position 50 of the amino acid sequence of Fig. 2, the amino acid Q has been replaced by amino acid L).

A food, feed, or pharmaceutical composition comprising the phytases of the invention is another aspect of the invention.

In this context, "at least three, preferably at least four amino acid sequences of such defined protein family" means that three, four, five, six to twelve, twenty, fifty, or even more sequences can be used for the alignment and the comparison to create the amino acid sequence of the consensus protein. "Sequences of a defined protein family" means that such sequences fold into a three-dimensional structure, wherein the alpha-helices, the beta-sheets and beta-turns are at the same position so that such structures are, as called by the man skilled in the art, largely superimposable. Furthermore these sequences characterize proteins that show the same type of biological activity, e.g. a defined enzyme class, e.g. the phytases. The three-dimensional structure of one such protein is sufficient to allow the modelling of the structure of the other homologous proteins of such a family. An example, how this can be done, is given in Example 1. "Evolutionary similarity" in the context of the present invention refers to a scheme which classifies amino acids regarding their structural similarity which allows that one amino acid can be replaced by another amino acid with a minimal influence on the overall structure, as this is done e.g. by programs, like "PRETTY", known in the art. The phrase "the degree of similarity provided by such a program...is set to less stringent number" means in the context of the present invention that values for the parameters which determine the degree of similarity in the program used in the practice of the present invention are chosen in a way to allow the program to define a consensus amino acid for a maximum of positions of the whole amino acid sequence, e. g. in case of the program PRETTY a value of 2 or 3 for the THRESHOLD and a value of 2 for the PLURALITY can be chosen. Furthermore, "a vote weight of one divided by the number of such sequences" means in the context of the present invention that the sequences which define a group of sequences with a higher degree of similarity as the other sequences used for the determination of the consensus sequence only contribute to such determination with a factor which is equal to one divided by the number of all sequences of this group.

As mentioned before, should the program not allow to select the consensus amino acid, the most frequent amino acid is selected; should the latter be impossible the man skilled in the art will select an amino acid from all the sequences used for the comparison which is known in the art for its property to improve the thermostability in proteins as discussed e.g. by Janecek, S. (1993), Process Biochem. 28, 435-445; Fersht, A. R. & Serrano, L. (1993), Curr. Opin. Struct. Biol. 3, 75-83; Alber, T. (1989), Annu. Rev. Biochem. 58, 765-798; Matthews, B. W. (1987), Biochemistry 26, 6885-6888; or Matthews, B. W. (1991), Curr. Opin. Struct. Biol. 1, 17-21.

The stability of an enzyme is relevant for many industrial applications. Therefore, a lot of attempts, more or less successful, have been made to improve the stability, preferably the thermostability of enzymes by rational or random approaches.

Here we present an alternative way to improve the thermostability of a protein.

The invention discloses a process for the preparation of a consensus protein comprising a process to calculate an amino acid residue for nearly all positions of a so-called consensus protein and to synthesize a complete gene from this sequence that can be expressed in a pro- or eukaryotic expression system.

DNA sequences of the present invention can be constructed starting from genomic or cDNA sequences encoding the proteins, e.g. phytases, of interest. For example, they can be constructed by methods of in vitro mutagenesis [see e.g. Sambrook et al., Molecular Cloning, Cold Spring Harbor Laboratory Press, New York]. A widely used strategy for "site-directed mutagenesis", as originally outlined by Hurchinson and Edgell [J. Virol. 8, 181 (1971)], involves the annealing of a synthetic oligonucleotide carrying the desired nucleotide substitution to a target region of a single-stranded DNA sequence wherein the mutation should be introduced [for review see Smith, Annu. Rev. Genet. 19, 423 (1985), and for improved methods, see references 2-6 in Stanssen et al., Nucl. Acids Res., 17, 4441-4454 (1989). Another possibility of mutating a given DNA sequence is the mutagenesis by using the polymerase chain reaction (PCR). DNA as starting material can be isolated by methods known in the art and described e.g. in Sambrook et al. (Molecular Cloning) from the respective strains.

For strain information, see e.g. EP 684313 or any depository authority indicated below. Aspergillus niger [ATCC 9142], Myceliophthora thermophila [ATCC 48102], Talaromyces thermophilus [ATCC 20186] and Aspergillus fumigatus [ATCC 34625] have been redeposited according to the conditions of the Budapest Treaty at the American Type Culture Cell Collection under the following accession numbers: ATCC 74337, ATCC 74340, ATCC 74338 and ATCC 74339, respectively. It is, however, understood that DNA encoding a consensus protein in accordance with the present invention can also be prepared in a synthetic manner as described, e.g. in EP 747483 or EP 897985, or in the examples, by methods known in the art.

For sequence information, see e.g. EP 684313, or sequence data bases, for example like Genbank (Intelligenetics, California, USA), European Bioinformatics Institute (Hinston Hall, Cambridge, GB), NBRF (Georgetown University, Medical Centre, Washington DC, USA) and Vecbase (University of Wisconsin, Biotechnology Centre, Madison, Wisconsin, USA).

The process can e.g. be used to improve the thermostability of the enzyme phytase.

Once complete DNA sequences of the present invention have been obtained they can be integrated into vectors by methods known in the art and described e.g. in Sambrook et al. (s.a.) to overexpress the encoded polypeptide in appropriate host systems. However, a man skilled in the art knows that also the DNA sequences themselves can be used to transform the suitable host systems of the invention to get overexpression of the encoded polypeptide. Appropriate host systems are for example fungi, like Aspergilli, e.g. Aspergillus niger [ATCC 9142] or Aspergillus ficuum [NRRL 3135] or like Trichoderma, e.g. Trichoderma reesei; or yeasts, like Saccharomyces, e.g. Saccharomyces cerevisiae or Pichia, like Pichia pastoris, or Hansenula polymorpha, e.g. H. polymorpha (DSM5215); or plants, as described, e.g. by Pen et al., Bio/Technology 11, 811-814 (1994). A man skilled in the art knows that such microorganisms are available from depository authorities, e.g. the American Type Culture Collection (ATCC), the Centraalbureau voor Schimmelcultures (CBS) or the Deutsche Sammlung für Mikroorganismen und Zellkulturen GmbH (DSM) or any other depository authority as listed in the Journal "Industrial Property" [(1991) 1, pages 29-40]. Bacteria which can be used are e.g. E. coli; Bacilli as, e.g., Bacillus subtilis; or Streptomyces, e.g. Streptomyces lividans (see e.g. Anné and Mallaert in FEMS Microbiol. Lett. 114, 121 (1993). Preferred E. coli strains, which can be used are E. coli K12 strains e.g. M15 [described as DZ 291 by Villarejo et al. in J. Bacteriol. 120, 466-474 (1974)], HB 101 [ATCC No. 33694] or E. coli SG13009 [Gottesman et al., J. Bacteriol. 148, 265-273 (1981)].

Vectors which can be used for expression in fungi are known in the art and described e.g. in EP 420358, or by Cullen et al. [Bio/Technology 5, 369-376 (1987)], Ward [Molecular Industrial Mycology, Systems and Applications for Filamentous Fungi, Marcel Dekker, New York (1991)], Upshall et al. [Bio/Technology 5, 1301-1304 (1987)], Gwynne et al. [Bio/Technology 5, 71-79 (1987)], or Punt et al. [J. Biotechnol. 17, 19-34 (1991)]; and for yeasts by Sreekrishna et al. [J. Basic Microbiol. 28, 265-278 (1988), Biochemistry 28, 4117-4125 (1989)], Hitzemann et al. [Nature 293, 717-722 (1981)] or in EP 183070, EP 183071, EP 248227, or EP 263311. Suitable vectors which can be used for expression in E. coli are mentioned, e.g. by Sambrook et al. [s.a.], Fiers et al. [Procd. 8th Int. Biotechnology Symposium", Soc. Franc. de Microbiol., Paris (Durand et al., eds.), pp. 680-697 (1988)], Bujard et al. [Meth. Enzymol. 155, 416-433 (1987)], or Stüber et al. [Immunological Methods, eds. Lefkovits and Pernis, Academic Press, Inc., Vol. IV, 121-152 (1990)]. Vectors that can be used for expression in Bacilli are known in the art and described, e.g. in EP 207459, EP 405370, Proc. Natl. Acad. Sci. USA 81, 439 (1984) or Yansura and Henner, Meth. Enzymol. 185, 199-228 (1990). Vectors which can be used for the expression in H. Polymorpha are known in the art and described, e.g. in Gellissen et al., Biotechnology 9, 291-295 (1991).

Either such vectors already carry regulatory elements, e.g. promotors, or the DNA sequences of the present invention can be engineered to contain such elements. Suitable promotor elements which can be used are known in the art and are, e.g. for Trichoderma reesei the cbh1- [Haarki et al., Biotechnology 7, 596-600 (1989)] or the pki1-promotor [Schindler et al., Gene 130, 271-275 (1993)]; for Aspergillus oryzae the amy-promotor [Christensen et al., Abstr. 19th Lunteren Lectures on Molecular Genetics F23 (1987), Christensen et al., Biotechnology 6, 1419-1422 (1988), Tada et al., Mol. Gen. Genet. 229, 301 (1991)]; and for Aspergillus niger the glaA- [Cullen et al., Bio/Technology 5, 369-376 (1987), Gwynne et al., Bio/Technology 5, 713-719 (1987), Ward in Molecular Industrial Mycology, Systems and Applications for Filamentous Fungi, Marcel Dekker, New York, 83-106 (1991)], alcA- [Gwynne et al., Bio/Technology 5, 718-719 (1987)], suc1- [Boddy et al., Curr. Genet. 24, 60-66 (1993)], aphA- [MacRae et al., Gene 71, 339-348 (1988), MacRae et al., Gene 132, 193-198 (1993)], tpiA- [McKnight et al., Cell 46, 143-147 (1986), Upshall et al., Bio/Technology 5, 1301-1304 (1987)], gpdA- [Punt et al., Gene 69, 49-57 (1988), Punt et al., J. Biotechnol. 17, 19-37 (1991)] and the pkiA-promotor [de Graaff et al., Curr. Genet. 22, 21-27 (1992)]. Suitable promotor elements that can be used for expression in yeast are known in the art and are, e.g. the pho5-promotor [Vogel et al., Mol. Cell. Biol., 2050-2057 (1989); Rudolf and Hinnen, Proc. Natl. Acad. Sci. 84, 1340-1344 (1987)] or the gap-promotor for expression in Saccharomyces cerevisiae; the aox1-promotor [Koutz et al., Yeast 5, 167-177 (1989); Sreekrishna et al., J. Basic Microbiol. 28, 265-278 (1988)] for Pichia pastoris; or the FMD promoter [Hollenberg et al., EPA No. 0299108] or MOX-promotor [Ledeboer et al., Nucl. Acids Res. 13, 3063-3082 (1985)] for H. polymorpha.

Accordingly vectors comprising DNA sequences of the present invention, preferably for the expression of said DNA sequences in bacteria or a fungal or a yeast host and such transformed bacteria or fungal or yeast hosts are also a part of the invention.

The invention also provides a system that allows for high expression of proteins, in particular of the phytases of the invention, such as recombinant Hansenula strains. To achieve that, the codons of the DNA sequence of such a protein may be selected on the basis of a codon frequency table of the organism used for expression, e.g. of yeast as in the present case (see e.g. in Example 1). Optionally, the codons for the signal sequence may be selected in a manner as described for the specific case in Example 1; that means that a codon frequency table is prepared on the basis of the codons used in the DNA sequences which encode the amino acid sequences of the given protein family. Then the codons for the design of the DNA sequence of the signal sequence are selected from a codon frequency table of the host cell used for expression whereby always codons of comparable frequency in both tables are used.

Once such DNA sequences have been expressed in an appropriate host cell in a suitable medium, the encoded protein can be isolated either from the medium in the case the protein is secreted into the medium or from the host organism in case such protein is present intracellularly by methods known in the art of protein purification or described in case of a phytase, e.g. in EP 420358. Accordingly, a process for the preparation of a polypeptide of the present invention wherein transformed bacteria or a host cell as described above are cultured under suitable culture conditions, and the polypeptide is recovered therefrom and a polypeptide when produced by such a process; or a polypeptide encoded by a DNA sequence of the present invention, are also a part of the present invention.

Once obtained, the polypeptides of the present invention can be characterized regarding their properties that make them useful in agriculture by any assay known in the art.

In general, the polypeptides of the present invention can be used without being limited to a specific field of application, e.g. in case of phytases for the conversion of inositol polyphosphates, like phytate, to inositol and inorganic phosphate.

Furthermore, the polypeptides of the present invention can be used in a process for the preparation of a pharmaceutical composition or compound food or feeds wherein the components of such a composition are mixed with at least one polypeptide of the present invention. Accordingly, compound food or feeds or pharmaceutical compositions comprising at least one polypeptide of the present invention are also a part of the present invention. A man skilled in the art is familiar with their process of preparation. Such pharmaceutical compositions or compound foods or feeds can further comprise additives or components generally used for such purpose and known in the state of the art.

The present invention also discloses a process for the reduction of levels of phytate in animal manure wherein an animal is fed such a feed composition in an amount effective in converting phytate contained in the feedstuff to lower inositol phosphates and/or inositol, and inorganic phosphate.

In the present context, a phytase is an enzyme or polypeptide that has phytase activity. A phytase can be e.g. a myo-inositol hexakisphosphate phosphohydrolase, such as (myo-inositol hexakisphosphate 3-phosphohydrolase, EC 3.1.3.8) and (myo-inositol hexakisphosphate 6-phosphohydrolase, EC 3.1.3.26).

In one embodiment, the phytase is purified, viz. at least 85%, preferably at least 86, 87, 88, 89, 90, 91, 92, 93, 94, 95, 96, 97, 98, 99% pure, as evaluated by SDS-PAGE. The phytase may be isolated. Phytase activity can be determined using any phytase assay known in the art, e.g. the assay described herein (see Example 9). The assay temperature may be the optimum temperature of the actual phytase, and the assay pH may be the optimum pH of the actual phytase.

The assay temperature may e.g. be selected within the range of 20-90°C, or 30-80°C, or 35-75°C, for instance temperatures of 37°C, 50°C, 60°C, or 70°C.

The assay pH may e.g. be selected within the range of pH 2-9, or 3-8, or 3-6, for instance assay pH values of 3, 4, 5, 6, or 7 may be chosen.

Amino acid sequence homology (or polypeptide or amino acid homology) is determined as the degree of identity between two sequences. This may suitably be determined by means of computer programs known in the art such as GAP provided in the GCG program package [Program Manual for the Wisconsin Package, Genetics Computer Group, 575 Science Drive, Madison, Wisconsin 53711, USA], see also Needleman, S.B. and Wunsch, C.D., (1970), J. Mol. Biol., 48, 443-453]. In release 9.1, for comparing polypeptide sequences, the Length Weight is set to 0, and the Gap Weight is set to 3.0.

The degree of identity or homology between two DNA (nucleic acid) sequences may be determined by means of computer programs known in the art such as GAP provided in the GCG program package [Program Manual for the Wisconsin Package, Genetics Computer Group, 575 Science Drive, Madison, Wisconsin 53711, USA), see also Needleman, S.B. and Wunsch, C.D., (1970), J. Mol. Biol., 48, 443-453]. In release 9.1, GAP is used with the following settings for DNA sequence comparison: GAP creation penalty of 50 and GAP extension penalty of 3.

Suitable experimental conditions for determining whether a given DNA or RNA sequence hybridizes to a specified nucleotide or oligonucleotide probe involves presoaking of the filter containing the DNA or RNA fragments to examine for hybridization in 5 x SSC (Sodium chloride/Sodium citrate; (J. Sambrook, E.F. Fritsch, and T. Maniatis, 1989, Molecular Cloning, A Laboratory Manual, 2nd edition, Cold Spring Harbor, New York) for 10 min, and prehybridization of the filter in a solution of 5 x SSC, 5 x Denhardt's solution, 0.5 % SDS and 100 µg/ml of denatured sonicated salmon sperm DNA (Sambrook et al. 1989), followed by hybridization in the same solution containing a concentration of 10 ng/ml of a random-primed (Feinberg, A. P. and Vogelstein, B. (1983) Anal. Biochem. 132:6-13), ³²P-dCTP-labeled (specific activity > 1 x 10⁹ cpm/µg) probe for 12 hours at approximately 45°C.

The filter is then washed twice for 30 minutes in 2 x SSC, 0.5 % SDS at at least 55°C (low stringency), at at least 60°C (medium stringency), at at least 65°C (medium/high stringency), at at least 70°C (high stringency), or at at least 75°C (very high stringency).

Molecules to which the oligonucleotide probe hybridizes under these conditions can be detected using an x-ray film.

Phytases of amended thermostability, or thermostable plytases, are one aspect of the present invention. A "thermostable" phytase is a phytase that has a Tm (melting temperature) - as measured on purified phytase protein by Differential Scanning Calorimetry (DSC) - of at least 65°C. For the DSC, a constant heating rate may be used, e.g. of 10°C/min. In alternative embodiments, the Tm is at least 66, 67, 68, 69, 70, 71, 72, 73, 74 or 75°C. Or, the Tm is equal to or lower than 150°C, or equal to or lower than 145, 140, 135, 130, 125, 120, 115 or 110°C. Accordingly, examples of intervals of Tm are: 65-150°C, 66-150°C, - (etc.) - 75-150°C; 65-145°C, 66-145°C, - (etc.) - 75-145°C; 65-140°C, - (etc.) - 75-140°C; - (etc.) - 65-110°C, 66-110°C, - (etc.) - 75-110°C.

Particular ranges for Tm are the following: between 65 and 110°C; between 70 and 110°C; between 70 and 100°C; between 75 and 95°C, or between 80 and 90°C.

In Examples 9 and 10 below, the measurement of Tm by DSC is described, and the Tm's of a number of phytases are shown.

The optimum temperatures are also indicated, since - as an alternative mean - a thermostable phytase can be defined as a phytase having a temperature-optimum of at least 60°C. Preferably, the optimum temperature is determined on the substrate phytate or phytic acid at pH 5.0 or 5.5. Example 9 describes an example of a phytase assay, including a definition of units.

In alternative embodiments, the optimum temperature is at least 61, 62, 63, 64, 65, 66, 67, 68, 69 or 70°C. In a particular embodiment, the optimum temperature is equal to or lower than 140°C, or equal to or lower than 135, 130, 125, 120, 115, 110, 105 or 100°C. Accordingly, examples of intervals of optimum temperature are: 60-140°C, 61-140°C, - (etc.) - 70-140°C; 60-135°C, 61-135°C, - (etc.) - 70-135°C; 60-130°C, - (etc.) - 70-130°C; - (etc.) - 60-100°C, 61-100°C, - (etc.) - 70-100°C.

Before describing the present invention in more detail a short explanation of the Figures enclosed is given below.

Figure 1: Design of the consensus phytase-1 sequence. The letters represent the amino acid residues in the one-letter code. The following sequences were used for the alignment: phyA from Aspergillus terreus 9A-1 [Mitchell, D. B., Vogel, K., Weimann, B. J., Pasamontes, L. & van Loon, A. P. G. M. (1997) The phytase subfamily of histidine acid phosphatases: isolation of genes for two novel phytases from the fungi Aspergillus terreus and Myceliophthora thermophila, Microbiology 143, 245-252); from amino acid (aa) 27; SEQ ID NO: 1]; phyA from A. terreus cbs116.46 [EP 897985]. A heat resistant phytase of Aspergillus fumigatus with superior performance in animal experiments. Phytase optimization and natural variability. In: The Biochemistry of phytate and phytases (eds. Rasmussen, S.K; Raboy, V.; Dalbøge, H. and Loewus, F.; Kluwer Academic Publishers); from aa 27; SEQ ID NO: 2; phyA from Aspergillus niger var. awamori (Piddington et al (1993) Gene 133, 55-62; from aa 27; SEQ ID NO: 3); phyA from A. niger T213 (EP 897985); from aa 27; SEQ ID NO: 4); phyA from A. niger strain NRRL3135 [van Hartingsveldt, W., van Zeijl, C. M. F., Harteveld, G. M., Gouka, R. J., Suykerbuyk, M. E. G., Luiten, R. G. M., van Paridon, P. A., Selten, G. C. M., Veenstra, A. E., van Gorcom, R. F. M., & van den Hondel, C. A. M. J. J. (1993) Cloning, characterization and overexpression of the phytase-encoding gene (phyA) of Aspergillus niger. Gene 127, 87-94; from aa 27; SEQ ID NO: 5]; phyA from Aspergillus fumigatus ATCC 13073 (Pasamontes, L., Haiker, M., Wyss, M., Tessier, M. & van Loon, A. P. G. M. (1997) Cloning, purification and characterization of a heat stable phytase from the fungus Aspergillus fumigatus, Appl. Environ. Microbiol. 63, 1696-1700; from aa 25; SEQ ID NO: 6]; phyA from A. fumigatus ATCC 32722 (EP 897985); from aa 27; SEQ ID NO: 7); phyA from A. fumigatus ATCC 58128 (EP 897985); from aa 27; SEQ ID NO: 8); phyA from A. fumigatus ATCC 26906 (EP 897985); from aa 27; SEQ ID NO: 9); phyA from A. fumigatus ATCC 32239 (EP 897985); from aa 30; SEQ ID NO: 10; phyA from Emericella nidulans [Pasamontes, L., Haiker, M., Henriquez-Huecas, M., Mitchell, D. B. & van Loon, A. P. G. M. (1997a). Cloning of the phytases from Emericella nidulans and the thermophilic fungus Talaromyces thermophilus. Biochim. Biophys. Acta 1353, 217-223; from aa 25; SEQ ID NO: 11]; phyA from Talaromyces thermophilus (Pasamontes et al., 1997a; from aa 24; SEQ ID NO: 12); and phyA from Myceliophthora thermophila (Mitchell et al., 1997; from aa 19; SEQ ID NO: 13). The alignment was calculated using the program PILEUP. The location of the gaps was refined by hand. Capitalized amino acid residues in the alignment at a given position belong to the amino acid coalition that establish the consensus residue. In bold, beneath the calculated consensus sequence, the amino acid sequence of the finally constructed consensus phytase (Fcp) is shown (SEQ ID NO: 14). The gaps in the calculated consensus sequence were filled by hand according to principals stated in Example 1.

Figure 2: DNA sequence (SEQ ID NO: 15) of the consensus phytase-1 gene (fcp) and of the primers used for the gene construction. The calculated amino acid sequence (Figure 1, SEQ ID NO: 14) was converted into a DNA sequence using the program BACKTRANSLATE [Devereux, J., Haeberli, P. & Smithies, O. (1984) A comprehensive set of sequence analysis programs for the VAX. Nucl. Acids Res. 12, 387-395], and the codon frequency table of highly expressed yeast genes (GCG program package, 9.0). The signal peptide of the phytase from A. terreus cbs 116.46 was fused to the N-terminus. The amino acid sequence shown in Fig. 2 is SEQ ID NO: 16. The bold bases represent the sequences of the oligonucleotides used to generate the gene. The names of the respective oligonucleotides are alternately noted above or below the sequence. The underlined bases represent the start and stop codon of the gene. The bases written in italics represent the two introduced Eco RI sites.

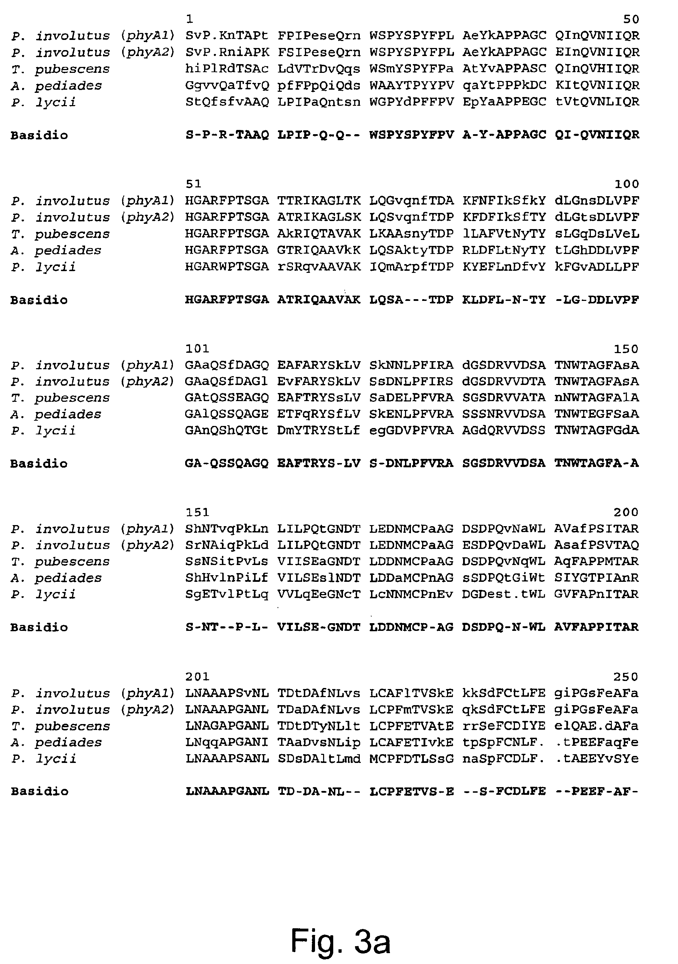

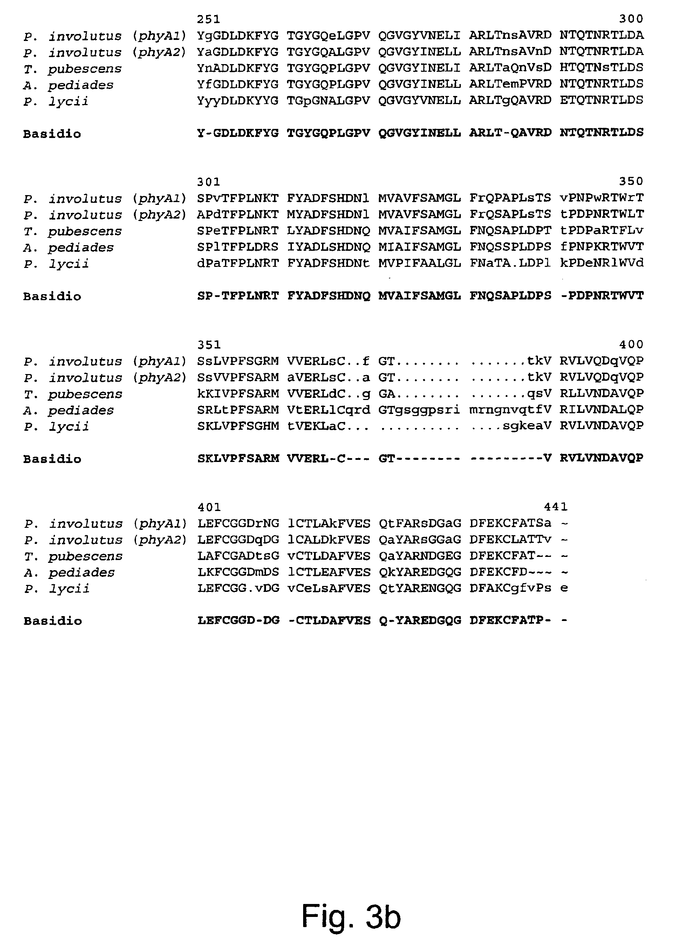

Figure 3: Alignment and consensus sequence of five Basidiomycete phytases. The letters represent the amino acid residues in the one-letter code. The amino acid sequences of the phytases from Paxillus involutus, phyA1 (from aa 21; SEQ ID NO: 17; and phyA2 (from aa 21, WO 98/28409; SEQ ID NO: 18); Trametes pubescens (from aa 24, WO 98/28409; SEQ ID NO: 19); Agrocybe pediades (from aa 19, WO 98/28409; SEQ ID NO: 20); and Peniophora lycii (from aa 21, WO 98/28409; SEQ ID NO: 21), starting with the amino acid residues mentioned in parentheses, were used for the alignment and the calculation of the corresponding consensus sequence called "Basidio" (Example 2; SEQ ID NO: 22). The alignment was performed with the program PILEPUP. The location of the gaps was refined by hand. The consensus sequence was calculated by the program PRETTY. While a vote weight of 0.5 was assigned to the two P. involutus phytases, all other genes were used with a vote weight of 1.0 for the consensus sequence calculation. At positions where the program was not able to determine a consensus residue, the Basidio sequence contains a dash. Capitalized amino acid residues in the alignment at a given position represent the amino acid coalition that established the consensus residue.

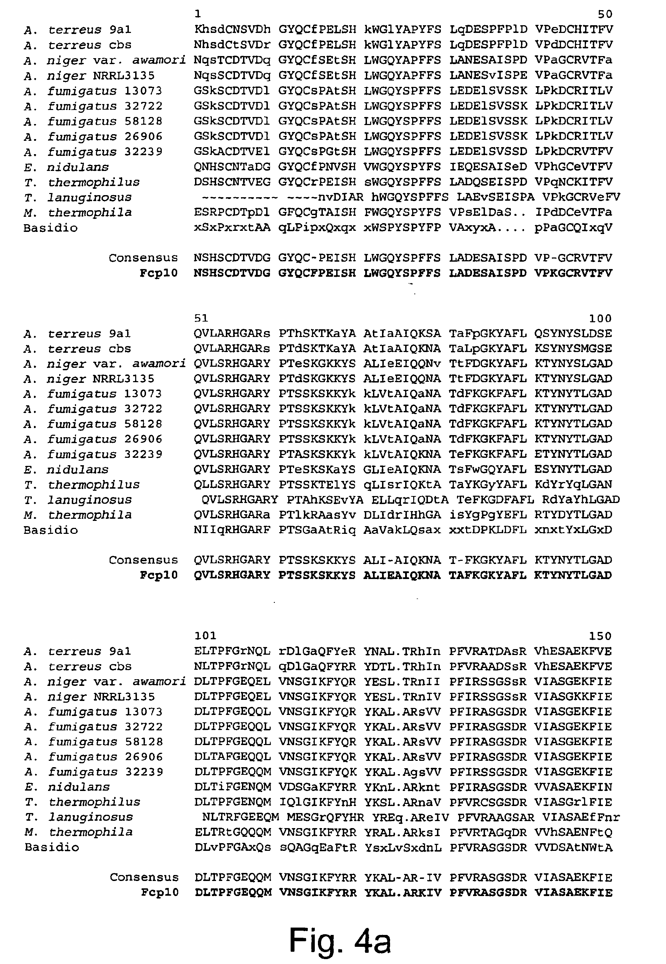

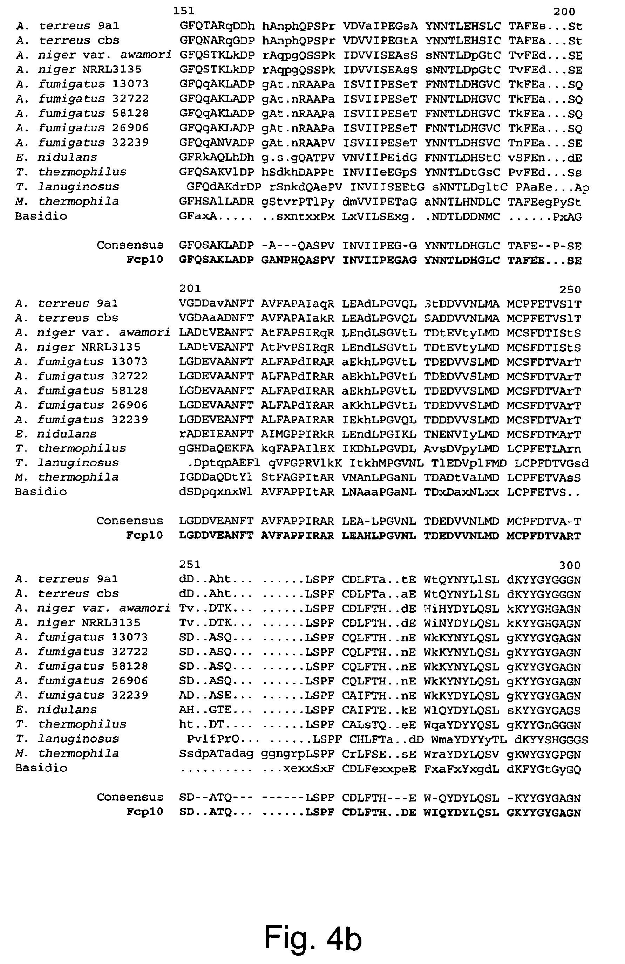

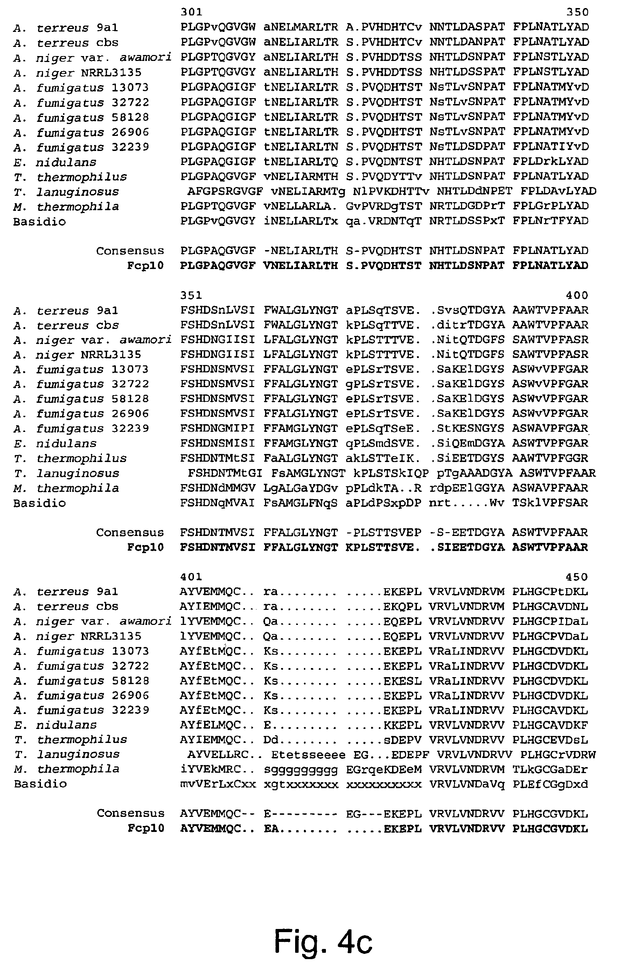

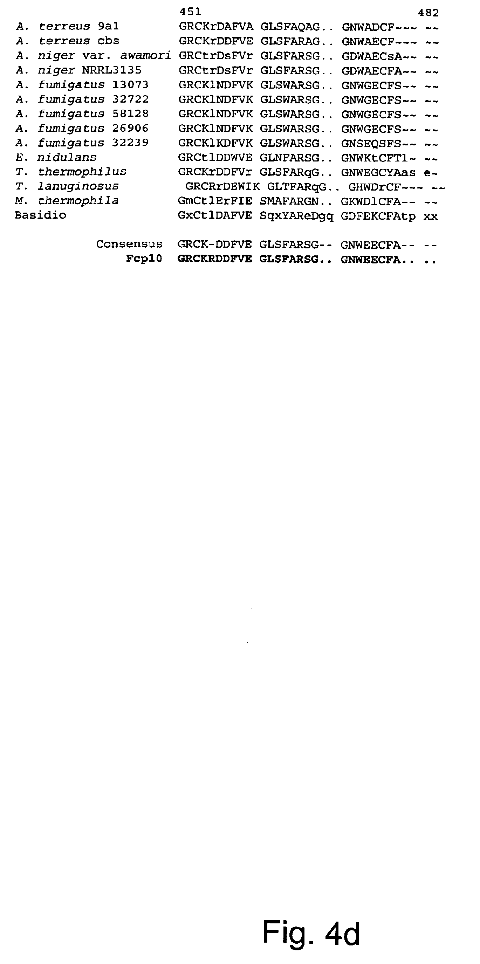

Figure 4: Design of consensus phytase-10 amino acid sequence. By adding the sequence of Thermomyces lanuginosus phytase [Berka, R. M., Rey, M. W., Brown, K. M., Byun, T. & Klotz, A. V. (1998) Molecular characterization and expression of a phytase gene from the thermophilic fungus Thermomyces lanuginosus. Appl. Environ. Microbiol. 64, 4423-4427; SEQ ID NO: 23] and the consensus sequence of the phytases from five Basidiomycetes (SEQ ID NO: 22) to the alignment of Figure 1, an improved consensus sequence was calculated by the program PRETTY. Additionally, the amino acid sequence of A. niger T213 was omitted, and a vote weight of 0.5 was assigned to the remaining two A. niger phytase sequences. For further information see Example 2.

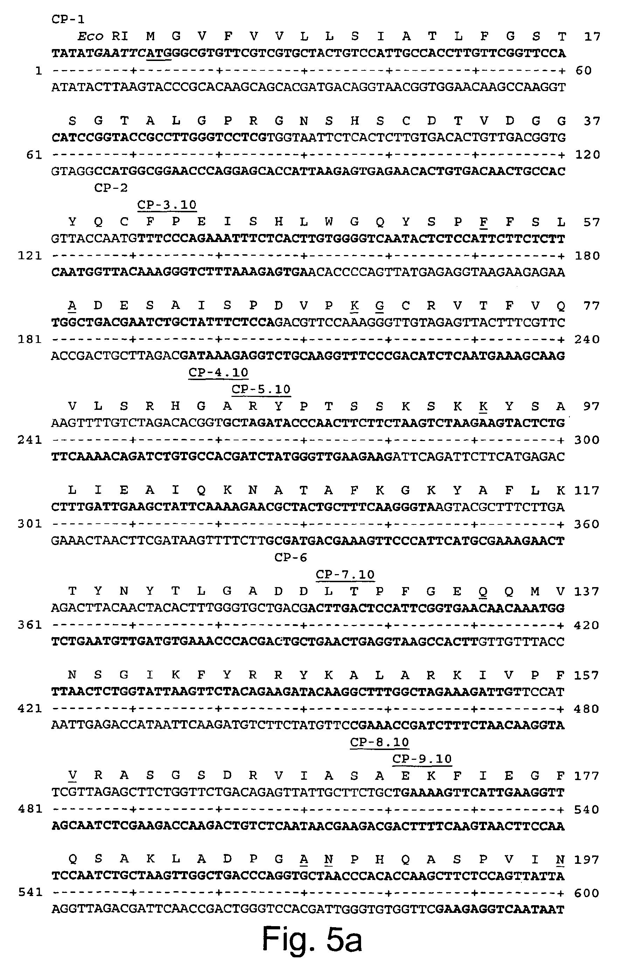

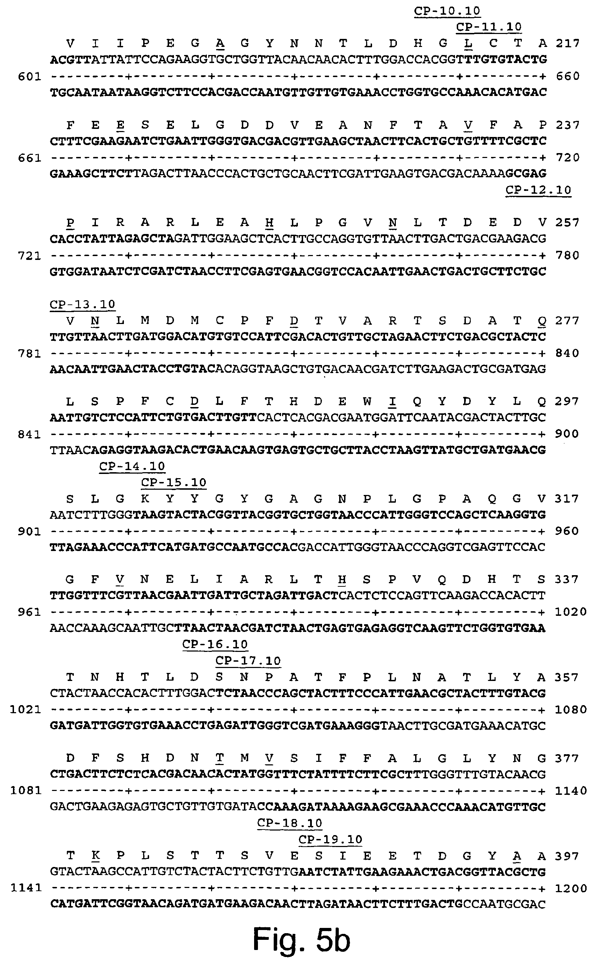

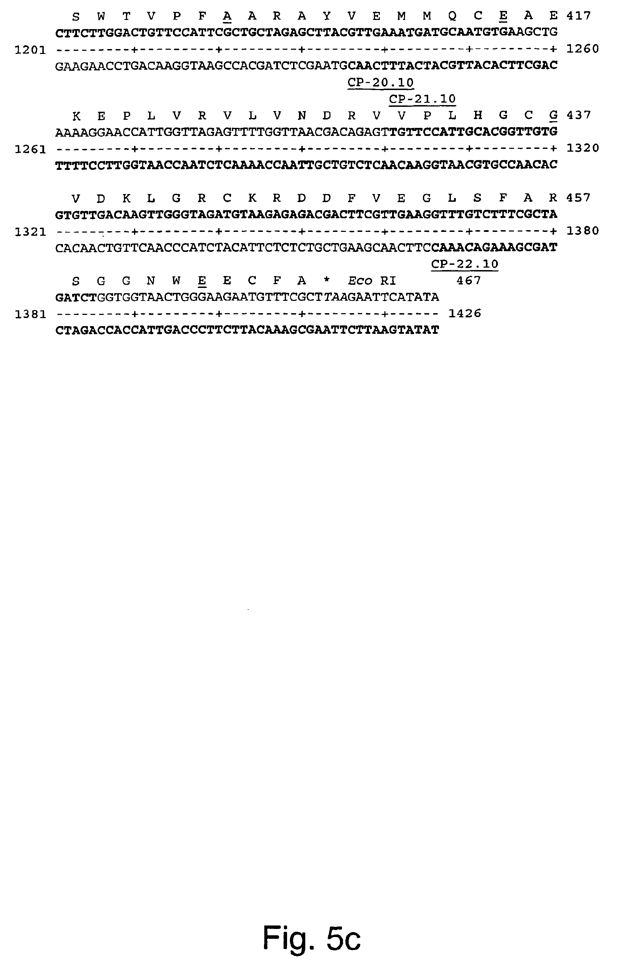

Figure 5: DNA and amino acid sequence of consensus phytase-10 (SEQ ID NO: 25, and SEQ ID NO: 26, respectively). The amino acid sequence is written above the corresponding DNA sequence using the one-letter code. The sequence of the oligonucleotides that were used to assemble the gene are in bold letters. The names of the respective oligonucleotides and the amino acids that differ relative to consensus phytase-1 are underlined. The fcp10 gene was assembled from the following oligonucleotides: CP-1, CP-2, CP-3.10, CP-4.10, CP-5.10, CP-6, CP-7.10, CP-8.10, CP-9.10, CP-10.10, CP-11.10, CP-12.10, CP-13.10, CP-14.10, CP-15.10, CP-16.10, CP-17.10, CP18.10, CP-19.10, CP-20.10, CP-21.10, and CP-22.10. The newly synthesized oligonucleotides are additionally marked by the number 10. The phytase contains the following 32 exchanges relative to consensus phytase-1: Y54F, E58A, D69K, D70G, A94K, N134Q, I158V, S187A, Q188N, D197N, S204A, T214L, D220E, L234V, A238P, D246H, T251N, Y259N, E267D, E277Q, A283D, R291I, A320V, R329H, S364T, I366V, A379K, S396A, G404A, Q415E, A437G, A463E. The underlined mutations revealed a stabilizing effect on consensus phytase-1 when tested as single mutations in consensus phytase-1.

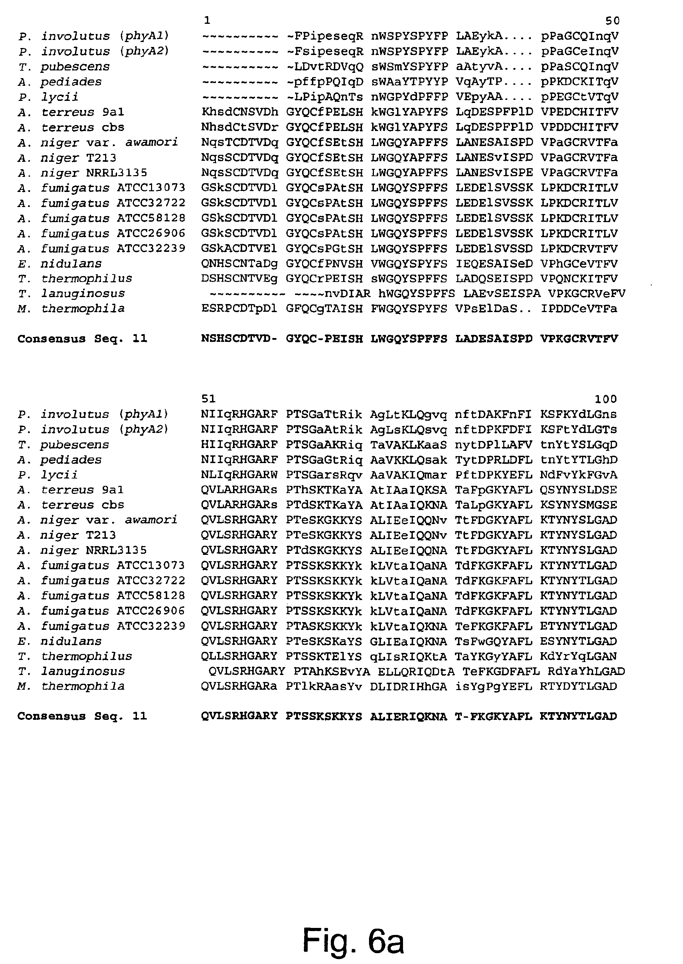

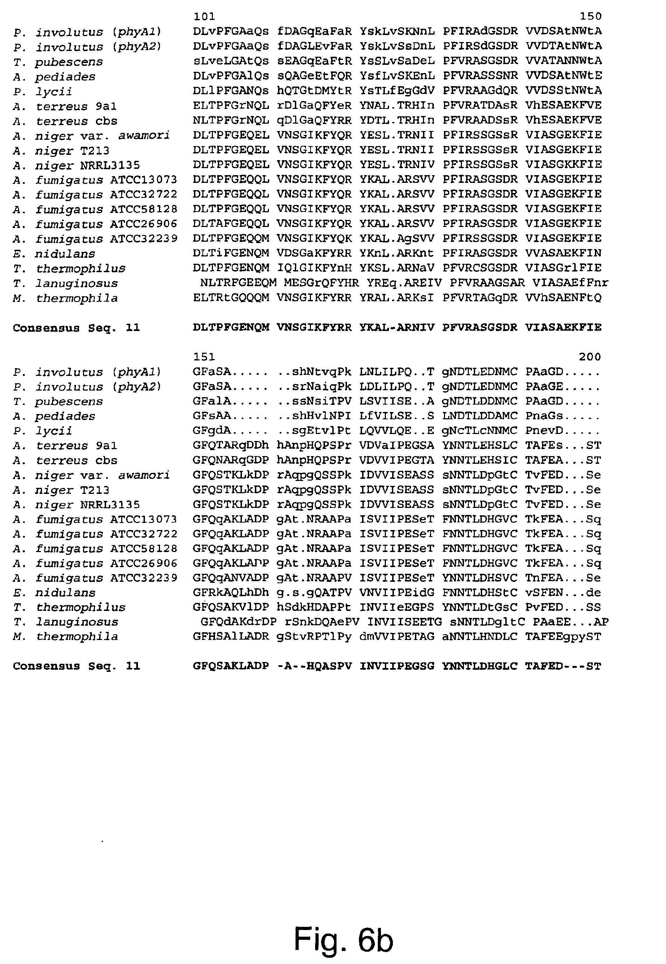

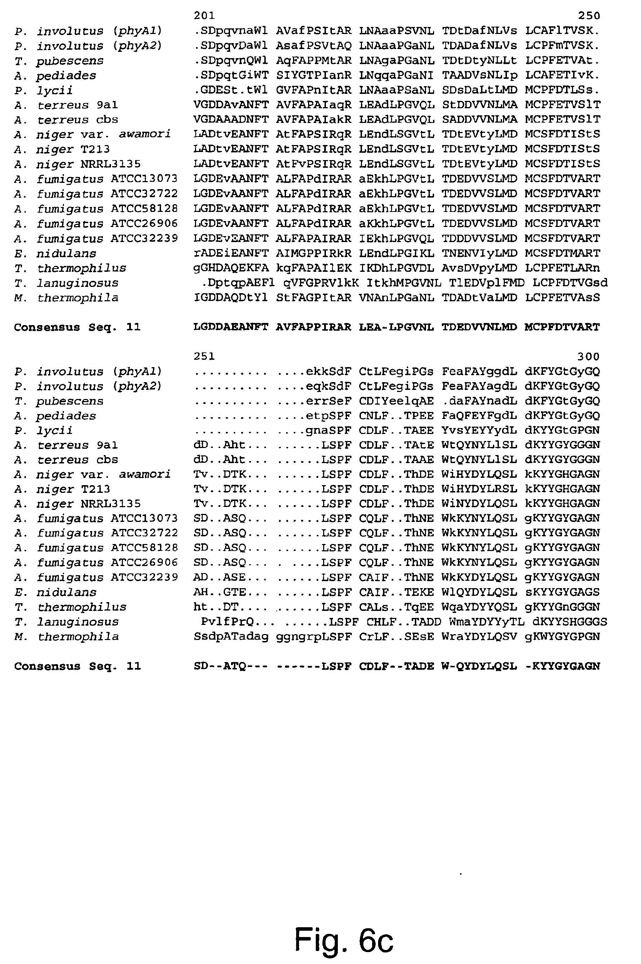

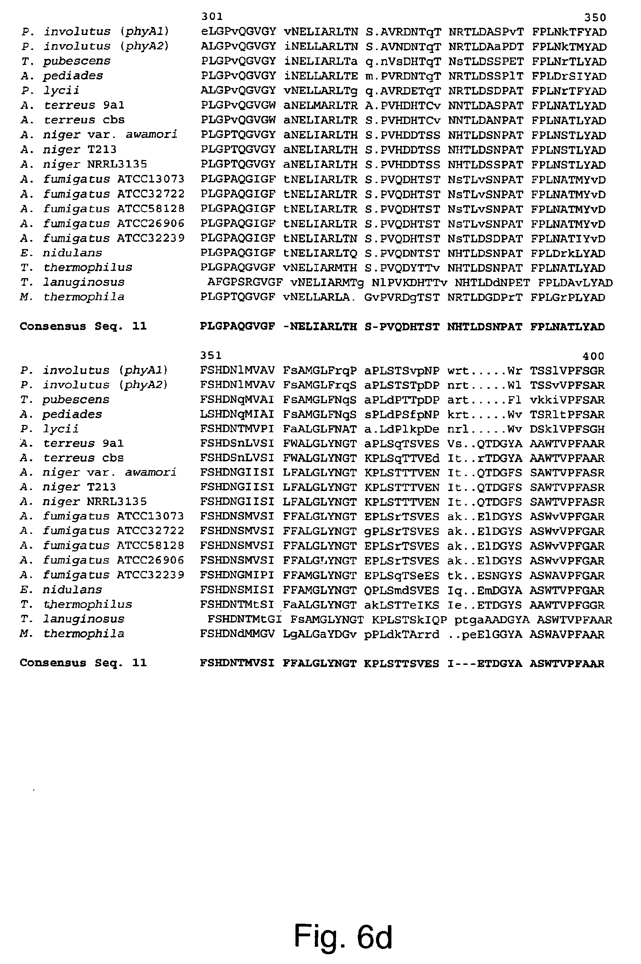

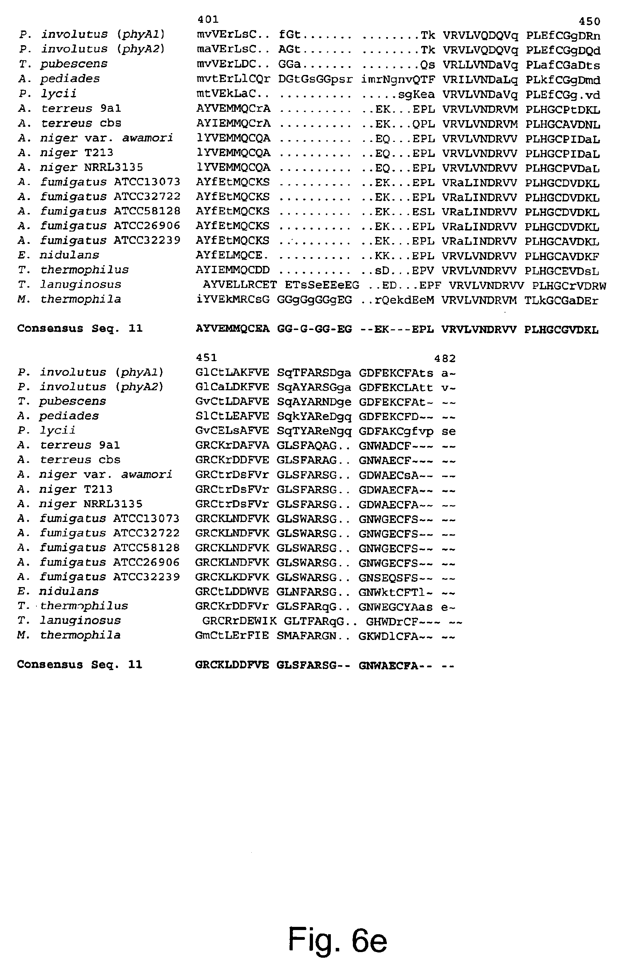

Figure 6: Alignment for the design of consensus phytase-11 (SEQ ID NO: 27). In contrast to the design of consensus phytase-10, for the design of the amino acid sequence of consensus phytase-11, all Basidiomycete phytases were used as independent sequences using an assigned vote weight of 0.2 for each Basidiomycete sequence. Additionally, the amino acid sequence of A. niger T213 was again used in this alignment.

Figure 7: DNA and amino acid sequence of consensus phytase-1-thermo[8]-Q50T-K91A (SEQ ID NO: 28, and SEQ ID NO: 29, respectively). The amino acid sequence is written above the corresponding DNA sequence using the one-letter code. The replaced amino acid residues (relative to consensus phytase-1) are underlined. The stop codon of the gene is marked by a star (*) .

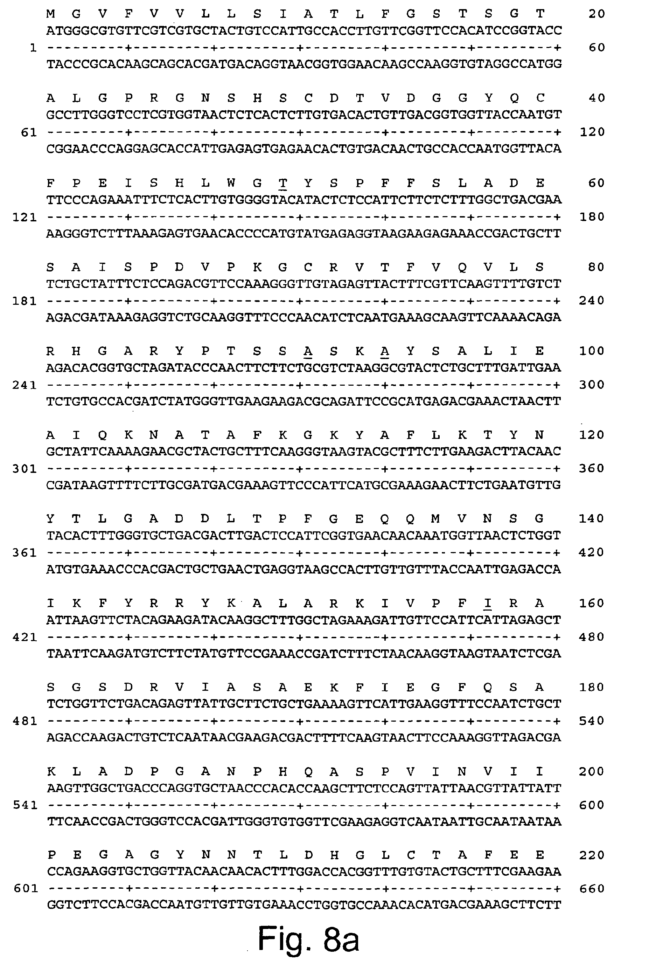

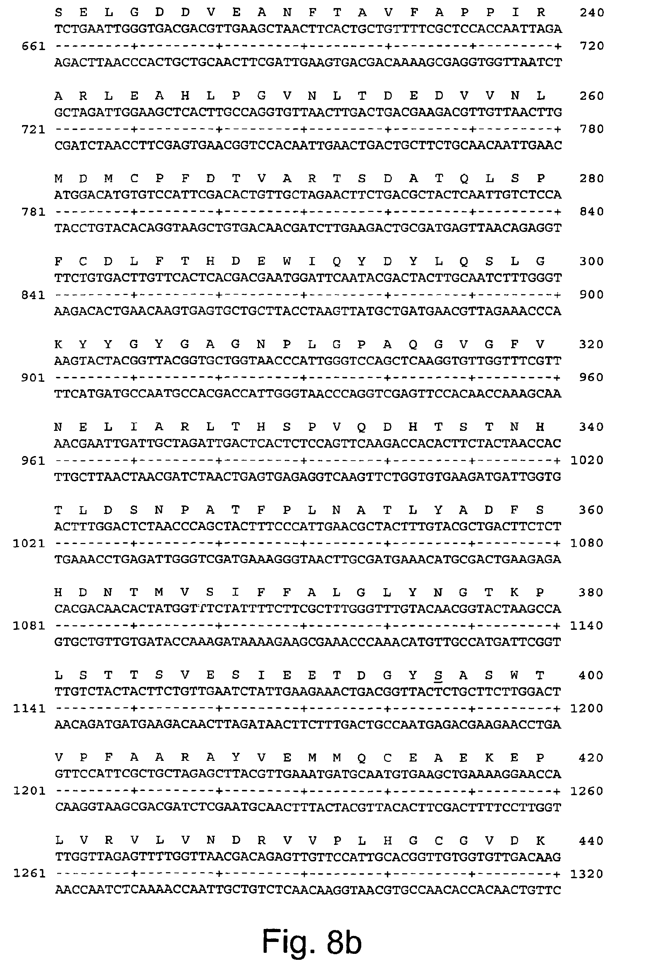

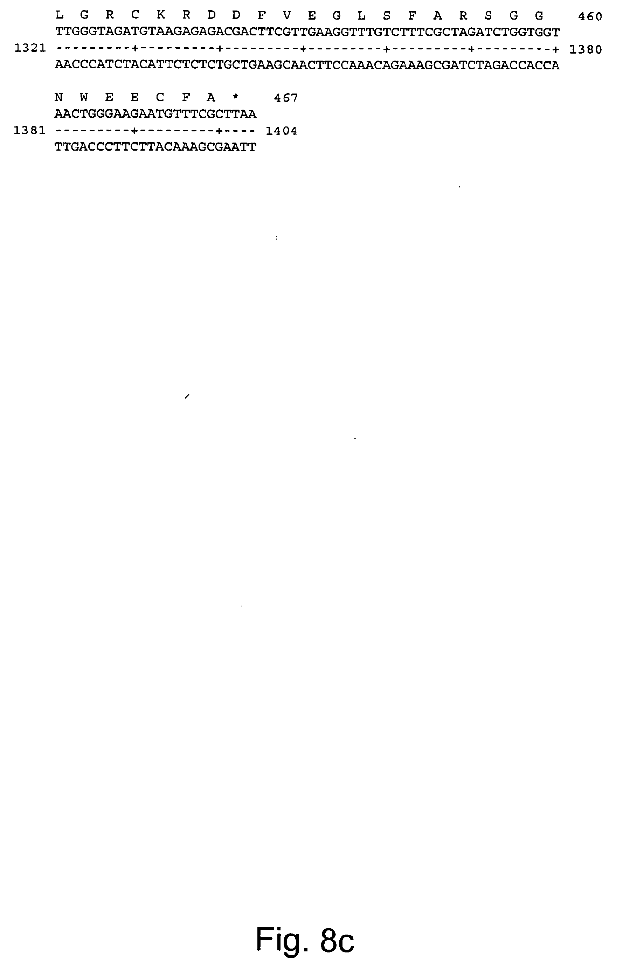

Figure 8: DNA and amino acid sequence of consensus phytase-10-thermo[3]-Q50T-K91A (SEQ ID NO: 30, and SEQ ID NO: 31, respectively). The amino acid sequence is written above the corresponding DNA sequence using the one-letter code. The replaced amino acid residues (relative to consensus phytase-10) are underlined. The stop codon of the gene is marked by a star (*) .

Figure 9: DNA and amino acid sequence of A. fumigatus ATCC 13073 phytase alpha-mutant Q51T (SEQ ID NO: 32, and SEQ ID NO: 33, respectively). The amino acid sequence is written above the corresponding DNA sequence using the one-letter code. The replaced amino acid residues (relative to A. fumigatus ATCC 13073 phytase) are underlined. The stop codon of the gene is marked by a star (*).

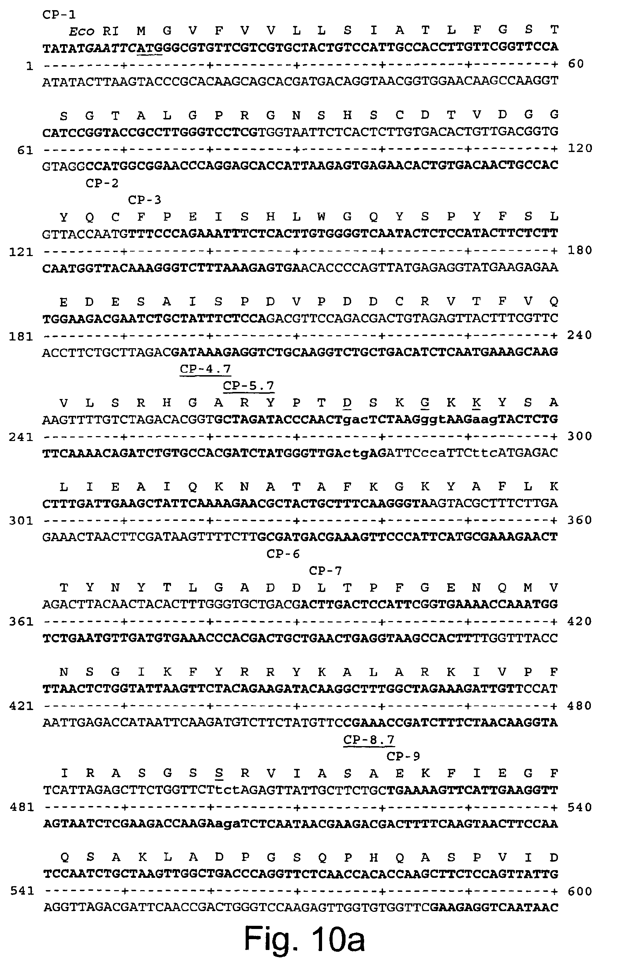

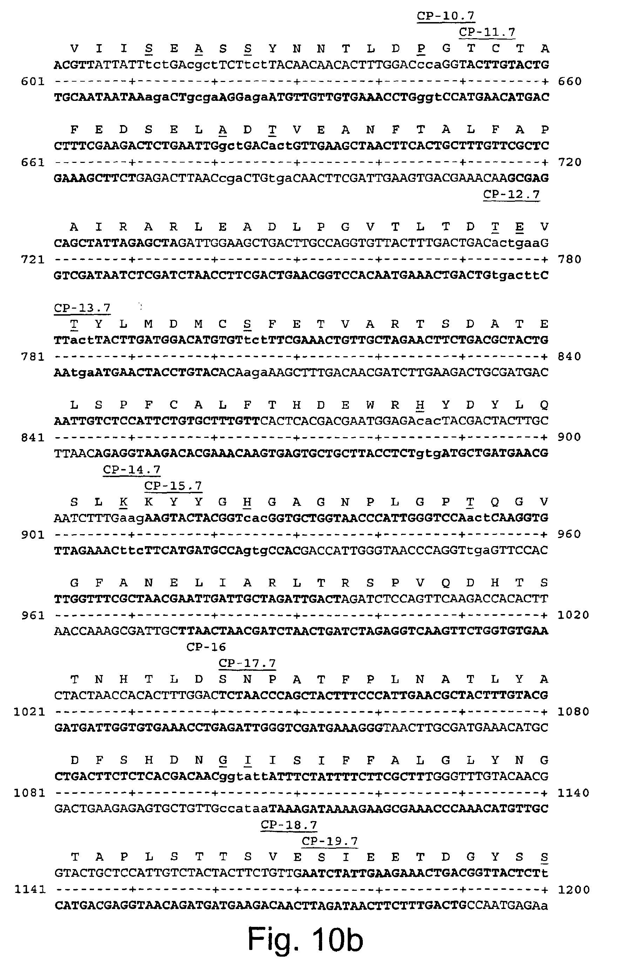

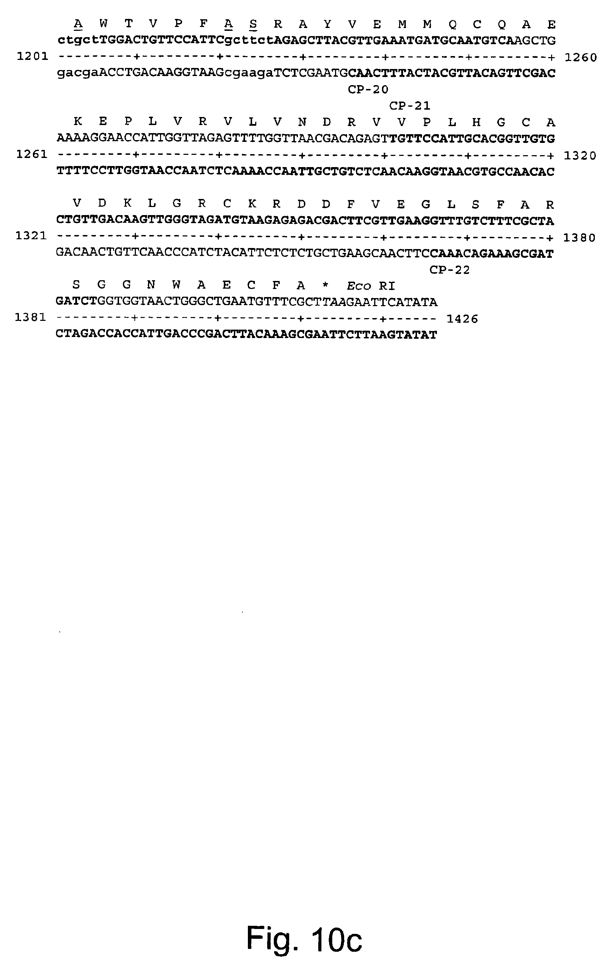

Figure 10: DNA and amino acid sequence of consensus phytase-7 (SEQ ID NO: 34, and SEQ ID NO: 35, respectively). The amino acids are written above the corresponding DNA sequence using the one-letter code. The sequence of the oligonucleotides used to assemble the gene are in bold letters. Oligonucleotides and amino acids that were exchanged (relative to consensus phytase-1) are underlined and the corresponding triplets are written in small case letters. The fcp7 gene was assembled from the following oligonucleotides: CP-1, CP-2, CP-3, CP-4.7, CP-5.7, CP-6, CP-7, CP-8.7, CP-9, CP-10.7, CP-11.7, CP-12.7, CP-13.7, CP-14.7, CP-15.7, CP-16, CP-17.7, CP-18.7, CP-19.7, CP-20, CP-21, and CP-22. The newly synthesized oligonucleotides are additionally marked by the number 7. Consensus phytase-7 contains the following 24 exchanges in comparison to the original consensus phytase-1: S89D, S92G, A94K, D164S, P201S, G203A, G205S, H212P, G224A, D226T, E255T, D256E, V258T, P265S, Q292H, G300K, Y305H, A314T, S364G, M365I, A397S, S398A, G404A, and A405S.

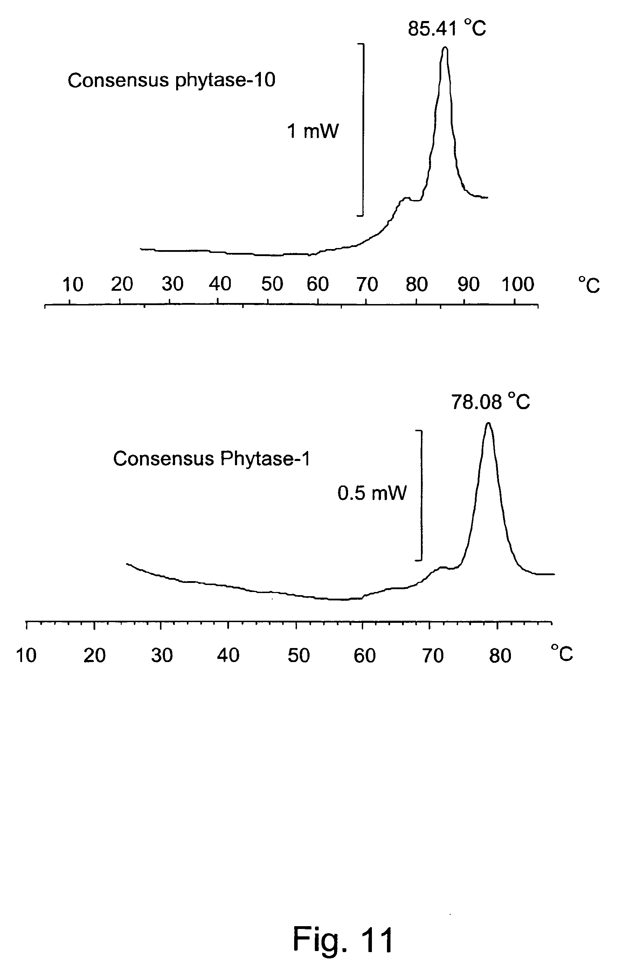

Figure 11: Differential scanning calorimetry (DSC) of consensus phytase-1 and consensus phytase-10. The protein samples were concentrated to about 50-60 mg/ml and extensively dialyzed against 10 mM sodium acetate, pH 5.0. A constant heating rate of 10°C/min was applied up to 95°C. DSC of consensus phytase-10 (upper graph) yielded a melting temperature of 85.4°C, which is 7.3°C higher than the melting point of consensus phytase-1 (78.1°C, lower graph).

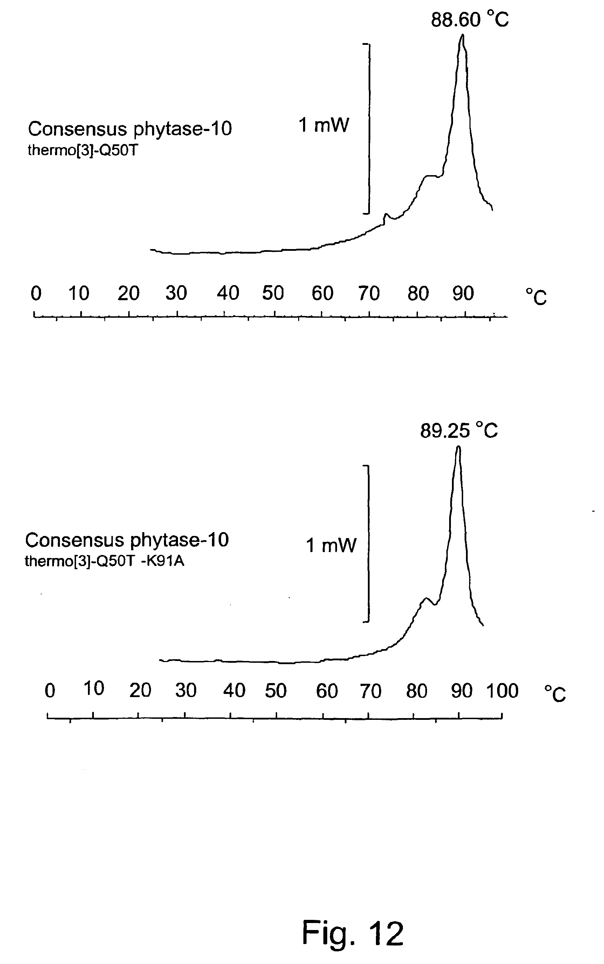

Figure 12: Differential scanning calorimetry (DSC) of consensus phytase-10-thermo[3]-Q50T and consensus phytase-10-thermo[3]-Q50T-K91A. The protein samples were concentrated to ca. 50-60 mg/ml and extensively dialyzed against 10 mM sodium acetate, pH 5.0. A constant heating rate of 10°C/min was applied up to 95°C. DSC of consensus phytase-10-thermo[3]-Q50T (upper graph) yielded a melting temperature of 88.6°C, while the melting temperature of consensus phytase-10-thermo-Q50T-K91A was determined to be 89.3°C.

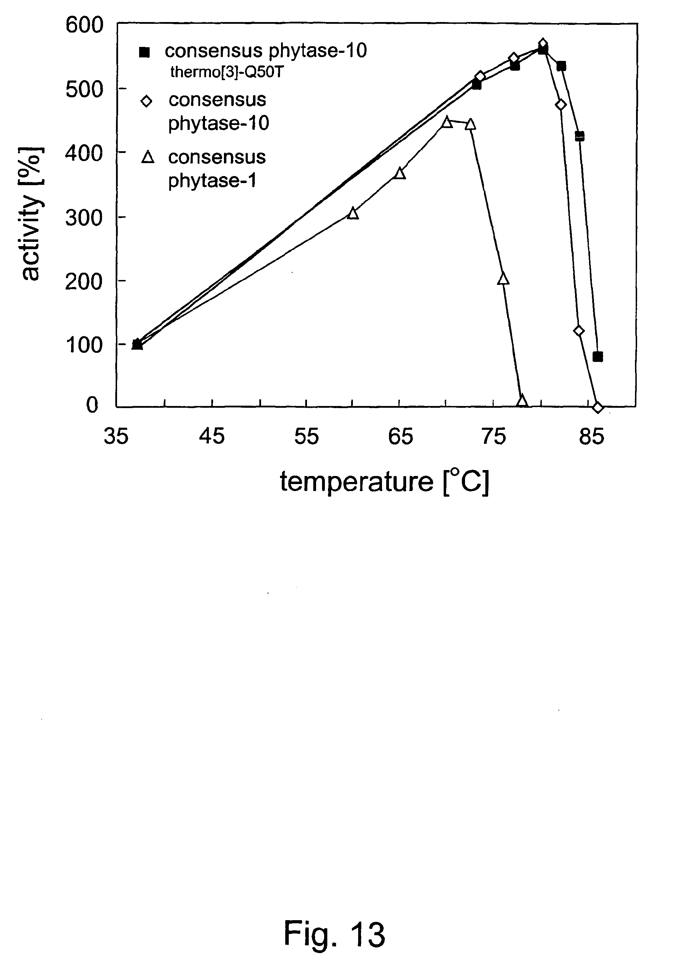

Figure 13: Comparison of the temperature optimum between consensus phytase-1, consensus phytase-10 and consensus phytase-10-thermo[3]-Q50T. For the determination of the temperature optimum, the phytase standard assay of Example 9 was performed at a series of temperatures between 37 and 86°C. The diluted supernatant of transformed S. cerevisiae strains was used for the determination. The other components of the supernatant had no influence on the determination of the temperature optimum: Λ, consensus phytase-1; ◇, consensus phytase-10; ■, consensus phytase 10-thermo[3]-Q50T.

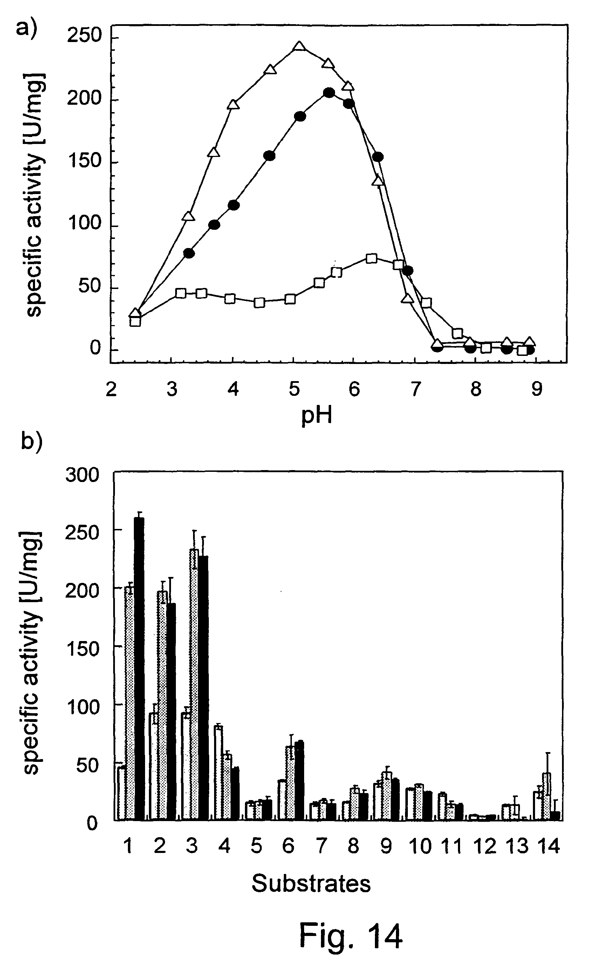

Figure 14: pH-dependent activity profile and substrate specificity of consensus phytase-10 and its variants thermo[3]-Q50T and thermo[3]-Q50T-K91A. The phytase activity was determined using the standard assay in appropriate buffers (see Example 9) at different pH-values. Graph a) shows the pH-dependent activity profile of consensus phytase-10 (□), consensus phytase-10-thermo[3]-Q50T (•), and consensus phytase-10-thermo[3]-Q50T-K91A (Λ). Graph b) shows the corresponding substrate specificity tested by replacement of phytate in the standard assay by the indicated compounds; open bars, consensus phytase-10; grey bars, consensus phytase-10-thermo[3]-Q50T; dark bars, consensus phytase-10-thermo[3]-Q50T-K91A). The numbers correspond to the following substrates: 1, phytate; 2, p-nitrophenyl phosphate; 3, phenyl phosphate; 4, fructose-1,6-bisphosphate; 5, fructose-6-phosphate; 6, glucose-6-phosphate; 7, ribose-5-phosphate; 8, DL-glycerol-3-phosphate; 9, glycerol-2-phosphate; 10, 3-phosphoglycerate; 11, phosphoenolpyruvate; 12, AMP; 13, ADP; 14, ATP.

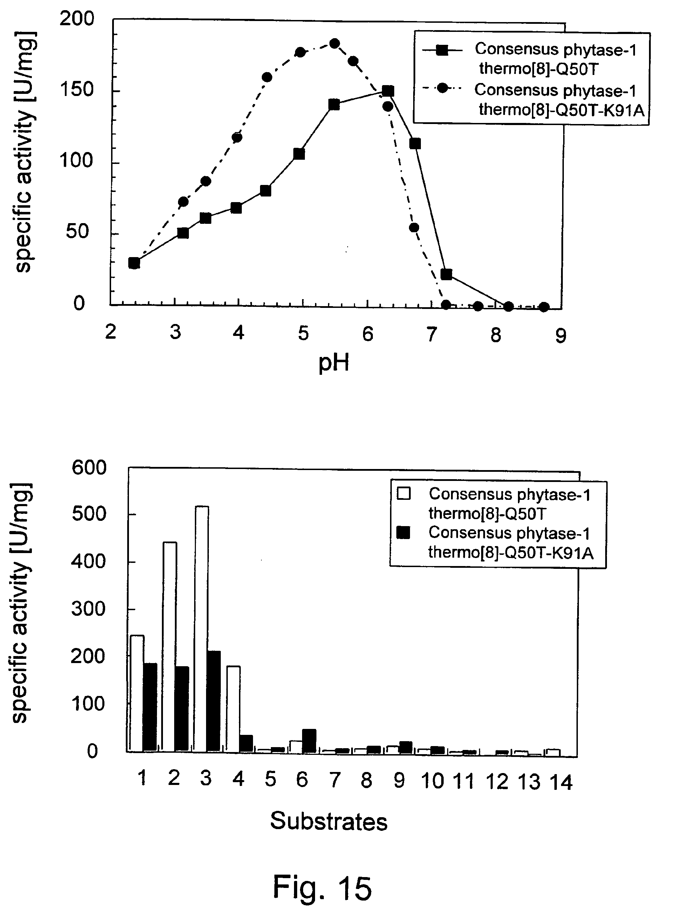

Figure 15: pH-dependent activity profile and substrate specificity of consensus phytase-1-thermo[8]-Q50T and of consensus phytase-1-thermo[8]-Q50T-K91A. The phytase activity was determined using the standard assay in appropriate buffers (see Example 9) at different pH-values. Graph a) shows the pH-dependent activity profile of the Q50T- (■) and the Q50T-K91A-variant (•). Graph b) shows the corresponding substrate specificities tested by replacement of phytate in the standard assay by the indicated compounds (open bars, consensus phytase-1-thermo[8]-Q50T; filled bars, consensus phytase-1-thermo[8]-Q50T-K91A). The substrates are listed in the legend of Figure 14.

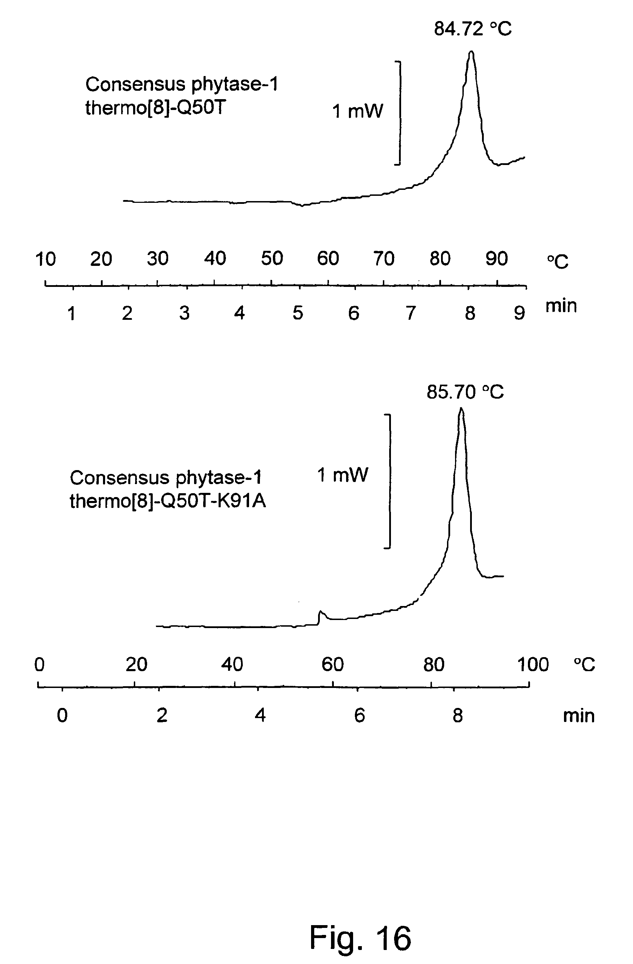

Figure 16: Differential scanning calorimetry (DSC) of consensus phytase-1-thermo[8]-Q50T and consensus phytase-1-thermo[8]-Q50T-K91A. The protein samples were concentrated to ca. 50-60 mg/ml and extensively dialyzed against 10 mM sodium acetate, pH 5.0. A constant heating rate of 10°C/min was applied up to 95°C. DSC of consensus phytase-1-thermo[8]-Q50T (upper graph) showed a melting temperature of 84.7°C, while the melting point of consensus phytase-1-thermo[8]-Q50T-K91A was found at 85.7°C.

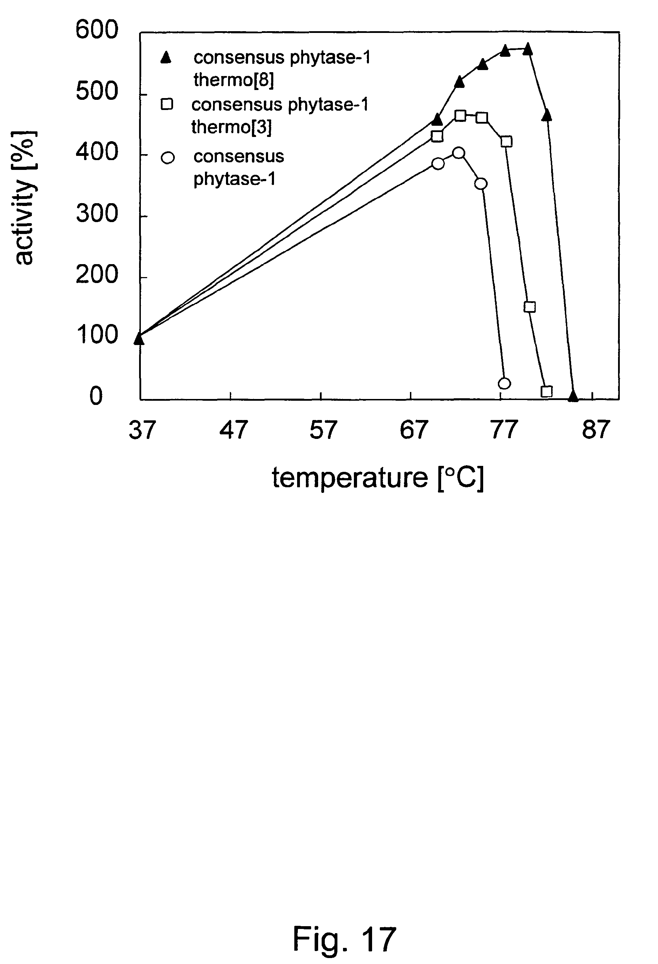

Figure 17: Comparison of the temperature optimum between consensus phytase-1, consensus phytase-1-thermo[3] and consensus phytase-1-thermo[8]. For the determination of the temperature optimum, the phytase standard assay was performed at a series of temperatures between 37 and 86°C. Protein purified from the supernatant of transformed S. cerevisiae strains was used for the determination. O, consensus phytase-1; □, consensus phytase-1-thermo[3]; ▲, consensus phytase 1-thermo[8].

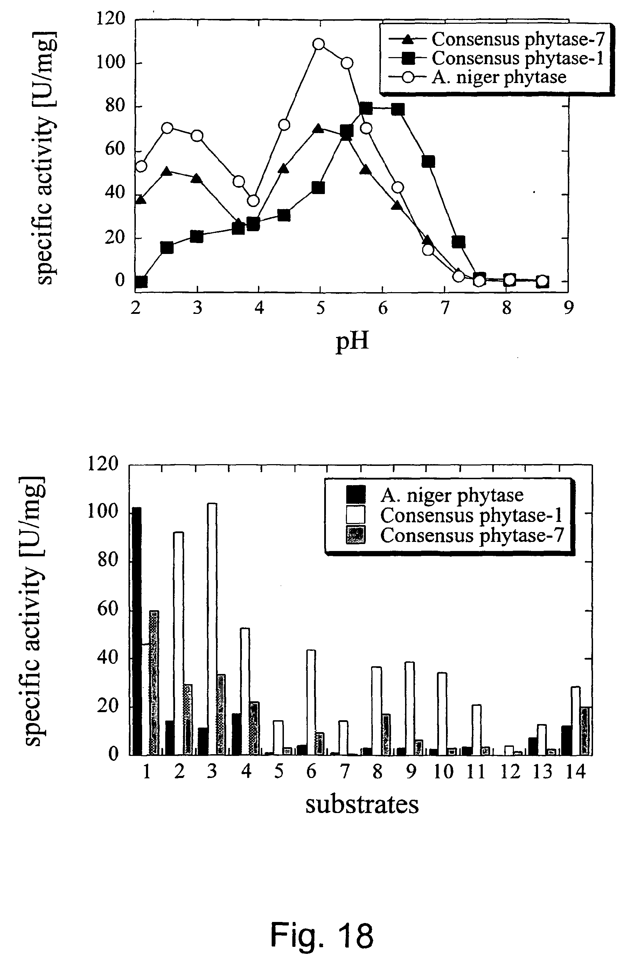

Figure 18: Comparison of the pH-dependent activity profile and substrate specificity between consensus phytase-1, consensus phytase-7, and the phytase from A. niger NRRL 3135. The phytase activity was determined using the standard assay in appropriate buffers (see Example 9) at different pH-values. Graph a) shows the pH-dependent activity profile of consensus phytase-1 (■), the phytase from A. niger NRRL 3135 (O), and of consensus phytase-7 (▲). Graph b) shows the corresponding substrate specificities tested by replacement of phytate in the standard assay by the indicated compounds (black bars, A. niger NRRL 3135 phytase; open bars, consensus phytase-1; dashed bars, consensus phytase-7). The substrates are listed in the legend of Figure 14.

Figure 19: Differential scanning calorimetry (DSC) of the phytase from A. fumigatus ATCC 13073 and of its stabilized alpha-mutant, which contains the following amino acid exchanges: F55Y, V100I, F114Y, A243L, S265P, and N294D.

The protein samples were concentrated to ca. 50-60 mg/ml and extensively dialyzed against 10 mM sodium acetate, pH 5.0. A constant heating rate of 10°C/min was applied up to 95°C. DSC of A. fumigatus 13073 phytase (lower graph) revealed a melting temperature of 62.5°C, while the melting point of the alpha-mutant was found at 67.0°C.

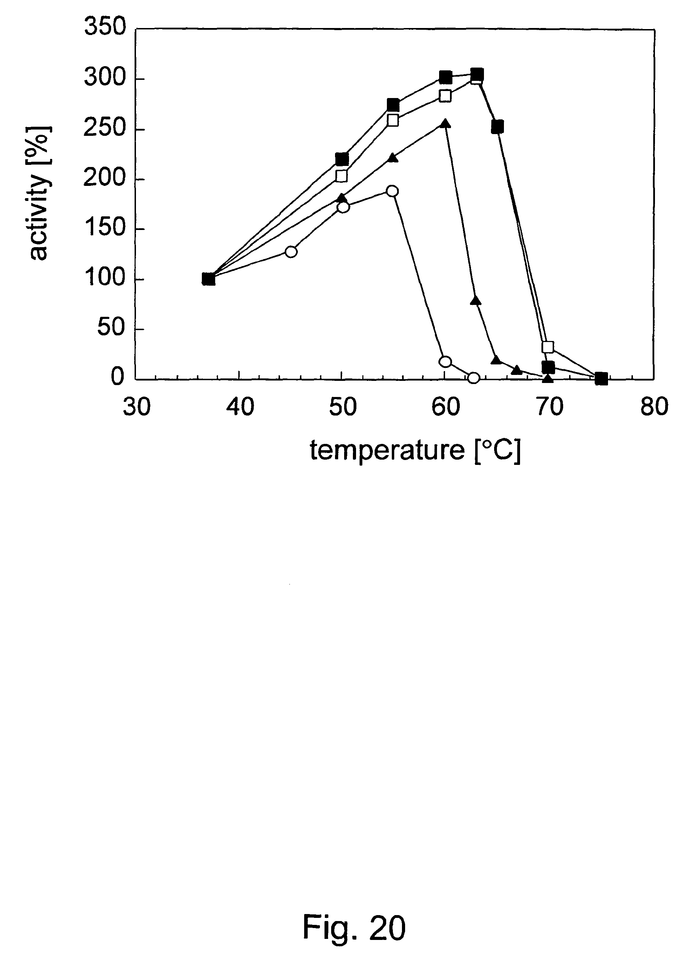

Figure 20: Comparison of the temperature optima of A. fumigatus 13073 wild-type phytase, its alpha-mutant, and a further stabilized alpha-mutant (E59A-S154N-R329H-S364T-G404A). For the determination of the temperature optimum, the phytase standard assay was performed at a series of temperatures between 37 and 75°C. The diluted supernatant of transformed S. cerevisiae strains was used for the determination. The other components of the supernatant had no influence on the determination of the temperature optimum. O, A. fumigatus ATCC 13073 phytase; ▲, A. fumigatus ATCC 13073 alpha-mutant; □, A. fumigatus ATCC 13073 alpha-mutant-(E59A-S154N-R329H-S364T-G404A)-Q27T; ■, A. fumigatus ATCC 13073 alpha-mutant-(E59AS154N-R329H-S364T-G404A)-Q51T-K92A. Q51T and K92A correspond to consensus phytase-1 substitutions Q50T and K91A, respectively.

Figure 21: Amino acid sequence of consensus phytase-12 (consphy12; SEQ ID NO: 36) which contains a number of active site residues transferred from the "basidio" consensus sequence to consensus phytase-10-thermo[3]-Q50T-K91A (underlined).

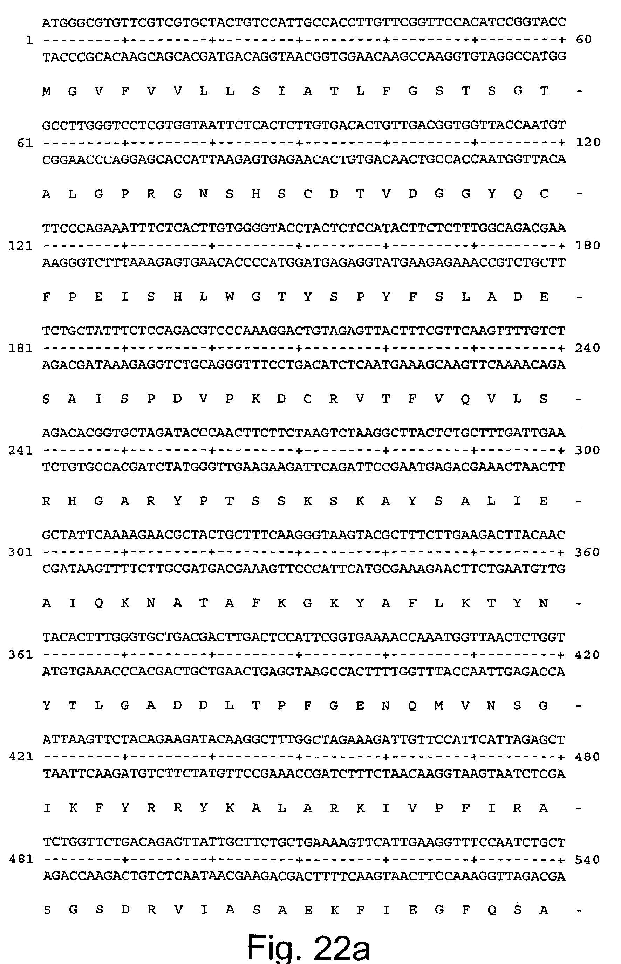

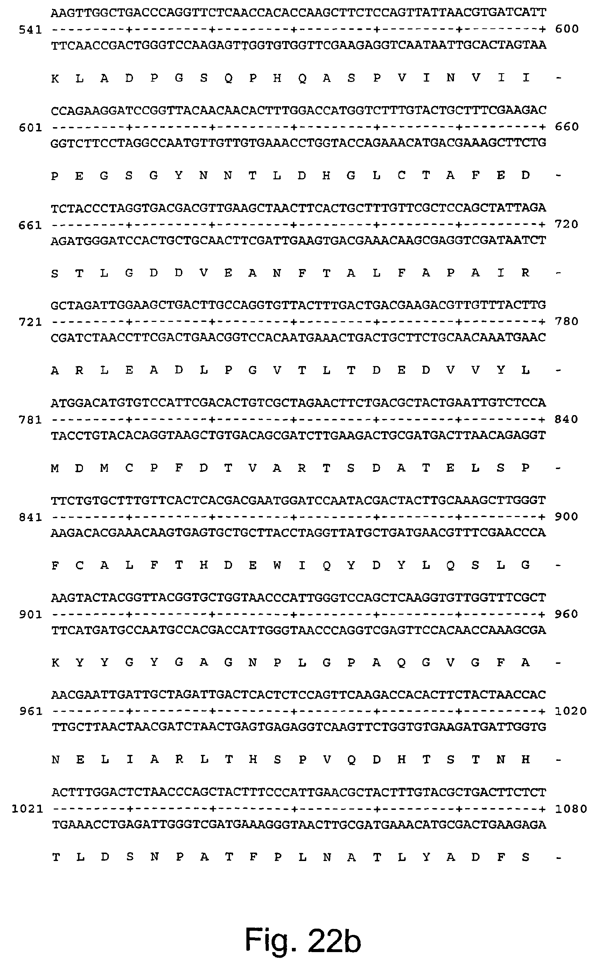

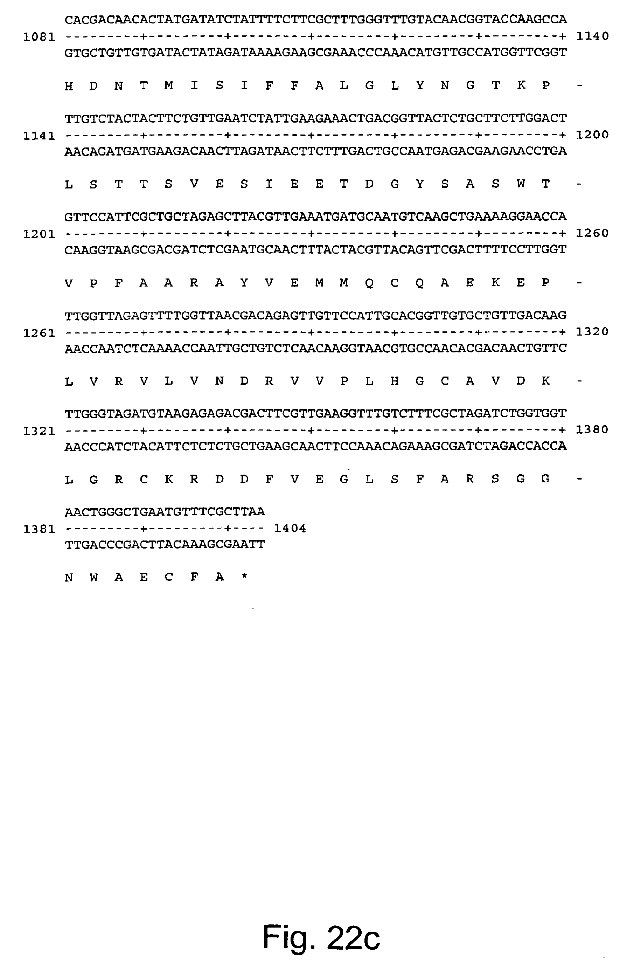

Figure 22: DNA and amino acid sequence of consensus phytase-3-thermo[11]-Q50T. The amino acids are written below the corresponding DNA sequence using the one-letter code.

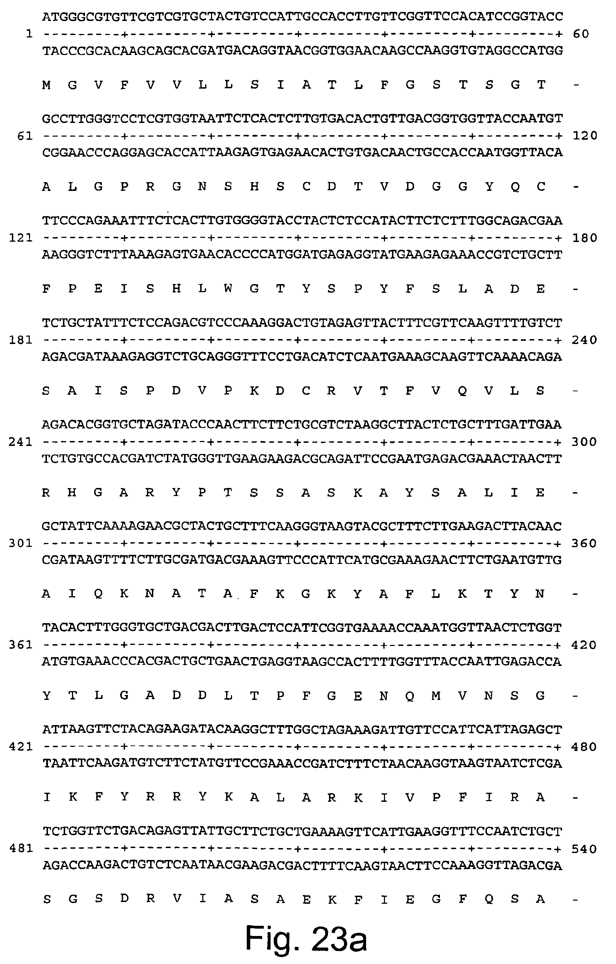

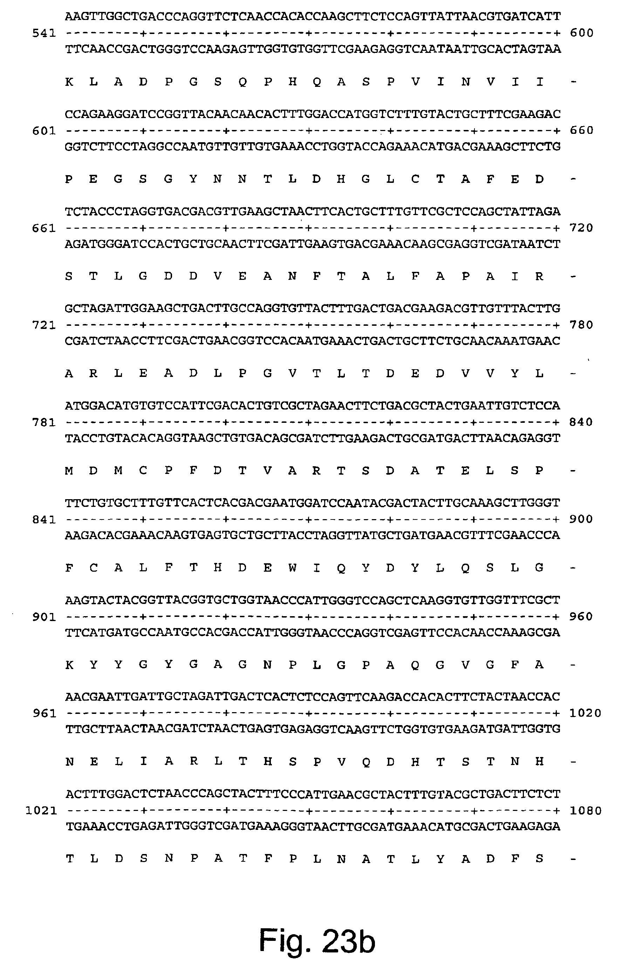

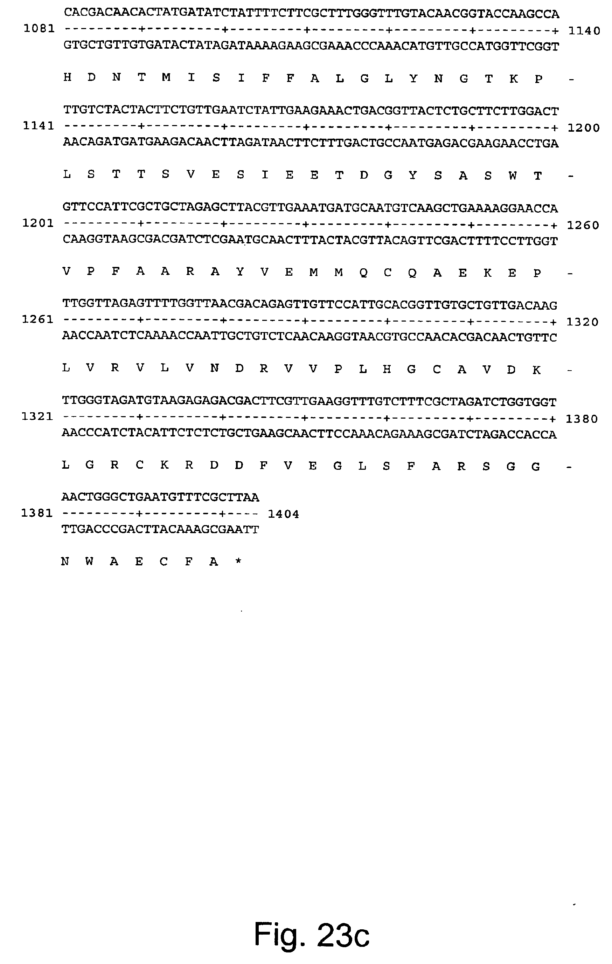

Figure 23: DNA and amino acid sequence of consensus phytase-3-thermo[11]-Q50T-K91A. The amino acids are written below the corresponding DNA sequence using the one-letter code.

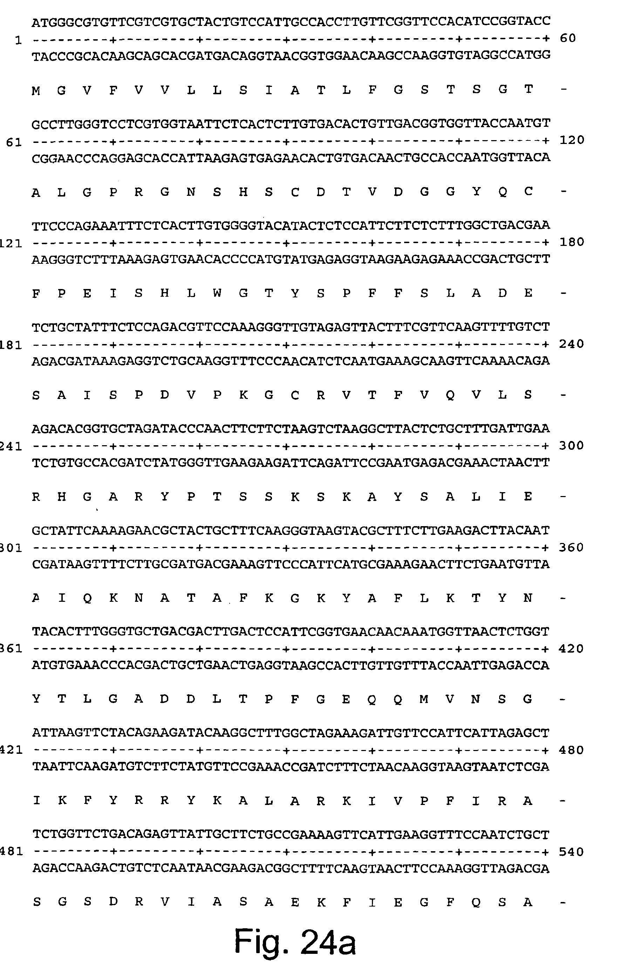

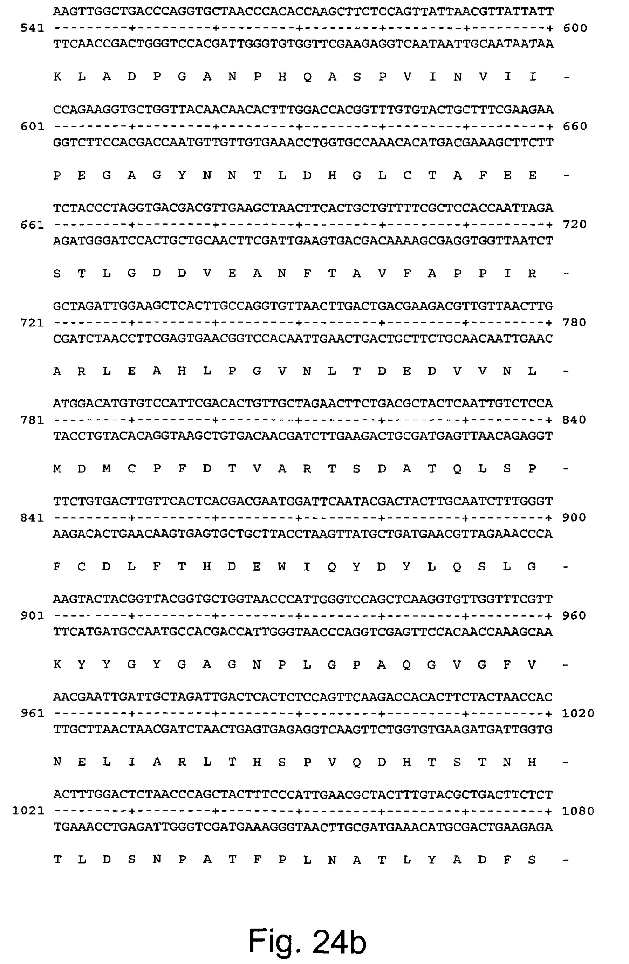

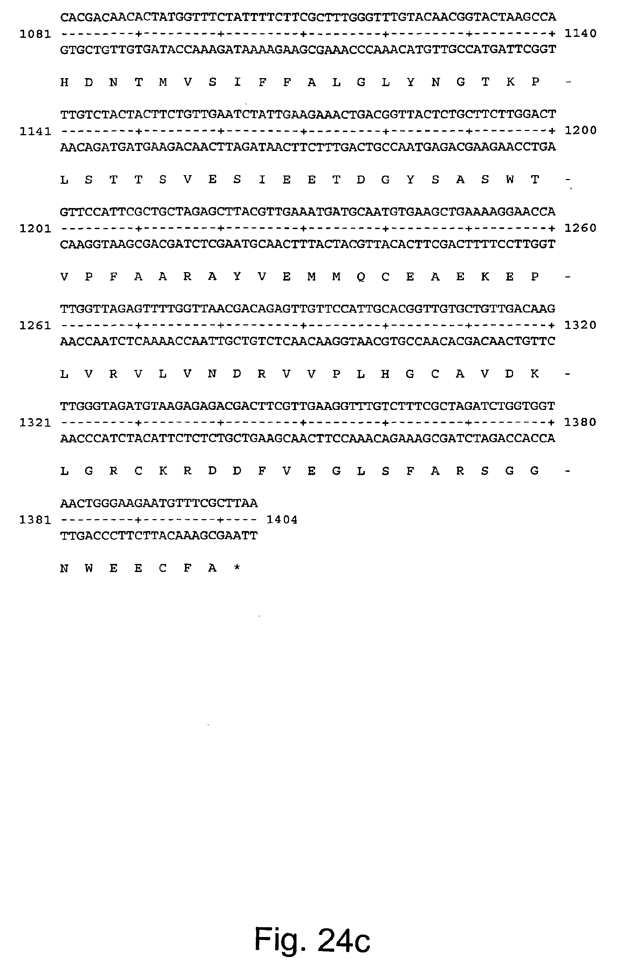

Figure 24: DNA and amino acid sequence of consensus phytase-10-thermo[5]-Q50T. The amino acids are written below the corresponding DNA sequence using the one-letter code.

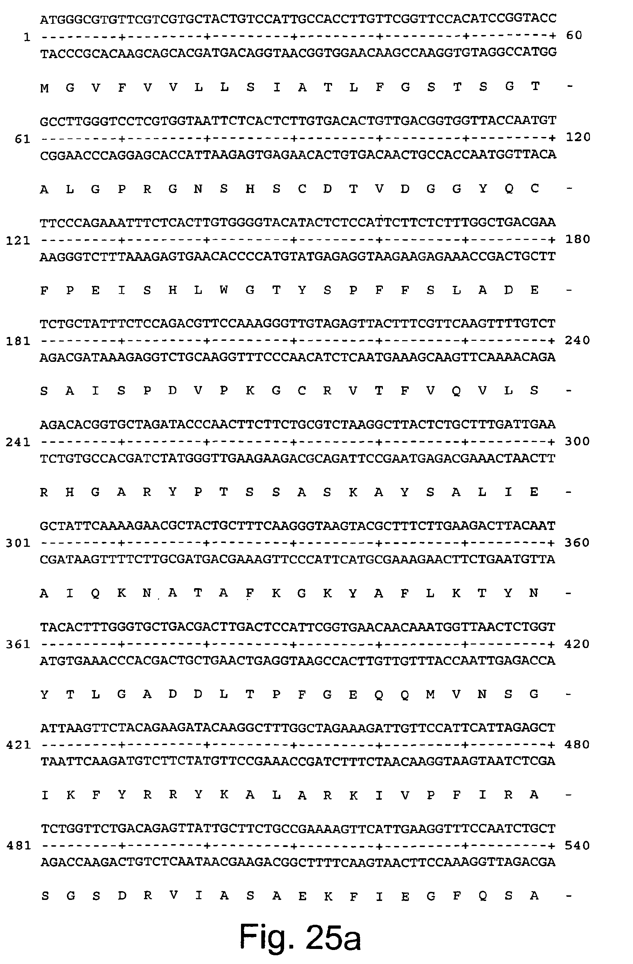

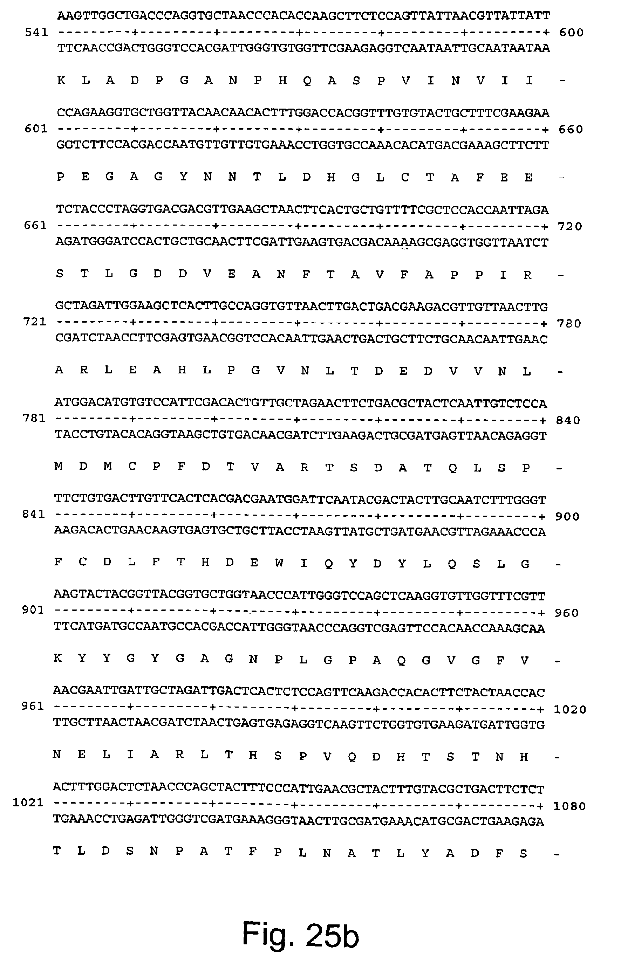

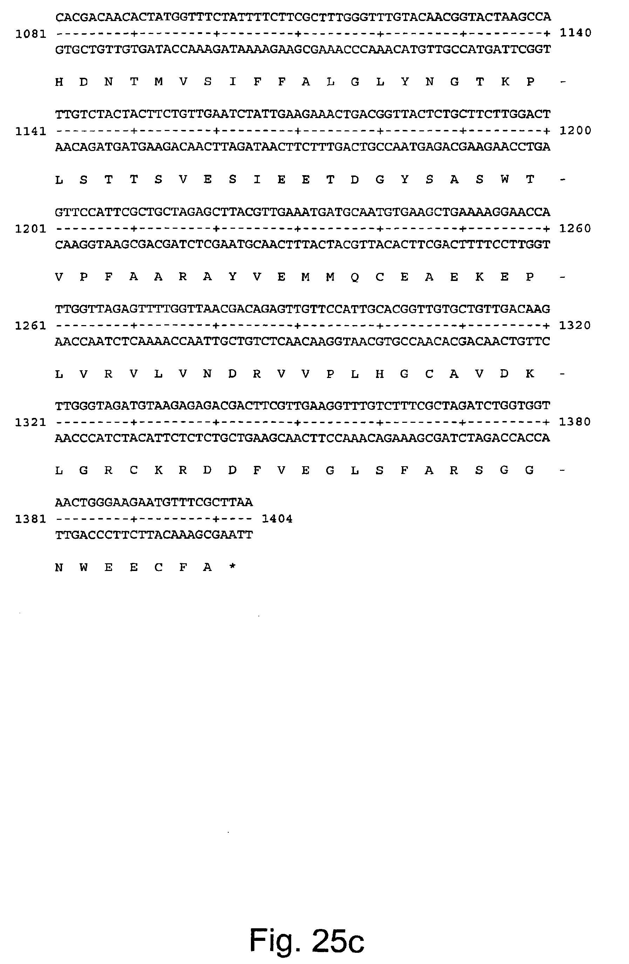

Figure 25: DNA and amino acid sequence of consensus phytase-10-thermo[5]-Q50T-K91A. The amino acids are written below the corresponding DNA sequence using the one-letter code.

The phytase-producing microorganism strains mentioned herein, viz. Paxillus involutus CBS 100231; Peniophora lycii CBS 686.96; Agrocybe pediades CBS 900.96; and Trametes pubescens CBS 100232; were isolated from natural samples originating from, respectively, Denmark; Denmark; Denmark; and Sweden (the Uppsala collection. The samples were collected in November 1992; October 1993; June 1995; and in November 1995, respectively.

### Example 1

### Consensus phytase-1

The amino acid sequence of consensus phytase-1 (fungal consensus phytase, fcp) was designed and calculated as described in Examples 1 and 2 of EP 897985. Table 1 below shows the origin and vote weight of the phytase amino acid sequences used for the design of consensus phytase-1. The consensus phytase-1 sequence was furthermore converted into a DNA sequence as described in Example 3 of EP 897985, and the consensus phytase-1 gene was constructed and cloned as described in Example 4 of EP 897985.

### Table 1

### Origin and vote weight of the phytase amino acid sequences

- phyA from Aspergillus terreus 9A-1, aa 27, vote weight 0.5 (Mitchell et al., 1997)
- phyA from Aspergillus terreus cbs116.46, aa 27, vote weight 0.5 (EP 897985)
- phyA from Aspergillus niger var. awamori, aa 27, vote weight 0.33 [Piddington, C. S., Houston, C. S., Paloheimo, M., Cantrell, M., Miettinen-Oinonen, A., Nevalainen, H., & Rambosek, J. (1993) The cloning and sequencing of the genes encoding phytase (phy) and pH 2.5-optimum acid phosphatase (aph) from Aspergillus niger var. awamori. Gene 133, 55-62].
- phyA from Aspergillus niger T213 (EP 897985), aa 27, vote weight 0.33
- phyA from Aspergillus niger strain NRRL3135, aa 27, vote weight 0.33 (van Hartingsveldt et al., 1993)
- phyA from Aspergillus fumigatus ATCC 13073, aa 26, vote weight 0.2 (Pasamontes et al., 1997)
- phyA from Aspergillus fumigatus ATCC 32722, aa 26, vote weight 0.2 (EP 897985)
- phyA from Aspergillus fumigatus ATCC 58128, aa 26, vote weight 0.2 (EP 897985)
- phyA from Aspergillus fumigatus ATCC 26906, aa 26, vote weight 0.2 (EP 897985)
- phyA from Aspergillus fumigatus ATCC 32239, aa 30, vote weight 0.2 (EP 897985)
- phyA from Emericella nidulans , aa 25, vote weight 1.0 (Pasamontes et al., 1997a)
- phyA from Talaromyces thermophilus ATCC 20186, aa 24, vote weight 1.0 (Pasamontes et al., 1997a)
- phyA from Myceliophthora thermophila, aa 19, vote weight 1.0 (Mitchell et al., 1997)

### Example 2

### Design of an improved consensus phytase (consensus phytase-10) amino acid sequence

The alignments used for the design of consensus phytase-10 were calculated using the program PILEUP from the GCG Sequence Analysis Package Release 9.0 (Devereux et al., 1984) with the standard parameters (gap creation penalty 12, gap extension penalty 4). The location of the gaps was refined using a text editor.

The following sequences were used for the alignment of the Basiodiomycete phytases starting with the amino acid (aa) mentioned in Table 2:

### Table 2

### Origin and vote weight of five Basidiomycete phytases used for the calculation of the corresponding consensus amino acid sequence (basidio)

- phyA1 from Paxillus involutus CBS No. 100231, aa 21, vote weight 0.5 (WO 98/28409)
- phyA2 from Paxillus involutus CBS No. 100231, aa 21, vote weight 0.5 (WO 98/28409)
- phyA from Trametes pubescens CBS No. 100232, aa 24, vote weight 1.0 (WO 98/28409)
- phyA from Agrocybe pediades CBS No. 900.96, aa 19, vote weight 1.0 (WO 98/28409)
- phyA from Peniophora lycii CBS No. 686.96, aa 21, vote weight 1.0 (WO 98/28409)

The alignment is shown in Figure 3.

In Table 3 the genes that were used for the final alignment are listed. The first amino acid (aa) of the sequence that is used in the alignment is mentioned behind the organism's designation.

### Table 3

### Origin and vote weight of the phytase sequences used for the design of consensus phytase-10

- phyA from Aspergillus terreus 9A-1, aa 27, vote weight 0.5 (Mitchell et al., 1997)
- phyA from Aspergillus terreus cbs116.46, aa 27, vote weight 0.5 (EP 897985)
- phyA from Aspergillus niger var. awamori, aa 27, vote weight 0.5 (Piddington et al., 1993)
- phyA from Aspergillus niger strain NRRL3135, aa 27, vote weight 0.5 (van Hartingsveldt et al., 1993)
- phyA from Aspergillus fumigatus ATCC 13073, aa 26, vote weight 0.2 (Pasamontes et al., 1997)
- phyA from Aspergillus fumigatus ATCC 32722, aa 26, vote weight 0.2 (EP 897985)
- phyA from Aspergillus fumigatus ATCC 58128, aa 26, vote weight 0.2 (EP 897985)
- phyA from Aspergillus fumigatus ATCC 26906, aa 26, vote weight 0.2 (EP 897985)
- phyA from Aspergillus fumigatus ATCC 32239, aa 30, vote weight 0.2 (EP 897985)
- phyA from Emericella nidulans , aa 25, vote weight 1.0 (Pasamontes et al., 1997a)
- phyA from Talaromyces thermophilus ATCC 20186, aa 24, vote weight 1.0 (Pasamontes et al., 1997a)
- phyA from Myceliophthora thermophila, aa 19, vote weight 1.0 (Mitchell et al., 1997)
- phyA from Thermomyces lanuginosus, aa 36, vote weight 1.0 (Berka et al., 1998)
- Consensus sequence of five Basidiomycete phytases, vote weight 1.0 (Basidio, Figure 3)

The corresponding alignment is shown in Figure 4.

### Calculation of the amino acid sequence of consensus phytase-10

To improve the alignment, we added the original consensus sequence of five phytases from four different Basidiomycetes (called Basidio; still containing the undefined sequence positions; see Figure 3), nearly all phytase sequences used for the calculation of the original consensus phytase sequences and one new phytase sequence from the Ascomycete Thermomyces lanuginosus to a larger alignment.

We set plurality on 2.0 and threshold on 3. The used vote weights are listed in Table 3. The alignment and the corresponding consensus sequence are presented in Figure 4. The new consensus phytase sequence has 32 different amino acids in comparison to the original consensus phytase-1. Positions for which the program PRETTY was not able to calculate a consensus amino acid residue were filled according to rules mentioned in Example 1. None of the residues suggested by the program was replaced.

Furthermore, in another calculation, we included all Basidiomycete phytases as single amino acid sequences but assigning a vote weight of 0.2 in the calculation. The corresponding alignment is shown in Figure 6. The calculated consensus amino acid sequence (consensus phytase-11) has the following differences to the sequence of consensus phytase-10. Letter X means that the program was not able to calculate a consensus amino acid; the amino acid in parenthesis corresponds to the amino acid finally included into consensus phytase-10.

D35X (first letter for consensus phytase-10, last letter for consensus phytase-11), X(K)69K, X(E)100E, A101R, Q134N, X(K)153N, X(H)190H, X(A)204S, X(E)220D, E222T, V227A, X(R)271R, H287A, X(D)288D, X(K)379K, X(I)389I, E390X, X(E)415E, X(A)416A, X(R)446L, E463A. The numbering is as in Fig. 5.

We also checked single amino acid replacements suggested by the improved consensus sequences 10 and 11 on their influence on the stability of the original consensus phytase-1. The approach is described in example 3.

### Conversion of the consensus phytase-10 amino acid sequence into a DNA sequence

The first 26 amino acid residues of A. terreus cbs116.46 phytase were used as signal peptide and fused to the N-terminus of consensus phytase-10. The used procedure is further described in Example 1.

The resulting sequence of the fcp10 gene is shown in Figure 5.

### Construction and cloning of the consensus phytase-10 gene (fcp10)

The calculated DNA sequence of fcp10 was divided into oligonucleotides of 85 bp, alternately using the sequence of the sense and the anti-sense strand. Every oligonucleotide overlaps 20 bp with the previous and the following oligonucleotide of the opposite strand. The location of all primers, purchased from Microsynth, Balgach (Switzerland) and obtained in a PAGE-purified form, is indicated in Figure 5.

### PCR-Reactions

In three PCR reactions, the synthesized oligonucleotides were composed to the entire gene. For the PCR, the High Fidelity Kit from Boehringer Mannheim (Boehringer Mannheim, Mannheim, Germany) and the thermo cycler "The ProtokolTM" from AMS Biotechnology (Europe) Ltd. (Lugano, Switzerland) were used. The following oligonucleotides were used in a concentration of 0.2 pMol/ml.

| | |
|---|---|
| Mix 1.10: | CP-1, CP-2, CP-3.10, CP-4.10, CP-5.10, CP-6, CP-7.10, CP-8.10, CP-9.10, CP-10.10 |
| Mix 2.10: | CP-9.10, CP-11.10, CP-12.10, CP-13.10, CP-14.10, CP-15.10, CP-16.10, CP-17.10, CP18.10, CP-19.10, CP-20.10, CP-21.10, CP-22.10 |

The newly synthesized oligonucleotides are marked by the number 10. Consensus phytase-10 contains the following 32 exchanges, which are underlined in Figure 5, in comparison to the original consensus phytase-1: Y54F, E58A, D69K, D70G, A94K, N134Q, I158V, S187A, Q188N, D197N, S204A, T214L, D220E, L234V, A238P, D246H, T251N, Y259N, E267D, E277Q, A283D, R291I, A320V, R329H, S364T, 1366V, A379K, S396A, G404A, Q415E, A437G, A463E.

Four short PCR primers were used for the assembling of the oligonucleotides:
CP-a: *Eco RI*
   5'-TATAT*GAATTC*ATGGGCGTGTTCGTC-3' (SEQ ID NO: 37)
CP-b:
   5'-TGAAAAGTTCATTGAAGGTTTC-3' (SEQ ID NO: 38)
CP-c.10:
   5'-TCTTCGAAAGCAGTACACAAAC-3' (SEQ ID NO: 39)
CP-e: *Eco RI*
   5'-TATAT*GAATTC*TTAAGCGAAAC-3' (SEQ ID NO: 40)

| | |
|---|---|
| PCR reaction a: | 10 µl Mix 1.10 (2.0 pmol of each oligonucleotide) |
| | 2 µl nucleotides (10 mM of each nucleotide) |
| | 2 µl primer CP-a (10 pmol/ml) |
| | 2 µl primer CP-c.10 (10 pmol/ml) |
| | 10,0 µl PCR buffer |
| | 0.75 µl polymerase mixture (2.6 U) |
| | 73.25 µl H₂O |
| PCR reaction b: | 10 µl Mix 2.10 (2.0 pmol of each oligonucleotide) |
| | 2 µl nucleotides (10 mM each nucleotide) |
| | 2 µl primer CP-b (10 pmol/ml) |
| | 2 µl primer CP-e (10 pmol/ml) |
| | 10,0 µl PCR buffer |
| | 0.75 µl polymerase mixture (2.6 U) |
| | 73.25 µl H₂O |

| Reaction conditions for PCR reactions a and b: | |
|---|---|
| step 1 | 2 min - 45°C |
| step 2 | 30 sec - 72°C |
| step 3 | 30 sec - 94°C |
| step 4 | 30 sec - 52°C |
| step 5 | 1 min - 72°C |

Steps 3 to 5 were repeated 40-times.

The PCR products (670 and 905 bp) were purified by agarose gel electrophoresis (0.9% agarose), followed by gel extraction (QIAEX II Gel Extraction Kit, Qiagen, Hilden, Germany). The purified DNA fragments were used for the PCR reaction c.

| | |
|---|---|
| PCR reaction c: | 6 µl PCR product of reaction a ≈50 ng) |
| | 6 µl PCR product of reaction b ≈50 ng) |
| | 2 µl primer CP-a (10 pmol/ml) |
| | 2 µl primer CP-e (10 pmol/ml) |
| | 10,0 µl PCR buffer |
| | 0.75 µl polymerase mixture (2.6 U) |
| | 73.25 µl H₂O |

| Reaction conditions for PCR reaction c: | |
|---|---|
| step 1 | 2 min - 94°C |
| step 2 | 30 sec - 94°C |
| step 3 | 30 sec - 55°C |
| step 4 | 1 min - 72°C |

Steps 2 to 4 were repeated 31-times.

The resulting PCR product (1.4 kb) was purified as mentioned above, digested with EcoRI, and ligated in an EcoRI-digested and dephosphorylated pBsk(-)-vector (Stratagene, La Jolla, CA, USA). 1 µl of the ligation mixture was used to transform E. coli XL-1 competent cells (Stratagene, La Jolla, CA, USA). All standard procedures were carried out as described by Sambrook et al. (1987). The DNA sequence of the constructed gene (fcp10) was checked by sequencing as known in the art.

### Example 3

### Increasing the thermostability of consensus phytase-1 by introduction of single mutations suggested by the amino acid sequences of consensus phytase-10 and consensus phytase-11

In order to increase the thermostability of homologous genes, it is also possible to test the stability effect of each differing amino acid residue between the protein of interest and the calculated consensus sequence and to combine all stabilizing mutations into the protein of interest. We used the consensus phytase-1 as protein of interest and tested the effect on the protein stability of 34 amino acid residues that differ relative to consensus phytase-10 and/or -11 by single site-directed mutagenesis.

To construct muteins for expression in A. niger, S. cerevisiae, or H. polymorpha, the corresponding expression plasmid containing the consensus phytase-1 gene was used as template for site-directed mutagenesis (see Examples 6-8). Mutations were introduced using the "quick exchangeTM site-directed mutagenesis kit" from Stratagene (La Jolla, CA, USA) following the manufacturer's protocol and using the corresponding primers. All mutations made and the corresponding primers are summarized in Table 4. Plasmids harboring the desired mutation were identified by DNA sequence analysis as known in the art.

### Table 4

### Primers used for site-directed mutagenesis of consensus phytase-1

Exchanged bases are highlighted in bold. The introduction of a restriction site is marked above the sequence. When a restriction site is written in parenthesis, the mentioned site was destroyed by introduction of the mutation.

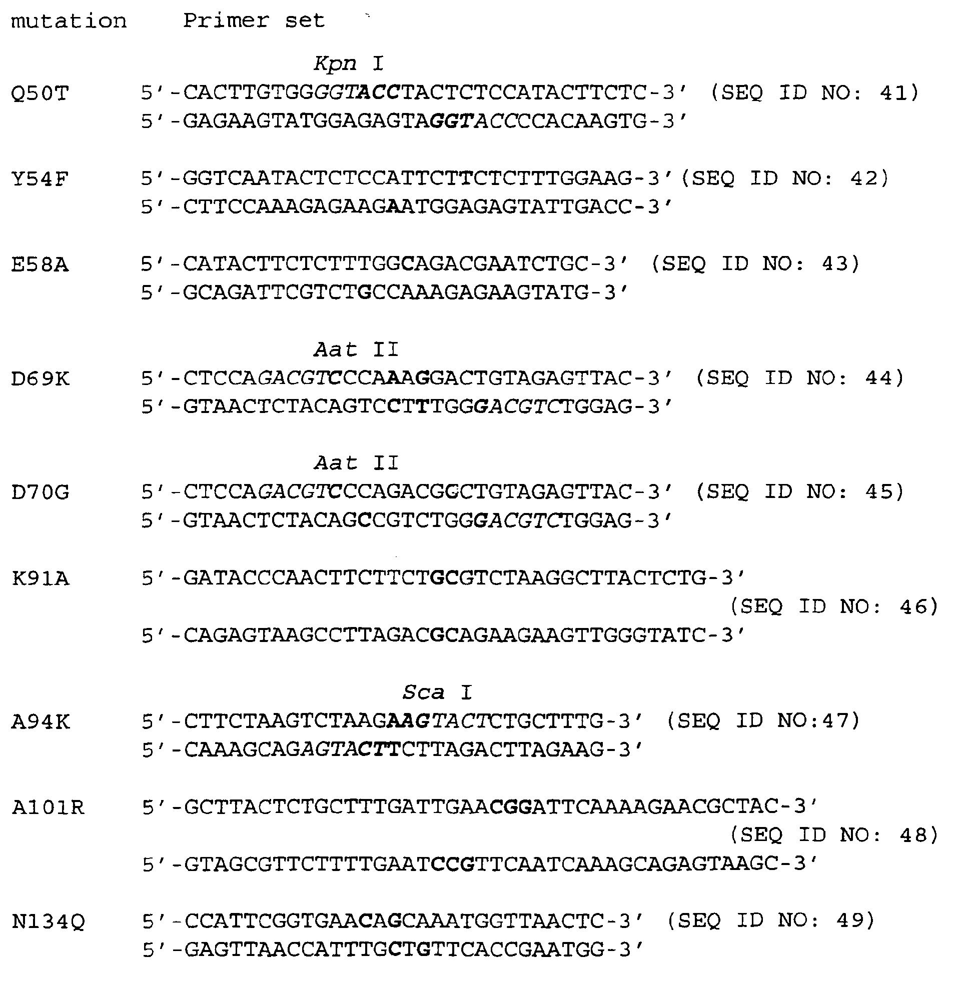

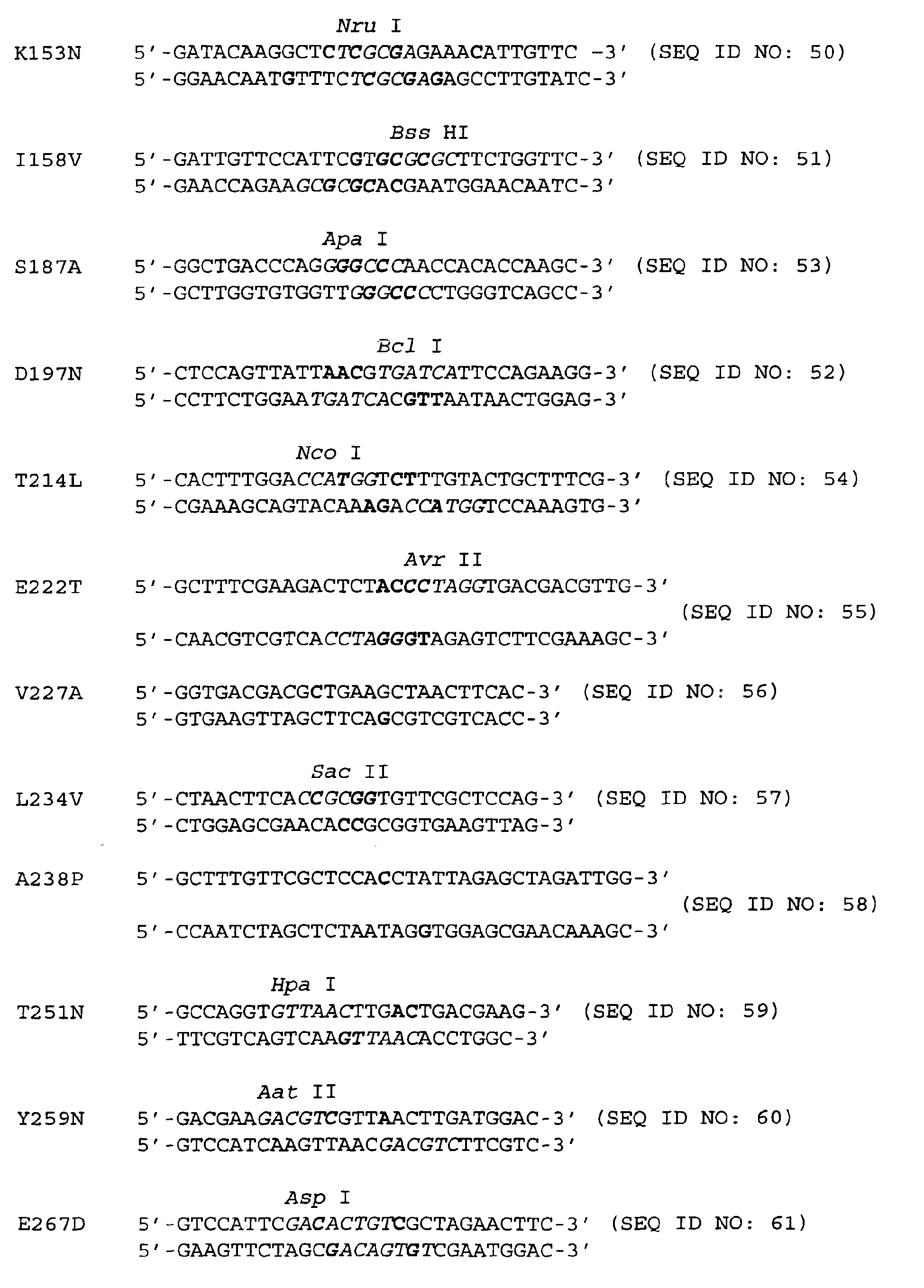

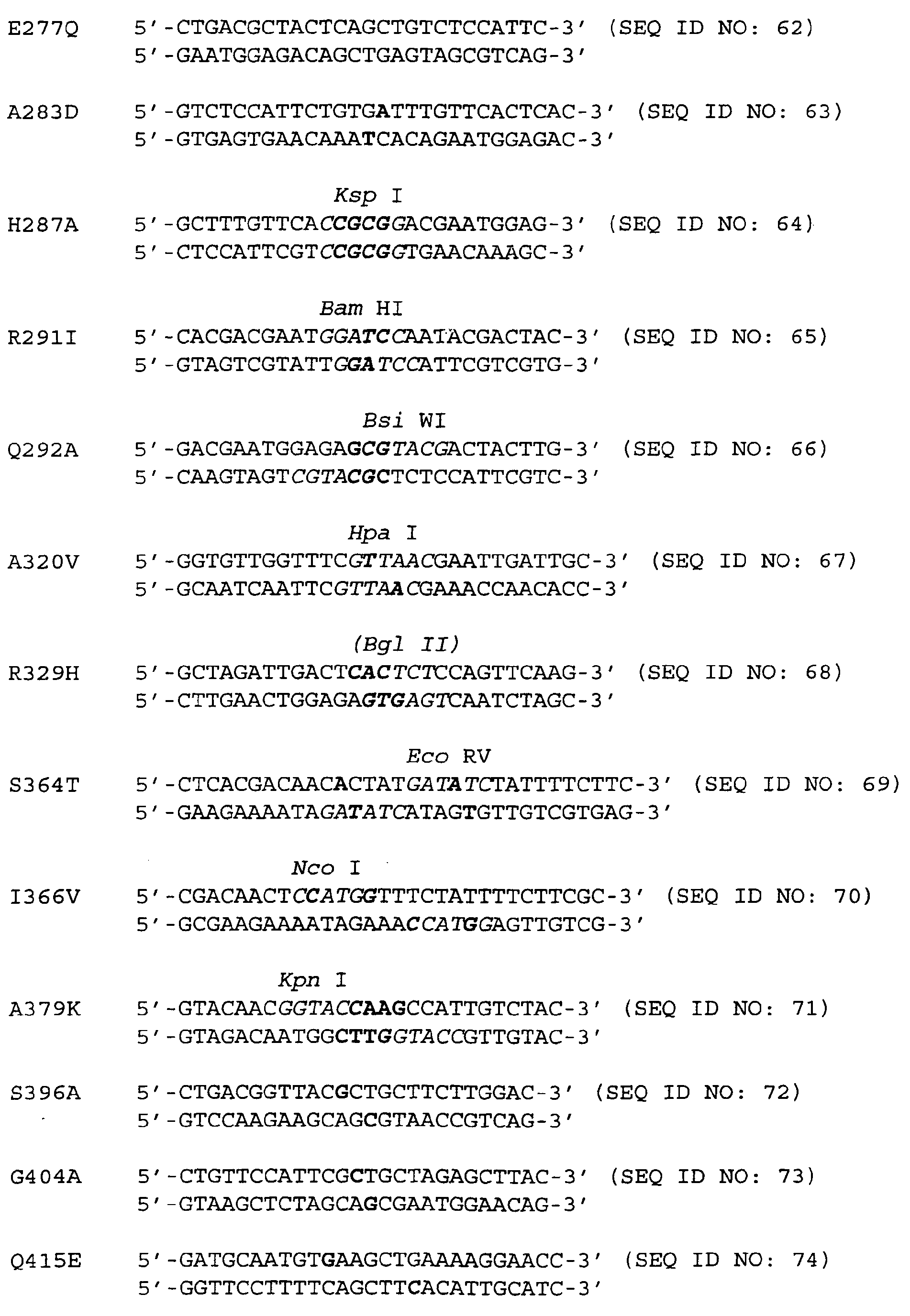

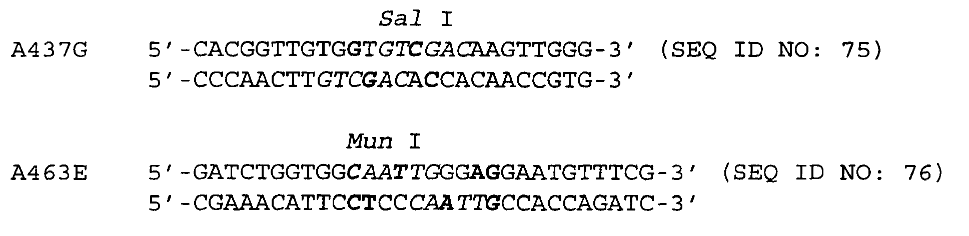

and, accordingly, for other mutations.

The temperature optimum of the purified phytases, expressed in Saccharomyces cerevisiae (Example 7), was determined as outlined in Example 9. Table 5 shows the effect of each mutation introduced on the stability of consensus phytase-1.

### Table 5

### Stability effect of the individual amino acid replacements in consensus phytase-1

+ or - means a positive, respectively, negative effect on the protein stability up to 1°C, ++ and -- means a positive, respectively, negative effect on the protein stability between 1 and 3°C; the numbers 10 or 11 in parentheses indicate the consensus phytase sequence that suggested the amino acid replacement.

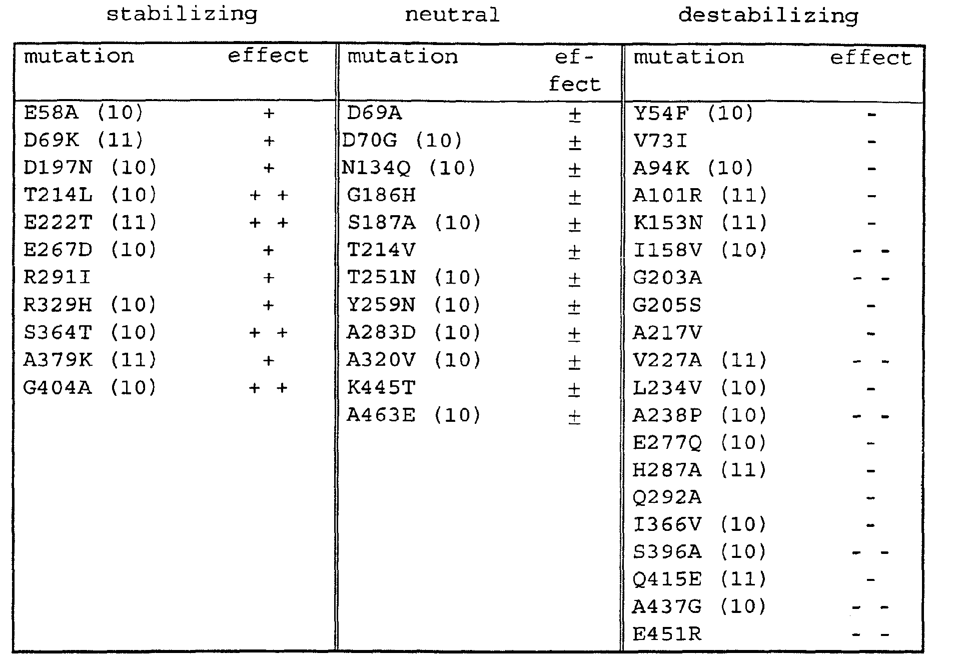

We combined eight positive mutations (E58A, D197N, E267D, R291I, R329H, S364T, A379K, G404A) in consensus phytase-1 thermo[8], using the primers and the technique mentioned above in this example. Furthermore, the mutations Q50T and/or K91A were introduced which mainly influence the catalytic characteristics of phytase (see patent applications EP 897010 and EP 897985, as well as Example 9). The DNA and amino acid sequence of the resulting phytase (consensus phytase-1-thermo[8]-Q50T-K91A) are shown in Figure 7. In this way, the temperature optimum and the melting point of the consensus phytase were increased by 7°C (Figures 15, 16, 17).

In a further consensus protein, we combined eleven positive mutations (E58A, D69K, D197N, T214L, E222T, E267D, R291I, R329H, S364T, A379K, G404A) in consensus phytase-1 thermo[11]. Furthermore, the mutations Q50T and/or K91A were introduced. In this way, the melting temperature was increased by another 3-4°C when compared to consensus phytase-1 thermo[8].

Using the results of Table 5, we further improved the thermostability of consensus phytase-10 by the back mutations K94A, V158I, and A396S, the reverse of which (A94K, I158V, and S396A) revealed a strong negative influence on the stability of consensus phytase-1. The resulting protein was called consensus phytase-10-thermo[3]. SEQ ID NO: 26 plus the three mutations K94A, V158I, and A396S. Furthermore, we introduced the mutations Q50T and K91A that mainly influence the catalytic characteristics of consensus phytase (see patent applications EP 897010 and EP 897985, as well as Example 9 and Figures 14 and 15). The resulting DNA and amino acid sequence are shown in Figure 8. The optimized phytase showed a 4°C higher temperature optimum and melting point than consensus phytase-10 (Figures 12 and 13). Furthermore, the phytase has also a strongly increased specific activity with phytate as substrate of 250 U/mg at pH 5.5 (Figure 14).

In a still further consensus protein, two additional mutations were introduced into consensus phytase-10 thermo[3] (E222T, G437A) which yielded consensus phytase-10 thermo[5]. Furthermore, the mutations Q50T and/or K91A were introduced. In this way, the melting temperature was increased by another 1-2°C when compared to consensus phytase-10 thermo[3].

### Example 4

### Stabilization of the phytase of A. fumigatus ATCC 13073 by replacement of amino acid residues with the corresponding consensus phytase-1 and/or consensus phytase-10 residues

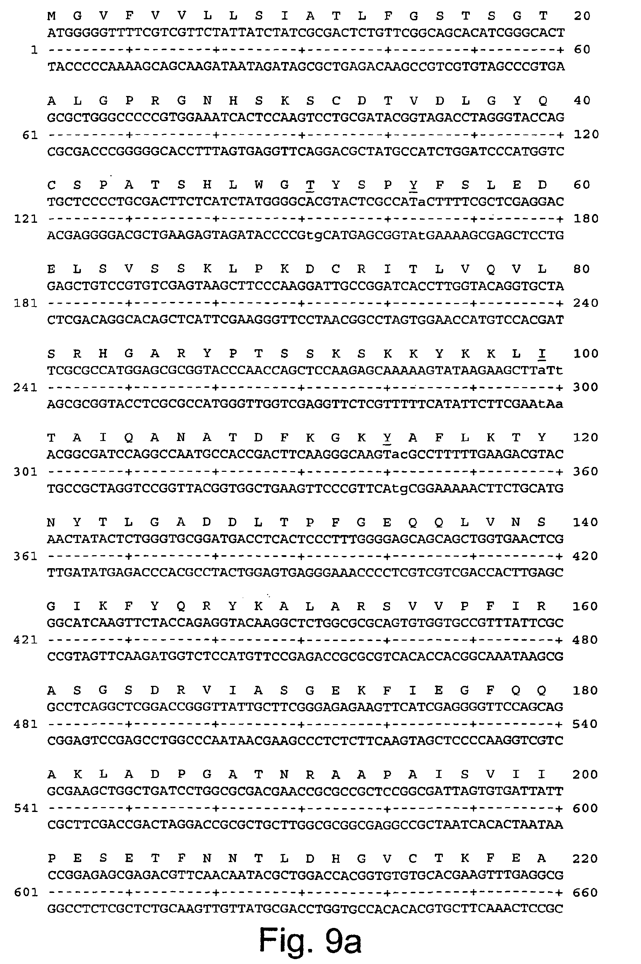

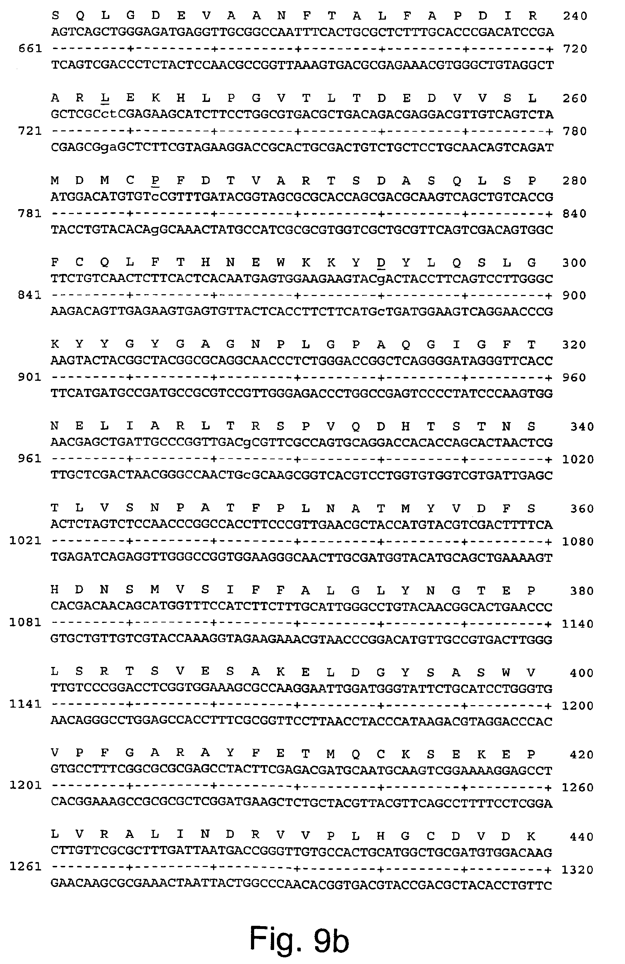

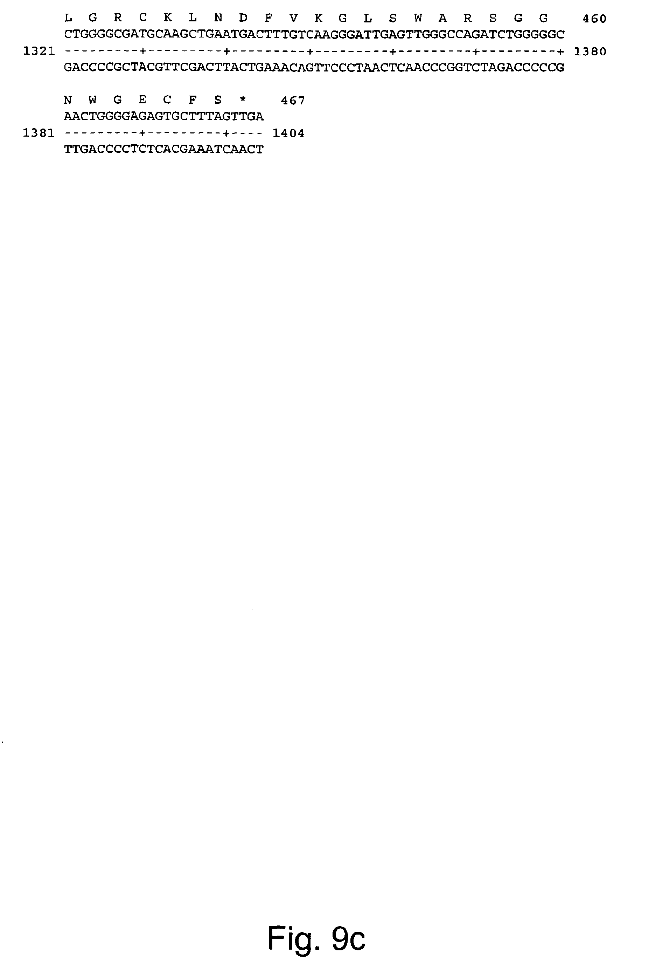

At six amino acid sequence positions where A. fumigatus 13073 phytase is the only or nearly the only phytase in the alignment of Figure 1 that does not contain the corresponding consensus phytase amino acid residue, the non-consensus amino acid residue was replaced by the consensus one. The following amino acids were substituted in A. fumigatus 13073 phytase, containing additionally the Q51(24)T substitution (influencing the catalytic properties and corresponding to the Q50T substitution in the consensus phytases) and the signal sequence of A. terreus cbs116.46 phytase (see European Patent Application No. 0897010, and Figure 9): F55(28)Y, V100(73)I, F114(87)Y, A243(220)L, S265(242)P, N294(282)D. The numbers in parentheses refer to the numbering in Figure 1.

In a second round, four of the seven stabilizing amino acid exchanges (E58A, R329H, S364T, G404A) identified in consensus phytase-10 and tested as single mutations in consensus phytase-1 (Table 5) were additionally introduced into the A. fumigatus alpha-mutant. Furthermore, the amino acid replacement S154N, shown to reduce the protease susceptibility of the phytase, was introduced.

The mutations were introduced as described in Example 3 (see Table 6) and expressed as described in Examples 6 to 8. The resulting A. fumigatus 13073 phytase variants were called alpha-mutant (i.e. the A. fumigatus ATCC 13073 phytase with the substitutions Q24T, F28Y, V73I, F87Y, A220L, S242P, N282D) and "optimized" alpha-mutant (i.e. the A. fumigatus alpha-mutant having the additional substitutions E59A-S154N-R329H-S364T-G404A). K92A is an additional preferred mutation.

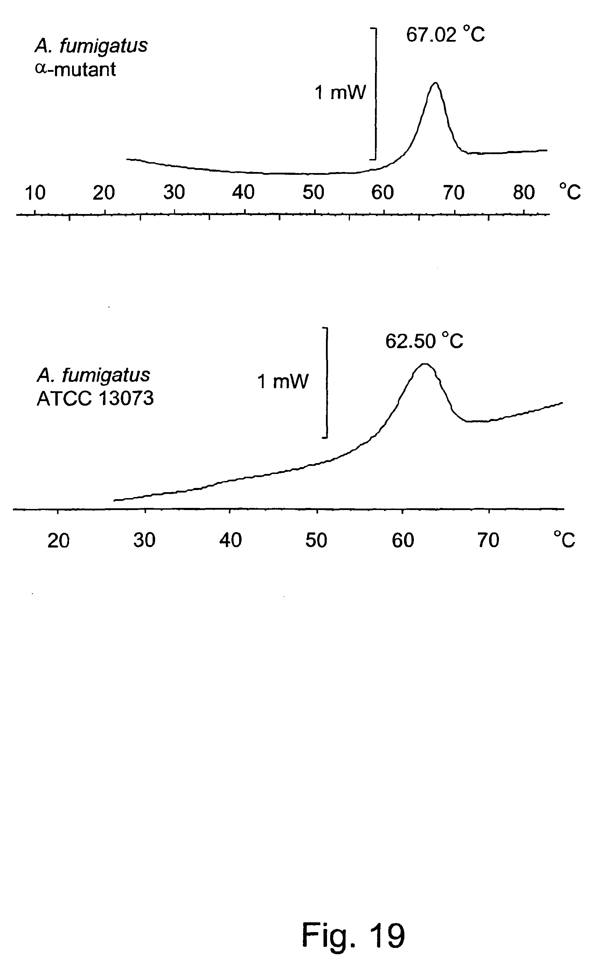

The temperature optimum (60°C, Figure 20) and the melting temperature (67.0°C, Figure 19) of the A. fumigatus 13073 alpha-mutant phytase were increased by 5-7°C in comparison to the values of the wild-type phytase (temperature optimum: 55°C, Tm: 60°C). The five additional amino acid replacements further increased the temperature optimum by 3°C (Figure 20).

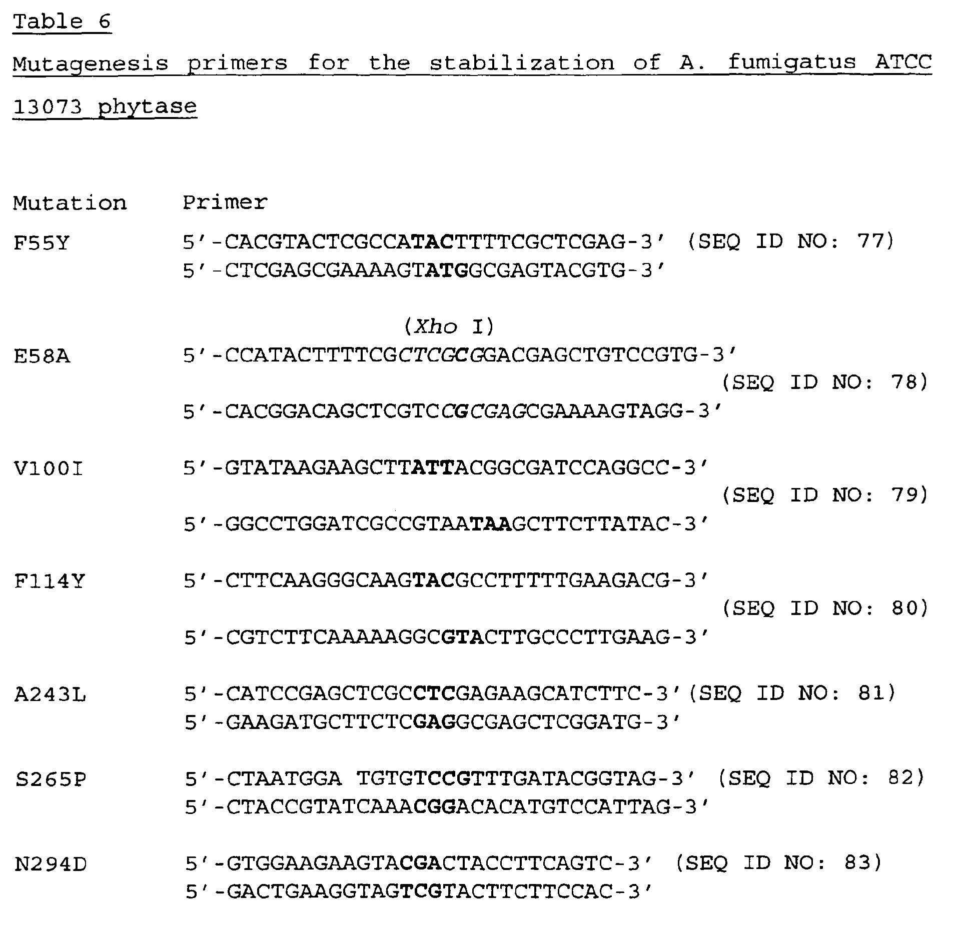

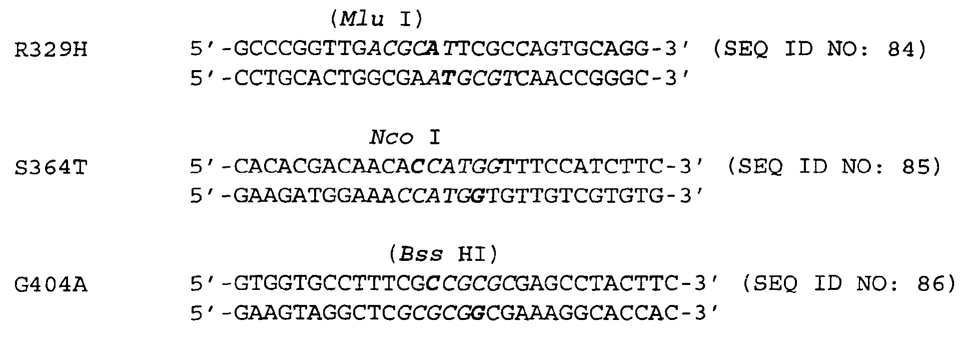

### Example 5

### Introduction of the active site amino acid residues of A. niger NRRL 3135 phytase into consensus phytase-1

We used the crystal structure of Aspergillus niger NRRL 3135 phytase to define all active site amino acid residues (see Example 1, and EP 897010). Using the alignment of Figure 1, we replaced the following active site residues and additionally the non-identical adjacent ones of consensus phytase-1 by those of A. niger phytase:

S89D, S92G, A94K, D164S, P201S, G203A, G205S, H212P, G224A, D226T, E255T, D256E, V258T, P265S, Q292H, G300K, Y305H, A314T, S364G, M365I, A397S, S398A, G404A, and A405S.

The new consensus phytase-7 protein sequence was backtranslated into a DNA sequence (Figure 10) as described in Example 1. The corresponding gene (fcp7) was generated as described in Example 1 using the following oligonucleotide mixes:

| | |
|---|---|
| Mix 1.7: | CP-1, CP-2, CP-3, CP-4.7, CP-5.7, CP-6, CP-7, CP-8.7, CP-9, CP-10.7 |
| Mix 2.7: | CP-9, CP-10.7, CP-11.7, CP-12.7, CP-13.7, CP-14.7, CP-15.7, CP-16, CP-17.7, CP-18.7, CP-19.7, CP-20, CP-21, CP-22. |

The DNA sequences of the oligonucleotides are indicated in Figure 10. The newly synthesized oligonucleotides are additionally marked by the number 7. After assembling of the oligonucleotides using the same PCR primers as mentioned in Example 1, the gene was cloned into an expression vector as described in Examples 6-8.

The pH-profile of the enzyme determined after expression in H. polymorpha and purification was very similar to that of A. niger phytase (see Figure 18).

### Example 6

### Expression of the consensus phytase genes in Hansenula polymorpha

The phytase expression vectors used to transform H. polymorpha RB11 [Gellissen, G., Hollenberg, C. P., Janowicz, Z. A. (1994) Gene expression in methylotrophic yeasts, in Smith, A. (ed.) Gene expression in recombinant microorganisms. Dekker, New York, pp. 395-439] were constructed by inserting the Eco RI fragment of pBsk-fcp or variants thereof into the multiple cloning site of the H. polymorpha expression vector pFPMT121, which is based on an ura3 selection marker from S. cerevisiae, a formate dehydrogenase (FMD) promoter element and a methanol oxidase (MO) terminator element from H. polymorpha. The 5' end of the fcp gene is fused to the FMD promoter, the 3' end to the MOX terminator (Gellissen et al., Appl. Microbiol. Biotechnol. 46, 46-54, 1996; EP 299108). The resulting expression vectors are designated pFPMTfcp, pFPMTfcp10, and pFPMTfcp7.

The constructed plasmids were propagated in E. coli. Plasmid DNA was purified using standard state of the art procedures. The expression plasmids were transformed into the H. polymorpha strain RB11 deficient in orotidine-5'-phosphate decarboxylase (ura3) using the procedure for preparation of competent cells and for transformation of yeast as described in Gellissen et al. (1996). Each transformation mixture was plated on YNB medium (0.14% w/v Difco YNB and 0.5% ammonium sulfate) containing 2% glucose and 1.8% agar, and incubated at 37 °C. After 4 to 5 days individual transformant colonies were picked and grown in the liquid medium described above for 2 days at 37 °C. Subsequently, an aliquot of this culture was used to inoculate fresh vials with YNB-medium containing 2% glucose. After seven further passages in selective medium, the expression vector had integrated into the yeast genome in multimeric form. Subsequently, mitotically stable transformants were obtained by two additional cultivation steps in 3 ml non-selective liquid medium (YPD, 2% glucose, 10 g/l yeast extract, and 20 g/l peptone). In order to obtain genetically homogeneous recombinant strains, an aliquot from the last stabilization culture was plated on a selective plate. Single colonies were isolated for analysis of phytase expression in YNB containing 2% glycerol instead of glucose to derepress the FMD promoter. Purification of the consensus phytases was done as described in Example 7.

### Example 7

### Expression of the consensus phytase genes in Saccharomyces cerevisiae and purification of the phytases from the culture supernatant

The consensus phytase genes were isolated from the corresponding Bluescript-plasmid (pBsk-fcp, pBSK-fcp10, pBsk-fcp7) and ligated into the Eco RI sites of the expression cassette of the Saccharomyces cerevisiae expression vector pYES2 (Invitrogen, San Diego, CA, USA) or subcloned between the shortened GAPFL (glyceraldhyde-3-phosphate dehydrogenase) promoter and the pho5 terminator as described by Janes et al., Curr. Genet. 18, 97-103. The correct orientation of the gene was checked by PCR. Transformation of S. cerevisiae strains, e.g. INVScl (Invitrogen, San Diego, CA, USA), was done according to Hinnen et al., Proc. Natl. Acad. Sci. USA 75, 1929-1933 (1978). Single colonies harboring the phytase gene under the control of the GAPFL promoter were picked and cultivated in 5 ml selection medium [SD-uracil; Sherman, J. P., Finck, G. R. & Hicks, J. B. (1986) Laboratory course manual for methods in yeast genetics. Cold Spring Harbor University] at 30°C under vigorous shaking (250 rpm) for one day. The preculture was then added to 500 ml YPD medium (Sherman et al., 1986) and grown under the same conditions. Induction of the gal1 promoter was done according to the manufacturer's instructions. After four days of incubation, the cell broth was centrifuged (7000 rpm, GS3 rotor, 15 min, 5°C) to remove the cells, and the supernatant was concentrated by way of ultrafiltration in Amicon 8400 cells (PM30 membranes; Grace AG, Wallizeller, Switzerland) and ultrafree-15 centrifugal filter devices (Biomax-30K, Millipore, Bedford, MA, USA). The concentrate (10 ml) was desalted on a 40 ml Sephadex G25 Superfine column (Pharmacia Biotech, Freiburg, Germany), with 10 mM sodium acetate, pH 5.0, serving as elution buffer. The desalted sample was brought to 2 M (NH₄)₂SO₄ and directly loaded onto a 1 ml Butyl Sepharose 4 Fast Flow hydrophobic interaction chromatography column (Pharmacia Biotech, Feiburg, Germany) which was eluted with a linear gradient from 2 M to 0 M (NH₄)₂SO₄ in 10 mM sodium acetate, pH 5.0. Phytase was eluted in the breakthrough, concentrated and loaded on a 120 ml Sephacryl S-300 gel permeation chromatography column (Pharmacia Biotech, Freiburg, Germany). Consensus phytases -1, -7 and -10 eluted as a homogeneous symmetrical peak and were shown by SDS-PAGE to be approx. 95% pure.

### Example 8

### Expression of the consensus phytase genes in Aspergillus niger

The Bluescript-plasmids pBsk-fcp, pBsk-fcp10, and pBsk-fcp7 were used as template for the introduction of a Bsp HI-site upstream of the start codon of the genes and an Eco RV-site downstream of the stop codon. The ExpandTM High Fidelity PCR Kit (Boehringer Mannheim, Mannheim, Germany) was used with the following primers:
Primer Asp-1:
   *Bsp HI*
   5'-TATA*TCATGA*GCGTGTTCGTCGTGCTACTGTTC-3' (SEQ ID NO: 87)
Primer Asp-2 used for cloning of fcp and fcp7:
   *Eco RV*
   3'-ACCCGACTTACAAAGCGAATT*CTATAG*ATATAT-5' (SEQ ID NO: 88)
Primer Asp-3 used for cloning of fcp10:
   *Eco RV*
   3'-ACCCTTCTTACAAAGCGAATT*CTATAGA*TATAT-5' (SEQ ID NO: 89)

The reaction was performed as described by the supplier. The PCR-amplified fcp-genes had a new Bsp HI site at the start codon, introduced by primer Asp-1, which resulted in a replacement of the second amino acid residue glycine by serine. Subsequently, the DNA-fragment was digested with Bsp HI and Eco RV and ligated into the Nco I site downstream of the glucoamylase promoter of Aspergillus niger (glaA) and the Eco RV site upstream of the Aspergillus nidulans tryptophan C terminator (trpC) (Mullaney et al., 1985). After this cloning step, the genes were sequenced to detect possible errors introduced by PCR. The resulting expression plasmids, which basically correspond to the pGLAC vector as described in Example 9 of EP 684313, contained the orotidine-5'-phosphate decarboxylase gene (pyr4) of Neurospora crassa as a selection marker. Transformation of Aspergillus niger and expression of the consensus phytase genes was done as described in EP 684313. The consensus phytases were purified as described in Example 7.

### Example 9

### Determination of phytase activity and of the pH and temperature optima

This example relates i.a. to the determination of phytase activity and of the temperature optimum. Various phytases have been tested.

The phytase of Aspergillus niger NRRL 3135 was prepared as described in EP 420358 and by van Hartingsveldt et al. (Gene 127, 87-94, 1993).

The phytases of Aspergillus fumigatus ATCC 13073, Aspergillus terreus 9A-1, Aspergillus terreus cbs116.46, Emericella nidulans, Myceliophthora thermophila, and Talaromyces thermophilus were prepared as described in EP-0897985 and in the references therein.

The remaining phytases tested were prepared as described herein.

Consensus phytase-1-thermo(8) designates a variant of consensus phytase-1, which further comprises the eight mutations which are underlined in the legend to Figure 5. Consensus phytase-1 is shown in Fig. 1 (SEQ ID NO: 14) without signal peptide, and in Fig. 2 (SEQ ID NO: 16) with the signal peptide.

Phytase activity was determined basically as described by Mitchell et al. (1997). The activity was measured in an assay mixture containing 0.5% phytic acid (≈5 mM) in 200 mM sodium acetate, pH 5.0. After 15 min of incubation at 37°C, the reaction was stopped by addition of an equal volume of 15% trichloroacetic acid. The liberated inorganic phosphate was quantified by mixing 100 µl of the assay mixture with 900 µl H₂O and 1 ml of 0.6 M H₂SO₄, 2% ascorbic acid and 0.5% ammonium molybdate. Standard solutions of potassium phosphate were used as reference. One unit of enzyme activity was defined as the amount of enzyme that releases 1 µmol phosphate per minute at 37°C. The protein concentration was determined using the enzyme extinction coefficient at 280 nm calculated according to Pace et al. [Pace N. C., Vajdos, F., Fee, L., Grimsley, G. & Gray, T. (1995) How to measure and predict the molar absorption coefficient of a protein. Prot. Sci. 4, 2411-2423]: 1 absorption unit (1 OD) at 280 nm corresponds to 1.101 mg/ml of consensus phytase-1, 1.068 mg/ml of consensus phytase-7, and 1.039 mg/ml of consensus phytase-10.

In case of pH-optimum curves, the purified enzymes were diluted in 10 mM sodium acetate, pH 5.0. Incubations were started by mixing aliquots of the diluted protein with an equal volume of 1% phytic acid (≈10 mM) in a series of different buffers: 0.4 M glycine/HCl, pH 2.5; 0.4 M acetate/NaOH, pH 3.0, 3.5, 4.0, 4.5, 5.0, 5.5; 0.4 M imidazole/HCl, pH 6.0, 6.5; 0.4 M Tris/HCl pH 7.0, 7.5, 8.0, 8.5, 9.0. Control experiments showed that pH was only slightly affected by the mixing step. Incubations were performed for 15 min at 37°C as described above.

For determination of the substrate specificities of the phytases, phytic acid in the assay mixture was replaced by 5 mM concentrations of the respective phosphate compounds. Besides, the activity tests were performed as described above.

For determination of the temperature optimum, enzyme (100 µl) and substrate solution (100 µl) were pre-incubated for 5 min at the given temperature. The reaction was started by addition of the substrate solution to the enzyme. After 15 min of incubation, the reaction was stopped with trichloroacetic acid, and the amount of phosphate released was determined.

The pH-optimum of consensus phytase-1 was around pH 6.0-6.5 (70 U/mg). Introduction of the Q50T mutation shifted the pH-optimum to pH 6.0 (130 U/mg). Introduction of the K91A mutation further shifted the pH optimum into the more acidic pH-range. Comparable effects of the Q50T and K91A mutations were also observed for consensus phytase-10 and for further stabilized consensus phytase variants (Figures 14 and 15).

Consensus phytase-7, which was constructed to transfer the catalytic characteristics of A. niger NRRL 3135 phytase to consensus phytase-1, had a pH-profile very similar to that of A. niger NRRL 3135 phytase (see Figure 18). The substrate specificity also resembled more that of A. niger NRRL 3135 phytase than that of consensus phytase-1.

The temperature optimum of consensus phytase-1 (71°C) was 16-26°C higher than the temperature optima of the wild-type phytases (45-55°C, Table 7) that were used to calculate the consensus sequence. The improved consensus phytase-10 showed a further increase of its temperature optimum to 80°C (Figure 13).

The temperature optimum of consensus phytase-1-thermo[8] was found to be in the same range (78°C) when using the supernatant of an overproducing S. cerevisiae strain. The highest temperature optimum reached of 82°C was determined for consensus phytase-10-thermo[3]-Q50T-K91A.Table 7

### Temperature optima and Tm-values of consensus phytase and of the phytases from A. fumigatus, A. niger, E. nidulans, and M. thermophila.

The determination of the temperature optimum was performed as described in Example 9. The Tm-values were determined by differential scanning calorimetry as described in Example 10.

| **Phytase** | **Optimum temperature (°C)** | **Tm (°C)** |
|---|---|---|
| Aspergillus niger NRRL 3135 | 55 | 63.3 |
| Aspergillus fumigatus ATCC 13073 | 55 | 62.5 |
| Aspergillus terreus 9A-1 | 49 | 57.5 |
| Aspergillus terreus cbs116.46 | 45 | 58.5 |
| Emericella nidulans | 45 | 55.7 |
| Myceliophthora thermophila | 55 | - |
| Talaromyces thermophilus | 45 | - |
| Consensus phytase-10-thermo[5]-Q50T-K91A | - | 90.4 |
| Consensus-phytase-10-thermo[3]-Q50T-K91A | 82 | 89.3 |
| Consensus-phytase-10-thermo[3]-Q50T | 82 | 88.6 |
| Consensus-phytase-10 | 80 | 85.4 |
| Consensus phytase-1-thermo[11]-Q50T-K91A | - | 88.0 |
| Consensus phytase-1-thermo[11]-Q50T | - | 88.5 |
| Consensus-phytase-1-thermo[8]-Q50T-K91A | - | 85.7 |
| Consensus-phytase-1-thermo[8]-Q50T | 78 | 84.7 |
| Consensus-phytase-1-thermo[8] | 81 | - |
| Consensus-phytase-1-thermo[3] | 75 | - |
| Consensus-phytase-1-Q50T | - | 78.9 |
| Consensus-phytase-1 | 71 | 78.1 |
| Aspergillus fumigatus α-mutant Q51T | 60 | 67.0 |
| Aspergillus fumigatus α-mutant, plus mutations E59A, S154N, R329H, S364T, G404A | 63 | - |
| Aspergillus fumigatus "optimized" alpha-mutant, plus mutation K92A | 63 | - |

### Example 10

### Determination of the melting temperature by differential scanning calorimetry (DSC)

In order to determine the unfolding temperature of the phytases, differential scanning calorimetry was applied as described by Brugger et al., 1997 [Brugger, R., Mascarello, F., Augem, S., van Loon, A. P. G. M. & Wyss, M. (1997). Thermal denaturation of phytases and pH 2.5 acid phosphatase studied by differential scanning calorimetry. In The Biochemistry of phytate and phytase (eds. Rasmussen, S.K.; Raboy, V.; Dalbøge, H. and Loewus, F.; Kluwer Academic Publishers, Dordrecht, the Netherlands]. Solutions of 50-60 mg/ml of homogeneous phytase were used for the tests. A constant heating rate of 10°C/min was applied up to 90-95°C.

The determined melting points confirm the results obtained for the temperature optima (Table 7). The most stable consensus phytase designed so far is consensus phytase-10-thermo[3]-Q50T-K91A showing a melting temperature under the chosen conditions of 89.3°C. This is 26.0 to 33.6°C higher than the melting temperature of the wild-type phytases used.

### Example 11

### Transfer of basidiomycete phytase active site into consensus phytase-10-thermo[3]-Q50T-K91A

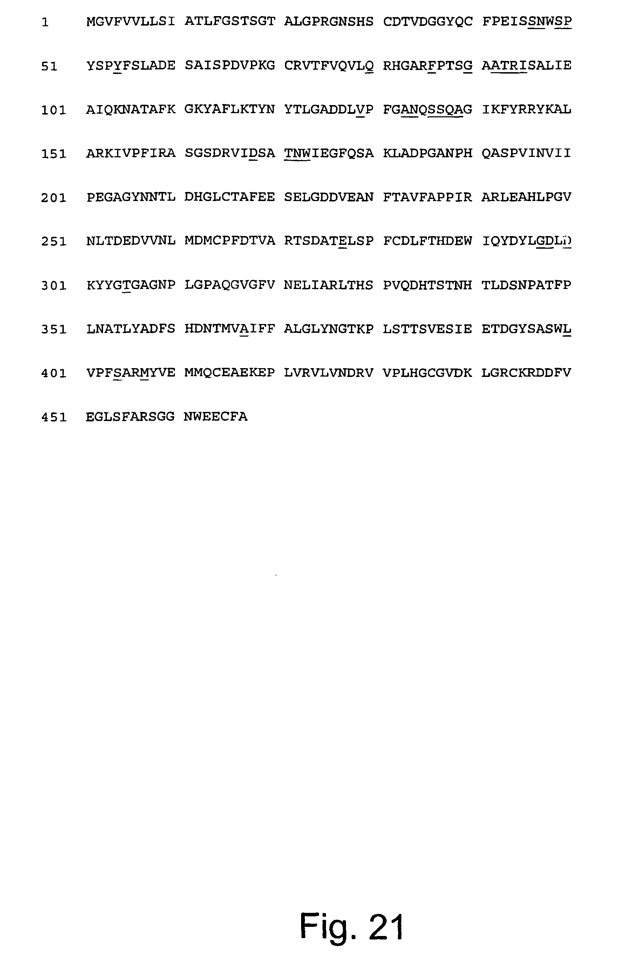

As described previously (Example 5), mutations derived from the basidiomycete phytase active sites were introduced into consensus phytase-10. The following five constructs a) to e) were prepared:
a) The construct called consensus phytase-12, and it comprises a selected number of active site residues of the basidio consensus sequence. Its amino acid sequence is shown in Fig. 21 (the first 26 amino acids form the signal peptide; positions differing from consensus phytase-10-thermo[3]-Q50T-K91A are underlined);
b) a cluster of mutations (Cluster II) was transferred to the consensus phytase-1 and -10 sequences, viz.: S80Q, Y86F, S90G, K91A, S92A, K93T, A94R, Y95I;
c) in a similar way, another cluster of mutations (Cluster III) was transferred, viz.: T129V, E133A, Q134N, M136S, V137S, N138Q, S139A;
d) in a similar way, a further cluster of mutations (Cluster IV) was transferred, viz.: A168D, E171T, K172N, F173W;
e) and finally, a further cluster of mutations (Cluster V) was transferred, viz.: Q297G, S298D, G300D, Y305T.

These constructs were expressed as described in Examples 6 to 8.

### Example 12

### Phytase alignment using GAP

The phytases described herein - i.e. the amino acid sequences as well as the corresponding DNA sequences - were aligned against each other. Also some other phytases were correspondingly aligned, viz. the following:
- the consensus phytase-1 described in EP 897985;
- the phytase derived from Aspergillus niger (ficuum) NRRL 3135 (A. niger NRRL3135) described in EP 420358;
- the phytases derived from Aspergillus fumigatus ATCC 13073 (A. fumigatus 13073); Aspergillus fumigatus ATCC 32239 (A. fumigatus 32239); Aspergillus terreus cbs116.46 (A.terreus cbs); Emericella nidulans (E. nidulans); and Talaromyces thermophilus (T. thermophilus) - all described in EP 897010;
- the phytases derived from Myceliophthora thermophila (M. thermophila); and Aspergillus terreus 9-A1 (A. terreus 9-A1) - both described in EP 684313;
- the phytase derived from Thermomyces lanuginosus (T. lanuginosus) described in WO 9735017 (PCT/US97/04559);
- the phytases derived from Agrocybe pediades (A. pediades), Paxillus involutus 1 and 2 (P. involutus phyA1 and phyA2); and Trametes pubescens (T. pubescens) - all described in WO 98/28409; and
- the phytase derived from Peniophora lycii (P. lycii) described in WO 98/28408.

For the alignments, the program GAP was used with the settings as described above.

For polypeptide comparisons, the signal peptides were included with the exception of comparisons with consensus phytase-11.

The results of the amino acid sequence comparisons are shown in Table 8 below. The first number in each cell is the amino acid similarity, the second number is the amino acid identity.

For DNA sequence comparisons, the signal sequence was always included. The results are shown in Table 9 below.

This invention discloses e.g. the following embodiments (A) to (J) that are described below.

In these embodiments, when determining % identity or % similarity at the amino acid level for another phytase, its amino acid sequence is aligned with the reference sequence (e.g. in embodiment (A) the consensus phytase-10 amino acid sequence), using an alignment program such as GAP referred to above. Percentage identity, as well as percentage similarity, is calculated by the program. The amino acid sequence of the other phytase may or may not include the signal peptide.

When determining % identity on the DNA level for another phytase-encoding DNA, this DNA sequence is aligned with the reference sequence [e.g. in embodiment (A) nucleotides 12-1412 of SEQ ID NO: 25 (the DNA sequence of consensus phytase-10 (Fcp10) as shown in Fig. 5], using an alignment program such as GAP referred to above. Percentage identity is calculated by the program. The DNA sequence encoding the other phytase can be a genomic DNA sequence including introns, or it can be a cDNA sequence. It may or may not include the signal peptide-encoding part.

When determining hybridization, the probe to be used is the specified DNA sequence [e.g. in embodiment (A) nucleotides 12-1412 of SEQ ID NO: 25 (the DNA sequence of consensus phytase-10 (Fcp10) as shown in Fig. 5)]. The DNA sequence encoding the other phytase can be a genomic DNA sample which contains a phytase-encoding DNA-sequence; a purified genomic DNA sequence (purified with respect to the phytase-encoding DNA sequence); or it can be a phytase-encoding cDNA sequence, preferably purified or amplified, e.g. PCR-amplified. The phytase-encoding DNA, whatever type, may or may not include the signal peptide-encoding part. Suitable hybridization conditions are referred to above.

The term "DNA sequence" includes such fragments or parts of the herein exemplified DNA sequences, as long as they are capable of encoding an active enzyme (e.g. phytase).

The term "amino acid sequence" includes such fragments or parts of the herein exemplified amino acid sequences, as long as they are enzymatically active (e.g. displaying phytase activity).

### (A) Phytases and corresponding DNA sequences related to consensus phytase-10 (CP10, Fcp 10)

A phytase that comprises an amino acid sequence which is at least 93.80%; or at least 94, 94.5, 95, 95.5, 96, 96.5, 97, 97.5, 98, 98.5, 99, 99.5% identical to the sequence of amino acids 1-467 of consensus phytase-10 (Fcp10) as shown in Fig. 5.

A phytase that comprises an amino acid sequence which is at least 95.09%; or at least 95.5, 96, 96.5, 97, 97.5, 98, 98.5, 99, 99.5% similar to the sequence of amino acids 1-467 of consensus phytase-10.

A phytase which is encoded by a DNA sequence which is at least 95.88%; or at least 96, 96.5, 97, 97.5, 98, 98.5, 99, 99.5% identical to nucleotides 12-1412 of the DNA sequence of consensus phytase-10 (Fcp10) as shown in Fig. 5.

A DNA sequence which encodes a phytase and which (i) is at least 95.88%; or at least 96, 96.5, 97, 97.5, 98, 98.5, 99, 99.5% identical; or (ii) hybridizes under low, or medium, medium/high, high, or very high stringency conditions to nucleotides 12-1412 of the DNA sequence of consensus phytase-10 (Fcp10) as shown in Fig. 5. A suitable negative control is DNA encoding consensus phytase-1. A suitable positive control is DNA encoding any of CP10, CP10-thermo[3]-Q50T, K91A, CP1-thermo[8], CP1-thermo[8]Q50T,K91A.

A DNA sequence which encodes a phytase comprising an amino acid sequence which is at least 93.80%; or at least 94, 94.5, 95, 95.5, 96, 96.5, 97, 97.5, 98, 98.5, 99, 99.5% identical to the sequence of amino acids 1-467 of consensus phytase-10 (Fcp10) as shown in Fig. 5.

### (B) Phytases and corresponding DNA sequences related to consensus phytase-10-thermo[3]-Q50T-K91A

A phytase which comprises an amino acid sequence which is at least 93.37%; or at least 93.5, 94, 94.5, 95, 95.5, 96, 96.5, 97, 97.5, 98, 98.5, 99, 99.5% identical to the sequence of amino acids 1-467 of consensus phytase-10-thermo[3]-Q50T-K91A as shown in Fig. 8.

A phytase which comprises an amino acid sequence which is at least 94.66%; or at least 95.0, 95.5, 96, 96.5, 97, 97.5, 98, 98.5, 99, 99.5% similar to the sequence of amino acids 1-467 of consensus phytase-10-thermo[3]-Q50T-K91A as shown in Fig. 8.

A phytase which is encoded by a DNA sequence which is at least 95.88%; or at least 96, 96.5, 97, 97.5, 98, 98.5, 99, 99.5% identical to nucleotides 12-1412 of the DNA sequence of consensus phytase-10-thermo[3]-Q50T-K91A as shown in Fig. 8.

A DNA sequence which encodes a phytase and which (i) is at least 95.88%; or at least 96, 96.5, 97, 97.5, 98, 98.5, 99, 99.5% identical; or (ii) hybridizes under low, or medium, medium/high, high, or very high stringency conditions to nucleotides 12-1412 of the DNA sequence of consensus phytase-10-thermo[3]-Q50T-K91A as shown in Fig. 8. A suitable negative control is DNA encoding consensus phytase-1. A suitable positive control is DNA encoding any of CP10, CP10-thermo[3]-Q50T-K91A, CP1-thermo[8], or CP1-thermo[8]-Q50T-K91A.

A DNA sequence which encodes a phytase comprising an amino acid sequence which is at least 93.37%; or at least 93.5, 94, 94.5, 95, 95.5, 96, 96.5, 97, 97.5, 98, 98.5, 99, 99.5% identical to the sequence of amino acids 1-467 of consensus phytase-10-thermo[3]-Q50T-K91A as shown in Fig. 8.

### (C) Phytases and corresponding DNA sequences related to consensus phytase-1-thermo[8]

A phytase which comprises an amino acid sequence which is at least 98.30%; or at least 98.5, 99, 99.5% identical to the sequence of amino acids 1-467 of consensus phytase-1-thermo[8] (as shown in Fig. 7; backmutations T50Q and A91K to be added).

A phytase which comprises an amino acid sequence which is at least 98.51%; or at least 99, 99.5% similar to the sequence of amino acids 1-467 of consensus phytase-1-thermo[8] (as shown in Fig. 7; backmutations T50Q and A91K to be added).

A phytase which is encoded by a DNA sequence which is at least 98.73%; or at least 99, 99.5% identical to nucleotides 1-1407 of the DNA sequence of consensus phytase-1-thermo[8] (as shown in Fig. 7; backmutations T50Q and A91K to be added).

A DNA sequence which encodes a phytase and which (i) is at least 98.73%; or at least 99, 99.5% identical; or (ii) hybridizes under low, or medium, medium/high, high, or very high stringency conditions to nucleotides 1-1407 of the DNA sequence of consensus phytase-1-thermo[8] (as shown in Fig. 7; backmutations T50Q and A91K to be added). A suitable negative control is DNA encoding consensus phytase-1. A suitable positive control is DNA encoding any of CP1-thermo[8], CP1-thermo[8]-Q50T-K91A.

A DNA sequence which encodes a phytase comprising an amino acid sequence which is at least 98.30%; or at least 98.5, 99, 99.5% identical to the sequence of amino acids 1-467 of consensus phytase-1-thermo[8] (as shown in Fig. 7; backmutations T50Q and A91K to be added).

### (D) Phytases and corresponding DNA sequences related to consensus phytase-1-thermo[8]-Q50T-K91A

A phytase which comprises an amino acid sequence which is at least 97.87%; or at least 98, 98.5, 99, 99.5% identical to the sequence of amino acids 1-467 of consensus phytase-1-thermo[8]-Q50T-K91A as shown in Fig. 7.

A phytase which comprises an amino acid sequence which is at least 98.08%; or at least 98.5, 99, 99.5% similar to the sequence of amino acids 1-467 of consensus phytase-1-thermo[8]-Q50T-K91A as shown in Fig. 7.

A phytase which is encoded by a DNA sequence which is at least 98.37%; or at least 98.5, 99, 99.5% identical to nucleotides 1-1407 of the DNA sequence of consensus phytase-1-thermo[8]-Q50T-K91A as shown in Fig. 7.

A DNA sequence which encodes a phytase and which (i) is at least 98.37%; or at least 98.5, 99, 99.5% identical; or (ii) hybridizes under low, or medium, medium/high, high, or very high stringency conditions to nucleotides 1-1407 of the DNA sequence of consensus phytase-1-thermo[8]-Q50T-K91A as shown in Fig. 7. A suitable negative control is DNA encoding consensus phytase-1. A suitable positive control is DNA encoding any of CP1-thermo[8], CP1-thermo[8]-Q50T-K91A.

A DNA sequence which encodes a phytase comprising an amino acid sequence which is at least 97.87%; or at least 98, 98.5, 99, 99.5% identical to the sequence of amino acids 1-467 of consensus phytase 1-thermo[8]-Q50T-K91A as shown in Fig. 7.

### (E) Phytases and corresponding DNA sequences related to consensus phytase-11

A phytase that comprises an amino acid sequence which is at least 90.71%; or at least 91, 91.5, 92, 92.5, 93, 93.5, 94, 94.5, 95, 95.5, 96, 96.5, 97, 97.5, 98, 98.5, 99, 99.5% identical to the sequence of amino acids 1-482 of consensus phytase-11 as shown in Fig. 6.

A phytase that comprises an amino acid sequence which is at least 92.07%; or at least 92.5, 93, 93.5, 94, 94.5, 95, 95.5, 96, 96.5, 97, 97.5, 98, 98.5, 99, 99.5% similar to the sequence of amino acids 1-482 of consensus phytase-11 as shown in Fig. 6.

A DNA sequence that encodes a phytase comprising an amino acid sequence which is at least 90.71%; or at least 91. 91.5, 92, 92.5, 93, 93.5, 94, 94.5, 95, 95.5, 96, 96.5, 97, 97.5, 98, 98.5, 99, 99.5% identical to the sequence of amino acids 1-482 of consensus phytase-11 as shown in Fig. 6.

### (F) Phytases and corresponding DNA sequences related to A. fumigatus alpha-mutant

A phytase that comprises an amino acid sequence which is at least 97.17%; or at least 97.5, 98, 98.5, 99, 99.5% identical to the sequence of amino acids 1-467 of A. fumigatus alpha-mutant (phytase) as shown in Fig. 9.

A phytase that comprises an amino acid sequence that is at least 97.82%; or at least 98, 98.5, 99, 99.5% similar to the sequence of amino acids 1-467 of A. fumigatus alpha-mutant (phytase) as shown in Fig. 9.

A phytase which is encoded by a DNA sequence which is at least 96.13%; or at least 96.5, 97, 97.5, 98, 98.5, 99, 99.5% identical to nucleotides 1-1401 of the DNA sequence of A. fumigatus ATCC 13073 alpha-mutant (phytase) as shown in Fig. 9.

A DNA sequence which encodes a phytase comprising an amino acid sequence which is at least 97.17%; or at least 97.5, 98, 98.5, 99, 99.5% identical to the sequence of amino acids 1-467 of A. fumigatus ATCC 13073 alpha-mutant (phytase) as shown in Fig. 9.

A DNA sequence which encodes a phytase and which (i) is at least 96.13%; or 96.5, 97, 97.5, 98, 98.5, 99, 99.5% identical; or (ii) hybridizes under low, or medium, medium/high, high, or very high stringency conditions to nucleotides 1-1401 of the DNA sequence of A. fumigatus ATCC 13073 alpha-mutant (phytase) shown in Fig. 9. A suitable negative control is DNA encoding A. fumigatus 13073 phytase. A suitable positive control is DNA encoding any one of the A. fumigatus ATCC 13073 alpha mutant phytase or the optimised alpha-mutant.

### (G) Phytases and corresponding DNA sequences related to the optimized A. fumigatus alpha-mutant

A phytase that comprises an amino acid sequence that is at least 96.08%; or at least 96.5, 97, 97.5, 98, 98.5, 99, 99.5% identical to the sequence of the phytase of the optimized A. fumigatus alpha-mutant.

A phytase that comprises an amino acid sequence that is at least 96.74%; or at least 97, 97.5, 98, 98.5, 99, 99.5% similar to the sequence of the phytase of the optimized A. fumigatus alpha-mutant.

A phytase which is encoded by a DNA sequence which is at least 95.63%; or at least 96, 96.5, 97, 97.5, 98, 98.5, 99, 99.5% identical to nucleotides 1-1401 of the DNA sequence encoding the optimized A. fumigatus alpha-mutant phytase.

A DNA sequence that encodes a phytase comprising an amino acid sequence that is at least 96.08%; or at least 96.5, 97, 97.5, 98, 98.5, 99, 99.5% identical to the optimized A. fumigatus alpha-mutant phytase.

A DNA sequence which encodes a phytase and which (i) is at least 95.63%; or at least 96, 96.5, 97, 97.5, 98, 98.5, 99, 99.5% identical; or (ii) hybridizes under low, or medium, medium/high, high, very high stringency conditions to nucleotides 1-1401 of the DNA sequence encoding the optimized A. fumigatus alpha-mutant phytase.

A suitable negative control is DNA encoding A. fumigatus ATCC 13073 phytase. A suitable positive control is DNA encoding any one of the A. fumigatus ATCC 13073 alpha mutant phytase of the optimised alpha-mutant.

### (H) Phytases and corresponding DNA sequences related to consensus phytase-7

A phytase that comprises an amino acid sequence which is at least 94.87%; or at least 95, 95.5, 96, 96.5, 97, 97.5, 98, 98.5, 99, 99.5% identical to the sequence of amino acids 1-467 of consensus phytase-7 as shown in Fig. 10.

A phytase that comprises an amino acid sequence which is at least 95.30%; or at least 95.5, 96, 96.5, 97, 97.5, 98, 98.5, 99, 99.5% similar to the sequence of amino acids 1-467 of consensus phytase-7 as shown in Fig. 10.

A phytase which is encoded by a DNA sequence which is at least 96.38%; or 96.5, 97, 97.5, 98, 98.5, 99, 99.5% identical to nucleotides 12-1412 of the DNA sequence of consensus phytase-7 shown in Fig. 10.

A DNA sequence which encodes a phytase and which (i) is at least 96.38%; or at least 96.5, 97, 97.5, 98, 98.5, 99, 99.5% identical; or (ii) hybridizes under low, or medium, medium/high, high, or very high stringency conditions to nucleotides 12-1412 of the DNA sequence of consensus phytase-7 as shown in Fig. 10.

A DNA sequence which encodes a phytase comprising an amino acid sequence which is at least 94.87%; or at least 95, 95.5, 96, 96.5, 97, 97.5, 98, 98.5, 99, 99.5% identical to the sequence of amino acids 1-467 of consensus phytase-7 as shown in Fig. 10.

### (I) Phytases related to basidio consensus phytase

A phytase which comprises an amino acid sequence which is at least 76.23%; or at least 77, 78, 79, 80, 81, 82, 83, 84, 85, 86, 87, 88, 89, 90, 91, 92, 93, 94, 94.5, 95, 95.5, 96, 96.5, 97, 97.5, 98, 98.5, 99, 99.5% identical to the combined sequence of (i) amino acids 1-441 of basidio consensus phytase shown in Fig. 3, and (ii) amino acids 1-26 shown in Fig. 5 (the sequence of (ii) to be added at the N-terminus of the sequence of (i)).

A phytase that comprises an amino acid sequence which is at least 79.50%; or at least 80, 81, 82, 83, 84, 85, 86, 87, 88, 89, 90, 91, 92, 93, 94, 95, 95.5, 96, 96.5, 97, 97.5, 98, 98.5, 99, 99.5% similar to the sequence of amino acids 1-441 of basidio consensus phytase as shown in Fig. 3.

### (J) Phytases related to consensus phytase-12

A phytase which comprises an amino acid sequence which is at least 70, 75, 76, 77, 78, 79, 80, 81, 82, 83, 84, 85, 86, 87, 88, 89, 90, 91, 92, 93, 94, 95, 95.5, 96, 96.5, 97, 97.5, 98, 98.5, 99, 99.5% identical to the sequence of amino acids 1-467 of consensus phytase-12 as shown in Fig. 21.

A phytase which comprises an amino acid sequence which is at least 75, 76, 77, 78, 79, 80, 81, 82, 83, 84, 85, 86, 87, 88, 89, 90, 91, 92, 93, 94, 95, 95.5, 96, 96.5, 97, 97.5, 98, 98.5, 99, 99.5% similar to the sequence of amino acids 1-467 of consensus phytase-12 as shown in Fig. 21.

**Table 8**

| Comparison of phytase amino acid sequences | | | | | | | | | |
|---|---|---|---|---|---|---|---|---|---|
| Phytase | CP10 | CP10-thermo[3]-Q50T-K91A | CP1-thermo[8] | CP1-thermo[8]-Q50T-K91A | CP11 | CP7 | Basidio | A. fumigatus alpha-mutant | A. fumigtus alpha-mutant (opt.) |
| Consensus phytase-1 | 95.08/93.79 | 94.65/93.36 | 98.50/98.29 | 98.07/97.86 | 92.06/90.70 | 95.29/94.86 | 69.42/62.16 | 85.59/82.58 | 84.73/81.72 |
| A. niger NRRL3135 | 79.48/76.46 | 79.05/76.03 | 80.35/77.75 | 79.91/77.32 | 79.27/76.31 | 84.02/81.64 | 67.19/59.32 | 74.07/70.11 | 74.95/70/99 |
| A. terreus 9-A1 | 76.04/72.11 | 75.82/71.90 | 76.47/72.33 | 76.25/72.11 | 76.51/73.02 | 75.76/71.18 | 65.39/58.02 | 69.67/64.84 | 69.45/64.84 |
| A. terreus cbs | 79.04/75.11 | 78.82/74.89 | 79.48/75.76 | 79.26/75.55 | 77.19/73.27 | 79.17/75.00 | 66.92/59.65 | 72.59/67.76 | 72.37/67.76 |
| E. nidulans | 78.70/74.35 | 78.26/73.91 | 79.78/75.87 | 79.35/75.44 | 80.56/76.62 | 76.96/73.04 | 67.20/58.13 | 72.39/67.83 | 72.11/67.54 |
| A. fumigatus 13073 | 82.93/80.31 | 82.50/79.87 | 82.31/79.04 | 81.88/78.60 | 81.36/78.64 | 80.13/76.20 | 63.54/57.91 | 97.82/97.16 | 96.73/96.07 |
| A. fumigatus 32239 | 81.30/77.39 | 80.87/76.96 | 81.09/77.61 | 80.65/77.17 | 79.95/76.08 | 79.13/75.22 | 63.61/54.97 | 90.22/86.52 | 89.57/85.87 |
| T. thermophilus | 77.83/73.84 | 77.38/73.39 | 78.67/74.89 | 78.22/74.44 | 78.47/74.76 | 76.51/73.15 | 61.54/54.36 | 72.01/66.82 | 72.69/67.49 |
| M . thermophila | 69.16/62.81 | 69.48/63.33 | 69.27/62.84 | 69.59/63.36 | 69.65/63.06 | 68.82/62.13 | 65.56/57.91 | 66.21/58.45 | 66.44/58.68 |
| T. lanuginosus | 73.52/66.70 | 73.06/66.44 | 71.92/64.61 | 71.46/64.16 | 74.21/68.86 | 69.50/62.62 | 67.20/57.41 | 68.91/61.02 | 69.61/61.72 |
| P. lycii | 64.92/59.10 | 64.91/59.37 | 64.46/58.09 | 64.46/58.36 | 65.03/59.84 | 63.13/56.50 | 77.75/73.07 | 64.08/57.11 | 62.47/55.91 |
| A. pediades | 64.51/51.81 | 64.86/51.94 | 62.98/51.41 | 63.33/51.54 | 64.50/52.30 | 63.05/51.15 | 78.92/74.71 | 61.64/52.38 | 62.13/53.07 |
| P. involutus 1 | 66.67/58.07 | 66.67/58.33 | 64.84/56.51 | 64.84/56.77 | 63.30/54.52 | 65.33/56.53 | 79.49/76.22 | 59.59/51.81 | 59.95/52.20 |
| P. involutus 2 | 65.54/55.70 | 65.30/55.53 | 66.85/56.87 | 66.58/56.68 | 66.30/56.35 | 64.27/54.13 | 78.09/74.59 | 61.26/52.62 | 61.04/52.47 |
| T. pubescens | 65.46/57.22 | 65.72/57.47 | 62.89/55.67 | 63.14/55.93 | 65.03/57.65 | 63.28/56.51 | 78.34/75.12 | 64.08/57.11 | 62.30/55.24 |
| CP10 | - | 99.57/99.57 | 96.57/95.50 | 96.15/95.08 | 95.02/94.56 | 91.01/89.29 | 70.22/62.28 | 85.13/82.76 | 85.99/83.62 |
| CP10t[3]Q50TK91A | 99.57/99.57 | - | 96.15/95.08 | 96.57/95.50 | 94.56/94.10 | 90.58/88.87 | 70.47/62.28 | 85.13/82.76 | 85.99/83.62 |
| CP1thermo[8] | 96.57/95.50 | 96.15/95.08 | - | 99.57/99.57 | 93.42/92.29 | 94.43/93.79 | 68.40/60.74 | 84.52/81.94 | 85.38/82.80 |
| CP1t[8]Q50TK91A | 96.15/95.08 | 96.57/95.50 | 99.57/99.57 | - | 92.97/91.84 | 94.00/93.36 | 68.64/60.74 | 84.52/81.94 | 85.38/82.80 |
| CP11 | 95.02/94.56 | 94.56/94.10 | 93.42/92.29 | 92.97/91.84 | - | 88.44/86.62 | 68.27/59.73 | 82.23/79.73 | 83.37/80.87 |
| CP7 | 91.01/89.29 | 90.58/88.87 | 94.43/93.79 | 94.00/93.36 | 88.44/86.62 | - | 69.80/62.69 | 81.94/78.71 | 81.72/78.50 |
| Basidio | 70.22/62.28 | 70.47/62.28 | 68.40/60.74 | 68.64/60.74 | 68.27/59.73 | 69.80/62.69 | - | 65.97/60.52 | 66.41/60.68 |
| A.fumigatus alpha-mut. | 85.13/82.76 | 85.13/82.76 | 84.52/81.94 | 84.52/81.94 | 82.23/79.73 | 81.94/78.71 | 65.97/60.52 | - | 98.93/98.93 |
| A. fum alpha-mut -opt. | 85.99/83.62 | 85.99/83.62 | 85.38/82.80 | 85.38/82.80 | 83.37/80.87 | 81.72/78.50 | 66.41/60.68 | 98.93/98.93 | - |

**Table 9**

| Comparison of phytase encoding DNA sequences | | | | | | | | |
|---|---|---|---|---|---|---|---|---|
| Phytase | CP10 | CP10-thermo[3]-Q50T-K91A | CP1-thermo[8] | CP1-thermo[8]-Q50T-K91A | CP7 | Basidio | A. fumigatus alpha-mutant | A. fumi-gatus alpha-mutant (opt.) |
| Consensus phytase-1 | 95.87 | 95.87 | 98.72 | 98.36 | 96.37 | 65.46 | 66.88 | 66.88 |
| A. niger NRRL3135 | 65.10 | 64.82 | 66.10 | 65.74 | 67.52 | 50.68 | 65.88 | 66.17 |
| A. terreus 9-A1 | 61.74 | 61.53 | 62.17 | 62.03 | 60.53 | 49.40 | 66.24 | 66.31 |
| A. terreus cbs | 62.52 | 62.30 | 63.02 | 62.88 | 61.45 | 49.74 | 68.17 | 68.24 |
| E. nidulans | 65.08 | 64.94 | 65.30 | 65.01 | 64.22 | 49.92 | 64.90 | 65.44 |
| A. fumigatus 13073 | 65.66 | 65.38 | 64.19 | 64.08 | 63.65 | 48.27 | 96.12 | 95.62 |
| T. thermophilus | 62.52 | 62.50 | 62.53 | 62.66 | 62.00 | 52.19 | 61.77 | 61.92 |
| M . thermophila | 55.51 | 55.15 | 55.36 | 55.22 | 53.91 | 48.44 | 58.17 | 58.24 |
| T. lanuginosus | 57.56 | 57.20 | 56.76 | 56.47 | 62.00 | 44.66 | 59.71 | 60.07 |
| P. lycii | 45.76 | 46.51 | 45.14 | 55.21 | 55.46 | 58.50 | 48.91 | 49.44 |
| A. pediades | 49.89 | 49.89 | 49.89 | 50.11 | 45.54 | 61.66 | 47.49 | 47.56 |
| P. involutus 1 | 48.32 | 49.03 | 47.81 | 47.96 | 49.59 | 59.80 | 49.96 | 50.19 |
| P. involutus 2 | 48.24 | 49.00 | 48.08 | 48.63 | 47.94 | 60.16 | 47.56 | 47.63 |
| T. pubescens | 47.00 | 47.17 | 46.46 | 47.62 | 46.83 | 60.37 | 49.89 | 49.96 |
| CP10 | - | 99.43 | 96.40 | 96.05 | 93.73 | 66.40 | 67.81 | 68.24 |
| CP10t[3]Q50TK91A | 99.43 | - | 96.37 | 96.58 | 93.45 | 66.29 | 67.81 | 68.24 |
| Cp1thermo[8] | 96.40 | 96.37 | - | 99.65 | 95.30 | 65.40 | 66.74 | 67.17 |
| CP1t[8]Q50TK91A | 96.05 | 96.58 | 99.65 | - | 94.94 | 65.47 | 66.74 | 67.17 |
| CP7 | 93.73 | 93.45 | 95.30 | 94.94 | - | 64.56 | 65.88 | 65.88 |
| Basidio | 66.40 | 66.29 | 65.40 | 65.47 | 64.56 | - | 50.41 | 50.49 |
| A.fumigatus alpha-mut. | 67.81 | 67.81 | 66.74 | 66.74 | 65.88 | 50.41 | - | 99.50 |
| A. fum alpha-mut -opt. | 68.24 | 68.24 | 67.17 | 67.17 | 65.88 | 50.49 | 99.50 | - |

## Claims

1. A phytase that
(i) comprises an amino acid sequence which is at least 93.80% identical to the sequence of amino acids -26 to +441 of SEQ ID NO: 26, and/or
(ii) is encoded by a DNA sequence that is at least 95.88% identical to nucleotides 12-1412 of SEQ ID NO: 25;
wherein for calculating identity the program GAP is used, as provided in the GCG program package, release 9.1, and with the following settings for DNA sequence comparisons: GAP creation penalty 50, and GAP extension penalty 3, and the following settings for amino acid sequence comparisons: Length Weight 0, and Gap Weight 3.0.

2. The phytase of claim 1 wherein
(i) the amino acid sequence is at least 94, 94.5, 95, 95.5, 96, 96.5, 97, 97.5, 98, 98.5, 99, or at least 99.5% identical to the sequence of amino acids -26 to +441 of SEQ ID NO: 26, and/or
(ii) the encoding DNA sequence is at least 96, 96.5, 97, 97.5, 98, 98.5, 99, or at least 99.5% identical to nucleotides 12-1412 of SEQ ID NO: 25.

3. A phytase that comprises amino acids +1 to +441 of SEQ ID NO: 26; is encoded by nucleotides 12-1412 or 90-1412 of SEQ ID NO: 25; or is variant Q24L, Q24T, Q24G, Q24L-Y25N, or Q24T-Y25N of the phytase of amino acids +1 to +441 of SEQ ID NO: 26.

4. The phytase of any one of claims 1-2 that comprises an amino acid sequence chosen from
(i) the sequences -26 to +441, +1 to +441, or +1 to +438 of SEQ ID NO: 26; or an amino acid sequence encoded by
(ii) nucleotides 12-1412, or 90-1412 of SEQ ID NO: 25.

5. The phytase of any one of claims 1-2 that comprises an amino acid sequence chosen from
(i) variant K68A+V1321+A370S of SEQ ID NO: 26,
(ii) variants Q24T, K65A, or Q24T+K65A of (i),
(iii) the sequences +1 to 441 of any of the sequences of (i) and (ii),
(iv) the sequences -26 to +441, or +1 to +441 of SEQ ID NO: 31; or an amino acid sequence encoded by
(v) nucleotides 1-1401, or 79-1401 of SEQ ID NO: 30.

6. The phytase of any one of claims 1-2 that comprises an amino acid sequence chosen from
(i) variant T24Q+A65K of SEQ ID NO: 29,
(ii) variants Q24T, K65A, or Q24T+K65A of (i),
(iii) the sequences +1 to +441 of any of the sequences of (i) and (ii), or
(iv) the sequences -26 to +441, or +1 to +441 of SEQ ID NO: 29; or an amino acid sequence encoded by
(v) nucleotides 1-1401, or 79-1401 of SEQ ID NO: 28.

7. A phytase that comprises the amino acid sequence of SEQ ID NO: 27, or is variant Q24L, Q24T, Q24G, Q24L-Y25N, or Q24T-Y25N thereof.

8. The phytase of claim 7 that comprises the amino acid sequence of SEQ ID NO: 27.

9. A DNA sequence that comprises
(i) a DNA-sequence encoding the phytase of any one of claims 1-8,
(ii) a DNA-sequence encoding a phytase that is at least 95.88% identical to nucleotides 12-1412 of SEQ ID NO: 25, and/or
(iii) a DNA-sequence that encodes a phytase, wherein the phytase comprises an amino acid sequence that is at least 93.80% identical to the sequence of amino acids -26 to +441 of SEQ ID NO: 26;
wherein for calculating identity the program GAP is used, as provided in the GCG program package, release 9.1, and with the following settings for DNA sequence comparisons: GAP creation penalty 50, and GAP extension penalty 3, and the following settings for amino acid sequence comparisons: Length Weight 0, and Gap Weight 3.0.

10. The DNA sequence of claim 9 wherein
(ii) the phytase-encoding DNA-sequence is at least 96, 96.5, 97, 97.5, 98, 98.5, 99, or at least 99.5% identical to nucleotides 12-1412 of SEQ ID NO: 25, and/or
(iii) the phytase-encoding DNA sequence encodes a phytase comprising an amino acid sequence that is at least 94, 94.5, 95, 95.5, 96, 96.5, 97, 97.5, 98, 98.5, 99, or at least 99.5% identical to the sequence of amino acids -26 to +441 of SEQ ID NO: 26.

11. The DNA sequence of any one of claims 9-10 that comprises a DNA-sequence that encodes a phytase, and wherein the phytase-encoding DNA-sequence comprises
(i) nucleotides 12-1412, or 90-1412 of SEQ ID NO: 25;
(ii) nucleotides 1-1401, or 79-1401 of SEQ ID NO: 30; or
(iii) nucleotides 1-1401, or 79-1401 of SEQ ID NO: 28.

12. A vector comprising the DNA sequence according to any one of claims 9-11.

13. A microbial host cell comprising the DNA sequence according to any one of claims 9-11, or the vector according to claim 12.

14. A process for producing a phytase, the process comprising culturing the host cell according to claim 13 under conditions permitting the production of the phytase, and recovering the phytase from the culture broth.

15. A food, feed or pharmaceutical composition comprising the phytase of any one of claims 1-8.

## Patentansprüche

1. Phytase, welche
(i) eine Aminosäuresequenz umfasst, welche mindestens 93,80 % identisch mit der Sequenz der Aminosäuren -26 bis +441 von SEQ ID NR: 26 ist und/oder
(ii) durch eine DNA-Sequenz kodiert wird, die mindestens 95,88 % identisch mit den Nukleotiden 12 bis 1412 von SEQ ID NR: 25 ist;
wobei zum Berechnen der Identität das Programm GAP verwendet wird, wie in dem GCG-Programmpaket, Release 9.1, bereitgestellt, und mit den folgenden Einstellungen für DNA-Sequenzvergleiche: GAP Creation Penalty 50 und GAP Extension Penalty 3 und den folgenden Einstellungen für Aminosäurewequenzvergleiche: Length Weight 0 und Gap Weight 3,0.

2. Phytase nach Anspruch 1, wobei
(i) die Aminosäuresequenz mindestens 94, 94,5, 95, 95,5, 96, 96,5, 97, 97,5, 98, 98,5, 99 oder mindestens 99,5 % identisch mit der Sequenz der Aminosäuren - 26 bis +441 von SEQ ID NR: 26 ist und/oder
(ii) die kodierende DNA-Sequenz mindestens 96, 96,5, 97, 97,5 98, 98,5, 99 oder mindestens 99,5 % identisch mit den Nukleotiden 12 bis 1412 von SEQ ID NR: 25 ist.

3. Phytase, welche die Aminosäuren +1 bis +441 von SEQ ID NR: 26 umfasst; welche durch die Nukleotide 12 bis 1412 oder 90 bis 1412 von SEQ ID NR: 25 kodiert wird; oder welche die Variante Q24L, Q24T, Q24G, Q24L-Y25N oder Q24T-Y25N von der Phytase der Aminosäuren +1 bis +441 von SEQ ID NR: 26 ist.

4. Phytase nach einem beliebigen der Ansprüche 1 bis 2, welche eine Aminosäuresequenz umfasst, die ausgewählt ist aus
(i) den Sequenzen -26 bis +441, +1 bis +441 oder +1 bis +438 von SEQ ID NR: 26; oder einer Aminosäuresequenz, die kodiert wird durch
(ii) die Nukleotide 12 bis 1412 oder 90 bis 1412 von SEQ ID NR: 25.

5. Phytase nach einem beliebigen der Ansprüche 1 bis 2, welche eine Aminosäuresequenz umfasst, die ausgewählt ist aus
(i) Variante K68A+V132I+A370S von SEQ ID NR: 26,
(ii) Varianten Q24T, K65A oder Q24T+K65A aus (i),
(iii) den Sequenzen +1 bis 441 von einer beliebigen der Sequenzen aus (i) und (ii),
(iv) den Sequenzen -26 bis +441 oder +1 bis +441 von SEQ ID NR: 31; oder einer Aminosäuresequenz, die kodiert wird durch
(v) die Nukleotide 1 bis 1401 oder 79 bis 1401 von SEQ ID NR: 30.

6. Phytase nach einem beliebigen der Ansprüche 1 bis 2, welche eine Aminosäuresequenz umfasst, die ausgewählt ist aus
(i) Variante T24Q+A65K von SEQ ID NR: 29,
(ii) Varianten Q24T, K65A oder Q24T+K65A aus (i),
(iii) den Sequenzen +1 bis +441 von einer beliebigen der Sequenzen aus (i) und (ii) oder
(iv) den Sequenzen -26 bis +441 oder +1 bis +441 von SEQ ID NR: 29; oder einer Aminosäuresequenz, die kodiert wird durch
(v) die Nukleotide 1 bis 1401 oder 79 bis 1401 von SEQ ID NR: 28.

7. Phytase, welche die Aminosäuresequenz von SEQ ID NR: 27 umfasst oder die Variante Q24L, Q24T, Q24G, Q24L-Y25N oder Q24T-Y25N hiervon ist.

8. Phytase nach Anspruch 7, welche die Aminosäuresequenz von SEQ ID NR: 27 umfasst.

9. DNA-Sequenz, welche umfasst
(i) eine DNA-Sequenz, welche die Phytase nach einem beliebigen der Ansprüche 1 bis 8 kodiert,
(ii) eine DNA-Sequenz, welche eine Phytase kodiert, welche mindestens 95,88 % identisch mit den Nukleotiden 12 bis 1412 von SEQ ID NR: 25 ist und/oder
(iii) eine DNA-Sequenz, welche eine Phytase kodiert, wobei die Phytase eine Aminosäuresequenz umfasst, welche mindestens 93,80 % identisch mit der Sequenz der Aminosäuren -26 bis +441 von SEQ ID NR: 26 ist;
wobei zum Berechnen der Identität das Programm GAP verwendet wird, wie in dem GCG-Programmpaket, Release 9.1, bereitgestellt, und mit den folgenden Einstellungen für DNA-Sequenzvergleiche: GAP Creation Penalty 50 und GAP Extension Penalty 3 und den folgenden Einstellungen für Aminosäuresequenzvergleiche: Length Weight 0 und Gap Weight 3,0.

10. DNA-Sequenz nach Anspruch 9, wobei
(ii) die Phytase-kodierende DNA-Sequenz mindestens 96, 96,5, 97, 97,5, 98, 98,5, 99 oder mindestens 99,5 % identisch mit den Nukleotiden 12 bis 1412 von SEQ ID NR: 25 ist und/oder
(iii) die Phytase-kodierende DNA-Sequenz eine Phytase kodiert, welche eine Aminosäuresequenz umfasst, welche mindestens 94, 94,5, 95, 95,5, 96, 96,5, 97, 97,5, 98, 98,5, 99 oder mindestens 99,5 % identisch mit der Sequenz der Aminosäuren -26 bis +441 von SEQ ID NR: 26 ist.

11. DNA-Sequenz nach einem beliebigen der Ansprüche 9 bis 10, welche eine DNA-Sequenz umfasst, welche eine Phytase kodiert, und wobei die Phytase-kodierende DNA-Sequenz umfasst
(i) Nukleotide 12 bis1412 oder 90 bis 1412 von SEQ ID NR: 25;
(ii) Nukleotide 1 bis 1401 oder 79 bis 1401 von SEQ ID NR: 30; oder
(iii) Nukleotide 1 bis 1401 oder 79 bis 1401 von SEQ ID NR: 28.

12. Vektor, welcher die DNA-Sequenz nach einem beliebigen der Ansprüche 9 bis 11 umfasst.

13. Mikrobielle Wirtszelle, welche die DNA-Sequenz nach einem beliebigen der Ansprüche 9 bis 11 oder den Vektor nach Anspruch 12 umfasst.

14. Verfahren zum Herstellen einer Phytase, welches Verfahren das Kultivieren der Wirtszelle nach Anspruch 13 unter Bedingungen, welche die Herstellung der Phytase erlauben, und das Gewinnen der Phytase aus der Kulturbrühe umfasst.

15. Nahrungsmittel, Futtermittel oder pharmazeutische Zusammensetzung, welche die Phytase nach einem beliebigen der Ansprüche 1 bis 8 umfasst.

## Revendications

1. Phytase qui
(i) comprend une séquence d'acides aminés qui est au moins 93,80 % identique à la séquence d'acides aminés -26 à +441 de SEQ ID n° 26, et/ou
(ii) est codée par une séquence ADN qui est au moins 95,88 % identique aux nucléotides 12-1412 de la SEQ ID n° 25 ;
dans laquelle, pour calculer l'identité, on utilise le programme GAP tel que fourni dans le paquet programme GCG, édition 9.1, et avec les réglages suivants pour les comparaisons de séquences ADN : pénalité de création GAP 50 et pénalité d'extension GAP 3, et les réglages suivants pour les comparaisons de séquences d'acides aminés : Poids Longueur 0 et Poids Gap 3,0.

2. Phytase de la revendication 1, dans laquelle
(i) la séquence d'acides aminés est au moins 94, 94,5, 95, 95,5, 96, 96,5, 97, 97,5, 98, 98,5, 99 ou au moins 99,5 % identique à la séquence d'acides aminés -26 à +441 de SEQ ID n° 26, et/ou
(ii) la séquence d'ADN de codage est au moins 96, 96,5, 97, 97,5, 98, 98,5, 99 ou au moins 99,5 % identique aux nucléotides 12-1412 de SEQ ID n° 25.

3. Phytase qui comprend des acides aminés +1 à +441 de SEQ ID n° 26 ; est codée par les nucléotides 12-1412 ou 90-1412 de SEQ ID n° 25 ; ou est une variante Q24L, Q24T, Q24G, Q24L-Y25N ou Q24T-Y25N de la phytase des acides aminés +1 à +441 de SEQ ID n° 26.

4. Phytase de l'une quelconque des revendications 1-2 qui comprend une séquence d'acides aminés choisie à partir de
(i) les séquences -26 à +441, +1 à +441 ou +1 à +438 de SEQ ID n° 26 ; ou une séquence d'acides aminés codée par
(ii) nucléotides 12-1412 ou 90-1412 de SEQ ID n° 25.

5. Phytase de l'une quelconque des revendications 1-2 qui comprend une séquence d'acides aminés choisie à partir
(i) d'une variante K68A+V132I+A370S de SEQ ID n° 26,
(ii) des variantes Q24T, K65A ou Q24T+K65A de (i),
(iii) des séquences +1 à +441 de l'une quelconque des séquences de (i) et (ii),
(iv) des séquences -26 à +441 ou +1 à +441 de SEQ ID n° 31 ; ou une séquence d'acides aminés codée par
(v) des nucléotides 1-1401 ou 79-1401 de SEQ ID n° 30.

6. Phytase de l'une quelconque des revendications 1-2 qui comprend une séquence d'acides aminés choisie à partir
(i) d'une variante T24Q+A65K de SEQ ID n° 29,
(ii) des variantes Q24T, K65A ou Q24T+K65A de (i),
(iii) des séquences +1 à +441 de l'une quelconque des séquences de (i) et (ii), ou
(iv) des séquences -26 à +441 ou +1 à +441 de SEQ ID n° 29 ; ou une séquence d'acides aminés codée par
(v) des nucléotides 1-1401 ou 79-1401 de SEQ ID n° 28.

7. Phytase qui comprend la séquence d'acides aminés de SEQ ID n° 27 ou est une variante Q24L, Q24T, Q24G, Q24L-Y25N ou Q24T-Y25N de celle-ci.

8. Phytase de la revendication 7 qui comprend la séquence d'acides aminés de SEQ ID n° 27.

9. Séquence d'ADN qui comprend
(i) une séquence ADN codant la phytase de l'une quelconque des revendications 1 à 8,
(ii) une séquence ADN codant une phytase qui est au moins 95,88 % identique aux nucléotides 12-1412 de SEQ ID n° 25, et/ou
(iii) une séquence ADN qui code une phytase dans laquelle la phytase comprend une séquence d'acides aminés qui est au moins 93,80 % identique à la séquence d'acides aminés -26 à +441 de SEQ ID n° 26 ;
dans laquelle, pour calculer l'identité, on utilise le programme GAP, tel que fourni dans le paquet programme GCG, édition 9.1, et avec les ajustements suivants pour les comparaisons de séquences ADN : pénalité de création GAP 50 et pénalité d'extension GAP 3, et les réglages suivants pour les comparaisons de séquences d'acides aminés : Poids Longueur 0 et Poids Gap 3,0.

10. Séquence d'ADN de la revendication 9, dans laquelle
(ii) la séquence ADN codant la phytase est au moins 96, 96,5, 97, 97,5, 98, 98,5, 99 ou au moins 99,5 % identique aux nucléotides 12-1412 de SEQ ID n° 25, et/ou
(iii) la séquence ADN codant la phytase code une phytase comprenant une séquence d'acides aminés qui est au moins 94, 94,5, 95, 95,5, 96, 96,5, 97, 97,5, 98, 98,5, 99 ou au moins 99,5 % identique à la séquence d'acides aminés -26 à +441 de SEQ ID n° 26.

11. Séquence d'ADN de l'une quelconque des revendications 9-10 qui comprend une séquence d'ADN qui code une phytase et dans laquelle la séquence d'ADN codant la phytase comprend
(i) des nucléotides 12-1412 ou 90-1412 de SEQ ID n° 25 ;
(ii) des nucléotides 1-1401 ou 79-1401 de SEQ ID n° 30 ; ou
(iii) des nucléotides 1-1401 ou 79-1401 de SEQ ID n° 28.

12. Vecteur comprenant la séquence d'ADN selon l'une quelconque des revendications 9-11.

13. Cellule hôte microbienne comprenant la séquence ADN selon l'une quelconque des revendications 9-11 ou le vecteur selon la revendication 12.

14. Procédé pour produire une phytase, le procédé comprenant la mise en culture de la cellule hôte selon la revendication 13 dans les conditions permettant la production de la phytase et la récupération de la phytase à partir du bouillon de culture.

15. Composition alimentaire, nutritionnelle ou pharmaceutique comprenant la phytase de l'une quelconque des revendications 1-8.
